# EUROPEAN PATENT SPECIFICATION

(11) **EP 0 533 833 B1**
(45) Date of publication and mention of the grant of the patent: **20.12.1995**
(21) Application number: 91912398.4
(22) Date of filing: 11.06.1991
(51) Int. Cl.: C07F 9/6558, A61K 31/675, C07F 9/6561, C07H 19/20, C07H 19/10

(54) **PHOSPHOROUS PRODUGS**
PHOSPHORYLIERTE PRODRUGS
PROMEDICAMENTS PHOSPHOREUX

(30) Priority: 13.06.1990 US 537332
(43) Date of publication of application: 31.03.1993
(73) Proprietor: GLAZIER, Arnold, Newton, MA 02159 (US)
(72) Inventor: GLAZIER, Arnold, Newton, MA 02159 (US)
(74) Representative: Holdcroft, James Gerald, Dr.
(86) International application number: US9104124
(87) International publication number: WO9119721

(56) References cited:
- EP-A- 0 331 032
- EP-A- 0 338 372
- EP-A- 0 360 609
- Jerry March, Advanced Organic Chemistry; J. Wiley, (1985) third edition; pp. 882-885

## Description

### Technical Field

This invention is in the fields of chemistry, medicine, and pharmacology.

### Background

Phosphate derivatives are key metabolic intermediates in virtually all aspects of cellular metabolism. In addition many antineoplastic and antiviral drugs require intracellular phosphorylation in order to be biologically active. However, the pharmacological utility of phosphate derivatives is severely hampered by the inability of negatively charged phosphate derivatives to permeate into cells and through the blood brain barrier. In addition phosphate and phosphonate compounds in general have a very low oral bioavailability. At the present time there is no generally applicable, pharmacologically viable approach which will allow the intracellular delivery of phosphorous bearing drugs.

Studies have been published describing lipophilic, **negatively charged** phosphate derivatives of Arabinofuranosylcytosine (ARA-C). E.K. Ryu, et. al., J. Medicinal Chem., 25: 1322 (1982), C.I. Hong et al., J.Medicinal Chem., 28:171 (1985), A. Rosowsky et al.,J.Medicinal Chem., 25:171 (1982), C.I.Hong et al.,J.Medicinal Chem., 33:1380 (1990). However, these prodrug derivatives form liposomal aggregates in water which lead to unacceptable pharmacological properties. MacCross et al.,Biochemical and Biophysical Research Comm., 116:368 (1983). Neutrally charged phosphorodiamidates have been suggested as phosphate prodrugs. M.E.Phelps et al.,J.Medicinal Chem., 23:1229 (1980) However, this method is ineffective. Chawla,R.R. et al. J.Medicinal Chemistry, 27:1733 (1984). Similarly, cyclic phosphoramidate derivatives have been tested as potential prodrugs without success. Kumar,A.; Coe,P; Jones,A.; Walker,R.; Balzarini,J.; De Clercq,E.; J.Medicinal Chemistry , 33:2368 (1990) Neutrally charged phosphate prodrugs with good leaving groups have been described. Chawla et al., ibid., Farrow,S.N. et al.,J.Medicinal Chemistry, 33:1400 (1990) However, neutrally charged phosphate esters which have a good leaving group on the phosphorous are in general extremely toxic inhibitors of acetylcholinesterase. Holmstedt,B. in Pharmacological Reviews , 11:567 (1959) Phosphotriester derivatives which lack acetylcholinesterase activity are in general not catabolized by cellular enzymes to phosphodiesters. Benzylic 3'5'cyclic monophosphate triesters have been employed as prodrugs for cyclic nucleotide monophosphate derivatives with variable success. Engels,J. et al.,J.Medicinal Chemistry, 20:907 (1977) , Beres,J.et al. J.Medicinal Chemistry, 29:494 (1986) , Beres,J.et al., J.Medicinal Chemistry, 29:1243 (1986) Primary benzylic phosphate esters are alkylating agents and potentially toxic. In addition the slow rate of hydrolysis of benzyl phosphate diesters to phosphate monoesters precludes the use of dibenzyl phosphotriesters as prodrugs. Acyloxymethyl phosphate esters have been described as potential prodrugs. Farquhar,D. , Srivastava,D., Kuttesch,N. , Saunders,P. ; J.Pharmaceutical Sciences , 72:325 (1983) Farquhar,D. Srivastava,D; Bioorganic Chemistry , 12:118 (1984) Farquhar,D.; Nowak,B.; Khan,S.; Plunkett,W.; Antiviral Research Supple.1:143 (1991) However, acyloxyalkyl bis phosphonate esters have been tested as prodrugs for a phosphonate compound and were found to be ineffective. Iyer,R. ; Phillips,L.; Biddle,J.; Thakker,D.; Egan,W.; Tetrahedron Let.; 30:7141 (1989). An additional problem with acyloxymethyl phospho-esters is the fact that 2 equivalents of a known carcinogen; formaldehyde is generated during prodrug hydrolysis. Accordingly at the present time there is no satisfactory pharmacologically viable method for facilitating the intracellular delivery of phosphorous bearing drugs.

### Summary of the Invention

The present invention relates to prodrugs which will allow the intracellular delivery of a wide range of phosphate and phosphorous bearing drugs, and methods for making the prodrugs. The method of the invention yields lipophilic, neutrally charged prodrugs which lack anticholinesterase activity.

In the present process , the parent phosphorous bearing drug is converted into a prodrug by coupling a unique class of substituted benzyl derivatives to each hydroxy group on the phosphorous. The benzyl derivatives are substituted with an acyloxy group in the para and or the ortho positions. Nonspecific intracellular esterases cleave the carboxylate esters and trigger heterolytic degradation of the hydroxy substituted benzylic phosphate esters to yield the desired phosphorous bearing drug. Since the prodrugs are lipid soluble enhanced oral absorption and improved penetration of the drug through the blood brain barrier is expected. The relationship between lipid solubility and enhanced drug absorption into the brain is well established. ( Dearden, J. in Comprehensive Medicinal Chemistry ; 4:402 ; Editors Hansch.C. et al.; Pergman Press, N.Y.,N.Y.)

### Detailed Description of the Invention

The present process is a widely applicable, pharmacologically viable, method for making prodrugs which will allow the delivery of phosphorous bearing drugs into cells and through the blood brain barrier. The term "prodrug" is meant for the present purposes to encompass the class of drugs the pharmacologic action of which results from conversion by metabolic processes within the body (i.e., biotransformation).

In the present process , the parent phosphorous bearing drug is transformed into a prodrug by converting one or more of the hydroxy groups on the phosphorous into a phosphate ester. The special nature of the class of phosphate esters employed in the prodrug design is central to the process. The phosphate ester is designed to undergo in vivo degradation via heterolytic cleavage of the C-O bond.

The methods described herein for producing prodrugs allow for the facile intracellular delivery of an enormous range of phosphorous derivatives. This is of special importance since phosphate derivatives are key metabolic intermediates of virtually all aspects of cellular metabolism. Analogs of naturally occurring phosphate compounds may be useful as both research tools and as drugs. The ability to deliver these phosphate analogs into cells in a pharmacologically viable manner should open up new possibilities in medicinal chemistry.

The present methods may also be used in chemical synthesis as a protective group for phosphate or phosphonates. In organic synthesis there is often the need to temporarily protect a phosphate or phosphonate moiety as a diester. The present protective groups are readily removed by purified pig liver esterase under very mild conditions.

Phosphorous derivatives for which the present prodrug methods are potentially applicable include: monophosphate esters; phosphate diesters; phosphonates; phosphonate esters; phosphorothioates; thio-phosphate esters; phosphonic acids; phosphinic acids; and phosphoramidates. In general any phosphorous containing drug which bears one or more hydroxy groups on the phosphorous atom(s) may be converted into lipid soluble, prodrugs with the present process. Drugs which may be converted into prodrugs may have the following STRUCTURE 1: 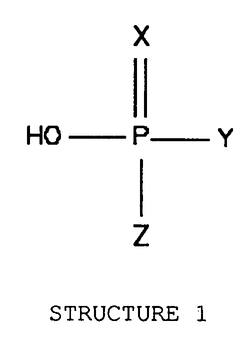
In this structure, X can be oxygen (O) or sulphur (S); The structure of groups Y and Z are defined by the structure of the parent drug. This prodrug method may not be used for phosphorous derivatives which possess a potential leaving group of low pKa attached to the phosphorous atom(s) as undesirable anticholinesterase activity will result.

Since the prodrug derivatives are lipid soluble ,they will exhibit enhanced penetration through biological membranes including the blood brain barrier. This is a desirable property for antineoplastic and antiviral drugs.

### GENERAL PRODRUG STRUCTURE

Prodrugs are preferably synthesized by replacing one or more of the hydroxy groups on the phosphorous atoms of the parent drug with a group of the general structure shown below as STRUCTURE 2: 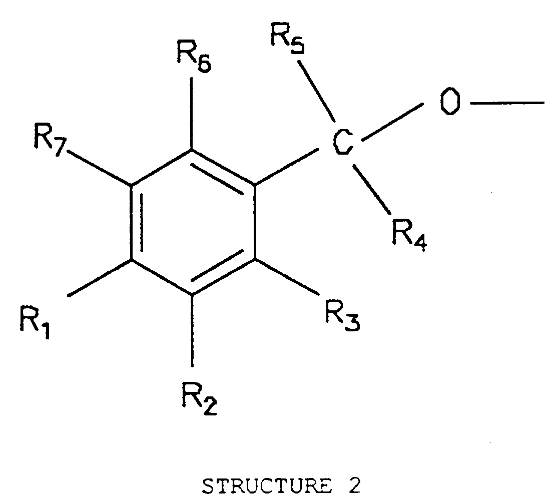
Wherein R1 can be an acyloxy group (-O-CO-R8) ; a (-O-CO₂-R8) group; a (-O-C(O)-NH₂) group; a (-O-C(O)-NHCH3 ) group; a (-O-C(O)-N(CH3)₂) group; hydrogen; a halogen; a ( -CO₂R9 ) group ; a methyl group or other alkyl group; or a hydroxymethyl group (HO-CH₂-)
R2 can be hydrogen; a ( -CO₂R10 ) group ; a methyl group or other alkyl group; a halogen; or a methoxy group; an acyloxy group (-O-CO-R8); or a hydroxymethyl group (HO-CH₂-)
R3 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; a methyl or other alkyl group; a hydroxymethyl group (-CH₂-OH ); a halogen; a hydroxyethyl group (-CH₂-CH₂-OH ); or a (-CH₂-CO₂-R11) group ;
R4 can be hydrogen; a methyl or other alkyl group; methoxymethyl (-CH₂-O-CH₃ ); a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group
R5 can be hydrogen; a methyl or other alkyl group; methoxymethyl (-CH₂-O-CH₃ ); a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group
R6 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; a methyl or other alkyl group; a hydroxymethyl group (-CH₂-OH ); a halogen; a hydroxyethyl group (-CH₂-CH₂-OH ); or a (-CH₂-CO₂-R11) group ;
R7 can be hydrogen; a ( -CO₂R10 ) group; a methyl group or other alkyl group; a halogen; or a methoxy group; an acyloxy group (-O-CO-R8); or a hydroxymethyl group (HO-CH₂-)
Wherein R8 may be a group of the following structure: (-(CH₂)_{N}-CH₃) for N= 0-18; R8 may be a phenyl group, the phenyl group may in turn bear substituents. The group R8 may also be selected such that R8-CO₂H is an amino acid; lactic acid; glycolic acid ( HO-CH₂-CO₂H), glyceric acid ( HO-CH₂-CH(OH)-CO₂H); or acetoacetic acid ( CH₃COCH₂-CO₂H). R8 may in general be any group such that the resulting ester moiety is degraded to the free phenolic hydroxy group in vivo.

Wherein R9 and R10, R11,and R12, may be a group such as methyl , ethyl, phenyl, or benzyl.

Wherein at least one of the following groups: R1; R3 ; R6 is an acyloxy group ( -O-CO-R8 ) or another group which undergoes biotransformation or spontaneous transformation in vivo to ultimately yield a hydroxy group on the phenyl ring in an ortho or para position.

The nature of the group R8 determines the rate at which the resulting ester is cleaved by nonspecific esterase. The solubility of the prodrug may be varied by changing the nature of the groups R1-R8. Water solubility may be enhanced by selecting substituents with hydrophilic groups. Alternatively one may select bulky substituents which disrupt intermolecular forces in the solid phase and lowers the prodrug's melting point. Anderson,B. in Physical Chemical Properties of Drugs, Edited by Yalkoswsky, S. page.231-266 ; Marcel Dekker Inc. New York.

For parent drugs of Structure 1 , the resulting prodrug has the general structure shown below as 3a: 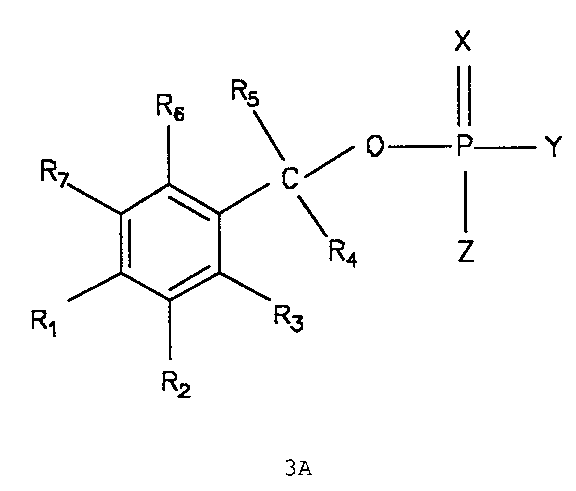
If the parent drug has the structure: 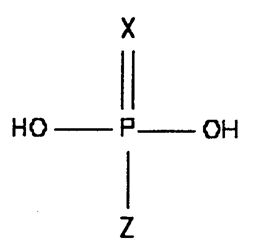
Then the prodrug will have the structure 3B: 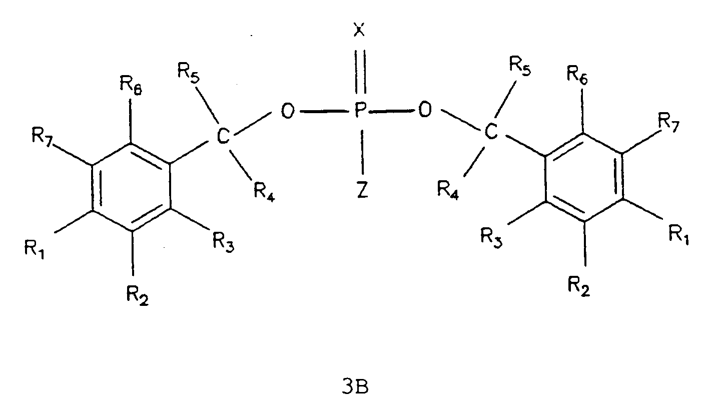
Wherein R1-R7 and X , Y, and Z are as defined previously for Structures 1 and 2.

A key feature of this class of prodrug is the presence of a masked hydroxy group in a para and or ortho position relative to the phospho-ester. Cellular enzymes trigger heterolytic fission of the phospho-ester bond by unmasking the hydroxy group. The ortho or para hydroxy group may be masked as an ester, carbonate, or carbamate. The hydroxy group may also be unmasked by nonenzymatic chemical reaction such as spontaneous hydrolysis.

The following basic chemical principles were exploited in the design of these prodrugs:
1.) Benzyl derivatives undergo solvolysis at rates that are well described by the Hammett equation. Okamoto,Y. and Brown,H.C., J.Org.Chem.,22:485 (1956). The value of the reaction constant depends upon the substituents on the carbon atom to which the phenyl analog is attached. The reaction constant for the solvolysis of t-cumyl derivatives is -4.5. A minimum estimate of the absolute value of the reaction constant for the SN1 solvolysis of the prodrugs is 4.5. Prodrugs in which the substituents R4 and R5 are less electron donating then the methyl group will have a larger + charge in the transition state and a more negative value or the reaction constant. Lowry,T.H.and Richardson,K.S.,in Mechanism and Theory in Organic Chemistry ,2nd ed. Harper and Row,Publishers, N.Y. N.Y. page 354-356 (1981)
2.) Acyloxy groups are only very slightly electron donating. For example the Hammett para sigma + constant for the acetoxy group is -.06. In contrast the hydroxy group is strongly electron donating. The Hammett sigma para + constant for the hydroxy group is -.92. The ionized hydroxy group (-O -) is even more electron donating with a Hammett para sigma + constant that has been estimated at -2.3. Chapman,N.B. and Shorter,J. in Correlation Analysis in Chemistry , Plenum Press, N.Y.,N.Y. page 483-484 ; Vogel,P. in Carbocation Chemistry, Elsevier, N.Y., N.Y. (1985) page 243. ; Hansch,C. in Comprehensive Medicinal Chemistry , Pergamon Press, N.Y., N.Y. , 4:235.
3.) Nonspecific esterase is ubiquitous within the cytoplasm of cells and is able to cleave a wide variety of carboxylate esters. Cleavage of the acyloxy group(s) of the prodrug will trigger heterolytic fission of the C-O bond of the phosphoester. Based on the above considerations the conversion of the group R1 into a hydroxy group will lead to a rate increase of at least 7000 fold. The compound in which R1 is ionized to an oxyanion, O- will undergo solvolysis at a rate of about 2 x 10¹⁰ fold greater. Based on an intracellular pH of 7 and a pKa of 10 for the phenolic hydroxy group about 0.1% of the hydroxy groups will be ionised under physiological conditions. The net result is that overall a rate increase on the order of 2 x 10⁷ fold will occur in the solvolysis reaction following cleavage of a para acyloxy group by nonspecific esterase. Similar rate enhancements are expected if the acyloxy group is in the ortho position
   The mechanism by which the prodrugs undergo transformation to the parent drug is shown below: 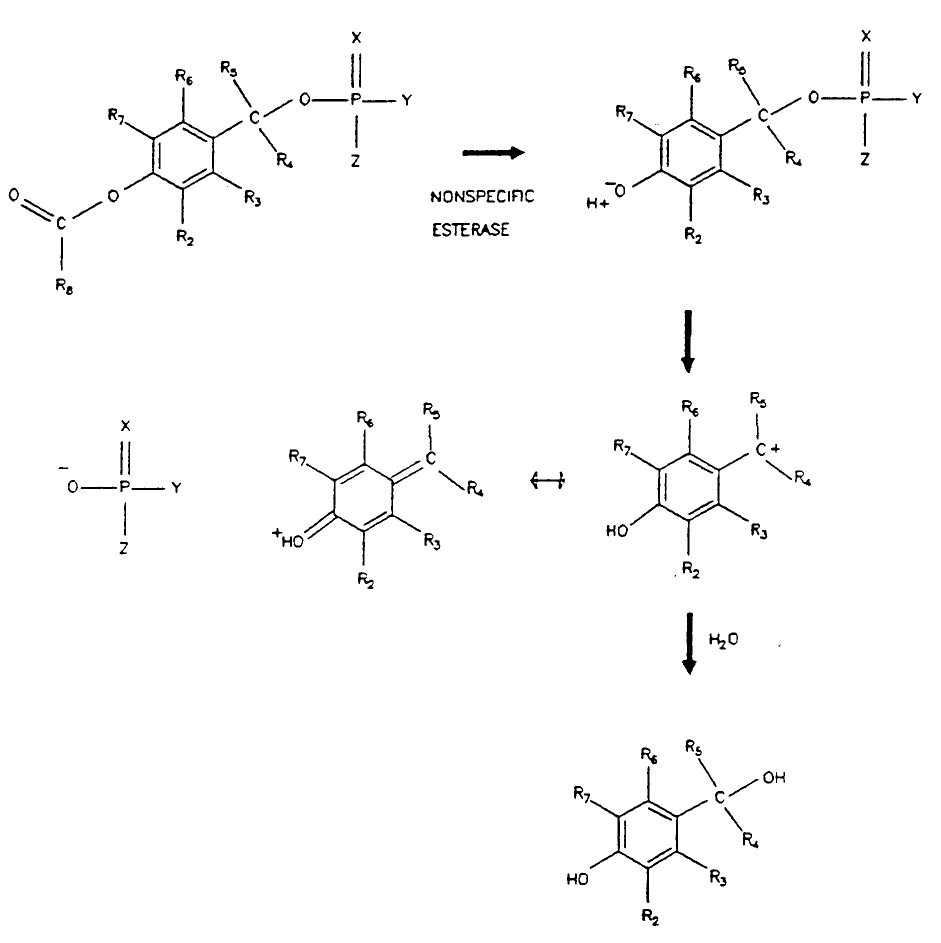 In this figure R1-R8 , X , Y, and Z are as described for Structures 1 and 2. The carbocation shown above may also decay by an elimination reaction which is not shown in the above figure.
4.) The rate of spontaneous hydrolysis of the prodrug is a function of the sum of the Hammet sigma+ constants of the substituents on the benzyl ring and a function of the Hammet sigma+ constants of the groups R4 and R5.
5.) The carbocation that arises from the solvolysis reaction will be consumed by an intramolecular nucleophilic reaction if a nucleophile is suitably positioned. For example, if R3 is a hydroxymethyl group then the effective intramolecular molarity for reaction at a benzylic carbocation would likely be in the range of 10⁶ to 10⁷ Molar. Kirby,A.; Advances in Physical Organic Chemistry; 17:183 ; 1980.

### Toxicological Considerations

Neutrally charged phosphorous derivatives are among the most toxic compound known. The toxicity is due to the ability of the compounds to cause suicide inhibition of the enzyme acetylcholinesterase. The present prodrugs were specifically designed to avoid this toxicity. The ability of phosphate esters and related compounds to inhibit acetylcholinesterase is a sensitive inverse function of the pKa of the esterified alcohol group. Ashani,Y. et al. J.Medicinal Chem., 16:446 (1973). The high pKa of the esterified alcohol group in the present prodrug method effectively precludes anticholinesterase activity.

The carbocation that results from the heterolytic cleavage of the C-O bond during the transformation of the prodrug to the parent drug will be consumed by reaction with water to form an alcohol , by an elimination reaction , or by an intramolecular nucleophilic reaction. The exact mechanism(s) of carbocation decay will depend upon the nature of the substituents on the carbocation.

### Preferred Embodiments of the Prodrug Method:

Some preferred embodiments of the prodrug method consist of replacing one or more of the hydroxy groups on the phosphorous with a group of the structures shown below as Structures 4A-D : 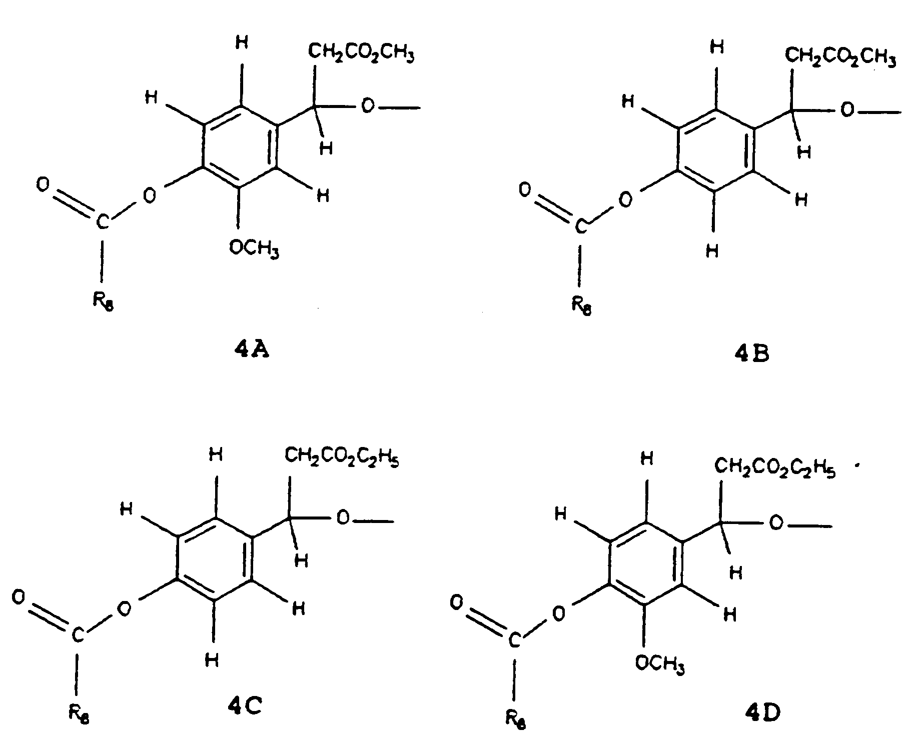
Wherein R8 may in general be any group such that the resulting acyloxy moiety is degraded to the free hydroxy group intracellularly. Some preferred embodiments for the group R8 are the following methyl ; ethyl; phenyl; isopropyl; t-butyl; and the selection of R8 such that the structure R8-CO₂H is an amino acid. For example R8 may be an aminomethyl group NH₂-CH₂- in which case R8-CO₂H would be the amino acid glycine. The group R8 may also be selected such that R8-CO₂H is; lactic acid; glycolic acid ( HO-CH₂-CO₂H), glyceric acid ( HO-CH₂-CH(OH)-CO₂H); or acetoacetic acid ( CH₃COCH₂-CO₂H).

### EXAMPLES:

To demonstrate the mechanism of action of the prodrug method methylphosphonic acid was employed as a model "drug" compound. Neutrally charged lipophilic derivatives of methylphosphonic acid were prepared using two representative embodiments of the prodrug methodology. The transformation of these compounds to methylphosphonic acid via nonspecific esterase was then demonstrated.

### Example 1: Bis (4-acetoxy-3-methoxybenzyl) methylphosphonate

### Synthesis: 4-acetoxy-3-methoxybenzyl alcohol

A solution of 4-acetoxy-3-methoxybenzaldehyde (Aldrich) 10 grams ( 0.05 Moles) in 200 ml of ethanol was treated with 10% palladium on carbon (1.0 gram) and hydrogenated on a Parr apparatus at a pressure of 20 PSI until no further uptake of hydrogen was observed. The catalyst was removed from the mixture by filtration with celite and washed with 50 ml of ethanol. The combined filtrates were concentrated with an aspirator at 40 C to give an oil. The oil was dissolved in 100 ml of toluene, concentrated at 40 C with an aspirator and dried at 0.01 mm Hg at room temperature to give 10 grams (ca. 100% yield) of product as a colorless oil.
Proton NMR in CDCL₃ at 60 MHZ with TMS as standard revealed: at 2.27 (3H ,singlet); at 3.76 (3H,singlet); at 4.53 (2H, singlet) 6.9-7.3 (3H, multiplet)

### Bis (4-acetoxy-3-methoxybenzyl) methylphosphonate

A solution of 4-acetoxy-3-methoxy-benzyl alcohol ( 2.94 gms, 15 mmol) in 20 ml of anhydrous diethyl ether containing n-methylimidazole (0.82 gms , 10 mmol) (Aldrich) and triethylamine (1.01 Gms 10 mmol) under a nitrogen atmosphere was treated with 0.66 gms (5 mmol) of methylphosphonic dichloride ( Aldrich). The mixture was stirred for 10 minutes at room temperature, diluted with 100 ml of methylene chloride and cooled to 5 C. Water 50 ml was added dropwise over a period of 20 minutes to the mixture which was kept at a temperature of 5-10 C. The organic phase was separated, washed times 2 with 25 ml of 5% aqueous sodium bicarbonate, water ( 25 ml x 2 ), and 25 ml of saturated aqueous sodium chloride. The organic phase was dried with MgSO₄ and concentrated with an aspirator at 25 C followed by drying at 0.01 mm Hg at room temperature overnight to give approximately 2.5 gms of product as a colorless oil. Proton NMR in CDCL₃ at 60 MHZ with TMS as standard revealed: at 1.5 (3H doublet, J 18 Hz); 2.30 (6H ,singlet); at 3.83 (6H singlet); at 5.0 (4H, doublet, J 10 Hz ); 6.8-7.3 (6H, multiplet)
For some studies the above preparation of bis (4-acetoxy-3-methoxybenzyl) methylphosphonate was subjected to further purification by additional partitioning between chloroform and aqueous sodium bicarbonate. The organic phase was then dried and removed exvacuo.

### DEGRADATION STUDIES

The conversion of the model prodrug compound; bis (4-acetoxy-3-methoxybenzyl) methylphosphonate was examined using a Varian Unity 300 MHZ NMR Spectrometer. Porcine liver esterase was purchased from Sigma Chemicals. The porcine liver esterase had an activity of 230 units/mg. The kinetics of decomposition of the model compound was followed by taking serial NMR spectra. The experiments were conducted with approximately 8 mg of the model compound in 0.75 ml of deuterium oxide buffered to pH 7.2 with Tris(hydroxy-d-methyl)amino-d₂-methane at 37 C . In early experiments the Tris buffer was .05 M. In later experiments the Tris molarity was increased to 0.10 M which provided for better pH control. Bis (4-acetoxy-3-methoxybenzyl) methylphosphonate ester, mono (4-acetoxy-3-methoxybenzyl) methylphosphonate ester, and methylphosphonic acid were readily distinguished by the differing chemical shifts of the methyl protons which were coupled to the phosphorous.

In the absence of esterase Bis (4-acetoxy-3-methoxybenzyl) methylphosphonate was relatively stable. The compound was observed to undergo slow solvolysis to mono (4-acetoxy-3-methoxybenzyl) methylphosphonate and 4-acetoxy-3-methoxybenzyl-alcohol. This occurred with a half life of approximately 10 hours.

In the presence of porcine liver esterase (40 micrograms) Bis (4-acetoxy-3-methoxybenzyl) methylphosphonate was degraded to acetic acid, 4-hydroxy-3-methoxybenzyl alcohol and mono (4-acetoxy-3-methoxybenzyl) methylphosphonate. This reaction occurred extremely rapidly with a half life much less then 1 minute. At 1 minute the bis ester was no longer detectable. The mono (4-acetoxy-3-methoxybenzyl) methylphosphonate was in turn degraded to methylphoshonic acid, acetic acid, and 4-hydroxy-3-methoxybenzyl alcohol. This reaction occurred with a half life of approximately 16 minutes. After 90 minutes monoester was no longer detectable. To verify that the observed degradation product was indeed methylphosphonic acid a small quantity of authentic methylphosphonic acid was added to the NMR tube. As expected the NMR absorption peaks were identical.

The likely reaction pathway is illustrated below: 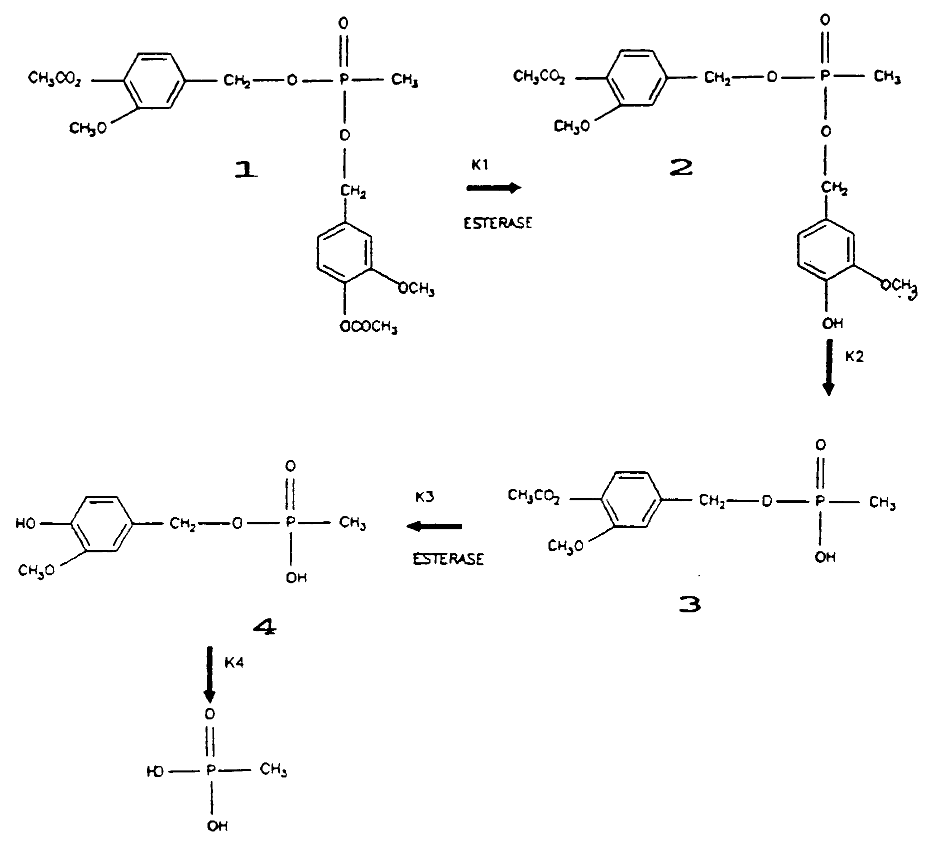
The rate constants K2 and K4 are likely very large since structures 2 and 4 are not detectable in the NMR spectra.

### Example 2

This second example of the prodrug method again employs methylphosphonic acid as a model phosphonate "drug" compound. The bis ester of ethyl 3-hydroxy-3-(4-acetoxyphenyl)propionate was synthesized. Treatment with nonspecific esterase resulted in the transformation of this compound to methylphosphonic acid and 4-hydroxycinnamic acid and ethyl 4-hydroxycinnamate.

### Synthesis: ethyl 3-hydroxy-3-(4-acetoxyphenyl)propionate

This novel compound was synthesized by the acylation of 4-hydroxyacetophenone with diethylcarbonate to yield ethyl 4-hydroxybenzoylacetate. Treatment with acetic anhydride and subsequent catalytic hydrogenation yielded the desired product. The experimental details are given below:

### Synthesis of ethyl 4-hydroxybenzoylacetate

50 ml of diethylcarbonate (Aldrich Chemicals) was heated to refluxed at 126 C under nitrogen with a Vigreaux column leading to a short path distillation apparatus. Then the oil bath was removed and 3.0 grams of sodium was slowly added. A thick purple-brown slurry formed. The mixture was then heated to 140 C. and a solution of 6.0 grams of 4-hydroxy-acetophenone, (Aldrich) in 60 ml of diethylcarbonate was slowly added. The reaction mixture became lighter in color and more viscous. Ethanol began to distill and 11 ml were collected. The oil bath temperature was increased to 180 C and the mixture was heated until the distillate temperature exceeded 120 C. The mixture was then cooled to 30 C and poured onto 30 ml of 3N HCL and 30 ml of crushed ice. The flask was then rinsed with another 10 ml of 3N HCL and 10 ml of crushed ice. The yellow organic layer was separated and the aqueous phase was extracted twice with 50 ml of ether. The combined organic phases were then dried over magnesium sulfate. The organic extracts were then filtered and dried to give 8.2 grams of a crude product as a yellow oil. The oil was then purified by flash chromatography on silica gel with 15% ethyl acetate/petroleum ether. 4.2 grams of the ethyl 4-hydroxybenzoylacetate were isolated. Proton NMR in CDCL₃ at 60 MHZ with TMS as standard revealed: at 1.27 (3H triplet) ; 4.0 (2H ,singlet); at 4.26 (2H quartet); at 6.9 (2H, doublet); at 7.9 (3H, doublet)

### Synthesis of ethyl 4-acetoxy-benzoylacetate

1.85 grams of ethyl 4-hydroxybenzoylacetate was added to 15 ml of anhydrous pyridine. 0.91 grams of acetic anhydride was added along with a few crystals of dimethylaminopyridine (Aldrich). After 1 hour at room temperature the pyridine was removed exvacuo. The oily residue was stirred with 25 ml of water and 25 ml of ether. The aqueous phase was separated and extracted twice with 50 ml of ether. The combined ether extracts were washed with 0.5N HCL (25 ml), then with saturated sodium bicarbonate solution (25 ml), and finally with 25 ml of brine. The extracts were then dried with magnesium sulfate, filtered and the ether removed exvacuo. To yield 2.07 grams of ethyl 4-acetoxy-benzoylacetate. Proton NMR in CDCL₃ at 60 MHZ with TMS as standard revealed: at 1.2 (3H triplet) ; at 2.27 (3H ,singlet); at 4.0 (2H ,singlet); at 4.2 (2H quartet); at 7.2 (2H, doublet); at 8.0 (2H, doublet)

### Synthesis: ethyl 3-hydroxy-3-(4-acetoxyphenyl)propionate

### METHOD A

A 50 ml flask was flushed with nitrogen and charged with 1.0 grams of ethyl 4-acetoxy-benzoylacetate and 15 ml of methanol. Platinum dioxide (Aldrich, 0.05 gm) was added and the flask was flushed with nitrogen before being filled with hydrogen. The mixture was stirred at room temperature and atmospheric pressure. After 26.5 hours of reaction time an additional 25 mg of platinum dioxide was added. After 93 hours the reaction mixture was filtered through celite. The celite was washed with methanol. The combined filtrates were rotary evaporated to yield 0.95 grams of crude product as an oil. The oil was purified by flash chromatography on silica to give 0.33 grams of a yellow solid, which was recrystallized from 25% diethyl ether/petroleum ether. A total of 0.24 gm of white crystalline product identified as ethyl 3-hydroxy-3-(4-acetoxyphenyl)propionate was obtained. The melting point was 74-75 C. Elemental analysis and NMR confirmed the product identity. Proton NMR in CDCL₃ at 300 MHZ with TMS as standard revealed: at 1.265 (3H, triplet) ; at 2.203 (3H, singlet) at 2.715 (2H, doublet with second order splitting); at 3.332 (1H doublet); at 4.184 (2H, quartet); at 5.125 (1H, triplet with second order splitting); at 7.084 (2H, doublet); at 7.377 (2H, doublet)

### Synthesis: ethyl 3-hydroxy-3-(4-acetoxyphenyl)propionate

### METHOD B

A 250 ml round bottom flask was charged with 11.25 grams ( 0.045 mol.) of ethyl 4-acetoxy-benzoylacetate and 150 ml of diethyl ether. The solution was stirred and ammonia-borane (1.39 gm., 0.045 mol., Alfa) was added in 4 portions. After being stirred 90 minutes at room temperature 50 ml of ether were added and the reaction mixture was slowly poured into a 400 ml beaker containing 20 ml of 3 N HCL and 100 ml of crushed ice. After 15 minutes the organic phase was separated. The aqueous phase was extracted with ether (2 X 100 ml). The combined organic phase was washed with brine, dried over magnesium sulfate, filtered and evaporated. The crude product was purified by flash chromatography, using a 7 cm. diameter column of 250 grams silica gel, packed and eluted with 25% ethyl acetate/petroleum ether. 6.53 grams of ethyl 3-hydroxy-3-(4-acetoxyphenyl)propionate was obtained. The product was identical to that obtained by method A as determined by NMR and elemental analysis.

### Synthesis of:

bis ((3-(ethyl 3-hydroxy-3-(4-acetoxyphenyl)propionate)) methylphosphonate 100 mg of ethyl 3-hydroxy-3-(4-acetoxyphenyl)propionate was dissolved in 1.5 ml of dichloromethane (Anachemia, distilled over phosphorous pentoxide). N-Methylimidazole (Aldrich, 0.029 ml) and triethylamine (Aldrich, distilled over calcium hydride, .050 ml) were stirred into the solution. Methylphosphonic acid dichloride (Aldrich, 0.024 gm) in 0.50 ml of anhydrous dichloromethane was added to the stirred reaction mixture. The solution was stirred for 105 minutes at room temperature and then refluxed for 30 minutes. The cooled solution was then diluted with 5 ml of dichloromethane and 3 ml of water was added. The organic layer was separated and washed successively with saturated sodium bicarbonate ( 2 X 5ml), water (2 X 5ml), and brine (5 ml). The organic phase was then dried over magnesium sulfate. The organic phase was then filtered and dried exvacuo to yield .107 grams of crude product. This product was then partially purified on a silica gel column which was eluted with 40% to 60% ethyl acetate in petroleum ether. The silica gel chromatography cleanly separated residual ethyl 3-hydroxy-3-(4-acetoxyphenyl)propionate from the products. The chromatography however, yielded incomplete separation of the mono and bis methylphosphonate esters from each other as evidenced by proton NMR and phosphorous NMR. The sample was further purified by hplc on a semiprep silica gel column (Rainin Dynamax Microsorb , 5 micrometerlx 25 cm) with chloroform:cyclohexane ,80:20, as eluent at a flow rate of 4.7 ml/min. Three fractions were isolated and examined by proton and phosphorous NMR. Fraction 1 was the d-l pair of the bis-ester, fraction 2 was the meso isomer of the bis isomer, fraction 3 was a mixture of the meso isomer of the bis ester and the mono-ester. Analytical data for fraction 1 (d-l pair) is given below. Proton NMR in CDCL₃ at 300 MHZ with TMS as standard revealed:1.11 (3H, doublet J= 17.7 hz); 1.207 (6H,triplet); 2.72 (2H, multiplet); 2.98 (2H, multiplet); 4.10 (4H, multiplet); 5.79(2H, quartet); 7.08(4H, doublet); 7.41 (4H,doublet). High resolution fab mass spectroscopy revealed a molecular weight of 564.176 (+/-.003) Calculated mass for C₂₇H₃₃O₁₁P is 564.1743. This data confirms that the isolated product is the desired bis ((3-(ethyl 3-hydroxy-3-(4-acetoxyphenyl)propionate)) methylphosphonate.

### DEGRADATION STUDIES

The degradation of Bis ((3-(ethyl 3-hydroxy-3-(4-acetoxyphenyl)propionate)) methylphosphonate was examined as described above in Example 1. Both the d-l pair and meso isomer were examined in separate experiments and behaved similarly. The compound was dissolved in D₂O containing 0.10 M tris buffer at pH 7.2 and a temperature of 20 C.

In the absence of esterase the NMR spectrum remained unchanged over a time period of 1 hour. This indicates bis esters are stable under these conditions.

In the presence of porcine liver esterase ( 33 micrograms) the d-l pair was rapidly converted into acetic acid, mono ((3-( ethy 3-hydroxy-3-(4-acetoxyphenyl) propionate ) ) methylphosphonate, and ethyl 4-hydroxycinnamate. By 30 minutes the bis ester was no longer detectable and 46% of the aromatic molecules present were ethyl 4-hydroxycinnamate. By 120 minutes hydrolysis of the acetoxy groups to acetic acid was essentially complete. An intermediate presumed to be mono ((3-(ethyl 3-hydroxy-3-(4-hydroxyphenyl)propionate)) methylphosphonatethe was noted by NMR. By 180 minutes 81% of the aromatic molecules were ethyl 4-hydroxycinnamate or 4-hydroxycinnamic acid. At 520 minutes 94% of the aromatic molecules were ethyl 4-hydroxycinnamate or 4-hydroxycinnamic acid. At 20 hours the NMR spectra revealed that the only products present were methylphosphonic acid, acetic acid, and trans 4-hydroxycinnamic acid, and ethanol.

To verify that the observed degradation product was indeed methylphosphonic acid a small quantity of authentic methylphosphonic acid was added to the NMR tube at 20 hours. As expected the NMR absorption peaks were identical.

The 4-hydroxycinnamic acid that was formed had the following NMR spectrum (300 MHz) in the buffered D₂O : at 6.39 (1H doublet, J 15.9); 6.94 (2H doublet, J=8.7); 7.36(1H, doublet J=16 ); 7.55(2H doublet J=9). A decoupling experiment demonstrated that the protons at shifts 6.4 and 7.4 were magnetically coupled.

The kinetics of the liberation of methylphosphonic acid is shown in the graph below: 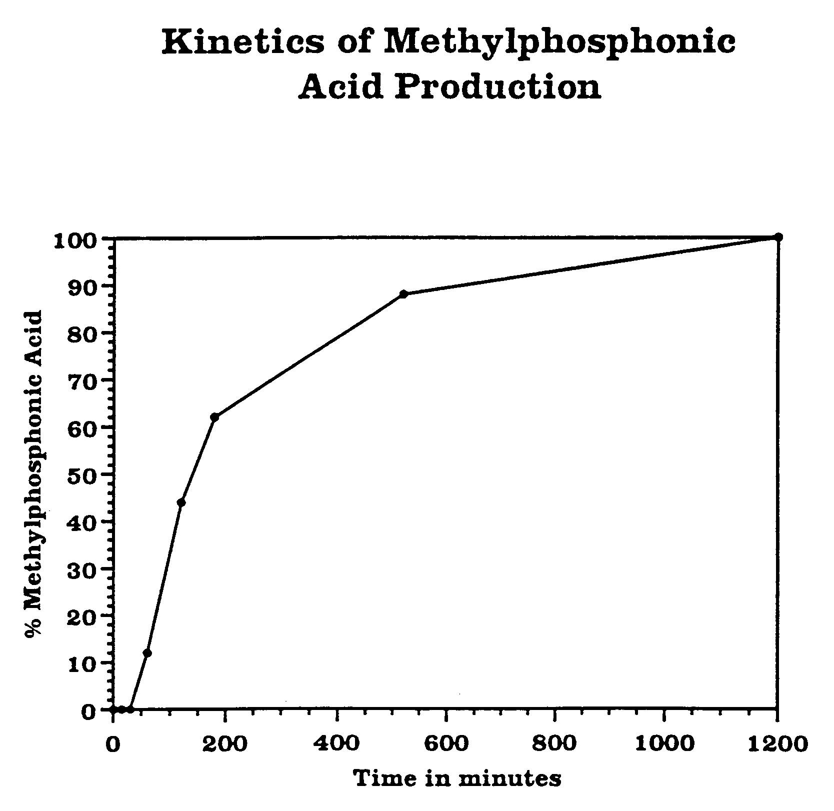
The precise mechanistic details of the esterase catalyzed decomposition of Bis ((3-(ethyl 3-hydroxy-3-(4-acetoxyphenyl)propionate)) methylphosphonate remains to be defined. However, it is clear that the compound is readily transformed by the action of nonspecific esterase into methylphosphonic acid and the elimination product 4-hydroxycinnamic acid. The overall reaction is summarized below: 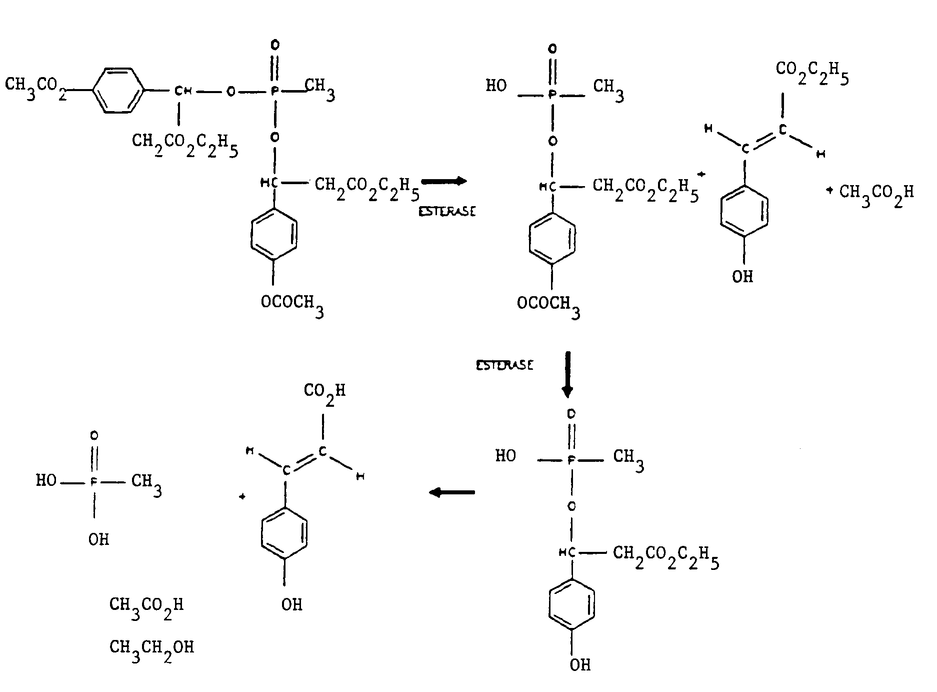
Regardless of the precise mechanistic details the results demonstrate given in Examples 1 and 2 demonstrate that a compound such as methylphosphonic acid may be converted into a neutrally charged ester which is lipophilic using the present prodrug method. This is evidenced by solubility in organic solvents such as methylene chloride, chloroform, and diethyl ether. In the presence of nonspecific esterase the esters are rapidly transformed to the parent phosphonic acid. In both Example 1 and 2 the function of the esterase is to unmask a hydroxy group which in turn leads to cleavage of the C-O bond of the phospho-ester.

Bis ((3-(ethyl 3-hydroxy-3-(4-acetoxyphenyl)propionate)) methylphosphonate is a representative example of a new class of phosphorous esters which undergo cleavage to the parent phosphorous acid by an elimination reaction which is triggered by the enzymatic unmasking of a hydroxy group in an ortho or para position on the phenyl ring. The powerful electron donating characteristics of the (ionized) hydroxy group promote heterolytic cleavage of the C-O bond of the phosphorous ester. An elimination reaction will result when the energy barrier for proton removal is significantly lower then the energy barrier for nucleophilic attack by solvent. The elimination reaction is favored by groups which lower the intrinsic barrier for proton removal. The transition state for proton removal is also likely stabilized by the partial formation of a conjugated double bond system. In the solvolysis of 1-phenylethyl chlorides only a small per cent of the carbocations decay via an elimination reaction to styrene. Richard,J.P. ; Jencks,W.P.; J.Am.Chem.Soc. 106:1373 ;1984. In the model compound of Example 2 the function of the ethoxycarbonyl group is to increase the pKa of the protons on the adjacent carbon atom and to lower the intrinsic energy barrier for proton removal. This causes the carbocation that results from the solvolysis of the phosphonate to decay exclusively via an elimination reaction. It should be emphasized that other groups such as -CO-CH₃ , -CO-NH₂, -NO₂ , CH₃-SO₂- ; which lower the intrinsic barrier for proton abstraction from an adjacent carbon atom may be employed in place of the ethoxycarbonyl group and will similarly lead to carbocation decay via an elimination reaction. One skilled in the arts of chemistry will recognize other groups which also lower the intrinsic barrier for proton abstraction. These groups will work equally well to insure that the carbocation that results from the solvolysis of the prodrug will decay via an elimination reaction. These are to be considered within the scope of the present invention.

In Examples 1 and 2 a para hydroxy group is unmasked by the action of esterase. Similar electrical and resonance effects are expected for the stabilization of carbocation formation by a hydroxy or oxy anion group located in the ortho position. Fujita,T. and Nidhioka,T. ; Progress in Physical Organic Chemistry ; 12:49. Accordingly, a prodrug in which an ortho hydroxy group is unmasked via cellular enzymes will degrade in a similar manner.

In Examples 1 and 2 an ester group is cleaved by esterase to expose a hydroxy group on the phenyl ring. Phenolic carbonates and carbamates are also degraded by cellular enzymes to yield phenols.
Dittert,L.,et al. J.Pharmaceutical Sci. ; 57:783, 1968; Dittert,L.,et al. J.Pharmaceutical Sci. ; 57:828, 1968; Dittert,L.,et al. J.Pharmaceutical Sci. ; 58:557, 1969; King,S., Lum,V., Fife,T.; Biochemistry ; 26:2294 , 1987 Lindberg,C., Roos,C.,Tunek,A., Svensson,L.; Drug Metabolism and Disposition ; 17:311 ; 1989. ; Tunek,A. Levin,E., Svensson,L.; Biochemical Pharmacology ; 37:3867 ; 1988
In addition a variety of carbonate and carbamate groups are known which undergo spontaneous cleavage in solution at kinetically favorable rates. Saari,W., et. al.;J.Medicinal Chem. ; 33:97 ; 1990 Rattie,E. et. al. ; J.Pharmaceutical Sci. ; 59:1741, 1970; The key requirement of the prodrug method is the presence of a group on the phenyl ring which is converted to a hydroxy group. Since carbonates, and carbamates, and esters all are known to undergo this transformation in vivo any of these groups may be employed to mask the hydroxy group in the prodrug. Advantage may be made of the relative rate of metabolism of various masking groups to provide selective activation of the prodrugs in certain tissues. For example, carbamates are almost exclusively metabolized in the liver. Prodrugs in which the hydroxy group is masked as a carbamate group will be perferentially metabolized in the liver. This will allow the selective delivery of antiviral nucleotide analogs into hepatic cells. This should prove quite useful in designing antiviral drugs against hepatitis. One skilled in the arts of medicinal chemistry will recognize other masking groups which enzymatically and/or spontaneously degrade to unmask a hydroxy group on phenyl ring. These groups may be employed in the present prodrug method and are to be considered to be within the scope of the present invention.

### EXAMPLE 3

### A PRODRUG FOR 3'AZIDO-3'DEOXYTHYMIDINE 5'PHOSPHATE

The prodrug method was employed to synthesize a neutrally charged, lipophilic prodrug for the 3'azido-3'deoxythymidine 5'phosphate. The resulting prodrug was an extremely potent inhibitor of the the AIDS virus in vitro. The structure of this prodrug is shown below as STRUCTURE 5:

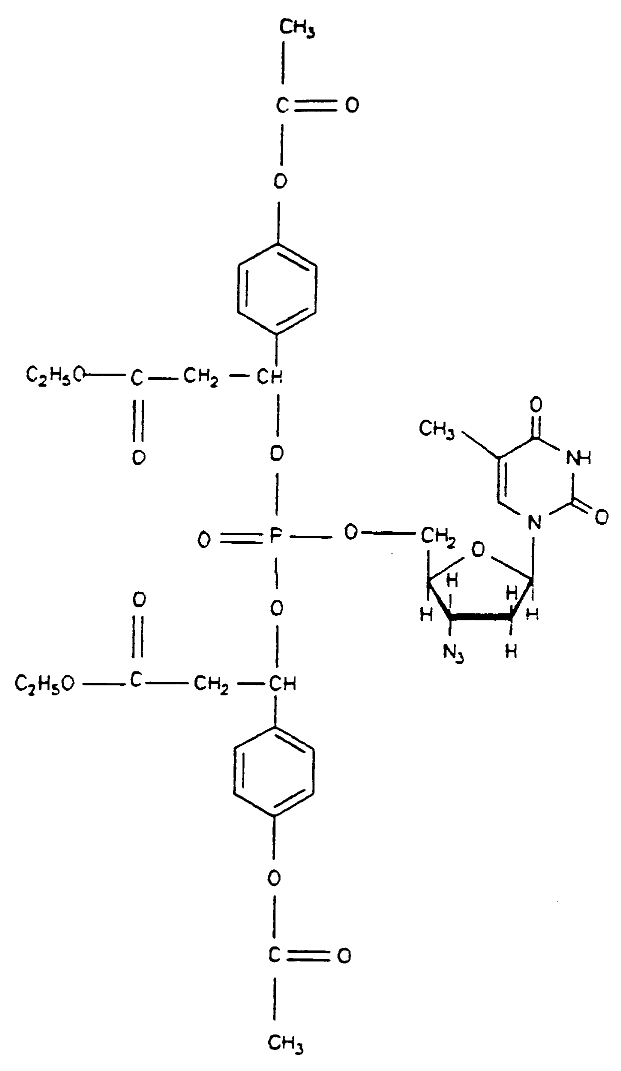

### SYNTHESIS OF A PRODRUG FOR AZT

The scheme utilized to synthesize the prodrug for AZT shown in Structure 5 is shown below:

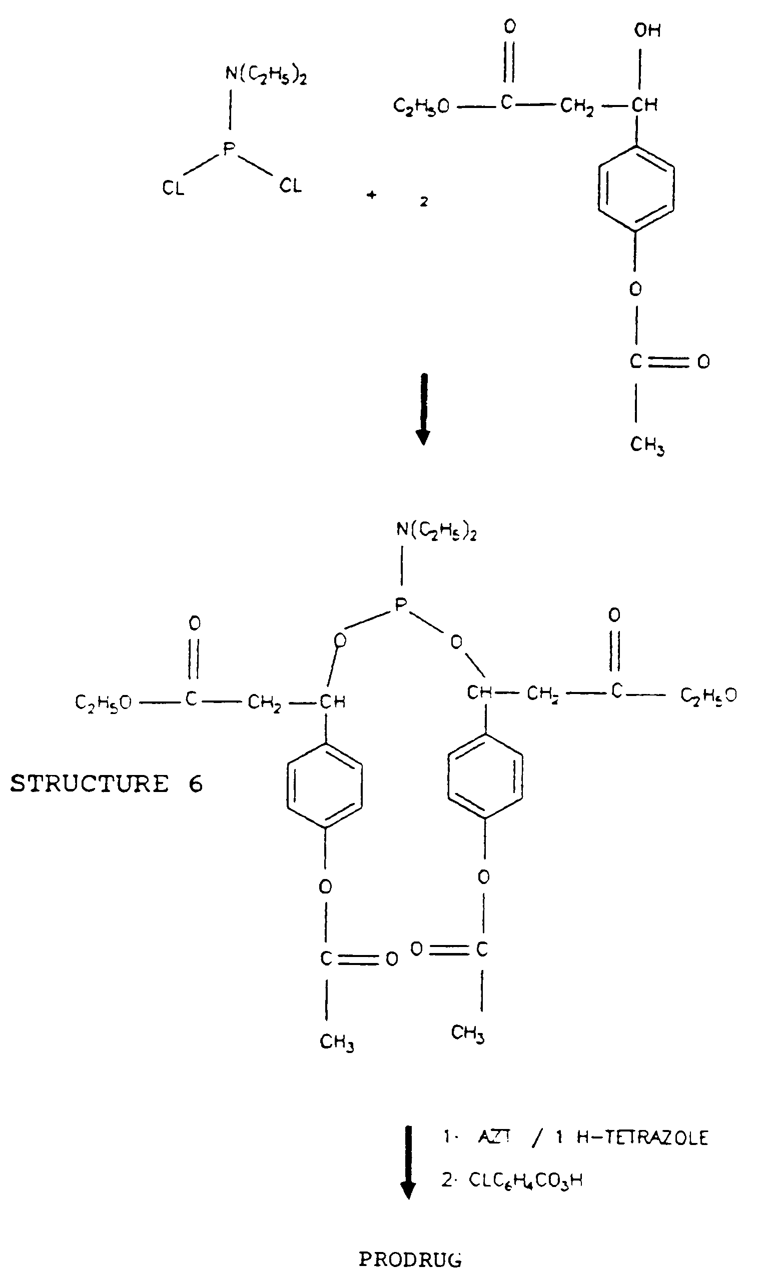

### SYNTHESIS OF DIETHYLPHOSPHORAMIDOUS DICHLORIDE

Under an atmosphere of dry nitrogen , diethylamine ( 68.9 grams, 0.942 moles ) was added dropwise over a period of 1 hour to a vigorously stirred solution of PCL₃ (64.7 grams, 0.47 moles) in 500 ml of anhydrous diethyl ether, which was maintained below 0 C by an external dry ice /acetone bath. When the addition was completed , the cooling bath was removed and stirring was continued for 3 hours. The precipitated diethylamine hydrochloride was removed by filtration and the solvent removed exvacuo. The residue was purified by distillation to yield 54.04 grams (66% yield) of product. (B.P. 61-62 C/ at 7 torr.) This was based on the method of Perich,J. and Johns,R. Synthesis 142 (1988)

### SYNTHESIS OF INTERMEDIATE GIVE BY STRUCTURE 6:

A solution of freshly distilled diethylphosphoramidous dichloride (0.607 gm, 3.49 mMoles) was added to 5 ml of anhydrous diethyl ether under a dry nitrogen atmosphere and cooled to - 20 C. A solution of ethyl 3-hydroxy-3-(4-acetoxyphenyl)propionate (1.760 gm, 6.98 mMoles) and dry triethylamine (0.777 gms, 7.68 mMoles) in 50 ml of diethyl ether was added dropwise over 15 minutes. After stirring for 15 more minutes the external cooling was removed and the reaction mixture was stirred at room temperature for 20 hours. The triethylamine hydrochloride was then removed by filtration under a nitrogen atmosphere. Evaporation of the filtrate yielded 2.29 grams of a viscous oil. The oil was then dissolved in 100 ml of methylene chloride, and was washed at 4 C with 50 ml of saturated aqueous saline, followed by 5% NaHCO₃ (50 ml X 2) , and 50 ml of water. The organic phase was then dried over sodium sulfate, filtered and dried exvacuo at room temperature. The result was 2.22 grams of a semi-solid gum.The product was not stable to silica chromatography and was employed without further purification in the next step. FAB mass spectroscopy and proton NMR were consistent with the product being that of the N,N diethylphosphoramidite shown in Structure 6.

### SYNTHESIS OF THE AZT PRODRUG SHOWN AS STRUCTURE 5 :

1-H-tetrazole (462 mg, 6.60 mMoles) was added to 2.00 grams of the N,N diethylphosphoramidite (Structure 6) and to 3'azido-3'deoxythymidine (707 mg, 2.63 mMoles) in 10 ml of anhydrous tetrahydrofuran. (The AZT provided by Aldrich contained 0.085 moles water per mole of AZT.) After 1.5 hours the solution was cooled to -40 C and and a solution of 3-chloroperoxybenzoic acid (873 mg, 4.3 mMoles as 85% reagent) in 15 ml of dry methylene chloride was added rapidly in a dropwise fashion. External cooling was removed and after 15 minutes, 20 ml of 10% aqueous NaHSO₄ was added and stirred vigorously for 10 minutes. The mixture was transferred to a separatory funnel with the addition of 50 ml of methylene chloride. Upon shaking an intractable emulsion was formed. After the addition of 500 ml of diethyl ether a clean phase separation was obtained. The organic phase was washed with 30 ml of 10% aqueous NaHSO₄ ,saturated NaHCO₃ (25 ml X 2) ,and water (25 ml X 2). The organic phase was then dried over sodium sulfate, filtered and dried exvacuo to yield 2.30 grams of crude product. The desired product was then purified by silica gel chromatography with CHCL₃/methanol 99:1 as eluant. 821 mg of product was obtained which was pure by TLC. NMR revealed a mixture of diastereomers. FAB mass spectroscopy revealed a molecular weight of 815. In an attempt to separate the d-l pair form the meso pair of isomers the sample was rechromatographed on a longer silica column and eluted with CHCL₃/methanol 99:1. This second chromatography was unsuccessful and complicated by the appearance of a hydrocarbon peak noted on NMR. This hydrocarbon peak was traced to a contaminant present in a commercially obtained reagent grade chloroform. The sample accordingly was subjected to a third chromatographic purification on a 3 inch silica column which was eluted with chloroform followed by CHCL₃/methanol 99:1. This removed all detectable traces of the hydrocarbon contaminant. 260 mg of product was isolated. This material was pure on TLC and by high field NMR. No AZT was noted on HPLC examination of a sample obtained after the first of the three chromatographic separation described above. High resolution FAB mass spectroscopy revealed a molecular weight of 815.2440 +/_ .0043 . The calculated mass is 815.2415. The proton NMR revealed the presence of 4 diastereomers. Two pairs of isomers were separated by HPLC on silica. However, attempts to further resolve the mixture were unsuccessful. P³¹ NMR demonstrated 2 phosphorous peaks in each mixture of diastereomers. The structure was further confirmed by proton NMR COSY analysis. The 300 MHZ NMR is shown below:

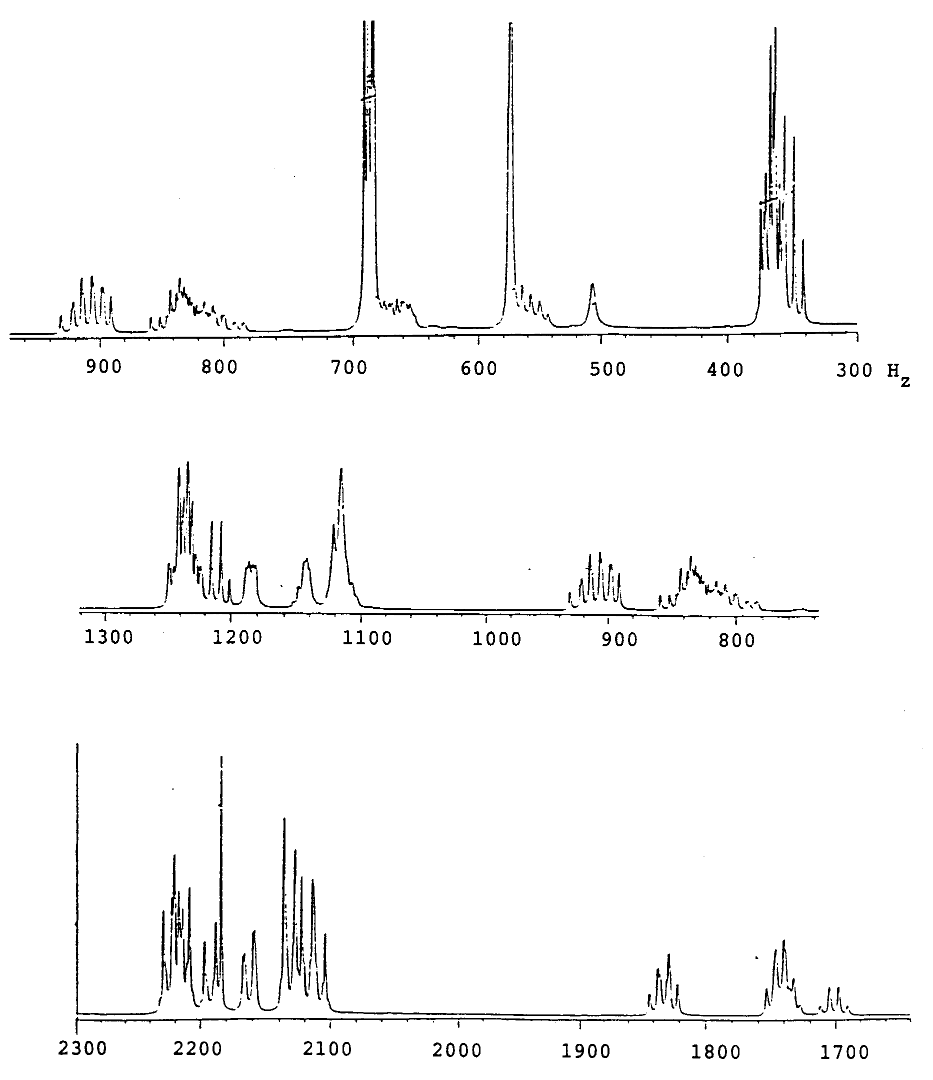

### ANTIVIRAL ACTIVITY OF THE PRODRUG FOR AZT

The antiviral activity of the AZT prodrug was evaluated in the Retrovirus Research Laboratory at Southern Research Institute. The AZT prodrug (Structure 5) was tested against HIV in CEM cells in a standardized assay system. In this assay the CEM cells were grown in microtitre plates and incubated for 6 day with or without a HIV. The cells were incubated with prodrug or AZT at varying concentrations. After 6 days the percentage of viable cells was measured by employing a standardized assay based upon the conversion of a tetrazolium salt to a formazan chromaphore by viable cells. (T.Mossman; J.Immunol.Methods 65:55 (1983)) The production of the chromaphore was measured spectrophotometrically with a Molecular Devices Vmax plate reader at 570 nm. From the optical density data a computer program calculated the per cent reduction in viral cytopathic effect (CPE), the prodrugs cytotoxic effect, and IC₅₀ values.

The prodrug for AZT proved to be an extremely potent inhibitor of the AIDS virus as measured in this assay. The IC₅₀ value; defined as that concentration of drug inhibited the HIV induced CPE 50% was 24 nanamolar for the AZT prodrug. The TC₅₀ value; defined as that concentration of drug that resulted in a 50% reduction of cell viability in uninfected cells was 7800 nanmolar. When the prodrug was added to the cell cultures 8 hours prior to infecting with HIV the IC₅₀ for the prodrug was 5 nanamolar. The IC9₉₀ for the prodrug was 10 nanamolar. The IC₅₀ for AZT under the same conditions was also 5 nanamolar. The IC₉₀ for AZT was 10 nanamolar. The data is summarized in the graph below for an experiment in which the prodrug or AZT were added 8 hours prior to the time of infection with the HIV. 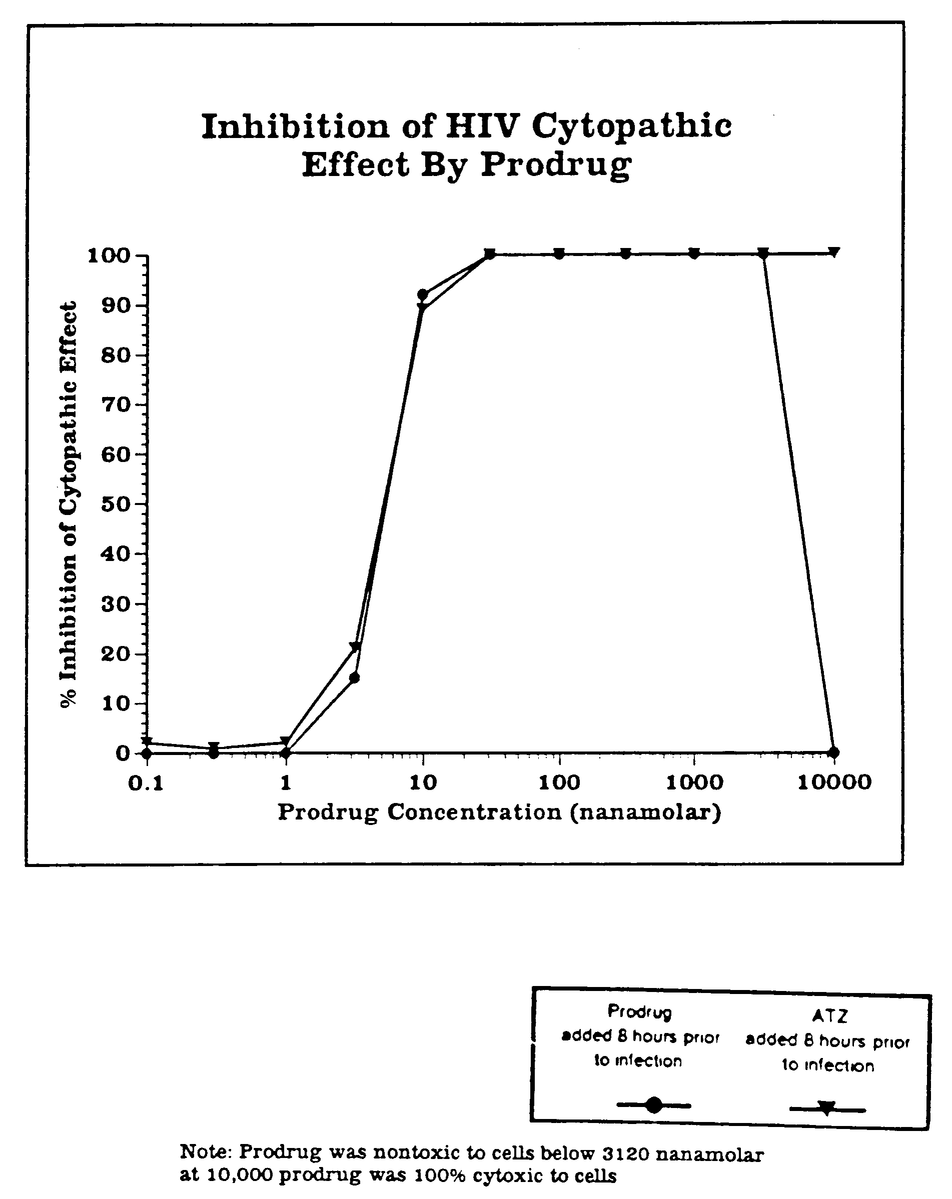
The graph below summarizes the results of an experiment in which the antiviral activity of the AZT prodrug was added 8 hours prior to infection or at the time of infection.

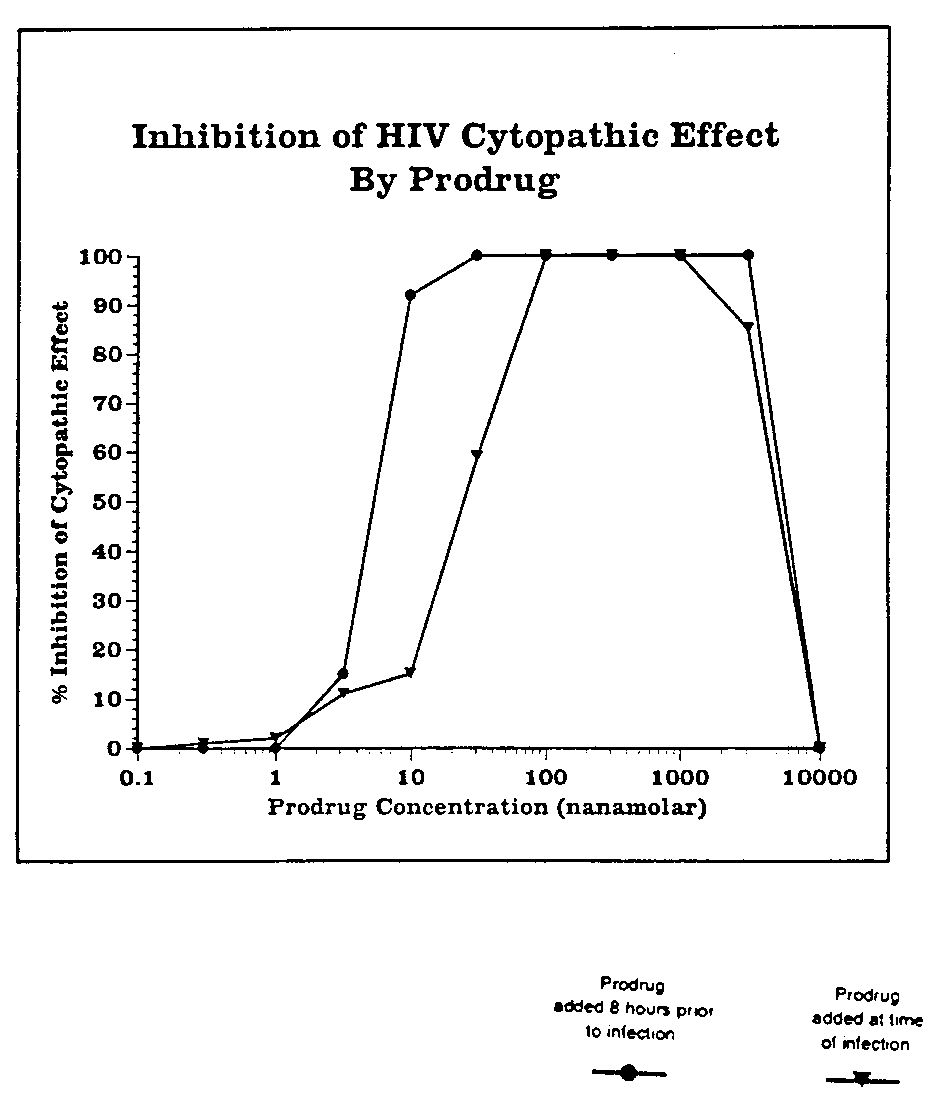

### LACK OF ANTICHOLINESTERASE ACTIVITY OF THE AZT PRODRUG

The AZT prodrug was evaluated for anticholinesterase activity at the Laboratory of Neurotoxicology and Pharmacology at Duke University using a standard well established acetylcholinesterase activity inhibition assay. Elleman et. al.; Biochemical Pharmacology ; 7:88 (1962) The prodrug was tested for anticholinesterase acitivity in chicken brain homogenate and electric eel extract. No inhibition of acetylcholinesterase activity was noted at 10⁻⁵ M.

The drug had no acute toxicity to mice when given intraperitoneally (IP) at a dose of 100 mg/kg as an emulsion in 5% Emulphor EL 620. One mouse was given a dose of 300 mg/kg IP without evident acute toxicity during a 24 hour period of observation.

### GENERAL METHODS OF PRODRUG SYNTHESIS REAGENTS

Prodrugs may be synthesized by a variety of chemical approaches from compounds of the structure shown below as Formula 1 and Formula 2. 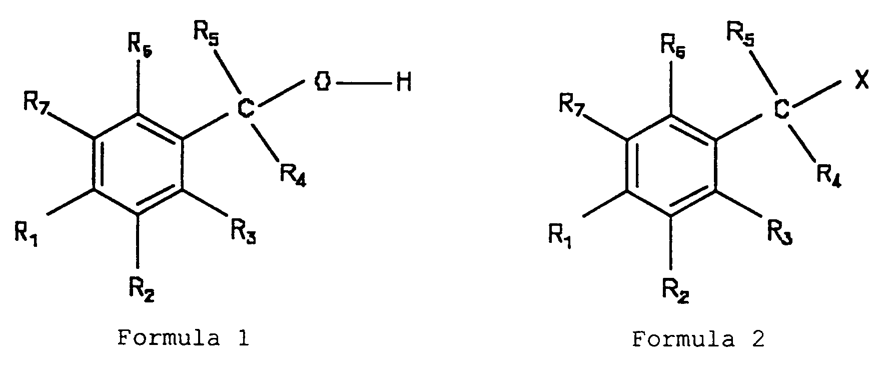
Wherein R1-R8 are as previously described for Structure 2. In Formula 2, X may be: CL; I; Br; o-tosyl; or o-trifyl.

If R1-R6 has a hydroxy group or an amino group then it is necessary to protect this group prior to employing the phosphorylation methods given below. Suitable hydroxy protective groups include: trimethylsilyl- ; tert-butyldimethylsilyl- ; beta-(trimethylsilyl) ethoxymethyl; and vinyl ethers . These protective groups may be coupled to ( and removed from) the hydroxy group of R1 and R6 using routine methods. Greene,T.W. in Protective Groups in Organic Synthesis ,John Wiley and Sons, New York, N.Y.(1981).
Suitable protective groups for amino substituents on R1-R6 include the 9-Fluorenylmethoxycarbonyl group and triazones. Carpino,L. Accounts Chem.Res. 20:401 (1987); Knapp,S., et. al; Tetrahedron Lett. 31:2109 (1990)
An especially useful set of intermediates in prodrug synthesis is given by FORMULA 1A below: 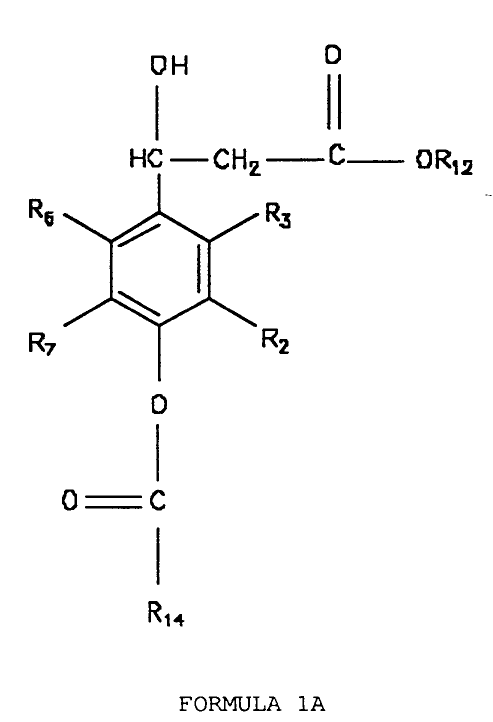
Wherein R2, R3, R6, R7, and R12 are as described for STRUCTURE 2. R14 may be R8 or ( R8-O-) where R8 is as defined in STRUCTURE 2. R14 may also be a group of the following such as: (-NH₂) ; (-NHCH3 ); (-N(CH3)₂)
Compounds given by FORMULA 1A may be synthesized by reduction of the corresponding ketone given by FORMULA 1B. The Structure of FORMULA 1B is shown below: 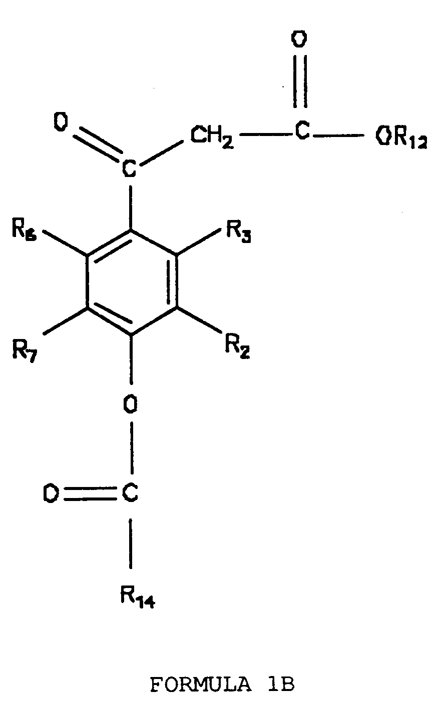
Wherein R2, R3, R6, R7, R12, and R14 are as described for FORMULA 1A.

The alcohol given by FORMULA 1A may be synthesized from the keto compound 1B by a variety of methods including catalytic hydrogenation with pallidium on carbon or platinum dioxide. Alternatively, the reduction may be effected by a reagent such as borane in ammonia.

### REDUCTION OF FORMULA 1B

### METHOD A

A 50 ml flask is flushed with nitrogen and charged with 1.0 gram of FORMULA 1B in 15 ml of methanol. Platinum dioxide (Aldrich, 0.05 gm) is added and the flask is flushed with nitrogen before being filled with hydrogen. The mixture is stirred at room temperature and atmospheric pressure. After 24 hours of reaction time an additional 25 mg of platinum dioxide is added. After 90 hours the reaction mixture is filtered through celite. The celite is washed with methanol. The combined filtrates are rotary evaporated to yield crude product which is then purified by flash chromatography on silica.
Note: The reaction may also be run with the addition of a trace amount of a base such as pyridine to suppress reduction of the desired alcohol.

### METHOD B

A 250 ml round bottom flask is charged with 0.045 moloes of FORMULA 1B and 150 ml of diethyl ether. The solution is stirred and ammonia-borane (1.39 gm., 0.045 mol., Alfa) is added in 4 portions. After being stirred 90 minutes at room temperature 50 ml of ether are added and the reaction mixture is slowly poured into a 400 ml beaker containing 20 ml of 3 N HCL and 100 ml of crushed ice. After 15 minutes the organic phase is separated. The aqueous phase is extracted with ether (2 X 100 ml). The combined organic phase is washed with brine, dried over magnesium sulfate, filtered and evaporated. The crude product is then purified by flash chromatography.

These basic methods were employed in the synthesis of ethyl 3-hydroxy-3-(4-acetoxyphenyl)propionate described in EXAMPLE 2.

### SYNTHESIS OF FORMULA 1B

compounds given by FORMULA 1B may be synthesized by the following Scheme:

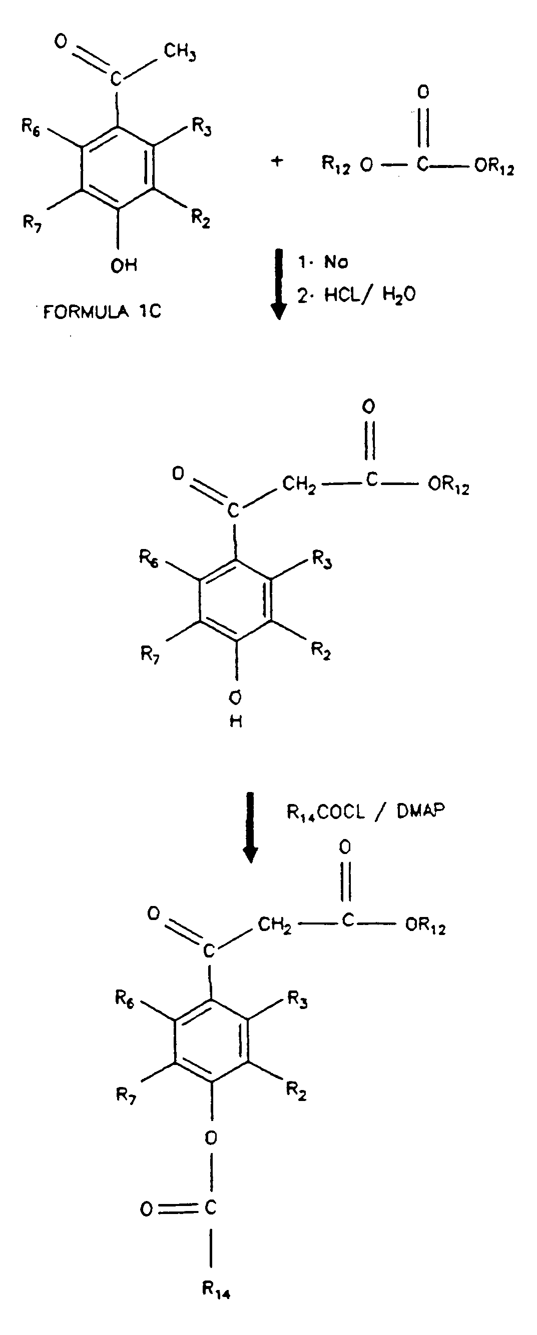

### SYNTHESIS OF FORMULA 1B

### METHOD A

50 ml of the carbonate (R12-O-CO-0-R12 ) is heated to refluxed under nitrogen with a Vigreaux column leading to a short path distillation apparatus. Then the oil bath is removed and 3.0 grams of sodium is slowly added. The mixture is then heated to 140 C. and a solution of 6.0 grams of the 4-hydroxy-acetophenone derivative (Formula 1C) in 60 ml of the carbonate is slowly added. The alcohol R12-OH is then removed by distillation. When the reaction has proceeded to completion the mixture is cooled to 30 C and poured onto 30 ml of 3N HCL and 30 ml of crushed ice. The flask is then rinsed with another 10 ml of 3N HCL and 10 ml of crushed ice. The organic layer is separated and the aqueous phase is extracted twice with 50 ml of ether. The combined organic phases are then dried over magnesium sulfate. The organic extracts are then filtered and dried to give crude product which is then purified by flash chromatography on silica gel. The product is dissolved in pyridine and treated with 1 equivalent of the acid chloride ( R14-C0-CL) or the related anhydride in the presence of a catalytic amount of dimethylaminopyridine (DMAP). After the reaction has proceeded to completion at room temperature the pyridine is removed exvacuo. The residue is stirred with 25 ml of water and 25 ml of ether. The aqueous phase is separated and extracted twice with 50 ml of ether. The combined ether extracts are washed with 0.5N HCL (25 ml), then with saturated sodium bicarbonate solution (25 ml), and finally with 25 ml of brine. The extracts are then dried with magnesium sulfate, filtered and the ether removed exvacuo.

This method was employed in the synthesis of ethyl 4-acetoxy-benzoylacetate described in EXAMPLE 2.

### SYNTHESIS OF FORMULA 1B

### METHOD B

This method is shown below:

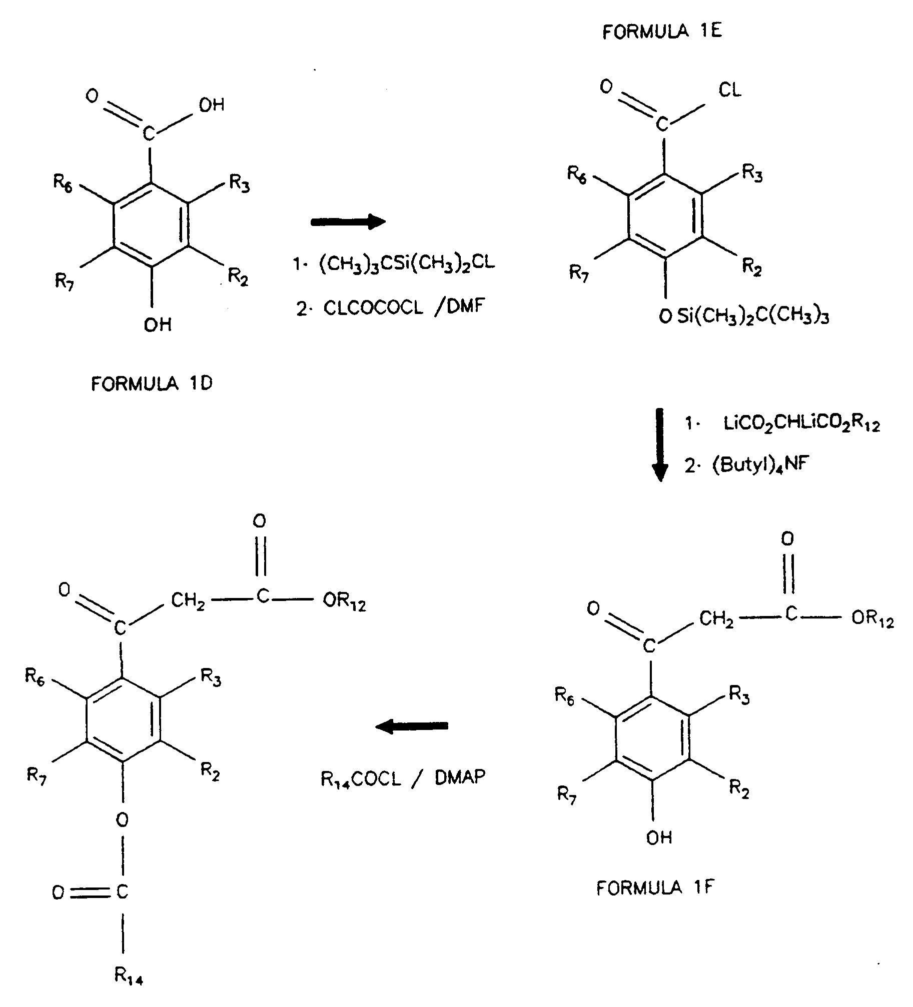

### METHOD B

In this method the 4-hydroxy-benzoic acid derivative (Formula 1D ) is treated with tert-butyldimethylsilyl chloride to yield the di-silylated product. Subsequent treatment with oxalyl chloride in the presence of dimethylforamide will yield the acid chloride derivative ( FORMULA 1E) These reactions may be carried out with published procedures. Wissner,A.; Grudzinskas,C.; J.Organic Chem. ; 43:3972 ; 1978. The acid chloride (FORMULA 1E) is then reacted with the LiCO₂CHliCO₂R12 using published procedures. Wierenga,W.; Skulnick,H.; Organic Synthesis Collective Volume ; 7:213 ; 1990. The protecting tert-butyldimethylsilyl group is then removed using well known procedures such as treatment with tetra-n-butylammonium fluoride in tetrahydrofuran to yield the corresponding 4-hydroxy derivative (FORMULA 1F). Treatment with R14-CO-Cl as described in Method A will yield the desired product (FORMULA 1B) General Methods of Prodrug Synthesis

### Method 1

In this method a compound given by Formula 1 is reacted with a chloride derivative of the parent phosphorous drug of the structure shown below as Formulae 3 and 4. 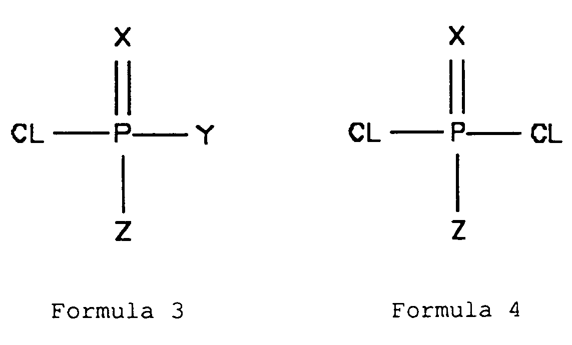
The reaction is carried out in a suitably inert anhydrous solvent such as pyridine, ether, or tetrahydrofuran in the presence of a base such as triethylamine or pyridine. Catalysts such as: n-methylimidazole ; 4-dimethylaminopyridine ; 3-nitro-1,2,4-triazole ; and 1-hydroxybenzotriazole may be employed.

Alternatively compounds of Formula 1 may be converted into the resepective alkoxides by treatment with a strong base such as n-butyl lithium in an inert solvent. This alkoxide may then be reacted at low temperatures with a compound of Formulae 3 or 4.

Compounds given by Formulae 3 and 4 may be synthesized from the parent phosphorous bearing drug shown in Structure 1 by the following methods:
1.) Treatment with thionyl chloride an in an inert solvent.
2.) Treatment with trimethylchlorosilane in pyridine followed by the reaction of the resulting trimethylsily ester with thionyl chloride
3.) Treatment with tris(2,4,6-tribromophenoxy)-dichlorophosphorane
   Hotoda,H. et al. Nucleic Acid Res., 17:5291 (1989)
The most expeditious route to the synthesis of compounds given by Fromulae 3 and 4 depends upon the nature of the groups X , Y, and Z. For example, if X is oxygen, and Y is an alkoxy group then the compound given by Formula 4 may be readily synthesized by the action of phosphoryl chloride on Y-OH in the presence of a base. The key point is that compounds of the structure given by Formulae 3 and 4 may readily be synthesized by one experienced in the art of organic chemistry using known methods.

### Method 2

In this method a compound of the structure given by Formula 1 is reacted with a compound of the structure shown below as Formulae 5 and 6. 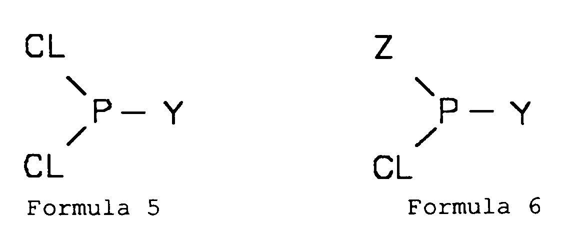
The reaction is carried out in a suitably inert anhydrous solvent such as pyridine, ether, or tetrahydrofuran in the presence of a base such as triethylamine or pyridine. Catalysts such as: n-methylimidazole ; 4-dimethylaminopyridine; and 3-nitro-1,2,4-triazole may be employed. The product is then oxidized to to yield the desired prodrug given by structure 3A or 3B . Suitable oxidants include: aqueous iodine; ethyl hypochlorite; and 3-chloroperoxybenzoic acid. Letsinger,R.L. et al.,J.AM.Chem.Soc., 98:3655 (1976)
Compounds given by Formulae 5 and 6 may be synthesized from the hydrogen phosphonate analog of the parent phosphorous bearing drug shown in Structure 1 by the the action of tris(2,4,6-tribromophenoxy)-dichlorophosphorane. Hotoda,H. et al. Nucleic Acid Res., 17:5291 (1989) , Wada,T. et al. Tetrahedron Lett., 29:4143 (1988)
If Y and Z are alkoxy groups then the compound given by Formulae 5 and 6 may be synthesized by the action of phosphorous trichloride chloride on Y-OH and Z-OH in the presence of a base. The key point is that compounds of the structure given by Formulae 5 and 6 may readily be synthesized by one experienced in the art of organic chemistry using known methods.

### Method 3

In this method a compound of the structure given by Formula 1 is reacted with a compound of the structure shown below as Formulae 7 and 8. 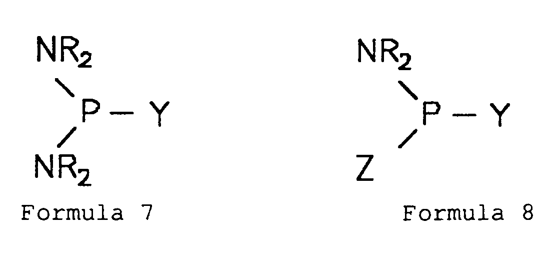
Wherein R is an alkyl group such as ethyl or isopropyl.

The reaction is carried out in a suitably inert anhydrous solvent such as ether, acetonitrile, or tetrahydrofuran in the presence of a a catalyst such as 1-H-tetrazole or 1H,5-methyltetrazole.
McBride,L.J. and Caruthers,M.H., Tetrahedron lett., 24:245 (1983) Perich,J.W. et al., Tetrahedron Lett. , 28:101 (1987) Hamamoto,S. and Takaku,H.,Chem.Lett. ,p.1410 (1986) Marugg,J.E. et al. Tetrahedron Lett., 27:2271 (1986) The product is then oxidized to to yield the desired prodrug given by structure 3A or 3B. Suitable oxidants include aqueous iodine or ethyl hypochlorite. Letsinger,R.L. et al.,J.AM.Chem.Soc., 98:3655 (1976)
Compounds of the structure given by Formulae 7 and 8 are readily obtained by the reaction of the desired dialkylamine with the chlorides derivatives given by Formulae 5 and 6.

### Method 4

This method is similar to Method 3 above except that a compound of the structure shown below as Formula 9 is reacted with the parent drug (Y-OH). The resulting phosphite ester is then oxidized as described in Method 3 to the corresponding phosphate. The corresponding phosphorothioate may be readily made by treating the phosphite with a reagent such as 3H-1,2,Benzodithiol-3-one 1,1,-Dioxide . Iyer,R.P., et. al. J.Organic Chem. 55:4693 ,1990 Compounds given by Formula 9 below may be readily synthesized by the reaction of a compound given by Formula 1 with a dialkylphosphoramidous Dichloride in an inert solvent in the presence of a base such as triethylamine. Formula 9: 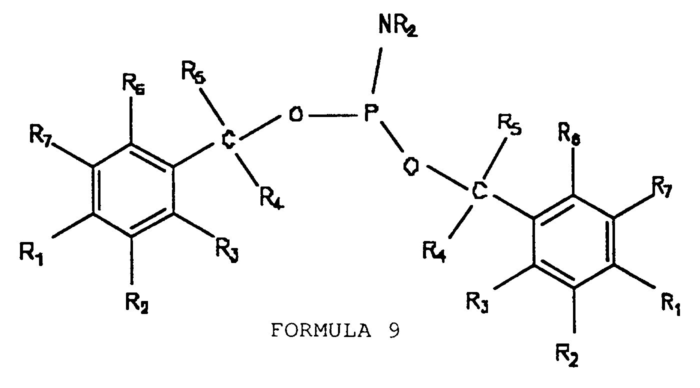
Wherein R is an alkyl group such as ethyl or isopropyl and R1-R8 are as describrd in Structure 2.

### Method 5

In this method a compound of the structure shown as Formula 2 is reacted with the parent phosphorous bearing drug. This reaction may be conducted by employing:
1.) the tetrabutylammonium salt of the parent drug. Zwierzak,A. et al. Synthetic Communications, p.770 (1978) , Davisson,V. et al.J.Org.Chem., 51:4768 (1986)
2.) The silver salt of the parent drug
3.) Cesium fluoride in acetonitrile Takaku,H.et al. Chem.Pharm.Bull., 31:2157 (1983)

### Prodrugs for Phosphonoformate

Phosphononformate is a potent antiviral agent which is active against a wide spectrum of viruses including the human immunodeficiency virus (HIV); Epstein Barr Virus (EBV); herpes simplex; and cytomegalovirus (CMV). Oberg,B. Pharmac.Ther. , 40:213 (1989) At concentrations of less then 2 micromolar, HIV reverse transcriptase is inhibited by 50%. However, hundred fold higher concentrations are required to inhibit viral replication in infected cells. Sarin,P. et al. Biochemical Pharm. , 34:4075 (1985), DeClercq,E. J.Medicinal Chem. , 29:1561 (1986) This is a reflection of the fact that phosphonoformate is negatively charged and poorly taken up by cells. Phosphonoformate has very poor oral bioavailability. Clinical use has been limited by the need to give very high intravenous doses which have been associated with renal toxicity. In addition the negative charge prevents penetration of the drug into the brain. Liposomes have been suggested as an approach to facilitate the delivery of phosphonoformate. Szoka,F.C. et al. Antimicrobial Agents and Chemotherapy ,32:858 (1988) However, the liposomal encapsulation of phosphonoformate does not allow for drug delivery into the central nervous system. In addition the liposome are not orally active and have a pharmacological distribution limited predominantly to the reticuloendothelial system. A variety of carboxylate and phosphoester derivatives of phosphonoformate have been examined as potential prodrugs. However, these derivatives are generally less active then the parent compound. Norin,J. et al. J.Medicinal Chem. , 26:264 (1982); Iyer,R. ; Phillips,L.; Biddle,J.; Thakker,D.; Egan,W.; Tetrahedron Let.; 30:7141 (1989).

The prodrug approach described below can be used to synthesize lipid soluble derivatives of phosphonformate which will readily diffuse into cells and through the blood brain barrier. The chemical structure of this class of drugs is shown below as Formula 10: 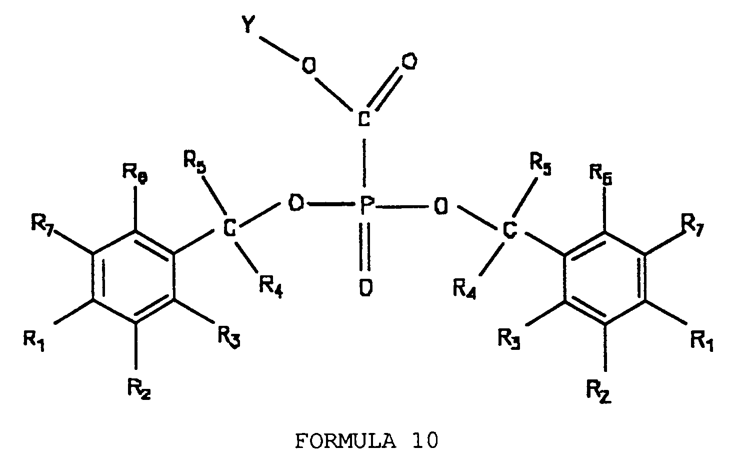
Wherein R1-R7 are as described for Structure 2. In Formula 10 Y is a benzyl, or phenyl group. The benzyl or phenyl group may in turn bear substituents. Y may also be a group of the same structure as attached to the phosphonate group of the molecule.

Compounds given by Formula 10 will be metabolized intracellularly to phosphonoformate via nonspecific esterase. An example of a prodrug for phosphonoformate is shown as Formula 11: 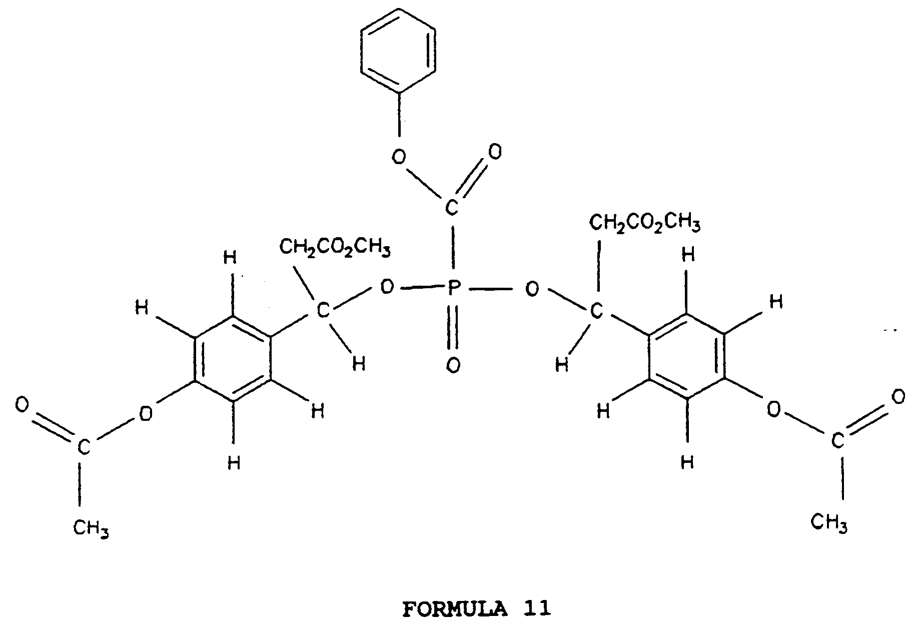
This compound will be metabolized to p-hydoxycinnamic acid and phosphonoformate.

The prodrug compounds given by formula 9 may be synthesized as follows:

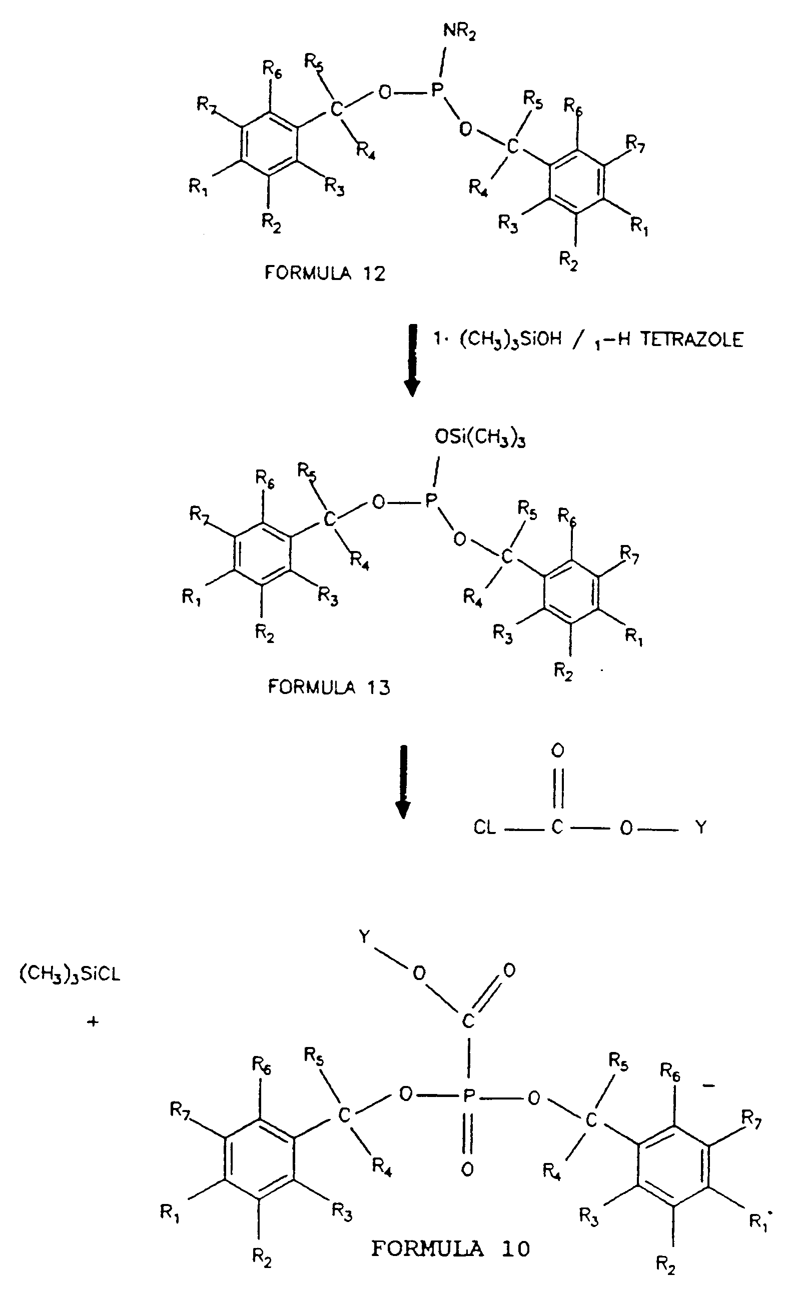

### General Method for Synthesizing Compounds of Formula 10

A solution of freshly distilled diethylphosphoramidous dichloride ( 100 mMoles) is added to 150 ml of anhydrous diethyl ether under a dry nitrogen atmosphere and cooled to - 20 C. A solution of 200 mMoles of Formula 1 and 200 mMoles of dry triethylamine in 500 ml of diethyl ether is slowly added. After stirring for 15 more minutes the external cooling is removed and the reaction mixture is stirred at room temperature for 20 hours. The triethylamine hydrochloride is then removed by filtration under a nitrogen atmosphere. The filtrate is then evaporated. The residue is then dissolved in 1000 ml of methylene chloride, and was washed at 4 C with 300 ml of saturated aqueous saline, followed by 5% NaHCO₃ (300 ml X 2) , and 500 ml of water. The organic phase is then dried over sodium sulfate, filtered and dried exvacuo at room temperature. The product will be that of the N,N diethylphosphoramidite shown as Formula 12.

1-H-tetrazole (180 mMoles) is added to 60 mMoles of the N,N diethylphosphoramidite (Formula 12) and 70 mMoles of anhydrous trimethylsilanol in 300 ml of anhydrous tetrahydrofuran. (Methanol may be emplyed in place of the trimethylsilanol) After 2 hours The solvent is removed exvacuo and the diethylamine are removed by distillation at reduced pressure. The resultant product will be that shown as Formula 13.

75 mMoles of Formula 13 is refluxed with 80 mMoles of the chloroformate ClCO₂Y. After the reaction has gone to completion the chlorotrimethylsilane and residual chloroformate is removed by fractional distillation at reduced pressure. The residue containing the desired product is then dissolved in methylene chloride and washed extensively with saturated aqueous sodium bicarbonate follwed by, water, follwed by saturated aqueous sodium chloride. The organic phase is then dried over sodium sulfate and the methylene chloride removed exvacuo. The desired product Formula 10 may then be purified by recrystallization from a suitable solvent or by chromatography.

### Synthesis of Formula 11

A solution of freshly distilled diethylphosphoramidous dichloride ( 100 mMoles) is added to 150 ml of anhydrous diethyl ether under a dry nitrogen atmosphere and cooled to - 20 C. A solution of 200 mMoles of ethyl 3-hydroxy-3-(4-acetoxyphenyl)propionate and 200 mMoles of dry triethylamine in 500 ml of diethyl ether is slowly added. After stirring for 15 more minutes the external cooling is removed and the reaction mixture is stirred at room temperature for 20 hours. The triethylamine hydrochloride is then removed by filtration under a nitrogen atmosphere. The filtrate is then evaporated. The residue is then dissolved in 1000 ml of methylene chloride, and was washed at 4 C with 300 ml of saturated aqueous saline, followed by 5% NaHCO₃ (300 ml X 2) , and 500 ml of water. The organic phase is then dried over sodium sulfate, filtered and dried exvacuo at room temperature. The product will be that of the N,N diethylphosphoramidite shown as Structure 6.

1-H-tetrazole (180 mMoles) is added to 60 mMoles of the N,N diethylphosphoramidite (Structure 6) and 70 mMoles of anhydrous trimethylsilanol in 300 ml of anhydrous tetrahydrofuran. (Methanol may be emplyed in place of the trimethylsilanol) After 2 hours The solvent is removed exvacuo. The diethylamine and 1-H-tetrazole are removed by distillation at reduced pressure. Then 75 mMoles of phenyl chloroformate is slowly added. After the reaction has gone to completion the chlorotrimethylsilane and residual chloroformate are removed by fractional distillation at reduced pressure. The residue containing the desired product is then dissolved in methylene chloride and washed extensively with saturated aqueous sodium bicarbonate follwed by, water, follwed by saturated aqueous sodium chloride. The organic phase is then dried over sodium sulfate and the methylene chloride removed exvacuo. The desired product Formula 11 may then be purified by recrystallization from a suitable solvent or by chromatography.

Another example of a prodrug for phosphonoformate is shown below as Formula 14: 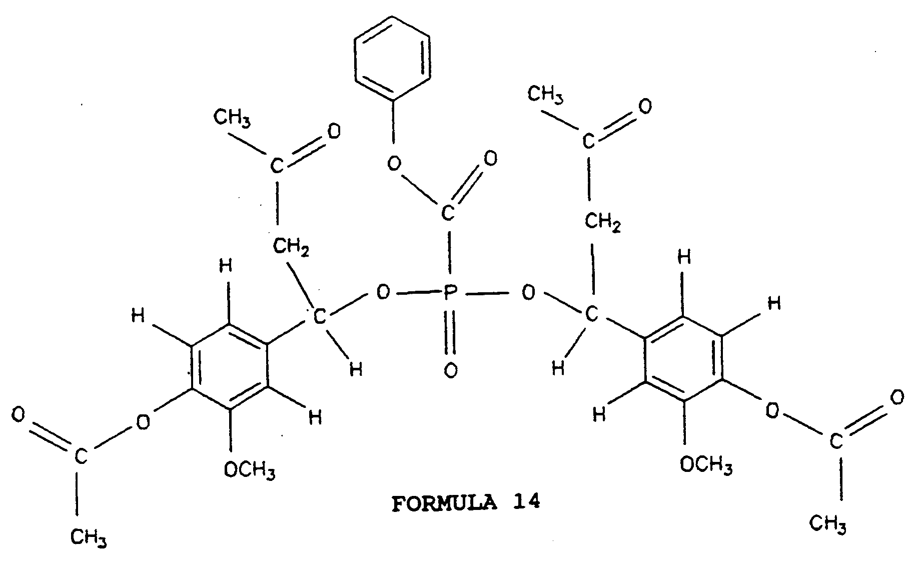
This compound will be metabolized intracellularly via the action of nonspecific esterases into phosphonoformate and methyl 4-hydroxy,3-methoxy-styryl ketone. It is known that methyl 4-hydroxy,3-methoxy-styryl ketone is of low toxicity with an oral LD₅₀ of greater then 2 grams/kg. Singh,G.B. et al. Arzneimittelforschung , 37:708 (1987) and 37:435 (1987)

### Synthesis of Formula 14

This compound may be synthesized using 4-(4-acetoxy,3-methoxyphenyl), 4-hydroxy,2-butanone in place of ethyl 3-hydroxy-3-(4-acetoxyphenyl)propionate in the previous method given for the synthesis of Formula 11.

### PRODRUGS FOR 9-(2-PHOSPHONYLMETHOXY-ETHYL)ADENINE

9-(2-phosphonylmethoxy-ethyl)adenine , (PMEA) is a potent antiviral agent which is active against a wide variety of viruses including: HIV, herpes simplex, cytomegalovirus , and hepatitis B. De Clercq,E.D., Sakuma,T.,M.Baba, Pauwels,R., Balzarini,J., Rosenberg,I., Holy,A.;.Antiviral Research, 8:261 (1987) , Gangemi,J.D. et al. Antimicrobial Agents and Chemotherapy, 33:1864 (1989) , De Clercq,E. et al. Antimicrobial Agents and Chemotherapy, 33:185 (1989), Balzarini,J. et al. Proc.Natl.Acad.Sci.(USA) , 86:332 (1989) Balzarini,J., Naesens,L., De Clercq,E. ;Int.J.Cancer ,46:337, 1990; Yokotta,T., Mochizuki,S., Konno,K., Mori,S., Shigeta,S., De Clercq,E., Antimicrobial Agents and Chemotherapy, 35:394 (1991)

PMEA is not absorbed following oral administration. In addition drug penetration into the brain is poor. Prodrugs of PMEA which will be readily absorbed following oral, topical or parenteral administration and which will readily diffuse into cells and into the brain may be synthesized using the present prodrug method.

The chemical structure of this class of drugs is shown below as Formula 15: 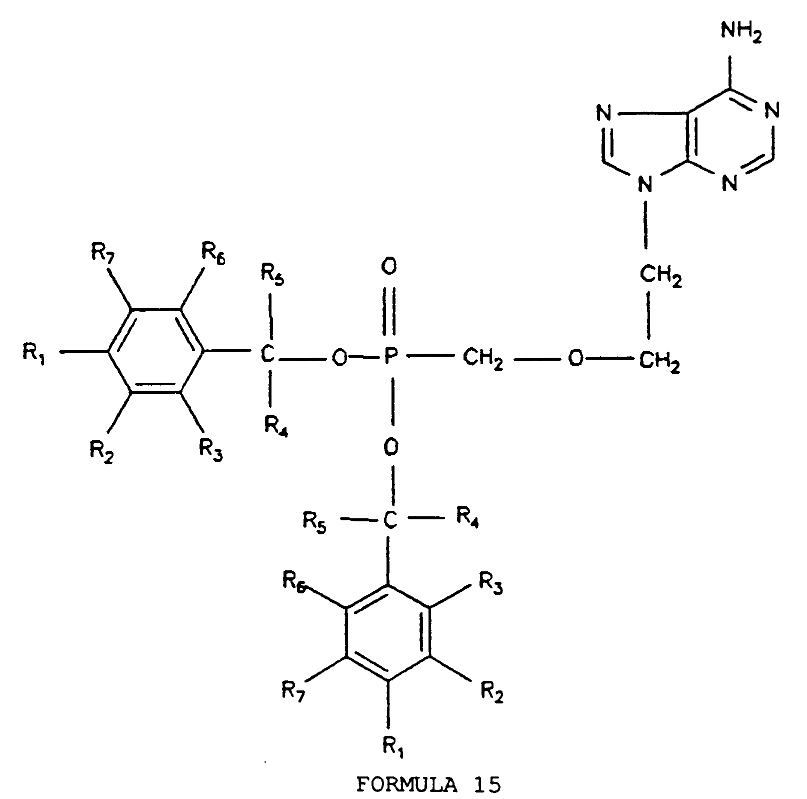
Wherein R1-R7 are as described for Structure 2.

### GENERAL METHODS OF SYNTHESIZING PRODRUGS FOR PMEA

### Method A

In this method the diethyl ester of PMEA is first protected with an 9-Fluorenylmethoxycarbonyl (FMOC) group. The use of FMOC to protect the exocyclic amino group of adenine derivatives is well established. Koole,L. et. al.;J.Organic Chem. 54:1657; 1989. The FMOC protected diethyl ester of PMEA is then treated with bromotrimethylsilane to convert the ethyl esters to trimethylsilyl esters. Reactions of this type have previously been described for diethyl esters of PMEA and other phosphonates. Rosenberg,I. Holy,A., Masojikkova,M; Collections Czechoslovak Chem.Commun. 53:2771. The bis trimethylsilyl ester of FMOC protected PMEA is then converted into the dichloride derivative by treatment with oxalyl chloride and a catalytic amount of dimethylforamide. This procedure is known to convert bis trimethylsilylphosphonates into the dichlorides in very high yield. Bhongle,N.; Notter,R.; Turcotte,J.; Synthetic Communications; 17:1071 ; 1987. In the presence of N-methylimadazole and triethylamine , phosphonic acid dichlorides rapidly react with alcohols in quantitative yield to yield phosphonate diesters. This was demonstrated in Example 1 and Example 2.

### METHOD A

50 mMoles of the diethyl ester of PMEA is dissolved in 200 ml of anhydrous pyridine to which is added 50 mMoles of 9-Fluorenylmethyl chloroformate. After the reaction has proceeded to completion as evidenced by TLC the solvent is removed exvacuo and the FMOC protected diethyl PMEA is dissolved in 200 ml of methylene chloride. The methylene chloride is then washed 3X with 150 ml of saturated aqueous sodium bicarbonate , followed by 150 ml of water x2, followed by 150 ml of saturated aqueous sodium chloride. The organic phase is then dried with sodium sulfate and and the solvent removed exvacuo. The FMOC protected diethyl ester of PMEA is then purified by chromatography on silica.

30 mMoles of the FMOC protected diethyl ester of PMEA is dissolved in 300 ml of anhydrous acetonitrile to which is added 120 mMoles of bromotrimethylsilsane at room temperature. After the reaction has proceeded to completion the bromoethane is removed exvacuo. The resulting FMOC protect bis-trimethylsilyl ester of PMEA is then converted into the FMOC protected dichlorophosphonate derivative of PMEA by treatment with 2.1 equivalents of oxalyl chloride and a catalytic amount of dimethylforamide in methylene chloride. After the reaction has proceed to completion the solvent, chlorotrimethylsilane and residual oxalyl chloride are removed at reduced pressure. 100 ml of anhydrous tolulene is added and removed exvacuo to effect azeotropic removal of any remaining traces of oxalyl chloride or chlorotrimethylsilane. Then the resulting FMOC protected dichlorophosphonate derivative of PMEA is dissolved in methylene chloride or acetonitrile. 70 mMoles of Formula 1 , 60 mMoles of triethylamine and 180 mMoles of n-methylimidazole are then added. After the reaction has proceeded to completion 75 mMoles of triethylamine is added in order to deprotect the exocyclic amino group on the adenine. After the deprotection is completed 500 ml of methylene chloride and 300 ml of water is added. The organic phase is separated, and washed x3 with 300 ml of saturated aqueous sodium bicabonate, followed by 300 ml x 3 of water, followed by 300 ml of saturated aqueous sodium chloride. The organic phase is then dried with sodium sulfate and the solvent removed exvacuo. The desired prodrug compound is then purified by chromatography on silica.

### METHOD B

In this method the FMOC protected diethyl ester or (dimethyl ester) of PMEA is prepared as described in Method A. The dichlorophosphonate derivative of the protected PMEA is then prepared by refluxing with thionyl chloride or oxalyl chloride in the presence of dimethylforamide. The ethyl chloride and thionyl chloride are then removed exvacuo to yield the desired dichlorophosphonate derivative of FMOC protected PMEA. This may in turn be converted into the prodrug as described previously in Method A.

### METHOD C

This method is the same as Method A except that thionyl chloride is employed in place of oxalyl chloride.

### METHOD D

In this method the diethyl ester of PMEA is protected with a triazone group in place of the FMOC group. Deprotection is accomplished with mild acid such as saturated aqueous ammonium chloride or citric acid. The use of triazone as amino protecting groups has been previuosly described. Knapp,S.; et.al. Tetrahedron Lett. ; 31:2109 , 1990. The triazone protected diethyl ester of PMEA may be used in place of the FMOC protected PMEA in Methods A-C above.
METHOD This method is outlined below:

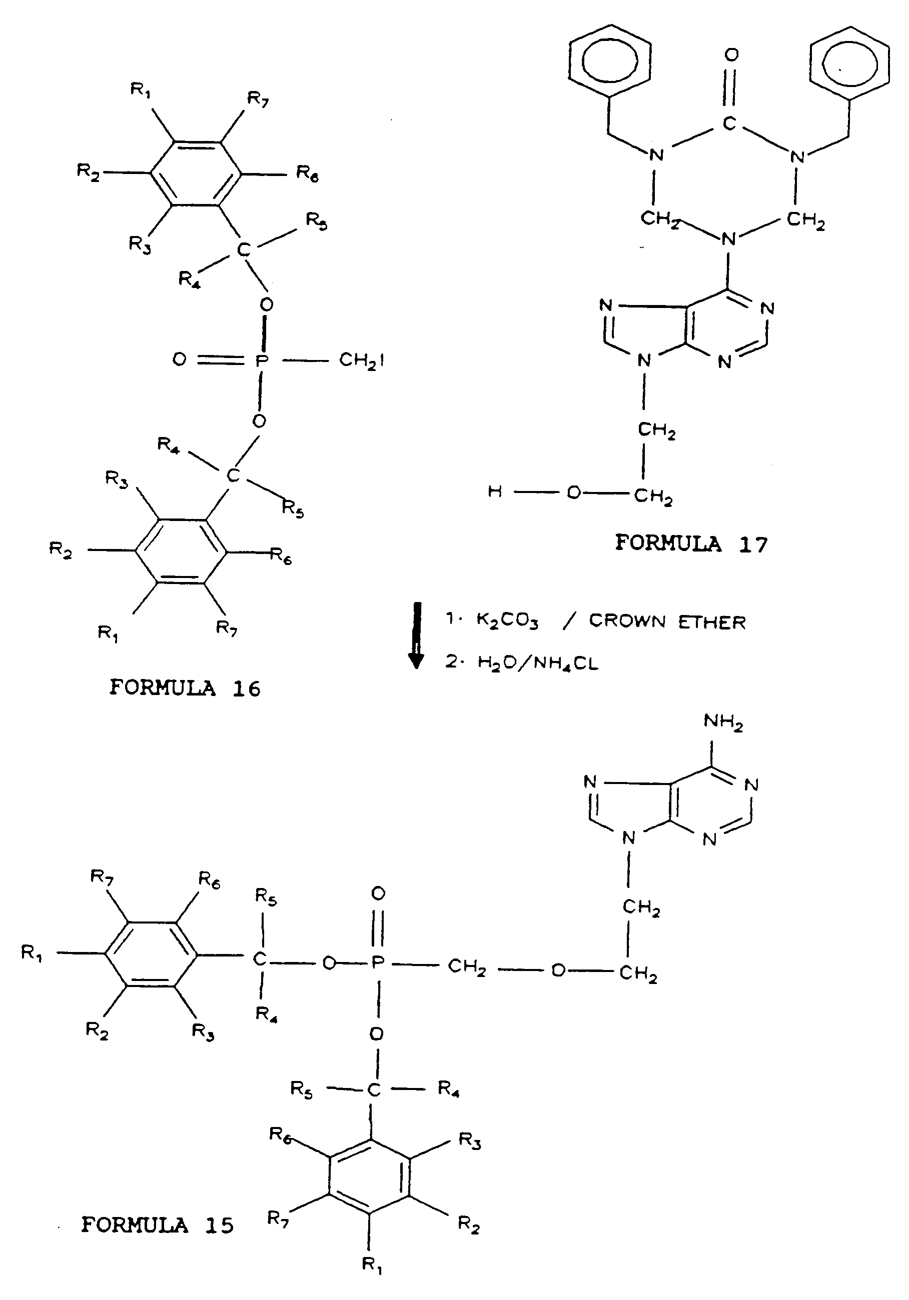

### METHOD E

### SYNTHESIS OF FORMULA 16

Chloromethylphosphonic dichloride (100 mMoles) is added to 300 ml of anhydrous methylene chloride under a dry nitrogen atmosphere. Formula 1 (200 mMoles) , triethylamine ( 200 mMoles) and N-methylimidazole (400 mMoles) is slowly added at - 20 C. After the addition is complete the reaction is warmed to room temperature. After the reaction has proceeded to completion as evidenced by TLC 100 ml of water is added dropwise at 4 C. Then the organic phase is separated and washed with 75 ml x2 of saturated aqueous sodium bicarbonate, followed by 75 ml x 3 of water, followed by 75 ml of saturated aqueous sodium chloride. The organic phase is then dried with sodium sulfate and the solvent removed exvacuo. The product which is the corresponding bis ester of chloromethylphosphonic acid is then purified by chromatography on silica or by recrystallization from a suitable solvent.

The chloride is then exchanged for iodide by treatment with sodium iodide in acetone in a flask shielded from light. 300 mMoles of sodium iodide is added to the compound in 100 ml of dry acetone. After refluxing for 4 hour the precipitated sodium chloride is removed by filtration. The acetone is then removed exvacuo and 200 ml of methylene chloride and 200 ml of water is added. The organic phase is separated and washed x 2 with 100 ml of water followed by 50 ml of saturated aqueous sodium chloride. The organic phase is then dried with sodium sulfate and the solvent removed exvacuo. The resulting product (Formula 16) is then dried under a high vacuum.

### SYNTHESIS OF FORMULA 17

100 mMoles of 9-(hydroxyethyl)adenine hydrochloride is added to 200 ml of 37% aqueous formaldehyde and the mixture is then neutralized by the addition of N,N-diisopropylethylamine. The mixture is stirred for six hours at reflux then 500 ml of tolulene is added. The mixture is then throughly dried exvacuo. Then 200 mMoles of N,N-dibenzyl urea and 400 ml of anhydrous pyridine (or acetonitrile) is added and the mixture is refluxed. When the reaction has proceeded to completion as measured by TLC the solvent is removed exvacuo. The organic phase is then dissolved in 500 ml of methylene chloride and washed with 100 ml x3 of water, followed by 100 ml of saturated aqueous sodium chloride. After drying with magnesium sulfate the organic solvennt is removed exvacuo. The desired product (Formula 17) is then purified by chromatography on silica or by recrystallization from a suitable solvent.

### SYNTHESIS OF FORMULA 15

50 mmoles of Formula 16 and 50 mMoles of Formula 17 are dissolved in 500 ml anhydrous acetonitrile. 50 mMoles of solid finely powdered anhydrous potassium carbonate is added along with 1 mMole of dibenzo-18-Crown-6 ether. The mixture is refluxed until the reaction has proceeded to completion as monitored by TLC. The solvent is then removed exvacuo and the residue is dissolved in 300 ml of methylene chloride. The organic phase is then wash x 3 with 100 ml of water, followed by 100 ml of saturated sodium chloride. The the solvent is then removed exvacuo. 200 ml of methanol and 200 ml of saturated aqueous ammonium chloride are added and the mixture is heated to 70 C. The hydrolysis of the triazone ring may be followed by TLC. After the deprotection is complete 700 ml of methylene chloride is added. The organic phase is separated, washed x 3 with 200 ml of 5% sodium bicarbonate, followed by 200 ml x 2 of water. The organic phase is then dried with sodium sulfate and the solvent removed exvacuo. The desired product (Formula 15) is then purified by chromatography on silica or by recrystallization form a suitable solvent.
NOTE: This ether synthesis may also be carried out by employing Ag₂O in place of the crown ether and potasssium carbonate. Alternatively, A compound of Formula 17 may be treated with one equivalent of potasium hydide in an inert solvent to yield the corresponding alkoxide derivative which may be reacted with the compound shown as Formula 16 in the presence of dibenzo-18-Crown-6 ether.
An example of a prodrug for PMEA is shown below as Formula 18:

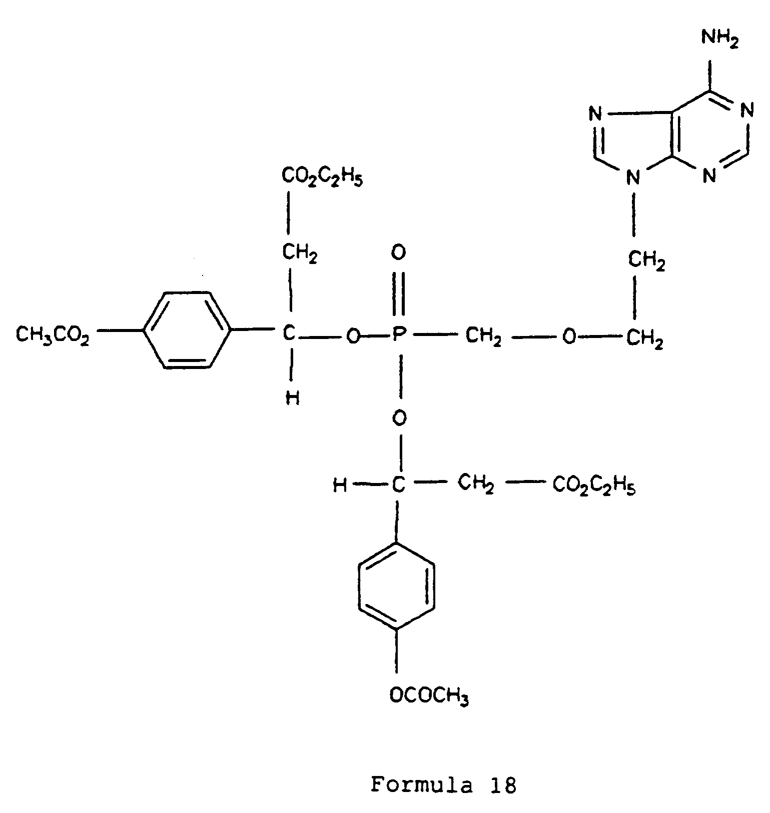

### Synthesis of Formula 18

### METHOD A

50 mMoles of the diethyl ester of PMEA is dissolved in 200 ml of anhydrous pyridine to which is added 50 mMoles of 9-Fluorenylmethyl chloroformate. After the reaction has proceeded to completion as evidenced by TLC the solvent is removed exvacuo and the FMOC protected diethyl PMEA is dissolved in 200 ml of methylene chloride. The methylene chloride is then washed 3X with 150 ml of saturated aqueous sodium bicarbonate , followed by 150 ml of water x2, followed by 150 ml of saturated aqueous sodium chloride. The organic phase is then dried with sodium sulfate and and the solvent removed exvacuo. The FMOC protected diethyl ester of PMEA is then purified by chromatography on silica.

30 mMoles of the FMOC protected diethyl ester of PMEA is dissolved in 300 ml of anhydrous acetonitrile to which is added 120 mMoles of bromotrimethylsilsane at room temperature. After the reaction has proceeded to completion the bromoethane is removed exvacuo. The resulting FMOC protect bis-trimethylsilyl ester of PMEA is then converted into the FMOC protected dichlorophosphonate derivative of PMEA by treatment with 2.1 equivalents of oxalyl chloride and a catalytic amount of dimethylforamide in 200 ml of methylene chloride. After the reaction has proceed to completion the solvent, chlorotrimethylsilane and residual oxalyl chloride are removed at reduced pressure. 100 ml of anhydrous tolulene is added and removed exvacuo to remove any traces of residual oxalyl chloride. Then the resulting FMOC protected dichlorophosphonate derivative of PMEA is dissolved in 200 ml of methylene chloride or acetonitrile. 70 mMoles of ethyl 3- hydroxy-3-(4-acetoxyphenyl)propionate ; 60 mMoles of triethylamine and 180 mMoles of n-methylimidazole are then added. After the reaction has proceeded to completion 75 mMoles of triethylamine is added in order to deprotect the exocyclic amino group on the adenine. after the deprotection is completed 500 ml of methylene chloride and 300 ml of water is added. The organic phase is separated, and washed x3 with 300 ml of saturated aqueous sodium bicabonate, followed by 300 ml x 3 of water, followed by 300 ml of saturated aqueous sodium chloride. The organic phase is then dried with sodium sulfate and the solvent removed exvacuo. The desired prodrug compound (Formula 18) is then purified by chromatography on silica.

### METHOD B

30 mMoles of the FMOC protected diethyl ester of PMEA is added to 150 ml of distilled thionyl chloride. Dimethylforamide (5 ml) is slowly added dropwise. The mixture is then refluxed under an anhydrous nitrogen atmosphere. After the reaction has proceeded to completion the chloroethane, and thionyl chloride is removed exvacuo. Anhydrous Tolulene (150 ml ) is added and removed exvacuo. The solvent is then removed under a high vacuum. Then the resulting FMOC protected dichlorophosphonate derivative of PMEA is dissolved in methylene chloride or acetonitrile. 70 mMoles of ethyl 3- hydroxy-3-(4-acetoxyphenyl)propionate ; 60 mMoles of triethylamine and 180 mMoles of n-methylimidazole are then added. After the reaction has proceeded to completion 75 mMoles of triethylamine is added in order to deprotect the exocyclic amino group on the adenine. Then 500 ml of methylene chloride and 300 ml of water is added. The organic phase is separated, and washed x3 with 300 ml of saturated aqueous sodium bicabonate, followed by 300 ml x 3 of water, followed by 300 ml of saturated aqueous sodium chloride. The organic phase is then dried with sodium sulfate and the solvent removed exvacuo. The desired prodrug compound (Formula 18) is then purified by chromatography on silica.

### METHOD C

This method is the same as Method A except that thionyl chloride is employed in place of oxalyl chloride.

### METHOD D

50 mMoles of the diethyl ester of PMEA is added to 200 ml of 37% aqueous formaldehyde and the mixture is then neutralized by the addition of N,N-diisopropylethylamine. The mixture is stirred for six hours at reflux. Then 500 ml of tolulene is added. The mixture is then throughly dried exvacuo. Then 200 mMoles of N,N-dibenzyl urea and 400 ml of acetonitrile ( or methylene chloride) is added and the mixture is refluxed. When the reaction has proceeded to completion as measured by TLC the solvent is removed exvacuo. The organic phase is then dissolved in 500 of methylene chloride and washed with 100 ml x3 of water, followed by 100 ml of saturated aqueous sodium chloride. After drying with magnesium sulfate the organic solvent is removed exvacuo. The desired product; triazone protected diethyl ester of PMEA is then purified by chromatography on silica or by recrystallization from a suitable solvent.
This compound may the be used in place of the FMOC protected diethyl ester of PMEA in Methods A-C above. In the final step of the prodrug synthesis the triazone protective group is removed with saturated aqueous ammoinum chloride at 70 C. A cosolvent such as methanol or DMSO may be employed in the deprotection step. The desired prodrug (Formula 18) is the purified chromatography on silica or by recrystallization from a suitable solvent.

### METHOD E

Chloromethylphosphonic dichloride (100 mMoles) is added to 300 ml of anhydrous methylene chloride under an dry nitrogen atmosphere. Ethyl 3- hydroxy-3-(4-acetoxyphenyl)propionate (200 mMoles) , triethylamine ( 200 mMoles) and N-methylimidazole (400 mMoles) is slowly added at - 20 C. After the addition is complete the reaction is warmed to room temperature. After the reaction has proceeded to completion as evidenced by TLC 100 ml of water is added dropwise at 4 C. Then the organic phase is separated and washed with 75 ml x2 of saturated aqueous sodium bicarbonate, followed by 75 ml x 3 of water, followed by 75 ml of saturated aqueous sodium chloride. The organic phase is then dried with sodium sulfate and the solvent removed exvacuo. The product which is the corresponding bis ester of chloromethylphosphonic acid is then purified by chromatography on silica or by recrystallization from a suitable solvent. The chloride is then exchanged for iodide by treatment with sodium iodide in acetone in a flask shielded from light. 300 mMoles of sodium iodide is added to the compound in 100 ml of dry acetone. After refluxing for 4 hour the precipitated sodium chloride is removed by filtration. The acetone is then removed exvacuo and 200 ml of methylene chloride and 200 ml of water is added. The organic phase is separated and washed x 2 with 100 ml of water followed by 50 ml of saturated aqueous sodium chloride. The organic phase is then dried with sodium sulfate and the solvent removed exvacuo. The resulting product; bis ((3-(ethyl 3-hydroxy-3-(4-acetoxyphenyl)propionate)) iodomethylphosphonate is then dried under a high vacuum.

100 mMoles of 9-(hydroxyethyl)adenine hydrochloride is added to 200 ml of 37% aqueous formaldehyde and the mixture is then neutralized by the addition of N,N-diisopropylethylamine. The mixture is stirred for six hours at reflux then 500 ml of tolulene is added. The mixture is then throughly dried ecvacuo. Then 200 mMoles of N,N-dibenzyl urea and 400 ml of anhydrous pyridine (or acetonitrile) is added and the mixture is refluxed. When the reaction has proceeded to completion as measured by TLC The solvent is removed exvacuo. The organic phase is then dissolved in 500 of methylene chloride and washed with 100 ml x3 of water, followed by 100 ml of saturated aqueous sodium chloride. After drying with magnesium sulfate the organic solvent is removed exvacuo. The desired triazone protected derivative of 9-(hydroxyethyl)adenine is then purified by chromatography on silica or by recrystallization from a suitable solvent.

50 mmoles of this triazone derivative of 9-(hydroxyethyl)adenine, and 50 mMoles of bis ((3-(ethyl 3-hydroxy-3-(4-acetoxyphenyl)propionate)) iodomethylphosphonate are dissolved in 250 ml anhydrous acetonitrile. 50 mMoles of solid finely powdered anhydrous potassium carbonate are added along with 1 mMole of dibenzo-18-Crown-6 ether. The mixture is refluxed and ultrasonicated until the reaction has proceeded to completion as monitored by TLC. The solvent is then removed exvacuo and the residue is dissolved in 300 ml of methylene chloride. The organic phase is then wash x 3 with 100 ml of water, followed by 100 ml of saturated sodium chloride. The the solvent is then removed exvacuo. 200 ml of methanol and 200 ml of saturated aqueous ammonium chloride are added and the mixture is heated to 70 C. The hydrolysis of the triazone ring may be followed by TLC. After the deprotection is complete 500 ml of methylene chloride is added. The organic phase is separated, washed x 3 with 200 ml of 5% sodium bicarbonate, followed by 200 ml x 2 of water. The organic phase is then dried with sodium sulfate and the solvent removed exvacuo. The desired product (Formula 18) is then purified by chromatography on silica or by recrystallization form a suitable solvent.

### METHOD F

In this method bis- ( ((3-(ethyl 3-hydroxy-3-(4-acetoxyphenyl)propionate)) trimethylsilyl phosphite is reacted via an Arbuzov reaction with chloromethoxyethyl chloride (CL-CH₂-O-CH₂-CH₂-CL ). The resulting phosphonate diester is then coupled to adenine with cesium carbonate in dimethylforamide to yield the desired prodrug given by Formula 18.

A solution of freshly distilled diethylphosphoramidous dichloride ( 100 mMoles) is added to 150 ml of anhydrous diethyl ether under a dry nitrogen atmosphere and cooled to - 20 C. A solution of 200 mMoles of ethyl 3-hydroxy-3-(4-acetoxyphenyl)propionate and 200 mMoles of dry triethylamine in 500 ml of diethyl ether is slowly added. After stirring for 15 more minutes the external cooling is removed and the reaction mixture is stirred at room temperature for 20 hours. The triethylamine hydrochloride is then removed by filtration under a nitrogen atmosphere. The filtrate is then evaporated. The residue is then dissolved in 1000 ml of methylene chloride, and was washed at 4 C with 300 ml of saturated aqueous saline, followed by 5% NaHCO₃ (300 ml X 2) , and 500 ml of water. The organic phase is then dried over sodium sulfate, filtered and dried exvacuo at room temperature. The product will be that of the N,N diethylphosphoramidite shown as Structure 6.

1-H-tetrazole (180 mMoles) is added to 60 mMoles of the N,N diethylphosphoramidite (Structure 6) and 70 mMoles of anhydrous trimethylsilanol in 300 ml of anhydrous tetrahydrofuran. (Methanol may be emplyed in place of the trimethylsilanol) After 2 hours The solvent is removed exvacuo. The diethylamine and 1-H-tetrazole are removed by distillation at reduced pressure. Then 65 mMoles of chloromethoxyethyl chloride (CL-CH₂-O-CH₂-CH₂-CL ) is added at 0 C in 100 of diethyl ether. The solution is then refluxed until the reaction has proceeded to completion. ( note: chloromethoxyethyl chloride is extremely poisonous). The solvent is then removed exvacuo. The product is dissolved in dimethylforamide to which is added 75 mMoles of adenine and 60 mMoles of cesium carbonate. The mixture is then refluxed until the reaction has proceeded to completion. Then the solvent is removed exvacuo and 300 ml of methylene chloride and 300 ml of water is added. The organic phase is separated, and washed x3 with 300 ml of saturated aqueous ammonium chloride , followed by 300 ml x 3 of water, followed by 300 ml of saturated aqueous sodium chloride. The organic phase is then dried with sodium sulfate and the solvent removed exvacuo. The desired prodrug compound (Formula 18) is thin purified by chromatography on silica.

### A CARBAMATE PRODRUG FOR PMEA

The prodrug for PMEA shown below will be selectively metabolized in the liver to PMEA. This will further enhance the therapeutic index of PMEA for the treatment of Hepatitis B. 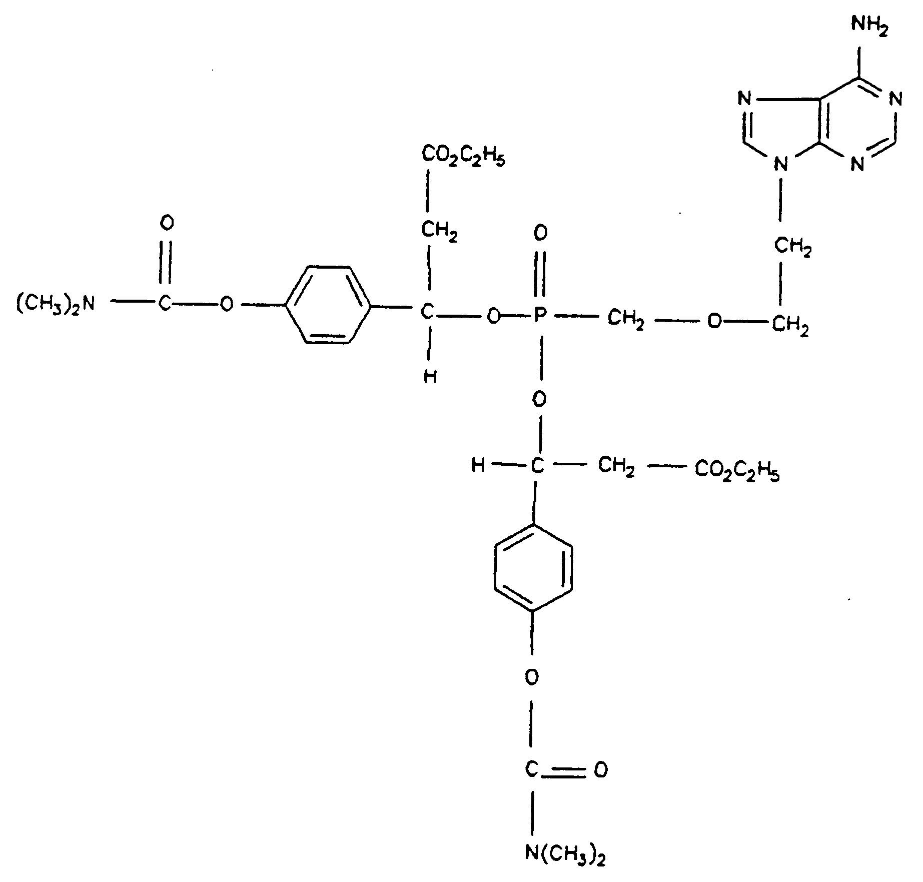
This carbamate prodrug of PMEA may be made by replacing ethyl 3-hydroxy-3-(4-acetoxyphenyl)propionate in Methods A-F above with the following compound: 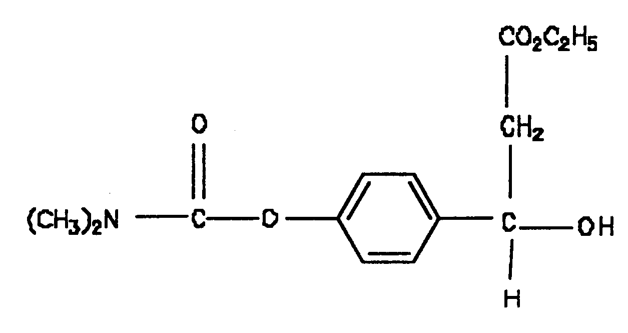
The compound above is readily made by treating ethyl 4-hydroxybenzoylacetate with dimethylcarbamoyl chloride and dimethylaminopyridine in pyridine. The procedure described for for the treatment of ethyl 4-hydroxybenzoylacetate with acetic anhydride may be used except that the dimethylcarbamoyl chloride is used in place of the acetic anhydride. The resulting compound is then reduced using the same procedure as described for the reduction of ethyl 4-acetoxy-benzoylacetate in EXAMPLE 2.

### PRODRUGS FOR FPMPA AND FPMPDAP

9-(RS)-(3-fluoro-2-phosphonylmethoxypropyly)Adenine (FPMPA) and 9-(RS)-(3-fluoro-2-phosphonylmethoxypropyly)2,6,Diaminopurine (FPMPDAP) are potent and selective inhibitors of HIV reverse transcriptase. Holy,A.; Balzarini,J.; Jindrich,J., Dvorakova,H.; Hao,Z.; Snoeck,R.; Herdewijn,P.; Johns,D.; De Clercq,E.; Antiviral Research; Supple.1:47 ; 1991. These agents may be converted into lipid soluble prodrugs using the same procedrures as described for the synthesis of prodrugs for PMEA in Methods A-D by substituting for PMEA FPMPA or FPMPDAP. The structures of two prodrugs for FPMPA are shown below:

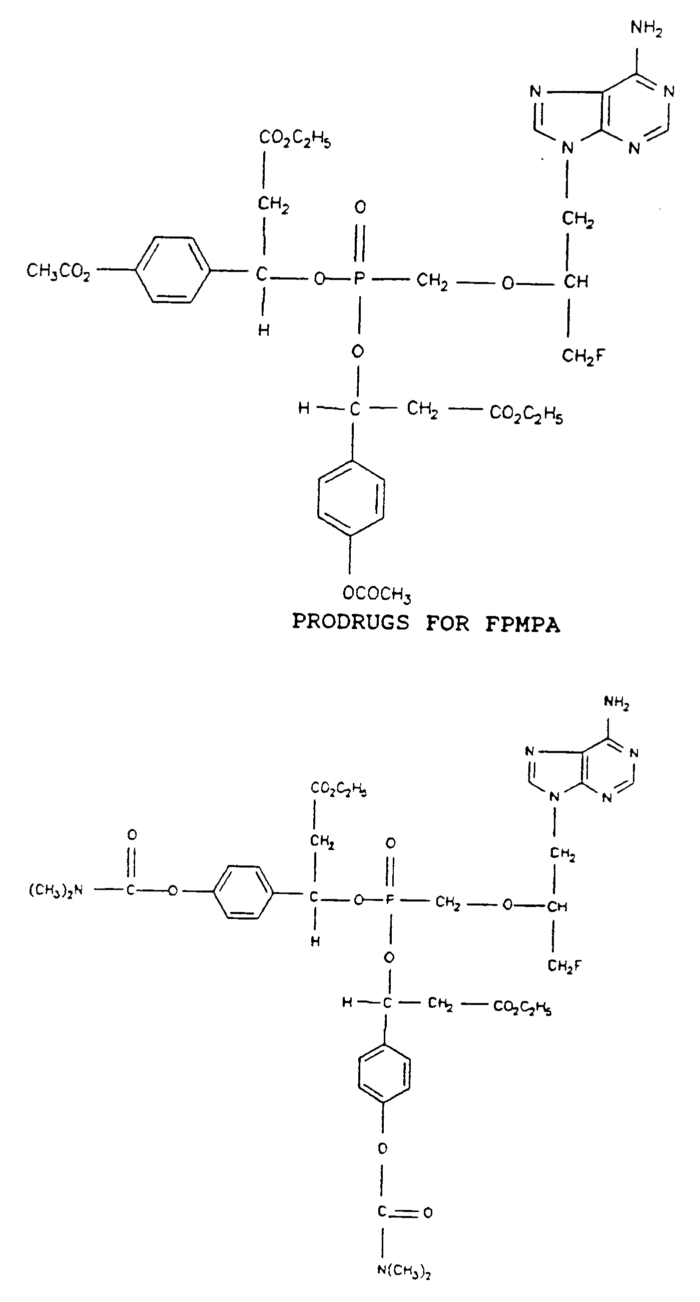

### NUCLEOTIDE MONOPHOSPHATE PRODRUGS

A wide variety of biologically active nucleoside derivatives require phosphorylation in order to exhibit biological activity. For example, a major factor which determines inhibitory activity of didexoynucleoside analogs against the AIDS virus is the ability of the nucleoside to be phosphorylated. Zhang,H. et al.Molecular Pharmacology, 34:431 (1988).Balzarini,J. et al. J.Biol.Chem. , 264:6127 (1989) The ability of cells from differing tissues to phosphorylate a nucleosides is quite variable. For example, 3'fluro-2'deoxythymidine (FLT) is phosphorylated efficently by spleen cells but not by liver cells. This results in the inactivity of FLT against Hepatitis B virus in liver cells. Lofgren,B. , Habteyesus, Eriksson,S.,Oberg,B.; Antiviral Chem.and Chemotherapy; 1:361 1990 . The present prodrug method will allow the delivery of the monophosphate derivatives of a wide variety of antiviral nucleotides into cells , including the liver and the brain.

The present prodrug method may also be employed to deliver nucleotide analogs which possess antineoplastic activity into cells. For example, chain terminating nucleoside analogs may be selectively toxic for leukemic cells that possess the enzyme deoxyribonucleotidyl transferase (TDT). Spigelman,Z. et. al.Blood, 71:1601 (1988) Cytotoxicity is likely dependent upon phosphorylation of the dideoxynucleoside into the corresponding triphosphate and incorporation of the chain terminating analog into the cellular DNA via TDT. The present prodrug method may be used to selectively kill TDT+ leukemic cells that are deficient in nucleoside kinase activity.

### General Structure of Nucleotide Monophosphate Prodrugs:

The general structure for nucleotide monophosphate prodrugs is shown below as Formula 19: 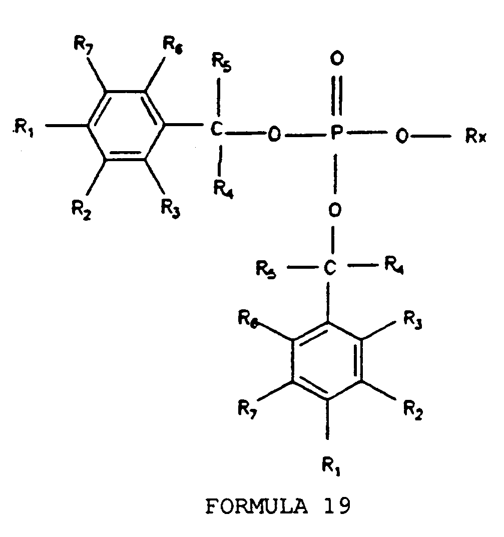
Wherein R1-R7 are as described for Structure 2 and Rx is the nucleosidyl group

### General Methods for Synthesizing Nucleotide Monophosphate Prodrugs

In the following sections the term "protected nucleoside" refers to a nucleoside derivative in which potentially interfering groups are suitably protected. Routine methods of nucleoside protection and deprotection may be employed. Sonveaux,E.Bioorganic Chem. , 14:274 (1986) The protecting groups are removed in the last step of prodrug synthesis.

### METHOD A

10 mMoles of phosphorous trichloride and 10 mmoles of triethylamine are added to 50 ml of anhydrous acetoniutrile at - 20 C. Then 10 mMoles of "protected nucleoside" in 30 ml of anhydrous acetonitrile is added to the rapidly stirred solution. After 4 hours an additional 20 mMoles of triethylamine and 35 mMoles of strictly anhydrous Formula 1 are added and the reaction is warmed to room termperature. After the reaction has proceeded to completion the solvent is removed exvacuo. 150 ml of diethyl ether is added and the precipitated triethylamine hydrochloride is removed by filtration. Then 40 ml of water is slowly added. The organic phase is then separated and the solvent removed exvacuo. The residue is then treated with 30 ml of a solution containing 450 mg of iodine in a 2:1 tetrahydrofuran-water mixture at 0 C for 5 minutes. Then 200 ml of methylene chloride and 100 ml of water are added. The organic phase is separated, washed times 3 with 50 ml of a 5% solution of aqueous sodium thiosulfate, followed by 50 ml x 3 of water. The protective groups are then removed using routine methods and the desired prodrug purified by crystallization from a suitable solvent or via chromatography on silica.

### Method B

10 mMoles of phosphorous trichloride and 10 mMoles of triethylamine are added to 50 ml of anhydrous acetonitrile at -20 C. Then 10 mMoles of "protected nucleoside" in 50 ml of anhydrous acetonitrile is added to the rapidly stirred solution. After 2 hours 40 mMoles of diisopropylamine is added and the mixture is warmed to room temperature for 3 hours. Then 30 mMoles of strictly anhydrous Formula 1 and 90 mMoles of 1-H-tetrazole are added. After the reaction has proceeded to completion the solvent is removed exvacuo. Then 200 ml of diethyl ether is added. The precipitated triethylamine hydrochloride and diisopropylamine hydrochloride are removed by filtration. Then the solvent is removed exvacuo. The residue is then treated with 30 ml of a solution containing 450 mg of iodine in a 2:1 tetrahydrofuran-water mixture at 0 C for 5 minutes. Then 200 ml of methylene chloride and 100 ml of water are added. The organic phase is separated, washed times 3 with 50 ml of a 5% solution of aqueous sodium thiosulfate, followed by 50 ml x 3 of water. The protective groups are then removed using routine methods and the desired prodrug purified by crystallization from a suitable solvent or via chromatography on silica.

### METHOD C

10 mMoles of phosphorous oxychloride and 10 mMoles of triethylamine are added to 50 ml of anhydrous acetonitrile at - 10 C. Then 10 mMoles of "protected nucleoside" in 50 ml of anhydrous acetonitrile is added to the rapidly stirred solution. After 4 hours an additional 20 mMoles of triethylamine, 20mMoles of n-methylimidazole, and 20 mMoles of 1-hydroxybenzotriazole and 35 mMoles of strictly anhydrous Formula 1 are added. After 6 hours the solvent is removed exvacuo and 200 ml of diethyl ether is added. The precipitated triethylamine hydrochloride is removed by filtration. Then 100 ml of water are added. The organic phase is separated, washed times 3 with 50 ml of 1 M aqueous sodium bicarbonate followed by 50 ml x 3 of water. The protective groups are then removed using routine methods and the desired prodrug purified by crystallization from a suitable solvent or via chromatography on silica.

### Method D

10 mMoles of phosphorous oxychloride and 30 mmoles of triethylamine and 30 mMoles of 3-nitro,1,2,4-triazole are added to 50 ml of anhydrous acetonitrile at room temperature. After 1 hour 10 mMoles of "protected nucleoside" in 30 ml of acetonitrile is added to the rapidly stirred solution. After 4 hours 35 mMoles of strictly anhydrous Formula 1 are added. After 6 hours the solvent is removed exvacuo and 200 ml of diethyl ether is added. The precipitated triethylamine hydrochloride is removed by filtration. Then 200 ml of water are added. The organic phase is separated, washed times 3 with 50 ml of 1 M aqueous sodium bicarbonate followed by 50 ml x 3 of water. The protective groups are then removed using routine methods and the desired prodrug purified by crystallization from a suitable solvent or via chromatography on silica.

### METHOD E

A solution of freshly distilled diethylphosphoramidous dichloride (10 mMoles) is added to 20 ml of anhydrous diethyl ether under a dry nitrogen atmosphere and cooled to - 20 C. A solution of Formula 1, ( 20 mMoles) and dry triethylamine (20 mMoles) in 150 ml of diethyl ether is added dropwise over 15 minutes. After stirring for 15 more minutes the external cooling is removed and the reaction mixture is stirred at room temperature for 20 hours. The triethylamine hydrochloride is then removed by filtration under a nitrogen atmosphere. The residue is then dissolved in 300 ml of methylene chloride, and washed at 4 C with 150 ml of saturated aqueous saline, followed by 5% NaHCO₃ (150 ml X 2) , and 150 ml of water. The organic phase is then dried over sodium sulfate, filtered and dried exvacuo at room temperature. 1-H-tetrazole ( 24 mMoles) is added along with the desired "protected nucleoside" ( 10 mMoles) in 30 ml of anhydrous tetrahydrofuran. After 1.5 hours the solution is cooled to -40 C and and a solution of 3-chloroperoxybenzoic acid ( 16.5 mMoles as 85% reagent) in 45 ml of dry methylene chloride is added rapidly in a dropwise fashion. External cooling is removed and after 15 minutes, 60 ml of 10% aqueous NaHSO₄ is added and stirred vigorously for 10 minutes. The mixture is transferred to a separatory funnel with the addition of 150 ml of methylene chloride. After the addition of 500 ml of diethyl ether the organic phase is washed with 100 ml of 10% aqueous NaHSO₄ ,saturated NaHCO₃ (75 ml X 2) ,and water (75 ml X 2). The organic phase is then dried over sodium sulfate, filtered and dried exvacuo to yield crude product. The desired product is then purified by silica gel chromatography.

### PRODRUGS FOR 3'AZIDO-3'DEOXY-THYMIDINE 5'PHOSPHATE

The general structure of prodrugs for 3'azido-dideoxythymidine 5'monophosphate (AZT-phosphate) is shown below as Formula 19: 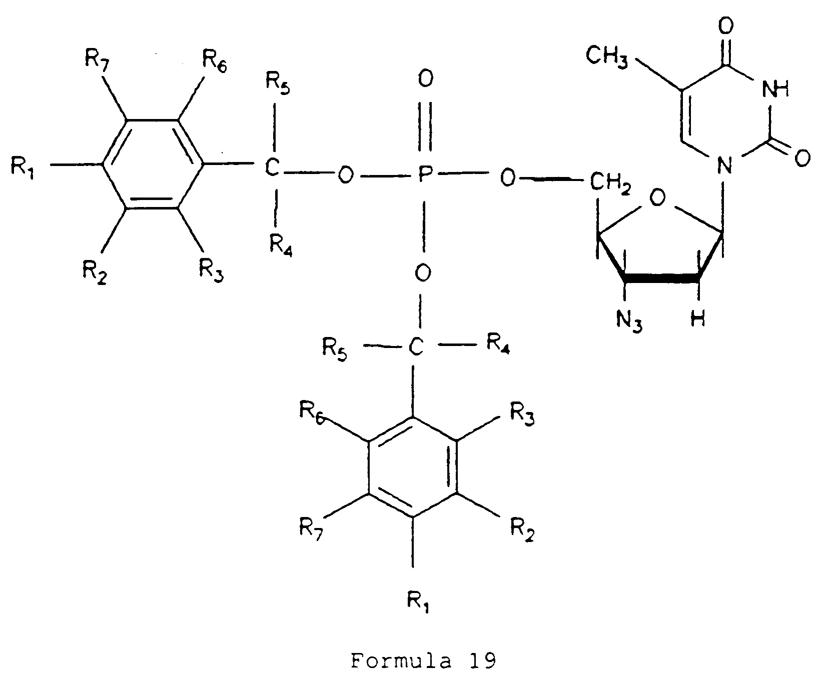
Wherein R1-R7 are as described for Structure 2 .
Prodrugs of Formula 19 may be employed to deliver AZT-phosphate into cells and into the brain. Although AZT has shown some promise in the treatment of HIV associated encephalitis the penetration of AZT into brain tissue is very poor. Terasaki,T. et al. J.Inf.Dis. 158:630 (1988), Doshi,K.J. et al. Drug Metab. Dispos. 17:590 (1989) The present prodrug will circumvent this problem. In addition the present prodrugs will prevent the multiplication of HIV in those tissues which do not adequately phosphorylate AZT.

The synthesis and biological activity for one prodrug for AZT was discussed previously in EXAMPLE 3. An example of a second prodrug for AZT-phosphate is shown below as Formula 20:

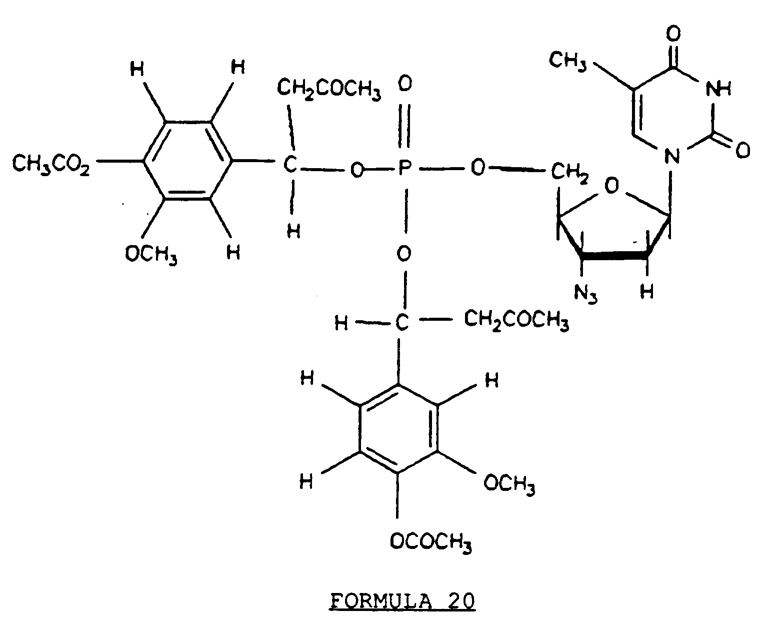

### Synthesis of Formula 20

### METHOD A

10 mMoles of phosphorous trichloride and 10 mmoles of triethylamine are added to 50 ml of anhydrous acetonitrile at - 20 C. Then 10 mMoles of 3'azido-2',3'deoxythymidine (AZT) in 30 ml of anhydrous acetonitrile is added to the rapidly stirred solution. After 4 hours an additional 20 mMoles of triethylamine and 35 mMoles of anhydrous 4-(4-acetoxy,3-methoxyphenyl), 4-hydroxy,2-butanone are added and the reaction is warmed to room temperature. After the reaction has proceeded to completion the solvent is removed exvacuo. 200 ml of diethyl ether are added. The precipitated triethylamine hydrochloride is removed by filtration. Then 40 ml of water is slowly added. The organic phase is then separated and the solvent removed exvacuo. The residue is then treated with 30 ml of a solution containing 450 mg of iodine in a 2:1 tetrahydrofuran-water mixture at 0 C for 5 minutes. Then 200 ml of methylene chloride and 100 ml of water are added. The organic phase is separated, washed times 3 with 50 ml of a 5% solution of aqueous sodium thiosulfate, followed by 50 ml x 3 of water. The desired prodrug (Formula 20) is then purified by crystallization from a suitable solvent or via chromatography on silica using routine methods. NOTE: the phosphite may also be oxidized to the phosphate using m-chloroperoxybenzoic acid as described in EXAMPLE 3.

### Method B

10 mMoles of phosphorous trichloride and 10 mmoles of triethylamine are added to 50 ml of anhydrous acetonitrile at - 20 C. Then 10 mMoles of AZT in 50 ml of anhydrous acetonitrile is added to the rapidly stirred solution. After 2 hours 40 mMoles of diisopropylamine is added and the mixture is warmed to room temperature for 3 hours. Then 30 mMoles of strictly anhydrous 4-(4-acetoxy,3-methoxyphenyl), 4-hydroxy,2-butanone and 90 mMoles of 1-H-tetrazole are added. After the reaction has proceeded to completion the solvent is removed exvacuo and 200 ml of diethyl ether are added. The precipitated triethylamine hydrochloride and diisopropylamine hydrochloride are removed by filtration. Then the solvent is removed exvacuo. The residue is then treated with 30 ml of a solution containing 450 mg of iodine in a 2:1 tetrahydrofuran-water mixture at 0 C for 5 minutes. Then 200 ml of methylene chloride and 100 ml of water are added. The organic phase is separated, washed times 3 with 50 ml of a 5% solution of aqueous sodium thiosulfate, followed by 100 ml x 3 of water. The desired prodrug may then be purified by crystallization from a suitable solvent or via chromatography on silica.

### METHOD C

10 mMoles of phosphorous oxychloride and 10 mmoles of triethylamine are added to 50 ml of anhydrous acetonitrile at 30 C. Then 10 mMoles of AZT in 50 ml of anhydrous acetonitrile is added to the rapidly stirred solution. After 4 hours an additional 20 mMoles of triethylamine, 20mMoles of n-methylimidazole, and 20 mMoles of 1-hydroxybenzotriazole and 35 mMoles of strictly anhydrous 4-(4-acetoxy,3-methoxyphenyl), 4-hydroxy,2-butanone are added. After the reaction has proceeded to completion the solvent is removed exvacuo and 200 ml of diethyl ether are added. The precipitated triethylamine hydrochloride is removed by filtration. Then 200 ml of water are added. The organic phase is separated, washed times 3 with 100 ml of 1 M aqueous sodium bicarbonate followed by 50 ml x 3 of water. The desired prodrug is then purified by crystallization from a suitable solvent or via chromatography on silica.

### Method D

10 mMoles of phosphorous oxychloride and 30 mmoles of triethylamine and 30 mMoles of 3-nitro,1,2,4-triazole are added to 50 ml of anhydrous acetonitrile at room temperature. After 1 hour 10 mMoles of AZT in 20 ml of anhydrous acetonitrile are added to the rapidly stirred solution. After 4 hours 35 mMoles of strictly anhydrous 4-(4-acetoxy,3-methoxyphenyl), 4-hydroxy,2-butanone are added. After the reaction has proceeded to completion as evidenced by TLC monitoring the solvent is removed exvacuo and 200 ml of diethyl ether are added. The precipitated triethylamine hydrochloride is removed by filtration. Then 200 ml of water are added. The organic phase is separated, washed times 3 with 50 ml of 1 M aqueous sodium bicarbonate followed by 50 ml x 3 of water. The desired prodrug purified is then by crystallization from a suitable solvent or via chromatography on silica.

### Prodrugs for 2,'3'-didehydro-2',3'dideoxythymidine-5'monophosphate

2',3'-didehydro-2',3'dideoxythymidine (D4T) is a potent inhibitor of HIV replication with a lower toxicity for meyloid progenitor cells then AZT. Martin,J.C. et al. Nucleosides and Nucleotides , 8:841 (1989). Antiviral activity requires conversion to the 5'triphosphate. The rate limiting step in the metabolism of D4T is conversion to the 5'monophosphate derivative. Hsu-Tso,Ho; Hitchcock,J.; Antimicrobial Agents and Chemo. ; 33:844 1989. The present prodrug method may be employed to facilitate the delivery of D4T-phosphate into cells and into the brain. An example of a prodrug for D4T-phosphate is shown below as Formula 21:

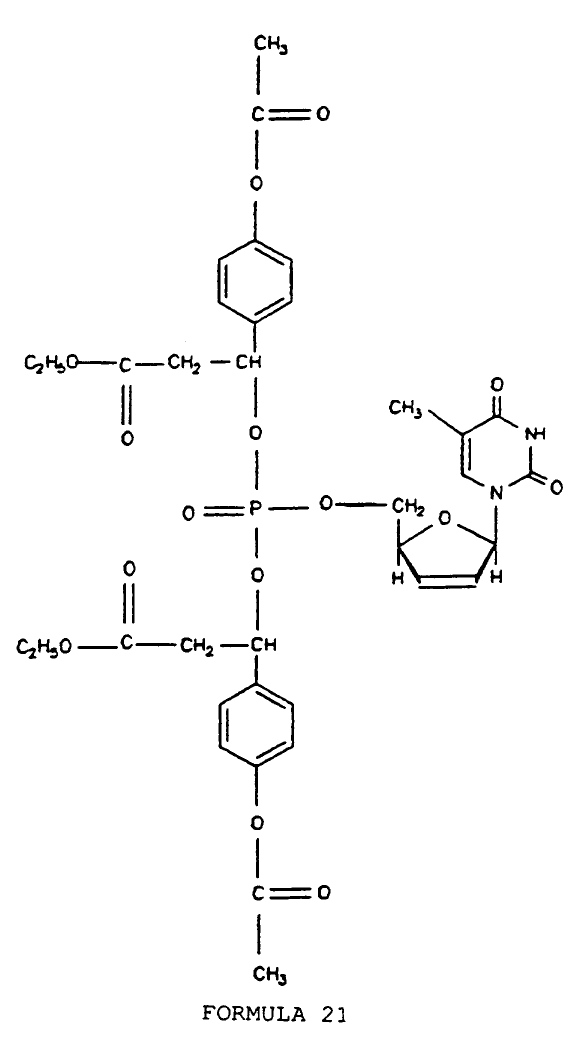

### Synthesis of Formula 21

Formula 21 may be synthesized by the same methods described above for the synthesis of prodrugs for AZT except that 2',3'-dihyhydro-2',3'dideoxythymidine (D4T) is used in place of AZT.

### Prodrugs for 2',3'-dideoxyadenosine 5'monophosphate

The present prodrug method may be employed to deliver 2'3'dideoxyadenosine 5'monophosphate into the cytoplasm of cells. This compund will be selectively toxic for TDT+ leukemia cells including those leukemic cells that are deficient in the kinase required for the phosphorylation of 2'3'dideoxyadenosine to the corrsponding 5'phosphate. The structure of a prodrug for 2'3'dideoxyadenosine 5'monophosphate is shown below as Formula 22:

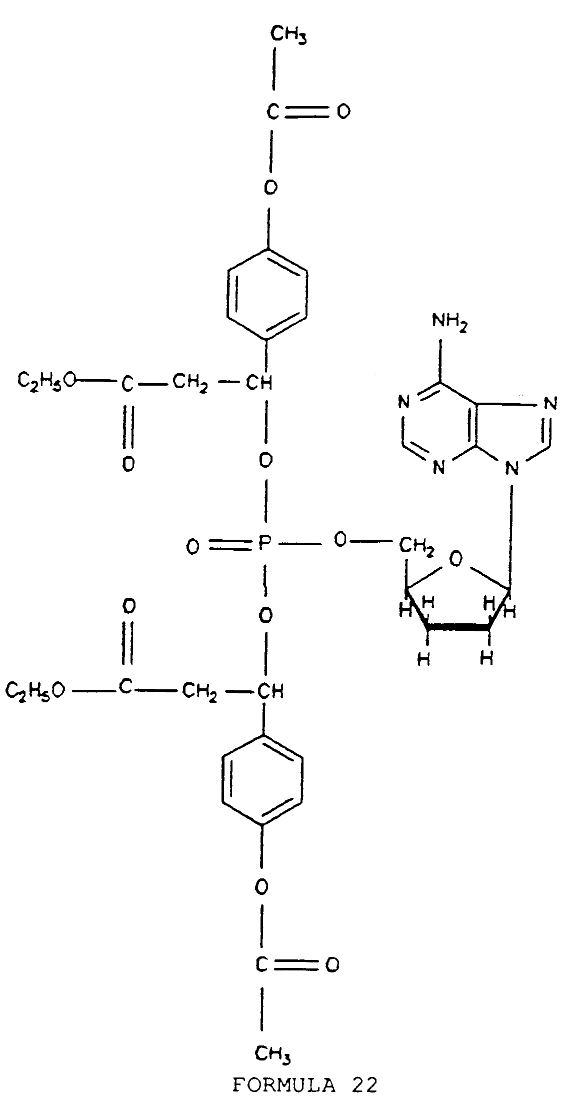

### SYNTHESIS OF FORMULA 22

Formula 22 may be synthesized by the same methods described above for the synthesis of prodrugs for AZT except that 2',3'-dideoxy-Adenosine (DDA) is used in place of AZT. Alternatively, a protected derivative of DDA may be employed. The protective group is then removed in the last step of the prodrug synthesis. For example, the exocyclic amine group on the adenine base may be protected by an FMOC group by treatment of DDA in pryidine with 9-Fluorenylmethyl chloroformate. In the last step of the prodrug synthesis the FMOC group is then removed by treatment with a base such as triethylamine.

### Treatment with and Administration of the Prodrugs

The present prodrugs can be used to treat certain disorders by allowing the facile penetration of the drug through the blood brain barrier and into cells by converting them into lipid-soluble compounds. The prodrugs then undergo biotransformation ,in vivo yielding the active phosphorylated form of the drug which is necessary for the treatment of the disorder. The prodrugs also allow for enhanced oral and topical drug absorbtion. For example, the prodrugs described for AZT-monophosphate, D4T-monophosphate, Phosphonoformate, PMEA, FPMPA, FPMPDAP, and 2'3'dideoxyadenosine-monophosphate can be administered to a patient with Acquired Immunodeficiency Syndrome or HIV infection. The prodrugs for 2'3'dideoxyadenosine-monophosphate can be administered to a patient with TDT+ leukemia. The prodrugs for PMEA can be administered to a patient with hepatitis B infection. The prodrugs will then be metabolized in vivo to the active phosphorous bearing drugs. The treatment program, i.e., drug dosage, frequency of administration,and length of administration will depend upon several factors such as the age, weight, and physical condition of the patient, the stage of the disease and the patients tolerance for the drug.

The prodrugs can be administered orally, parenterally or topically. The form in which the drugs are given (e.g. , powder, tablet, capsule, solution, emulsion) will depend upon the route by which it is to be administered. The quantity of the drugs to be administered will be determined on an individual basis and will be determined in part on consideration of the individuals size, the severity of the symptoms, and the result sought.

The composition of the present invention can optionally include, in addition to the prodrug other components. The other components included in a particular composition are determined primarily by the route of administration. For example, a composition to be administered orally in tablet form can include, in addition to the drug, a filler (e.g., lactose) , a binder (e.g. carboxy-methyl cellulose, gum arabic, gelatin) a flavoring agent, a coloring agent, and a coating material (e.g., wax,or a plasticizer). A composition to be administered in a liquid form can include the drugs of the present invention, and optionally an emulsifying agent, a carrier (e.g. saline or water), a flavoring agent, and or a coloring agent. A composition to be administered in a topical form may include an emulsifying agent, a solvent, stabilizing agents and a base such as polyethylene glycol.

## Claims (Claims for the following Contracting State(s): AT, BE, CH, LI, DE, DK, FR, GB, IT, LU, NL, SE)

1. A prodrug compound for a parent drug, wherein the parent drug has the general structure: 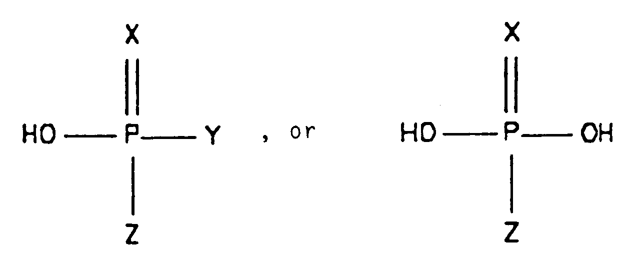 wherein X is oxygen (O), or sulphur (S), and Y and Z are defined by the remainder of the parent drug; and wherein the parent drug is not phosphoric acid; wherein the prodrug compound has the following structure: 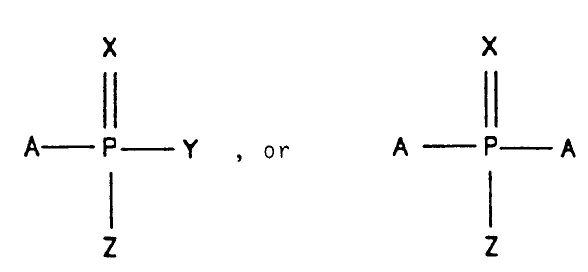 wherein the group "A" has the following structure: 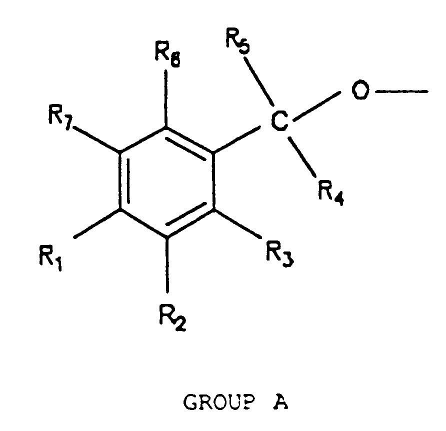 Wherein R1 can be an acyloxy group (-O-CO-R8) ; a (-O-CO₂-R8) group;
a (-O-C(O)-NH₂) group; a (-O-C(O)-NHCH3 ) group; a (-O-C(O)-N(CH3)₂) group; hydrogen; a halogen; a ( -CO₂R9 ) group; an alkyl (e.g. methyl) group; or a hydroxymethyl group (HO-CH₂-);
R2 can be hydrogen; a ( -CO₂R10 ) group; an alkyl (e.g. methyl) group; a halogen; a methoxy group; an acyloxy group (-O-CO-R8); or a hydroxymethyl group (HO-CH₂-);
R3 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; an alkyl (e.g. methyl) group; a hydroxymethyl group (-CH₂-OH ); a halogen; a hydroxyethyl group (-CH₂-CH₂-OH ); or a (-CH₂-CO₂-R11) group ;
R4 can be hydrogen ; an alkyl (e.g. methyl) group; methoxy-methyl (-CH₂-O-CH₃ ), a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group, or a methylene substituted with a functional group which favors an elimination reaction by lowering the intrinsic barrier for removal of a proton from said methylene;
R5 can be hydrogen ; an alkyl (e.g. methyl) group; methoxy-methyl (-CH₂-O-CH₃ ); a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group, or a methylene substituted with a functional group which favors an elimination reaction by lowering the intrinsic barrier for removal of a proton from said methylene;
R6 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; an alkyl (e.g. methyl) group; a hydroxymethyl group (-CH₂-OH ); a halogen; a hydroxyethyl group (-CH₂-CH₂-OH ); or a (-CH₂-CO₂-R11) group ;
R7 can be hydrogen; a ( -CO₂R10 ) group; an alkyl (e.g. methyl) group; a halogen; or a methoxy group; an acyloxy group (-O-CO-R8); or a hydroxymethyl group (HO-CH₂-)
Wherein R8 can be a group of the following structure: (-(CH₂)_{N}-CH₃) for N= 0-18; R8 may be a phenyl group, wherein the phenyl group may in turn bear substituents, wherein R8 may also be selected such such that R8-CO₂H is; an amino acid; lactic acid; glycolic acid ( HO-CH₂-CO₂H); glyceric acid ( HO-CH₂-CH(OH)-CO₂H); or acetoacetic acid ( CH₃COCH₂-CO₂H);
Wherein R9, and R10, R11, and R12, is a methyl, ethyl, phenyl, or benzyl; and
Wherein at least one of the following groups: R1; R3 ; R6 is an acyloxy group ( -O-CO-R8 ) or another group which undergoes biotransformation in vivo to ultimately yield a hydroxy group on the phenyl ring

2. A prodrug of Claim 1, wherein the parent drug is (a) a monophosphate ester drug; or
(b) a phosphodiester drug; or
(c) a phosphonic acid drug or phosphonate drug; or
(d) a phosphinic acid drug; or
(e) a nucleotide monophosphate drug; or is
(f) selected from the the group consisting of:
9-(2-phosphonylmethoxy-ethyl)adenine , (PMEA); 9-(RS)-(3-fluoro-2-phosphonylmethoxypropyly)Adenine (FPMPA), 9-(RS)-(3-fluoro-2-phosphonylmethoxypropyly) 2,6,diaminopurine (FPMPDAP); 3'azido-dideoxythymidine 5'monophosphate (AZT-phosphate);
2',3'-didehydro-2',3'dideoxythymidine 5' monophosphate (D4T-phosphate); 2'3'dideoxyadenosine 5'monophosphate;a carboxylate ester of phosphonoformate

3. A prodrug of Claim 1, wherein the structure of group "A" is selected from the group consisting of:

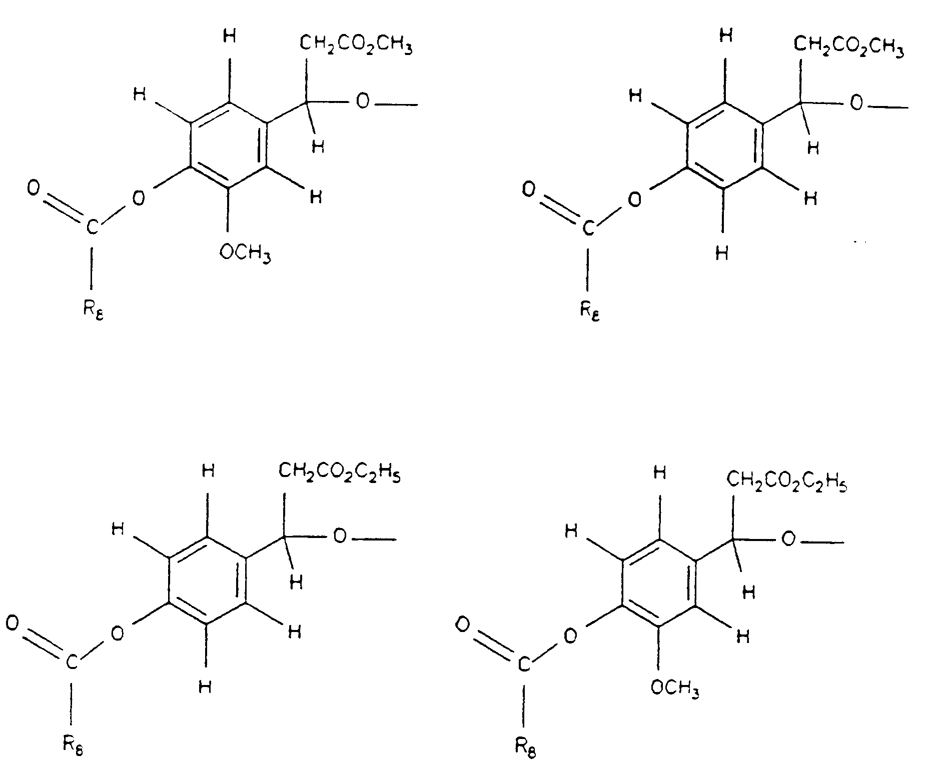

4. A prodrug of Claim 3 wherein the parent drug is (a) a nucleotide monophosphate; or is
(b) selected from the following group:
9-(2-phosphonylmethoxy-ethyl)adenine , (PMEA); 9-(RS)-(3-fluoro-2-phosphonylmethoxypropyly)Adenine (FPMPA), 9-(RS)-(3-fluoro-2-phosphonylmethoxypropyly)2,6,Diaminopurine (FPMPDAP); 3'azido-dideoxythymidine 5'monophosphate (AZT-phosphate); 2',3'-didehydro-2',3'dideoxythymidine 5' monophosphate (D4T-phosphate); 2'3'dideoxyadenosine 5'monophosphate; or is
(c) a carboxylate ester of phosphonoformate

5. A prodrug of Claim 1 wherein the group "A" has the following structure:

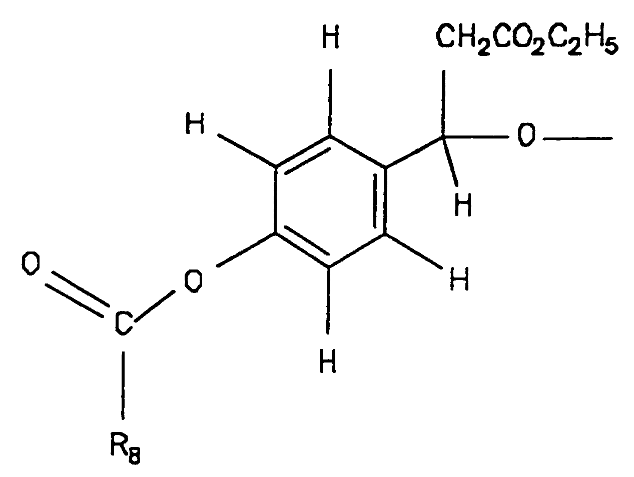

6. A prodrug of Claim 5 wherein the parent drug is a nucleotide monophosphate; or is selected from the following group:
9-(2-phosphonylmethoxy-ethyl)adenine , (PMEA); 9-(RS)-(3-fluoro-2-phosphonylmethoxypropyly)Adenine (FPMPA), 9-(RS)-(3-fluoro-2-phosphonylmethoxypropyly)2,6,Diaminopurine (FPMPDAP); 3'azido-dideoxythymidine 5'monophosphate ; 2',3'-didehydro-2',3'dideoxythymidine 5' monophosphate (D4T-phosphate); 2'3'dideoxyadenosine 5'monophosphate; a carboxylate ester of phosphonoformate

7. A prodrug of Claim 1 of the following structure: 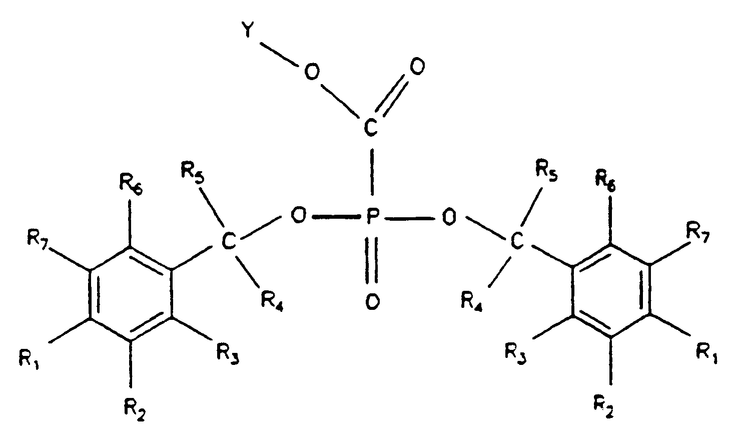 wherein Y is a benzyl, or phenyl group and wherein the benzyl or phenyl group may in turn bear substituents

8. A prodrug of Claim 7 having the structure:

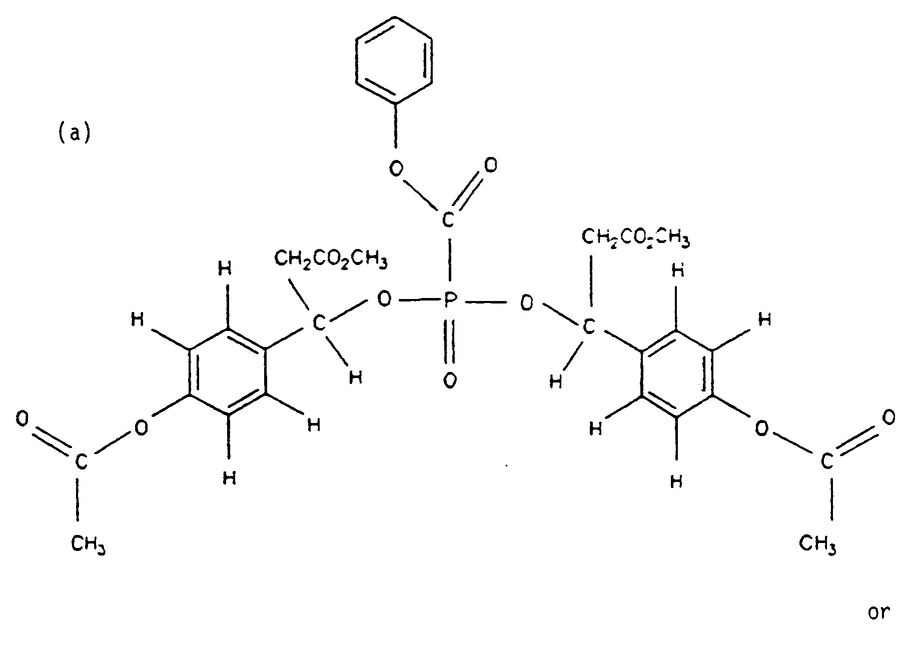

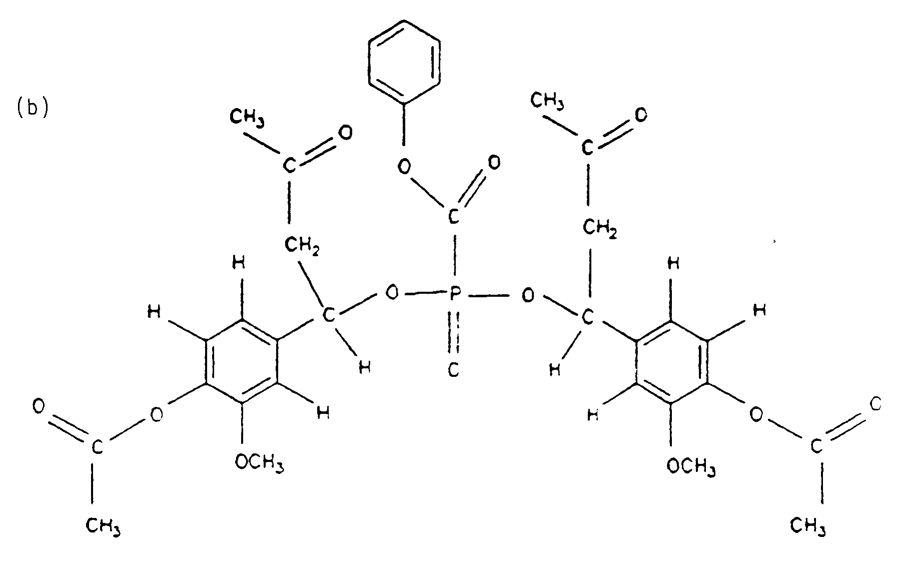

9. A prodrug of Claim 1 of the following structure:

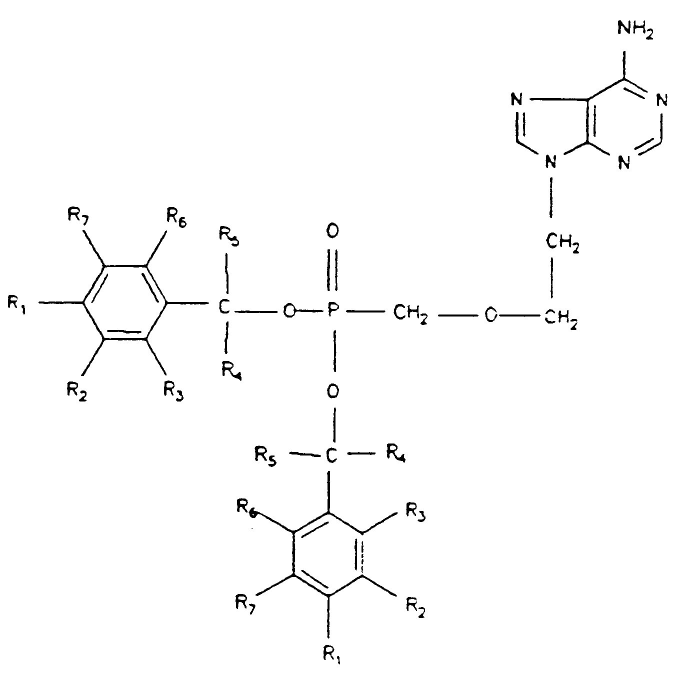

10. A prodrug of claim 9 having the structure:

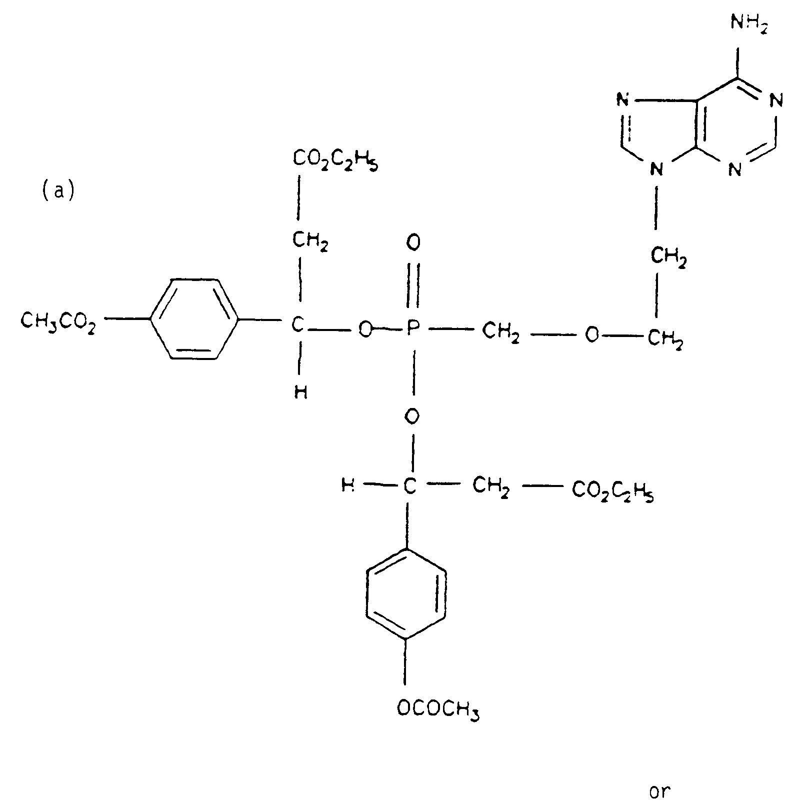

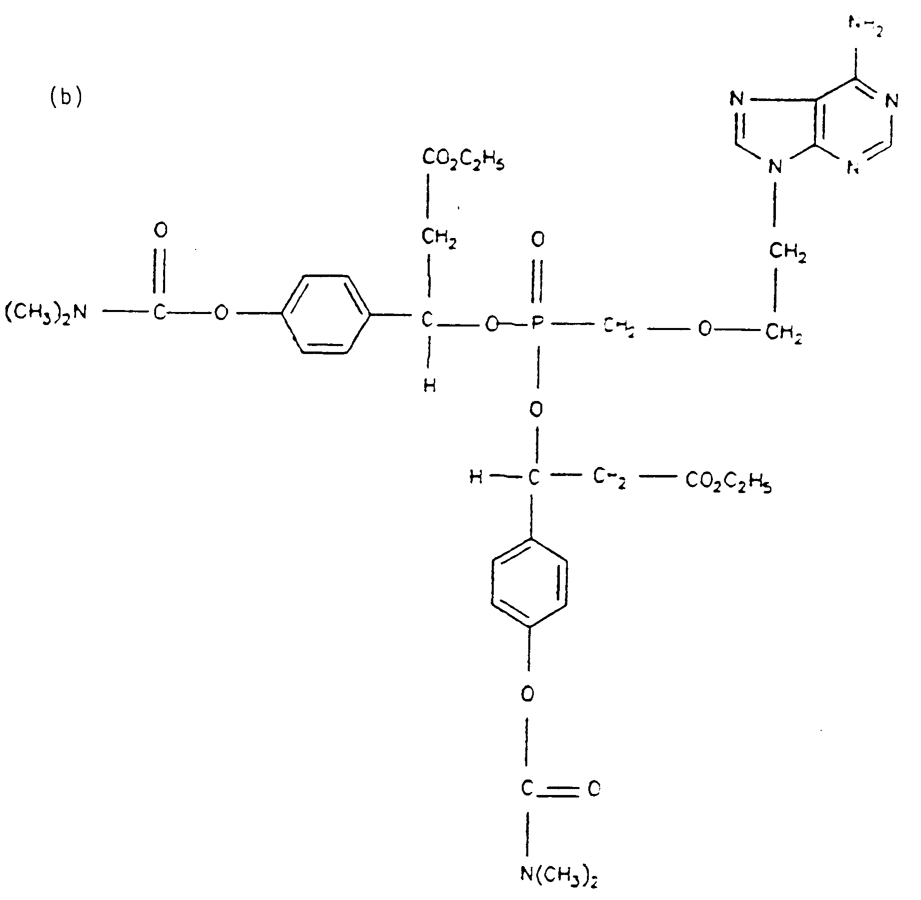

11. A prodrug of claim 1 of the following structures:

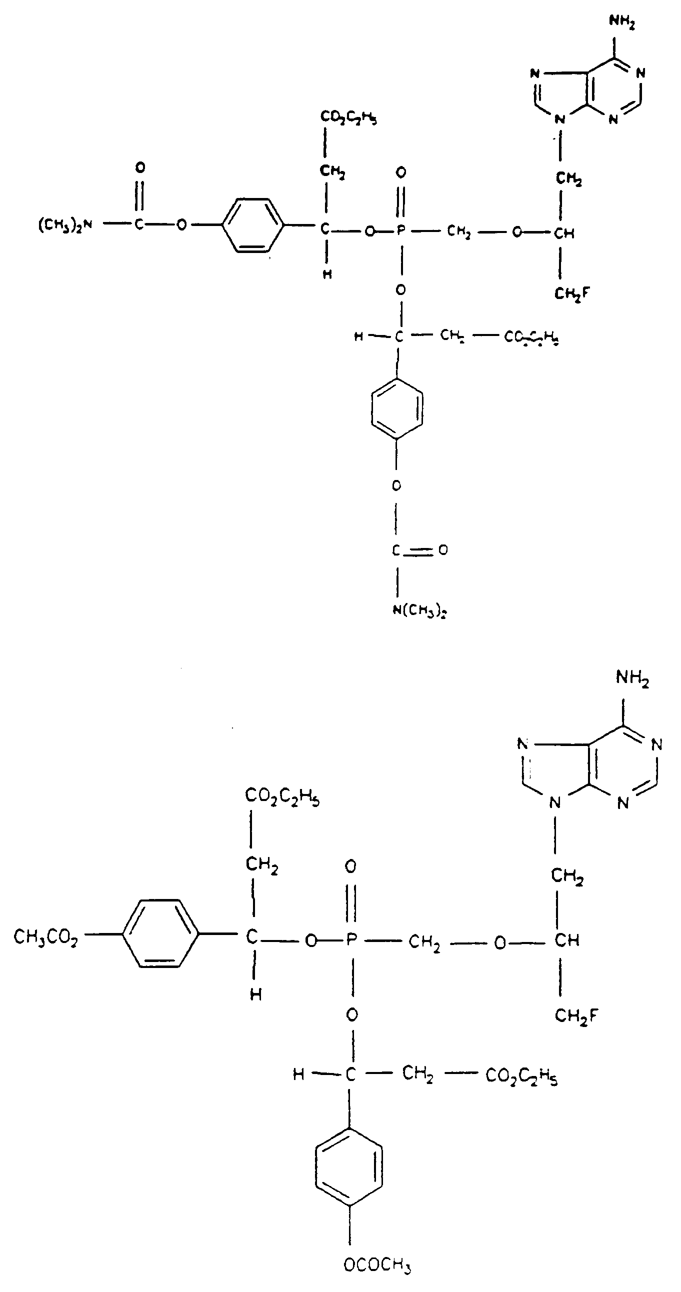

12. A prodrug of Claim 1 of the following structure: 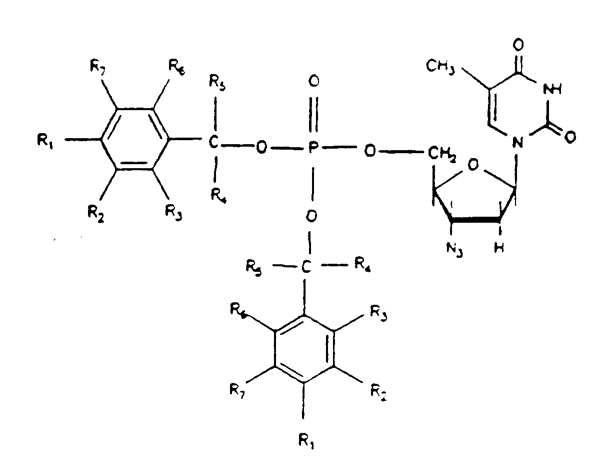 for example:

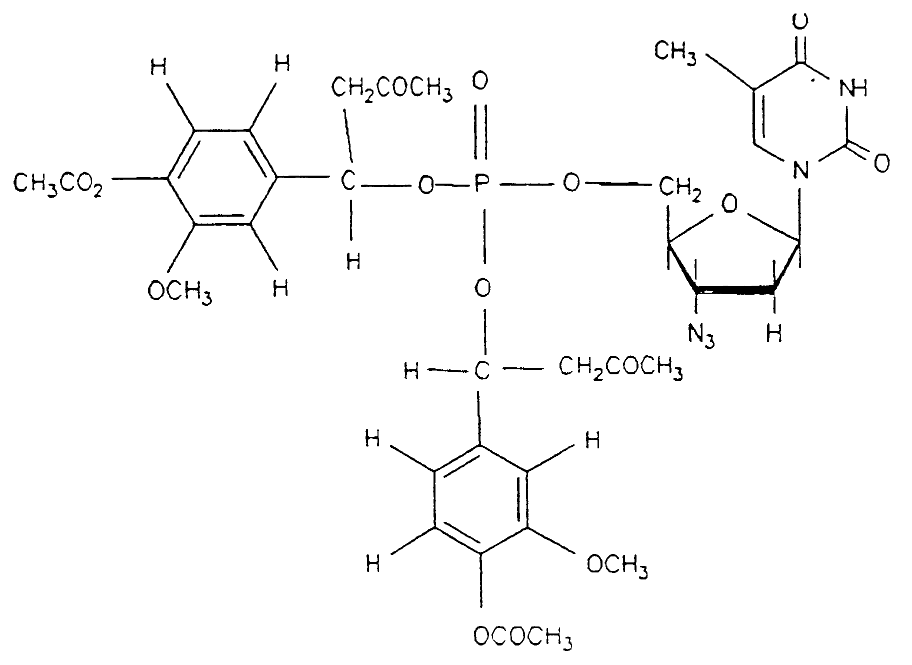

13. A prodrug of claim 1 having the structure:

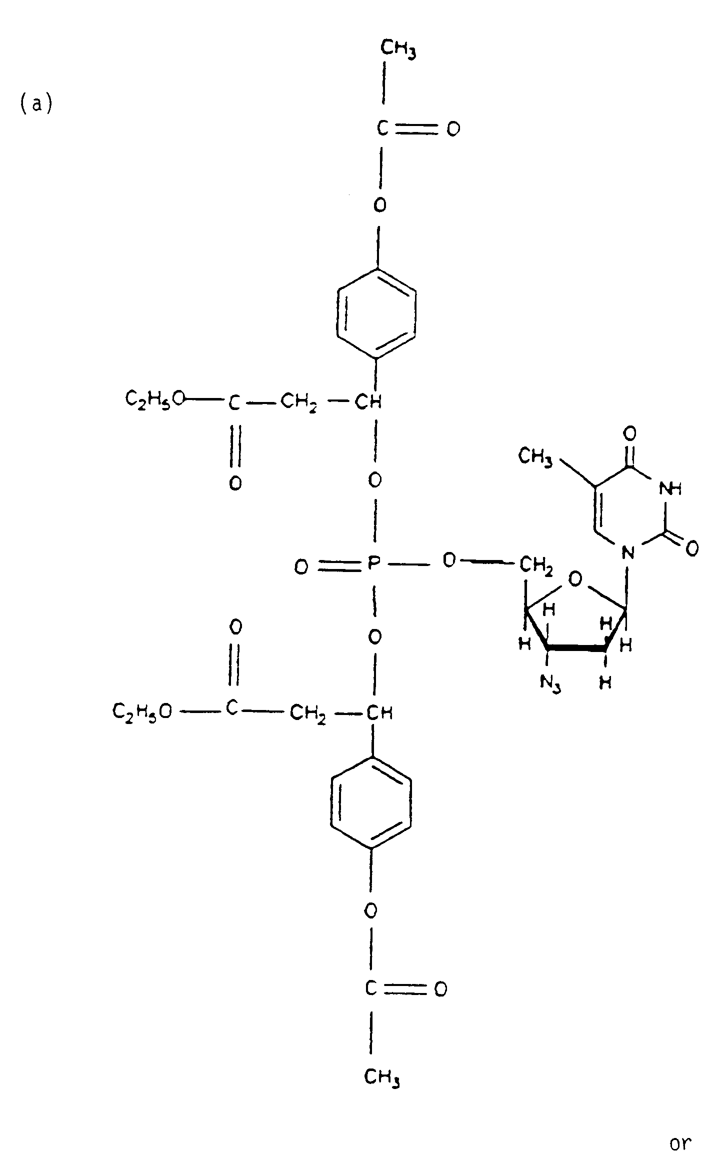

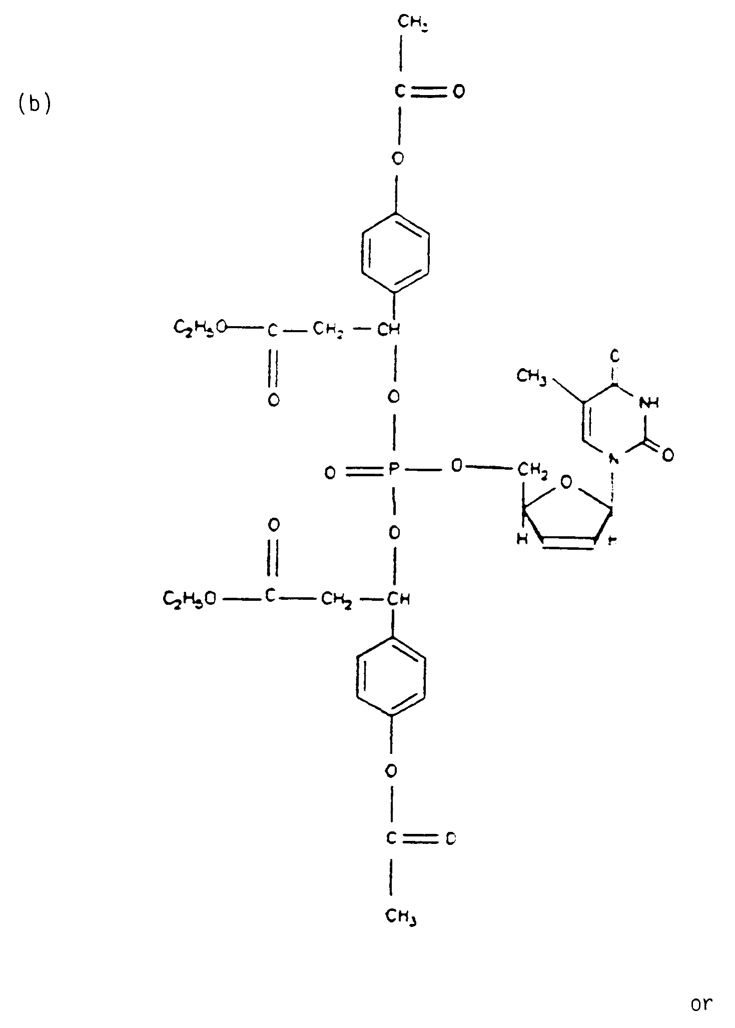

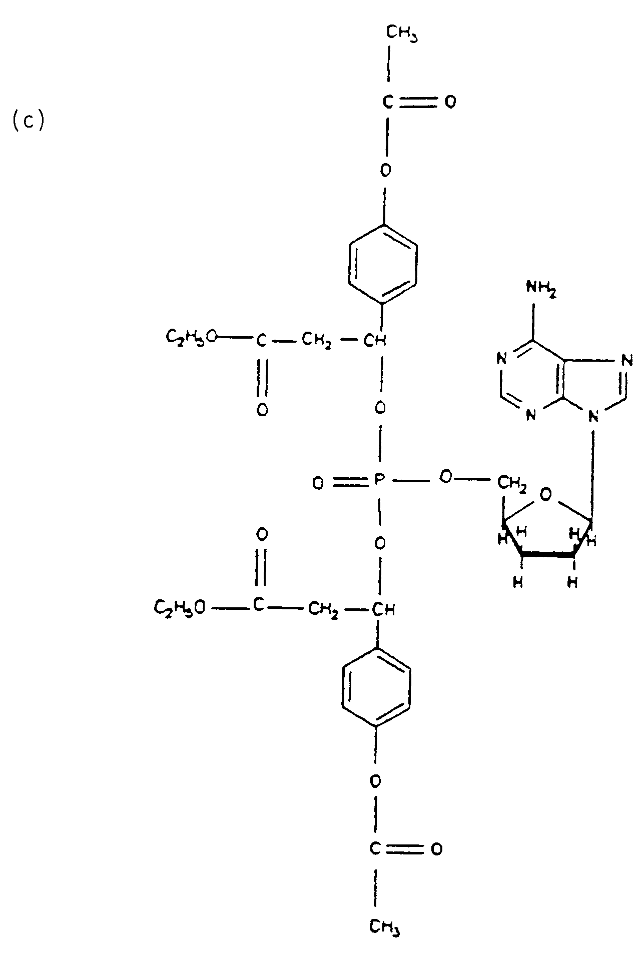

14. A compound for use as an intermediate in prodrug synthesis of the following structure: 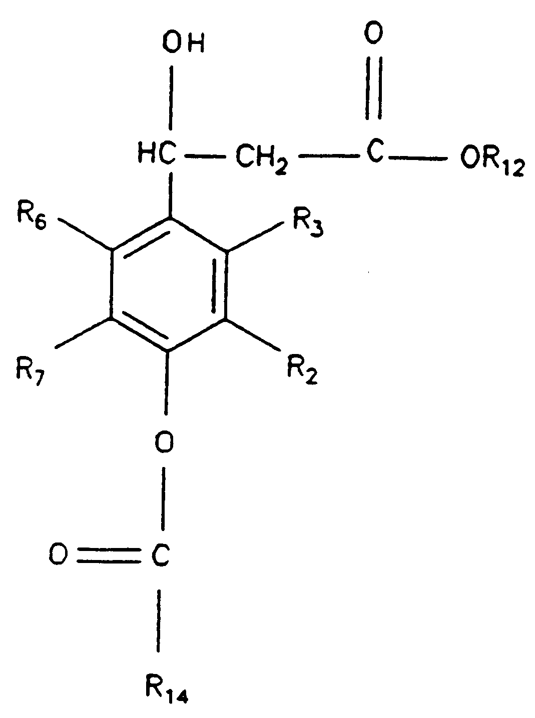 Wherein R2 and R7 can be hydrogen, a ( -CO₂R10 ) group , a methyl group , a halogen, or a methoxy group , or a hydroxymethyl group (HO-CH₂-); wherein R10 may be a methyl or ethyl group;
Wherein R3 and R6 can be hydrogen; a methyl group; a hydroxymethyl group (-CH₂-OH ); a halogen; a hydroxyethyl group (-CH₂-CH₂-OH );
Wherein R12 is a methyl, ethyl, phenyl or benzyl group;
Wherein R14 can be a group of the following structure: (-(CH₂)_{N}-CH₃) for N= 0-18; a phenyl group, wherein the phenyl group may in turn bear substituents, wherein R14 may be selected such that R14-CO₂H is; an amino acid; lactic acid; glycolic acid ( HO-CH₂-CO₂H); glyceric acid ( HO-CH₂-CH(OH)-CO₂H); or acetoacetic acid ( CH₃COCH₂-CO₂H); wherein R14 may be (-O-(CH₂)_{N}-CH₃) for N= 0-18; a phenyloxy-group, wherein the phenyloxy group may in turn bear substituents; and wherein R14 may be a group of the following such as: (-NH₂) ; (-NHCH3 ); or (-N(CH3)₂)

15. A compound of Claim 14 of the following structure: 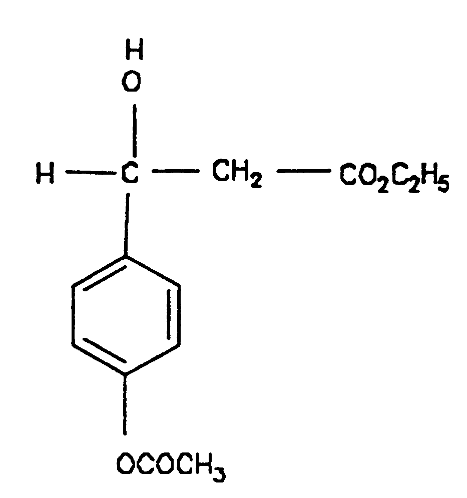 ethyl 3-hydroxy-3-(4-acetoxyphenyl)propionate

16. A processs for preparing a prodrug to facilitate the delivery of phosphorous bearing parent drugs into cells and through biological membranes, comprising the steps of:
a. Selecting a phosphorous bearing parent drug; and
b. Converting one or more of the hydroxy groups on each phosphorous of the parent drug into a group of the structure A to yield a prodrug, wherein the structure A comprises: 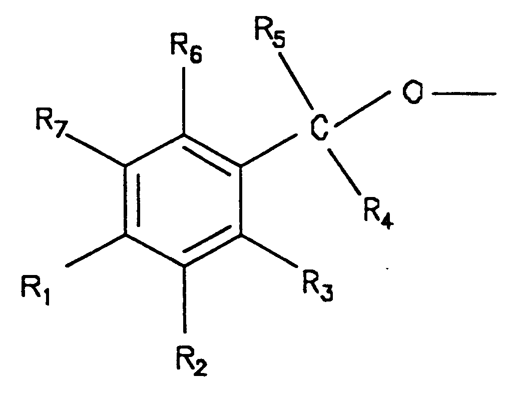 Wherein R1 can be an acyloxy group (-O-CO-R8) ; a (-O-CO₂-R8) group;
a (-O-C(O)-NH₂) group; a (-O-C(O)-NHCH3 ) group; a (-O-C(O)-N(CH3)₂) group; hydrogen; a halogen; a ( -CO₂R9 ) group; an alkyl (e.g. methyl) group; or a hydroxymethyl group (HO-CH₂-);
R2 can be hydrogen; a ( -CO₂R10 ) group; an alkyl (e.g. methyl) group; a halogen; a methoxy group; an acyloxy group (-O-CO-R8); or a hydroxymethyl group (HO-CH₂-);
R3 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; an alkyl (e.g. methyl) group; a hydroxymethyl group (-CH₂-OH ); a halogen; a hydroxyethyl group (-CH₂-CH₂-OH ); or a (-CH₂-CO₂-R11) group ;
R4 can be hydrogen ; an alkyl (e.g. methyl) group; methoxy-methyl (-CH₂-O-CH₃ ), a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group, or a methylene substituted with a functional group which favors an elimination reaction by lowering the intrinsic barrier for removal of a proton from said methylene;
R5 can be hydrogen ; an alkyl (e.g. methyl) group; methoxy-methyl (-CH₂-O-CH₃ ); a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group, or a methylene substituted with a functional group which favors an elimination reaction by lowering the intrinsic barrier for removal of a proton from said methylene;
R6 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; an alkyl (e.g. methyl) group; a hydroxymethyl group (-CH₂-OH ); a halogen; a hydroxyethyl group (-CH₂-CH₂-OH ); or a (-CH₂-CO₂-R11) group ;
R7 can be hydrogen; a ( -CO₂R10 ) group; an alkyl (e.g. methyl) group; a halogen; or a methoxy group; an acyloxy group (-O-CO-R8); or a hydroxymethyl group (HO-CH₂-)
Wherein R8 can be a group of the following stucture: (-(CH₂)_{N}-CH₃) for N= 0-18; R8 may be a phenyl group, wherein the phenyl group may in turn bear substituents, wherein R8 may also be selected such such that R8-CO₂H is; an amino acid; lactic acid; glycolic acid
( HO-CH₂-CO₂H); glyceric acid ( HO-CH₂-CH(OH)-CO₂H); or acetoacetic acid ( CH₃COCH₂-CO₂H);
Wherein R9, and R10, R11, and R12, is a methyl, ethyl, phenyl, or benzyl group;
and
Wherein at least one of the following groups: R1; R3 ; R6 is an acyloxy group ( -O-CO-R8 ) or another group which undergoes biotransformation in vivo to ultimately yield a hydroxy group on the phenyl ring

17. A process of Claim 16, Wherein the parent drug is (a) a monophosphate ester drug; or
(b) a phosphodiester drug; or
(c) a phosphonic acid drug; or
(d) a phosphonate drug; or
(e) a phosphinic acid drug; or
(f) a nucleotide monophosphate drug; or
(g) is selected from the the group consisting of:
9-(2-phosphonylmethoxy-ethyl)adenine , (PMEA); 9-(RS)-(3-fluoro-2-phosphonylmethoxypropyly)Adenine (FPMPA), 9-(RS)-(3-fluoro-2-phosphonylmethoxypropyly)2,6,diaminopurine 3'azido-dideoxythymidine 5'monophosphate (AZT-phosphate);
2',3'-didehydro-2',3'dideoxythymidine 5' monophosphate (D4T-phosphate);
2'3'dideoxyadenosine 5'monophosphate;
a carboxylate ester of phosphonoformate

18. A process of claim 16 or claim 17, wherein (a) the structure of group "A" is selected from the group consisting of: 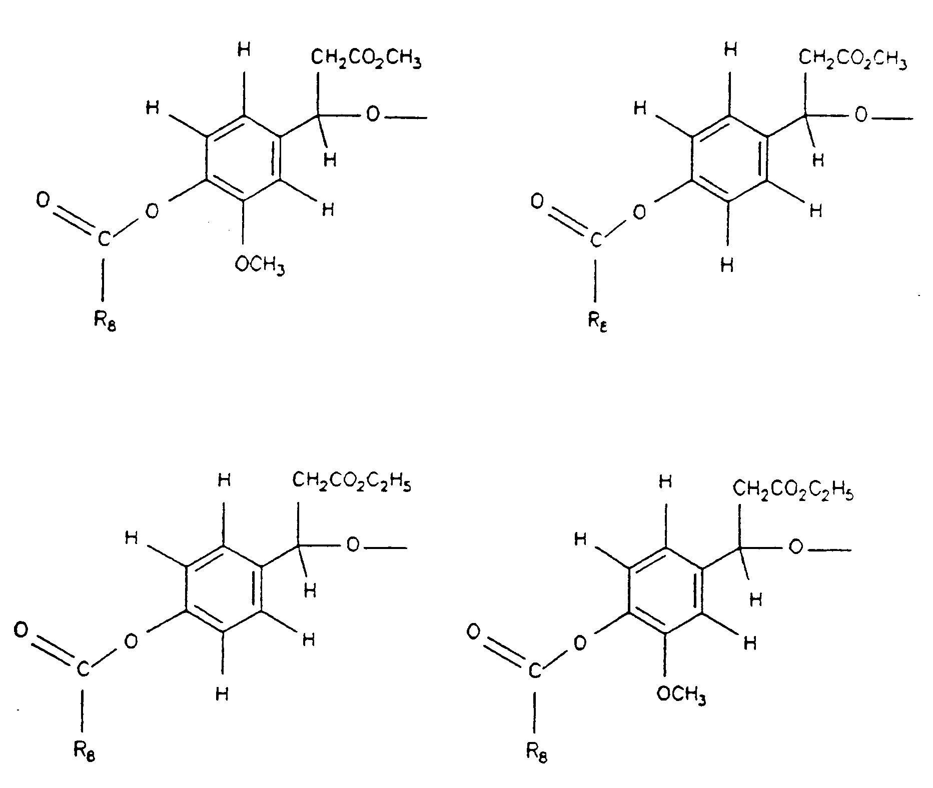 Wherein R8 can be a group of the following structure: (-(CH₂)_{N}-CH₃) for N= 0-18; R8 may be a phenyl group, wherein the phenyl group may in turn bear substituents, wherein R8 may also be selected such such that R8-CO₂H is; an amino acid; lactic acid; glycolic acid ( HO-CH₂-CO₂H); glyceric acid ( HO-CH₂-CH(OH)-CO₂H); or acetoacetic acid ( CH₃COCH₂-CO₂H); or Wherein R8 is any group selected such that the resulting prodrug undergoes biotransformation or spontaneous transformation in vivo to ultimately yield a hydroxy group on the phenyl ring; or (b) the group "A" has the following structure:

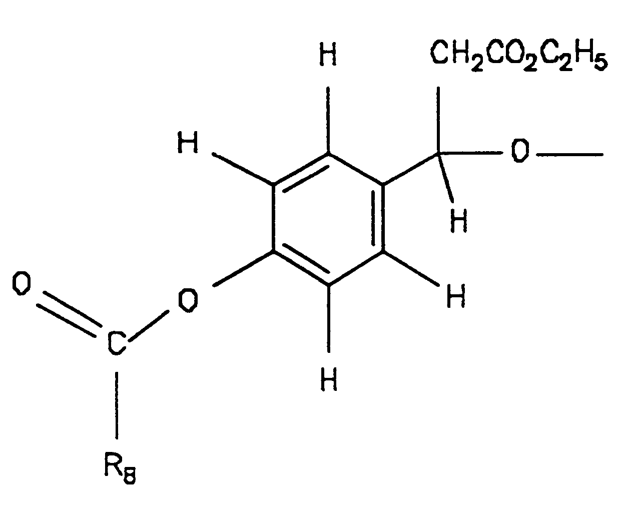

19. A process of Claim 16 for preparing a prodrug to facilitate the delivery of phosphonoformate into cells and through biological membranes, comprising preparing a prodrug of the following structure: 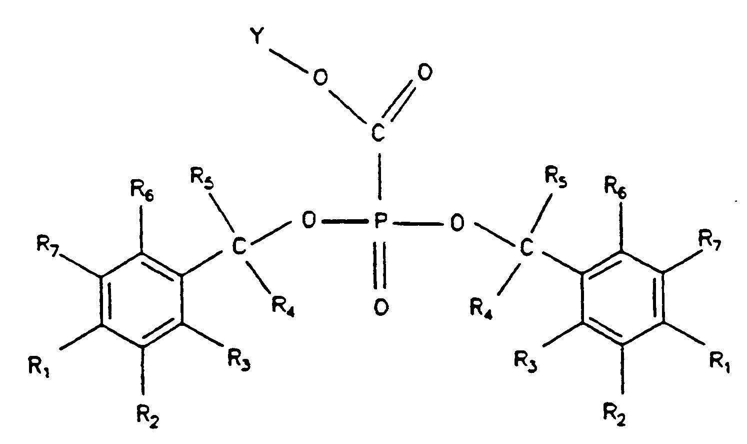 wherein Y is a benzyl, phenyl , wherein the benzyl or phenyl group may in turn bear substituents

20. A process of Claim 19 which comprises preparing a prodrug of the structure:

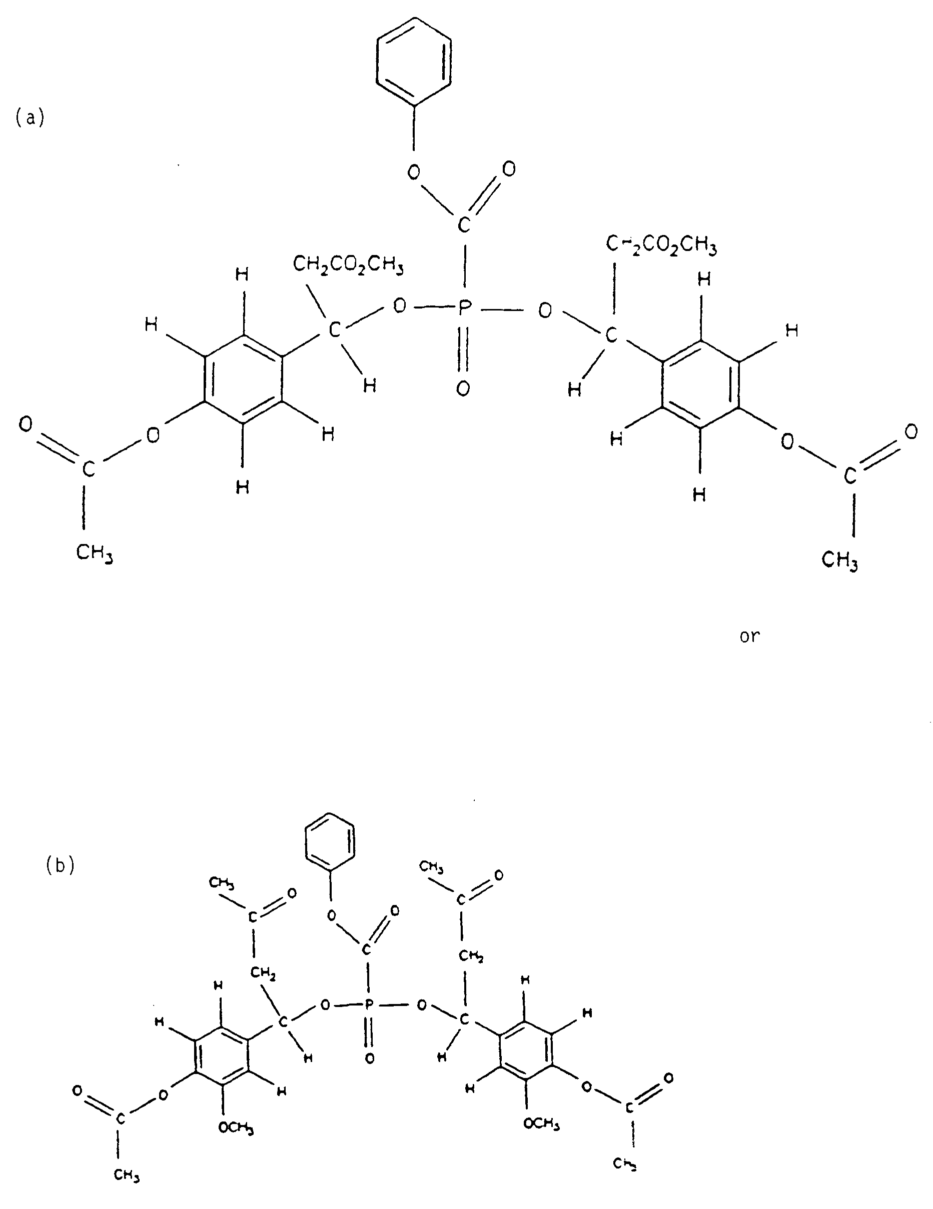

21. A process of Claim 16 wherein the phosphorous bearing drug and structure A are selected such that the resulting prodrug is:

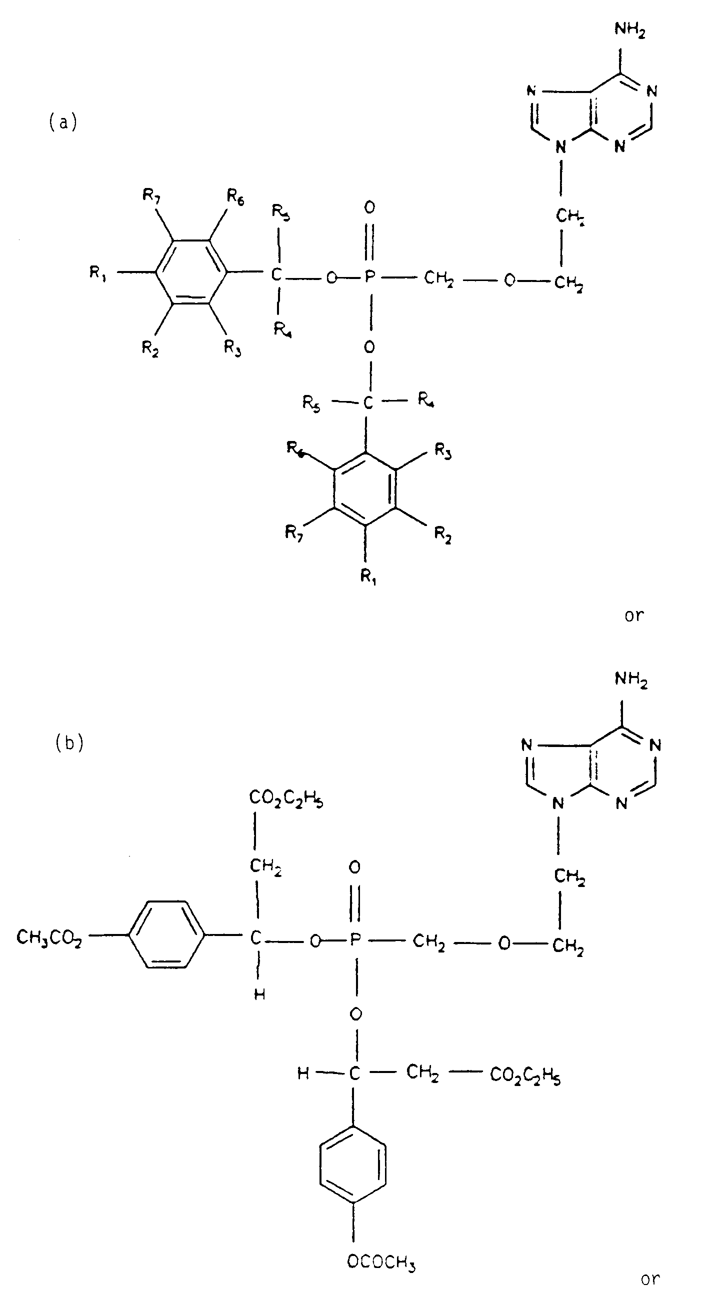

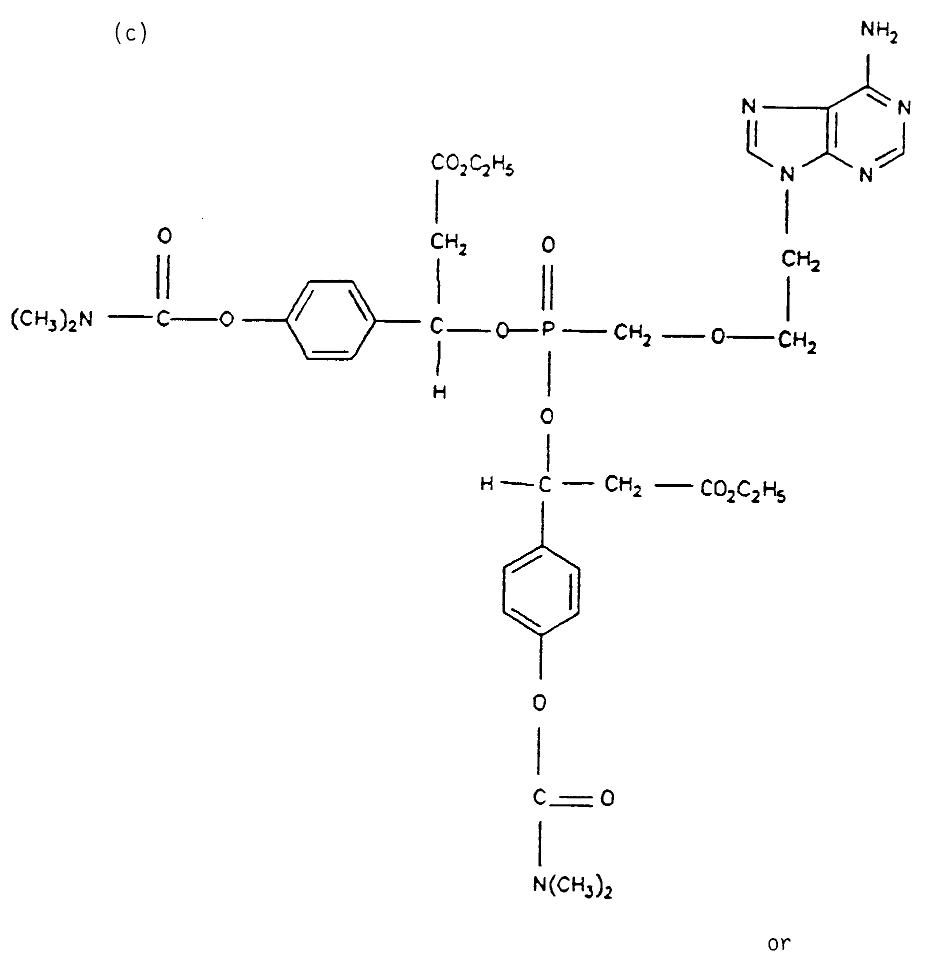

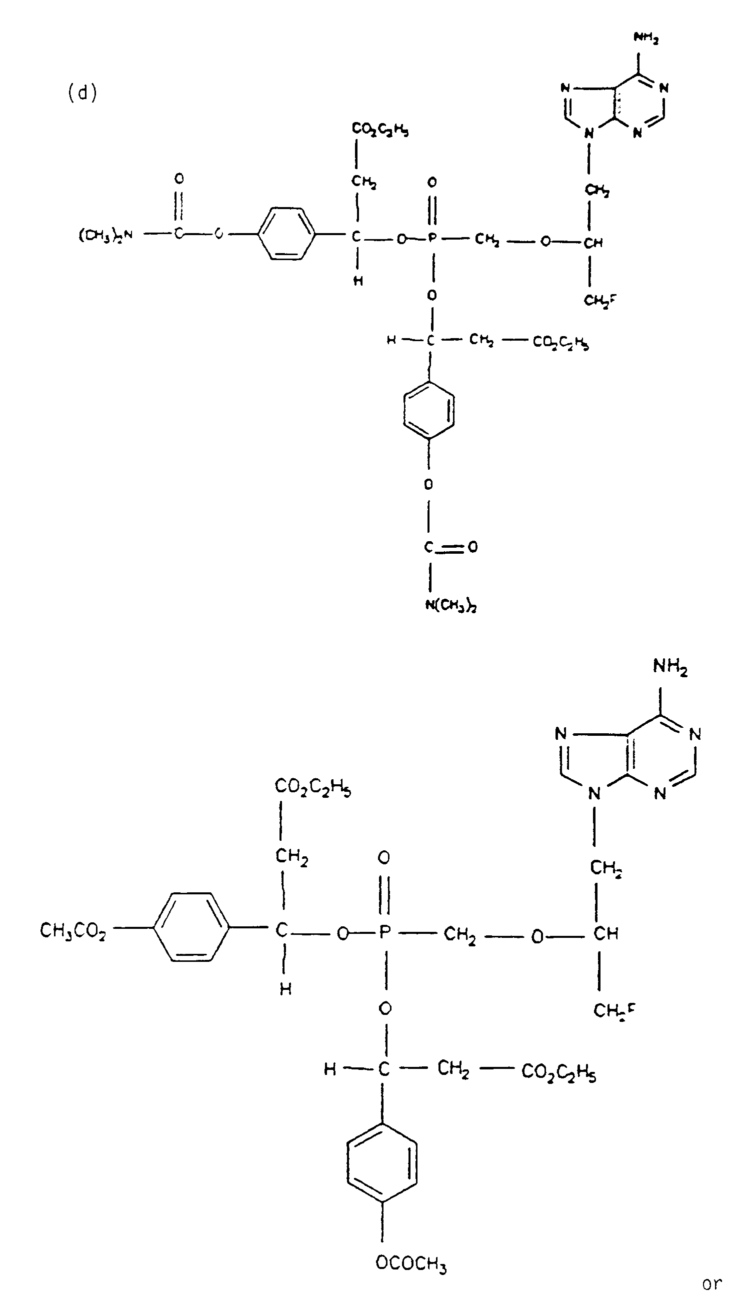

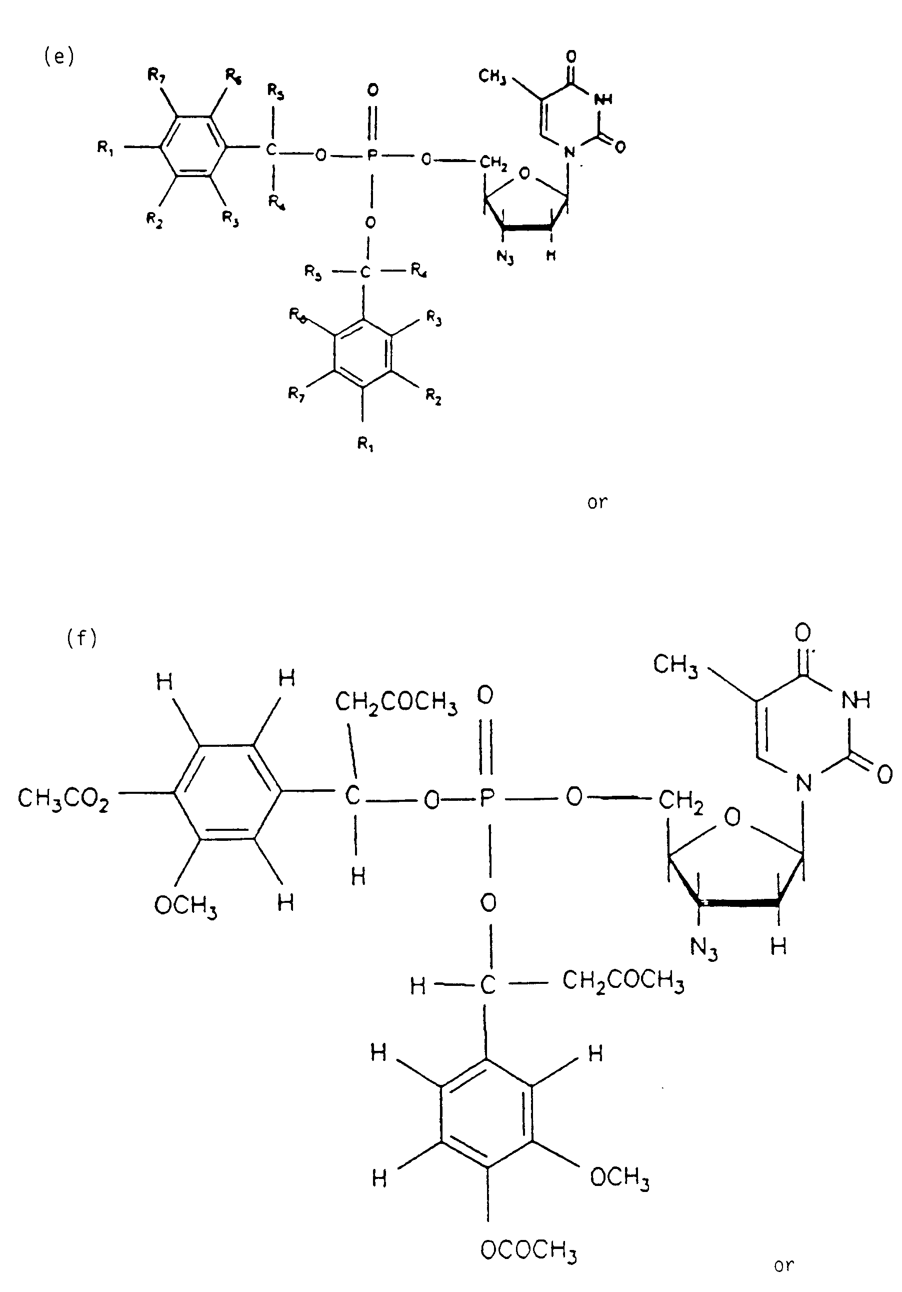

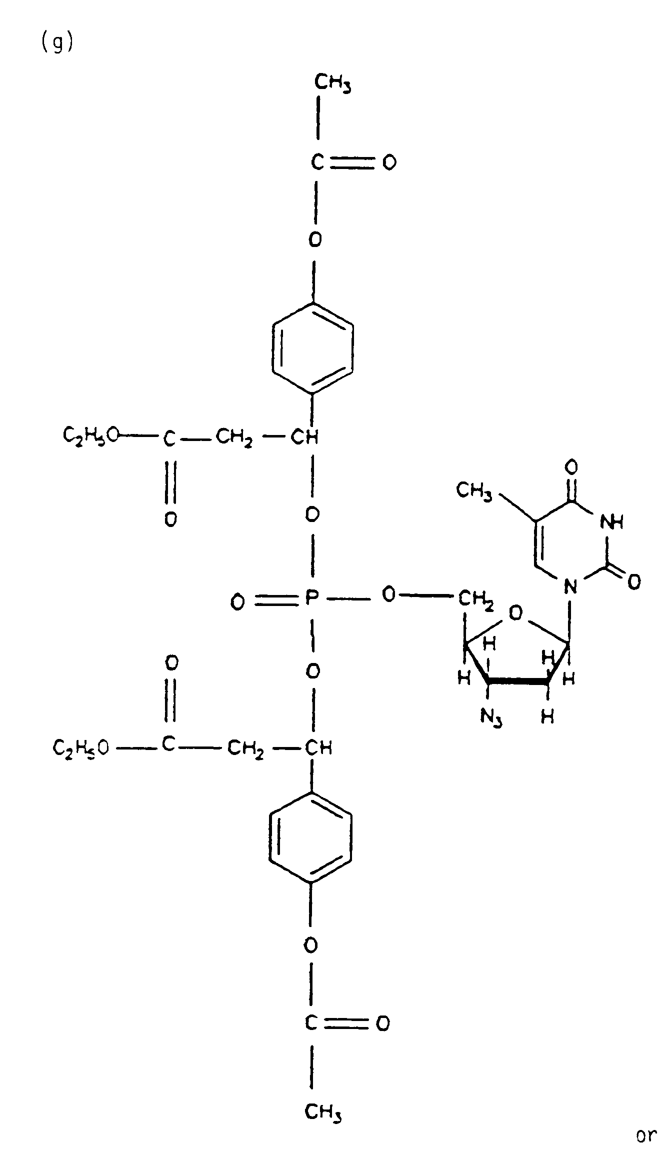

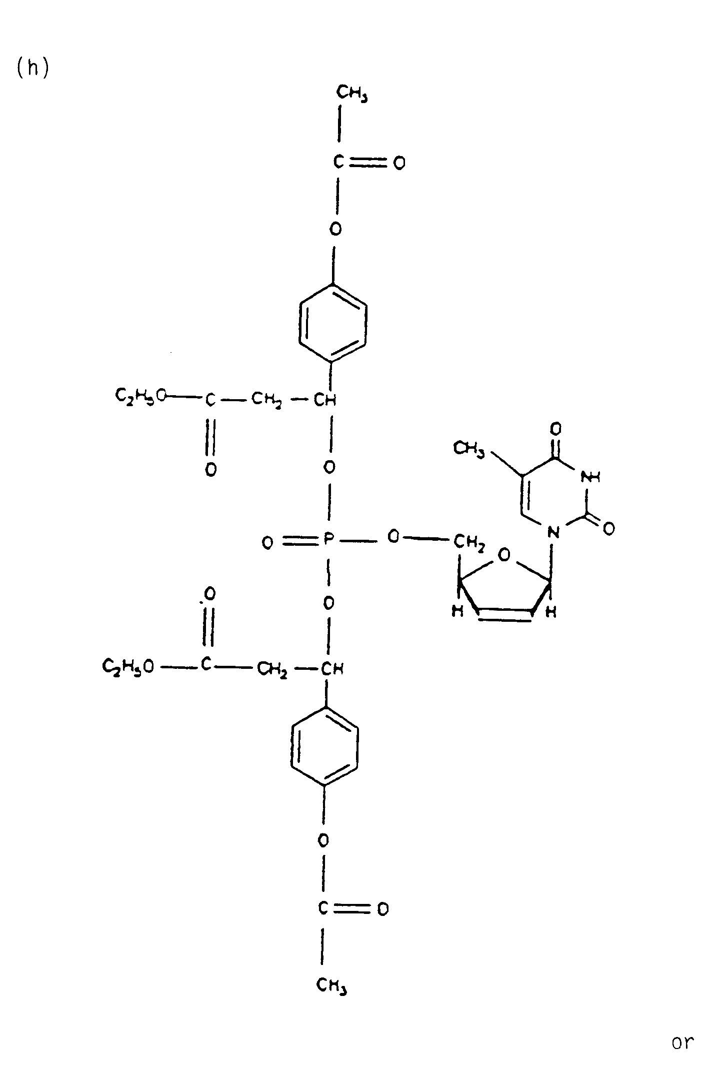

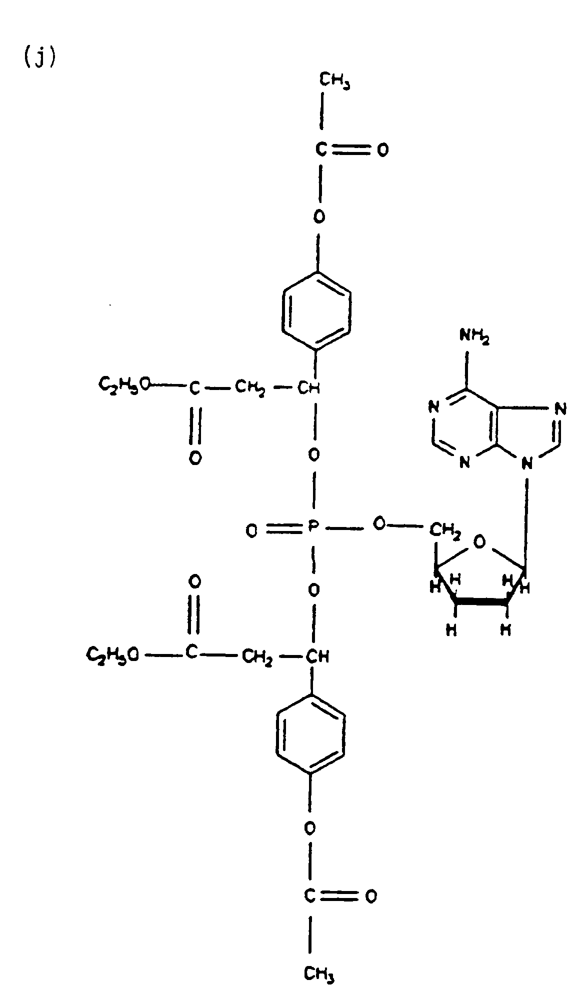

22. A process of preparing a prodrug to facilitate the delivery of phosphorous bearing parent drugs into cells and through biological membranes comprising the steps of:
a. Selecting a phosphorous bearing drug; and
b. Preparing a mono or bis phospho-ester from a suitably activated derivative of the parent drug and an alcohol of Structure A to yield a prodrug, wherein the Structure A comprises: 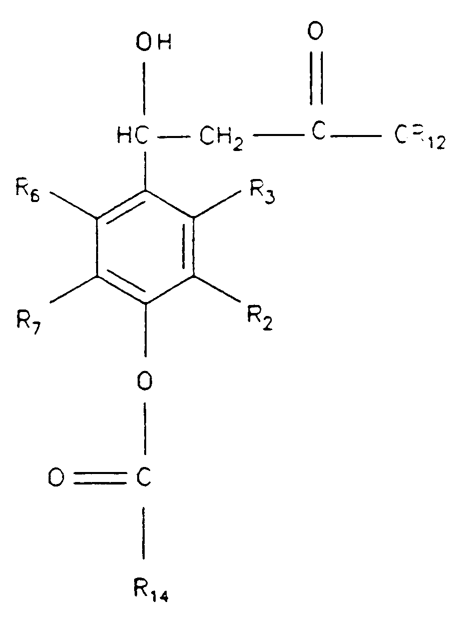 Wherein R2 and R7 can be hydrogen, a ( -CO₂R10 ) group , a methyl group , a halogen, or a methoxy group , or a hydroxymethyl group (HO-CH₂-); wherein R10 may be a methyl or ethyl group;
Wherein R3 and R6 can be hydrogen; a methyl group; a hydroxymethyl group (-CH₂-OH ); a halogen; a hydroxyethyl group (-CH₂-CH₂-OH );
Wherein R12 is a methyl, ethyl, phenyl or benzyl group;
Wherein R14 can be a group of the following structure: (-(CH₂)_{N}-CH₃) for N= 0-18; a phenyl group, wherein the phenyl group may in turn bear substituents, wherein R14 may be selected such that R14-CO₂H is; an amino acid; lactic acid; glycolic acid ( HO-CH₂-CO₂H); glyceric acid ( HO-CH₂-CH(OH)-CO₂H); or acetoacetic acid ( CH₃COCH₂-CO₂H); wherein R14 may be (-O-(CH₂)_{N}-CH₃) for N= 0-18; a phenyloxy- group, wherein the phenyloxy group may in turn bear substituents; and wherein R14 may be a group of the following such as: (-NH₂) ;(-NHCH3 ); or (-N(CH3)₂)

23. A process of Claim 22 wherein the Structure A is: 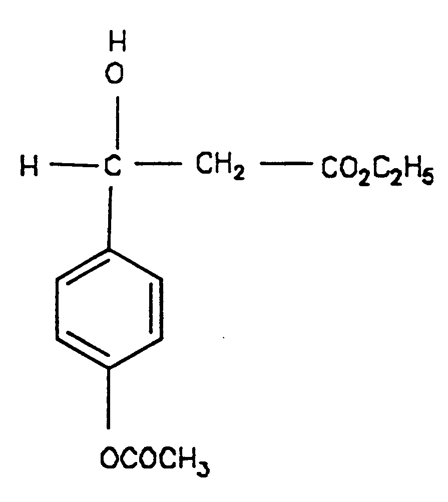 ethyl 3-hydroxy-3-(4-acetoxyphenyl)propionate

24. A prodrug prepared by a process according to any one of claims 16 to 23 for use in therapy in facilitating the delivery of phosphorous bearing drugs into cells and through biological membranes by contacting the cells and biological membranes with the prodrug.

25. Use of a prodrug prepared by a process according to any one of claims 16 to 23 for the manufacture of a medicament to facilitate the delivery of phosphorous bearing drugs into cells and through biological membranes by contacting the cells and biological membranes with the prodrug.

26. A prodrug compound for a parent drug, wherein the parent drug has the general structure: 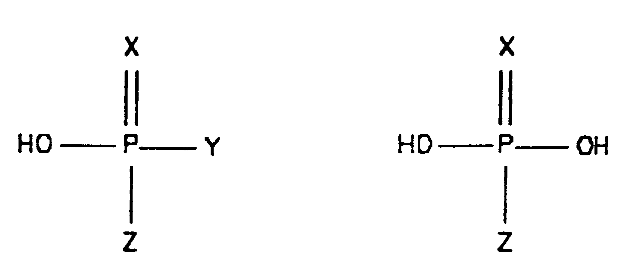 wherein X is oxygen (O), or sulphur (S), and Y and Z are defined by the remainder of the parent drug; and wherein the parent drug is not phosphoric acid; wherein the prodrug compound has the following structure: 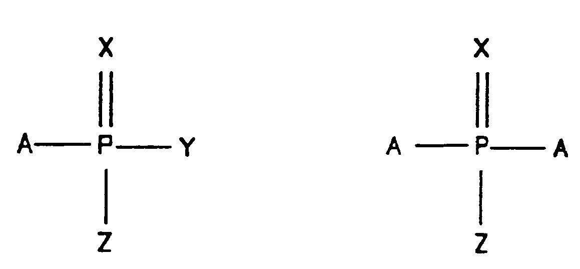 wherein the group "A" is a benzyl-oxy- derivative with one or more acyloxy groups in para or ortho positions relative to the phosphoester, and wherein the parent drug is liberated following conversion of the acyloxy group by esterase into the corresponding hydroxy group

27. A processs for preparing a prodrug to facilitate the delivery of phosphorous bearing parent drugs into cells and through biological membranes, comprising the steps of:
a. Selecting a phosphorous bearing parent drug; and
b. Converting one or more of the hydroxy groups on each phosphorous of the parent drug into a group of the structure A to yield a prodrug, wherein the group "A" is a benzyl-oxy- derivative with one or more acyloxy groups in para or ortho positions relative to the phosphoester,and wherein the parent drug is liberated following conversion of the acyloxy group by esterase into the corresponding hydroxy group

28. Use of a prodrug compound for the manufacture of a phosphate or phosphonate bearing parent drug which is a mono or diester of the phosphate or phosphonate for use in therapy, in which therapy the prodrug is degraded to the parent phosphorous bearing drug by an elimination reaction to form a carbon-carbon double bond; and wherein the elimination reaction is triggered by the spontaneous or enzymatic unmasking of a group which is known to be strongly electron donating, e.g. a hydroxy group.

29. A prodrug prepared by a process according to claim 27 for use in therapy in facilitating the delivery of phosphorous bearing drugs into cells and through biological membranes by contacting the cells with the prodrug.

30. Use of a prodrug prepared by a process according to claim 27 for the manufacture of a medicament to facilitate the delivery of phosphorous bearing drugs into cells and through biological membranes by contacting the cells with the prodrug.

## Claims (Claims for the following Contracting State(s): ES, GR)

1. A processs for preparing a prodrug to facilitate the delivery of phosphorous bearing parent drugs into cells and through biological membranes, comprising the steps of:
a. Selecting a phosphorous bearing parent drug; and
b. Converting one or more of the hydroxy groups on each phosphorous of the parent drug into a group of the structure A to yield a prodrug, wherein the structure A comprises: 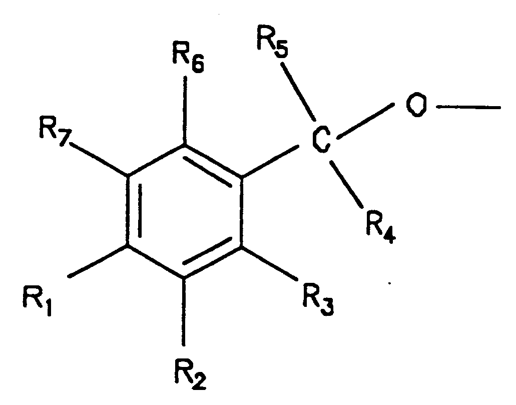 Wherein R1 can be an acyloxy group (-O-CO-R8) ; a (-O-CO₂-R8) group;
a (-O-C(O)-NH₂) group; a (-O-C(O)-NHCH3 ) group; a (-O-C(O)-N(CH3)₂) group; hydrogen; a halogen; a ( -CO₂R9 ) group; an alkyl (e.g. methyl) group; or a hydroxymethyl group (HO-CH₂-);
R2 can be hydrogen; a ( -CO₂R10 ) group; an alkyl (e.g. methyl) group; a halogen; a methoxy group; an acyloxy group (-O-CO-R8); or a hydroxymethyl group (HO-CH₂-);
R3 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; an alkyl (e.g. methyl) group; a hydroxymethyl group (-CH₂-OH ); a halogen; a hydroxyethyl group (-CH₂-CH₂-OH ); or a (-CH₂-CO₂-R11) group ;
R4 can be hydrogen ; an alkyl (e.g. methyl) group; methoxy-methyl (-CH₂-O-CH₃ ), a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group, or a methylene substituted with a functional group which favors an elimination reaction by lowering the intrinsic barrier for removal of a proton from said methylene;
R5 can be hydrogen ; an alkyl (e.g. methyl) group; methoxy-methyl (-CH₂-O-CH₃ ); a (-CH₂-CO₂-R12 ) group; a (-CH₂-CO-CH₃ ) group; a (-CH(CH3)-CO₂-R12 ) group; a (-CH₂-CO-NH₂) group; a (-CH₂-NO₂ ) group; or a (- CH₂-SO₂-CH₃) group, or a methylene substituted with a functional group which favors an elimination reaction by lowering the intrinsic barrier for removal of a proton from said methylene;
R6 can be an acyloxy group ( -O-CO-R8 ); a (-O-CO₂-R8) group; a (-O-C(O)-NH₂) group; a (-O-C(O)-NHCH3 ) group; a (-O-C(O)-N(CH3)₂) group; hydrogen; an alkyl (e.g. methyl) group; a hydroxymethyl group (-CH₂-OH ); a halogen; a hydroxyethyl group (-CH₂-CH₂-OH ); or a (-CH₂-CO₂-R11) group ;
R7 can be hydrogen; a ( -CO₂R10 ) group; an alkyl (e.g. methyl) group; a halogen; or a methoxy group; an acyloxy group (-O-CO-R8); or a hydroxymethyl group (HO-CH₂-)
Wherein R8 can be a group of the following structure: (-(CH₂)_{N}-CH₃) for N= 0-18; R8 may be a phenyl group, wherein the phenyl group may in turn bear substituents, wherein R8 may also be selected such such that R8-CO₂H is; an amino acid; lactic acid; glycolic acid
( HO-CH₂-CO₂H); glyceric acid ( HO-CH₂-CH(OH)-CO₂H); or acetoacetic acid ( CH₃COCH₂-CO₂H);
Wherein R9, and R10, R11, and R12, is a methyl, ethyl, phenyl, or benzyl group;
and
Wherein at least one of the following groups: R1; R3 ; R6 is an acyloxy group ( -O-CO-R8 ) or another group which undergoes biotransformation in vivo to ultimately yield a hydroxy group on the phenyl ring

2. A process of Claim 1, Wherein the parent drug is (a) a monophosphate ester drug; or
(b) a phosphodiester drug; or
(c) a phosphonic acid drug; or
(d) a phosphonate drug; or
(e) a phosphinic acid drug; or
(f) a nucleotide monophosphate drug; or
(g) is selected from the the group consisting of:
9-(2-phosphonylmethoxy-ethyl)adenine , (PMEA); 9-(RS)-(3-fluoro-2-phosphonylmethoxypropyly)Adenine (FPMPA), 9-(RS)-(3-fluoro-2-phosphonylmethoxypropyly)2,6,diaminopurine 3'azido-dideoxythymidine 5'monophosphate (AZT-phosphate);
2',3'-didehydro-2',3'dideoxythymidine 5' monophosphate (D4T-phosphate);
2'3'dideoxyadenosine 5'monophosphate;
a carboxylate ester of phosphonoformate

3. A process of claim 1 or claim 2, wherein (a) the structure of group "A" is selected from the group consisting of: 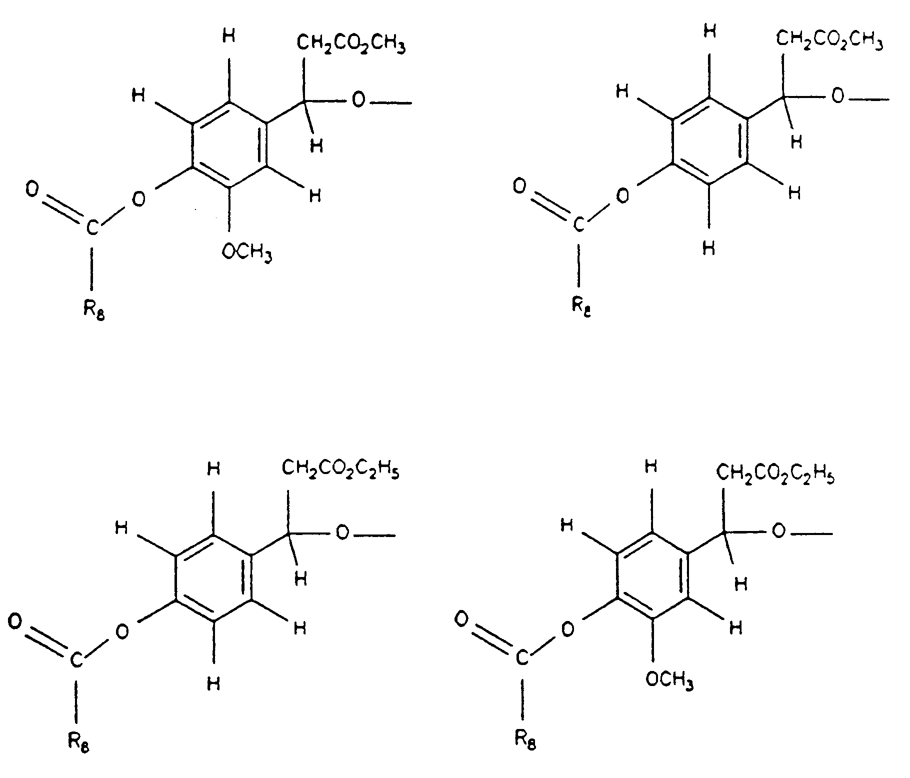 Wherein R8 can be a group of the following structure: (-(CH₂)_{N}-CH₃) for N= 0-18; R8 may be a phenyl group, wherein the phenyl group may in turn bear substituents, wherein R8 may also be selected such such that R8-CO₂H is; an amino acid; lactic acid; glycolic acid ( HO-CH₂-CO₂H); glyceric acid ( HO-CH₂-CH(OH)-CO₂H); or acetoacetic acid ( CH₃COCH₂-CO₂H); or Wherein R8 is any group selected such that the resulting prodrug undergoes biotransformation or spontaneous transformation in vivo to ultimately yield a hydroxy group on the phenyl ring; or (b) the group "A" has the following structure:

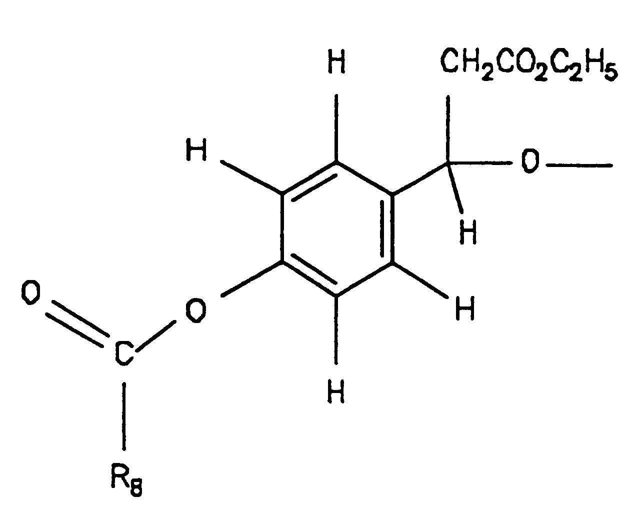

4. A process of Claim 1 for preparing a prodrug to facilitate the delivery of phosphonoformate into cells and through biological membranes, comprising preparing a prodrug of the following structure: 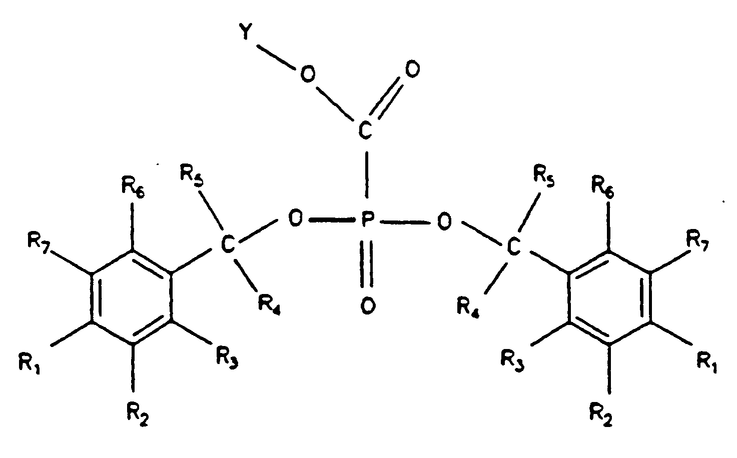 wherein Y is a benzyl, phenyl , wherein the benzyl or phenyl group may in turn bear substituents

5. A process of Claim 4 which comprises preparing a prodrug of the structure:

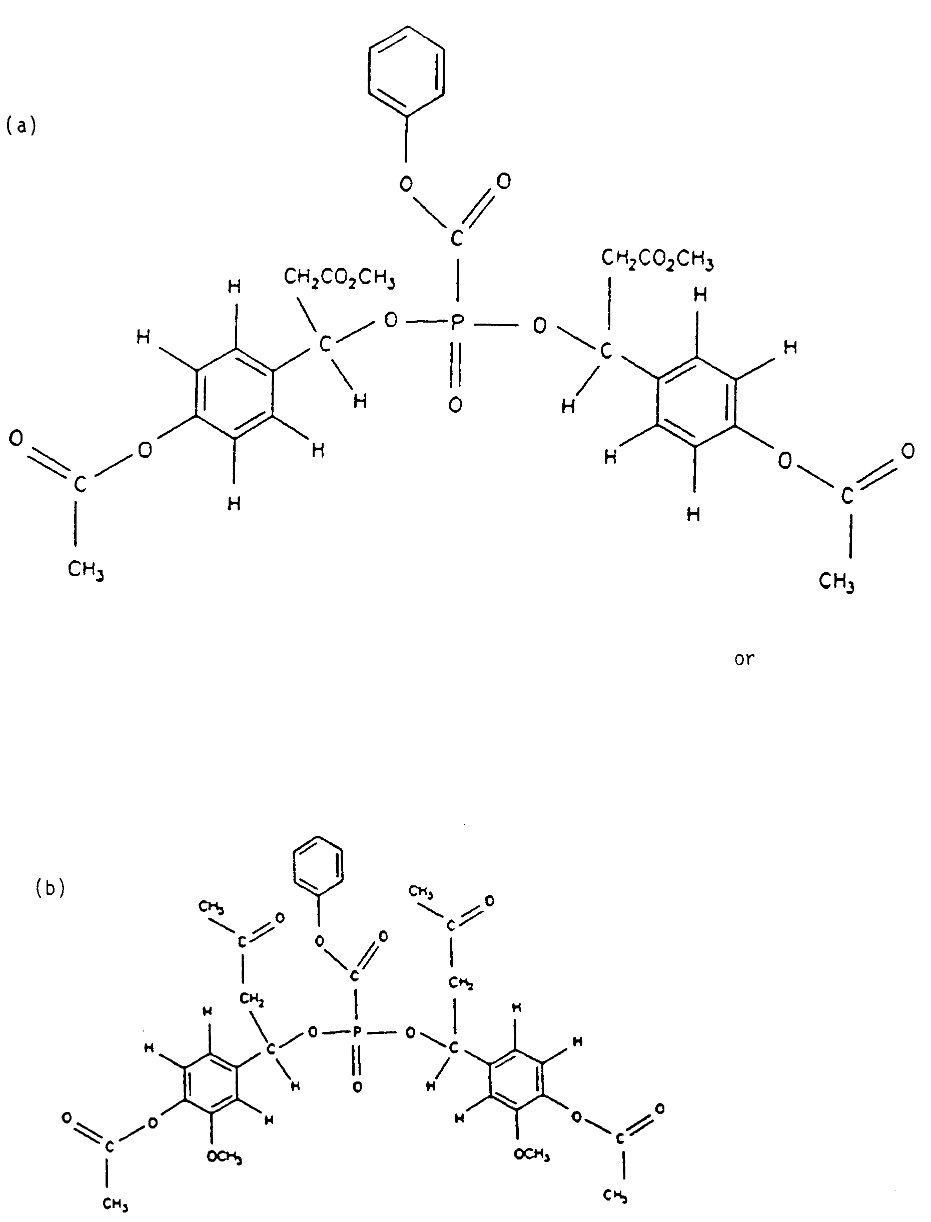

6. A process of Claim 1 wherein the phosphorous bearing drug and structure A are selected such that the resulting prodrug is:

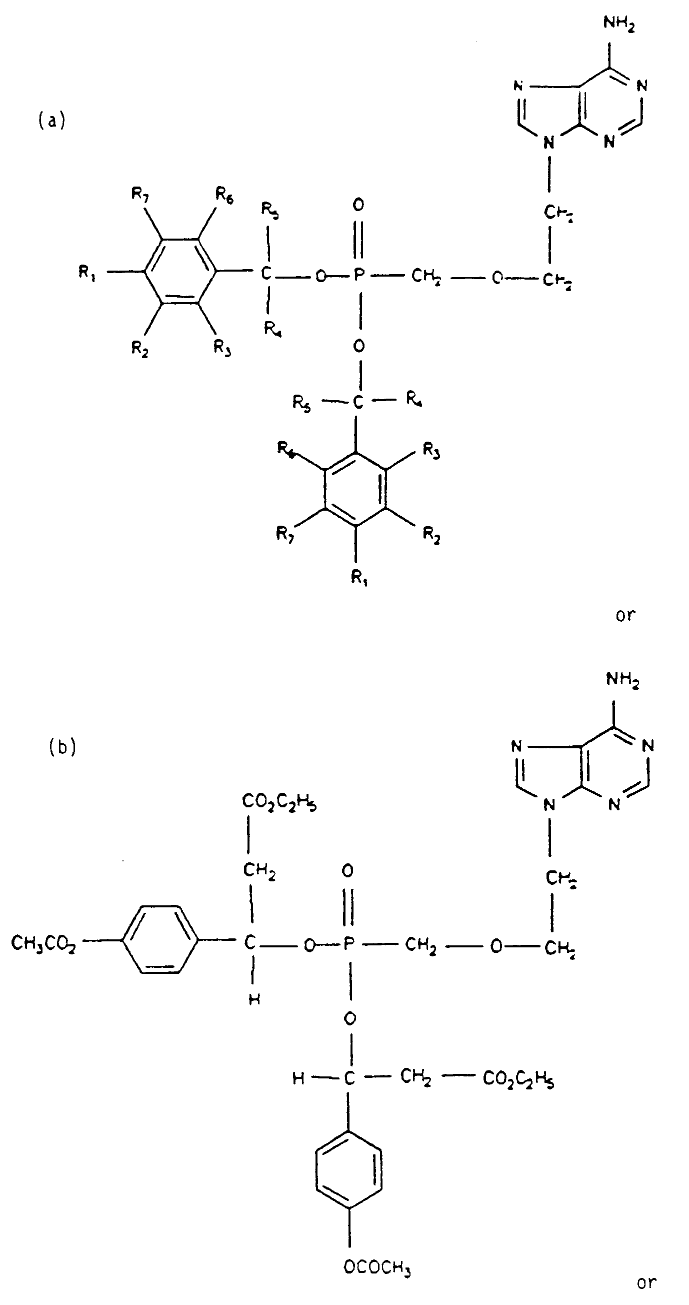

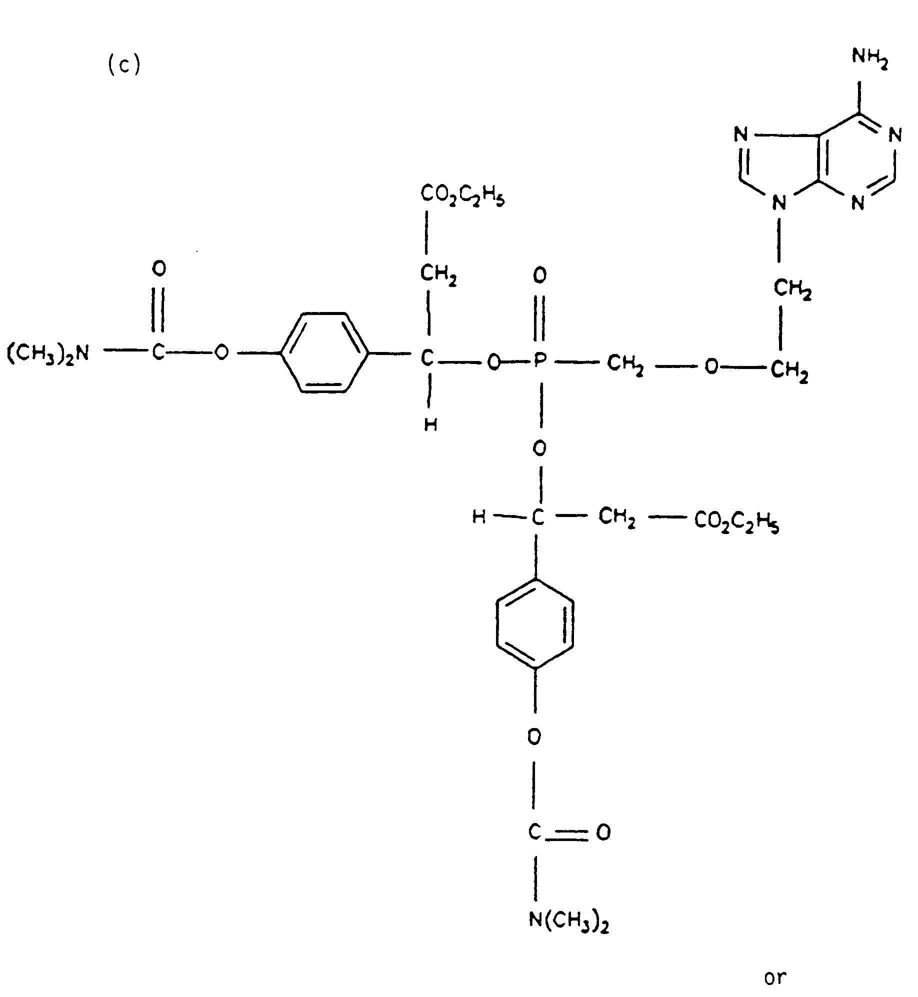

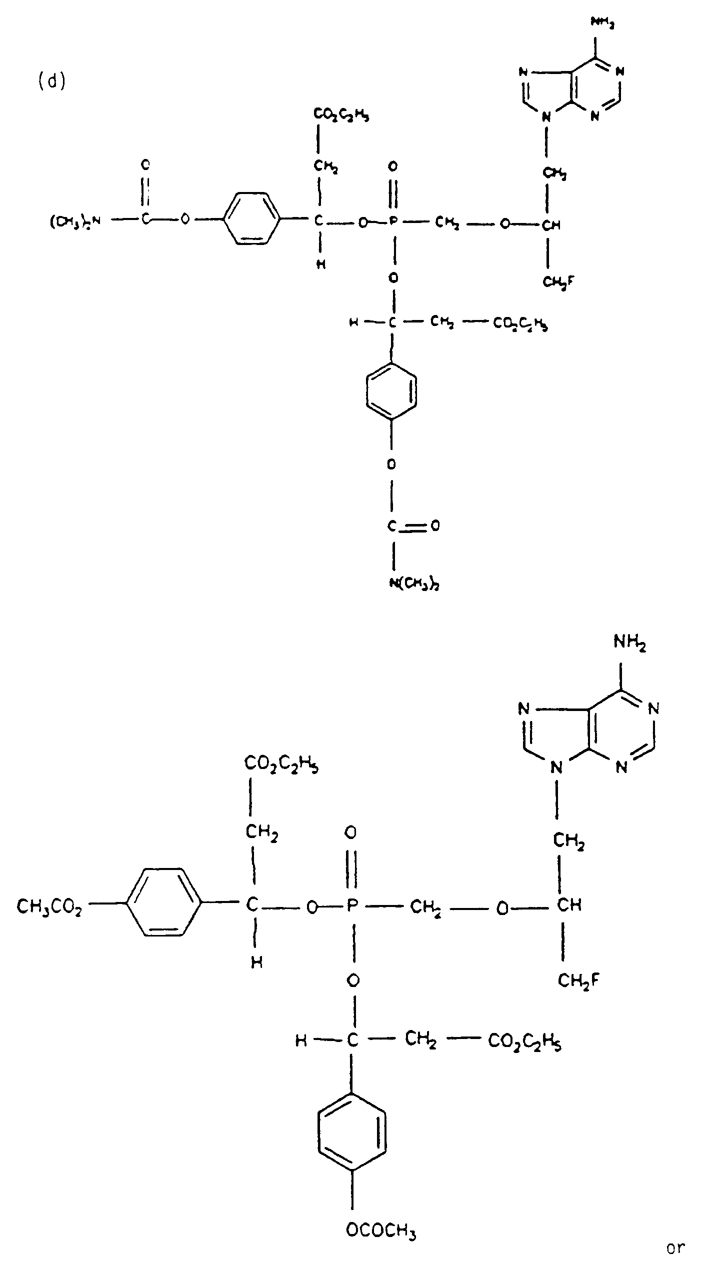

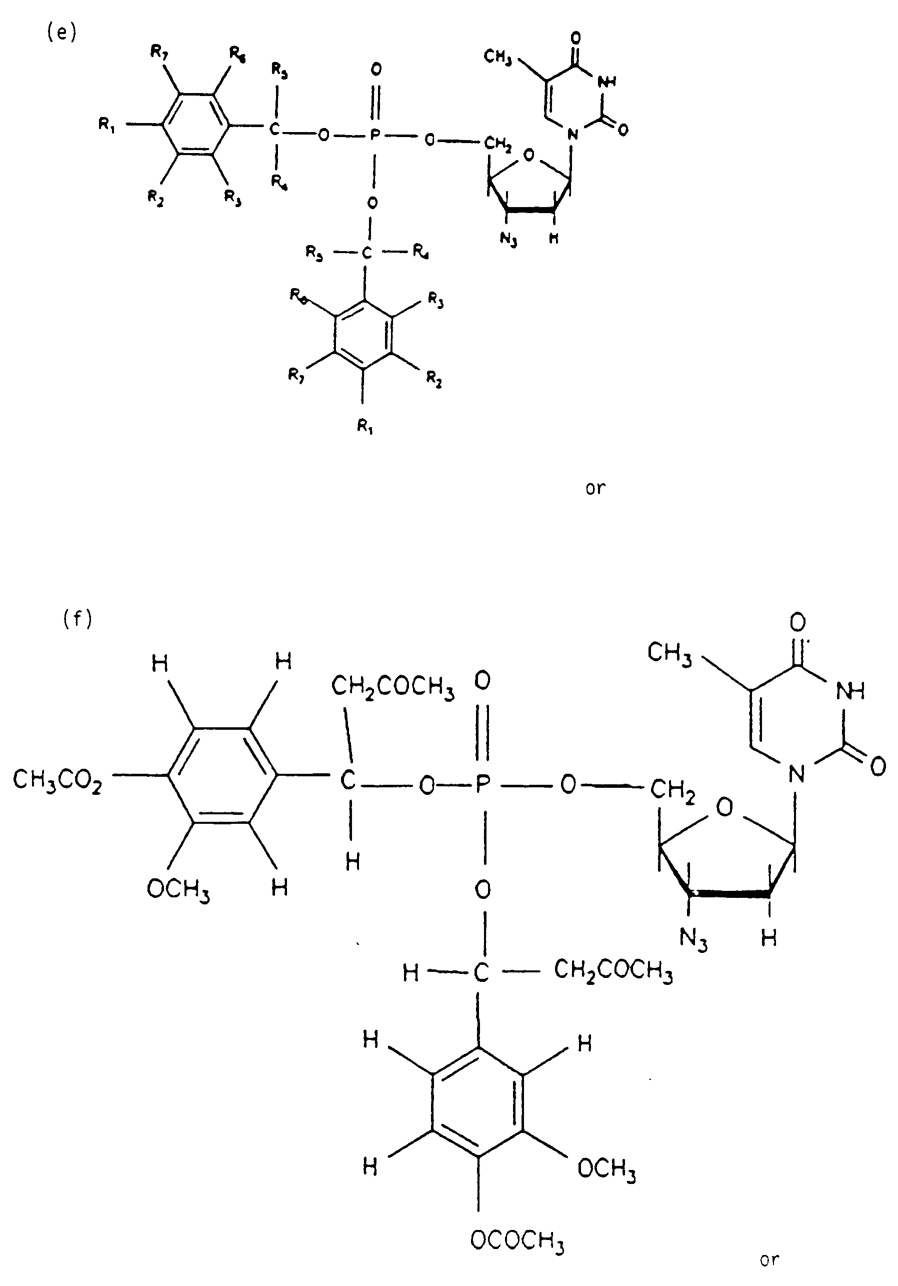

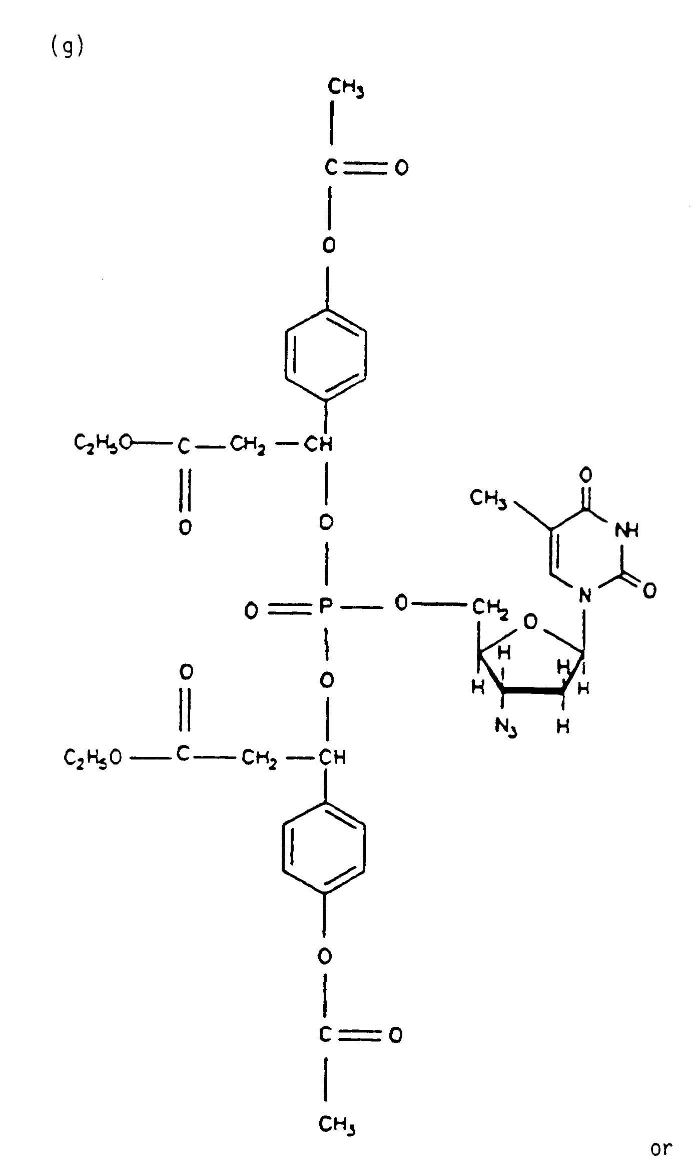

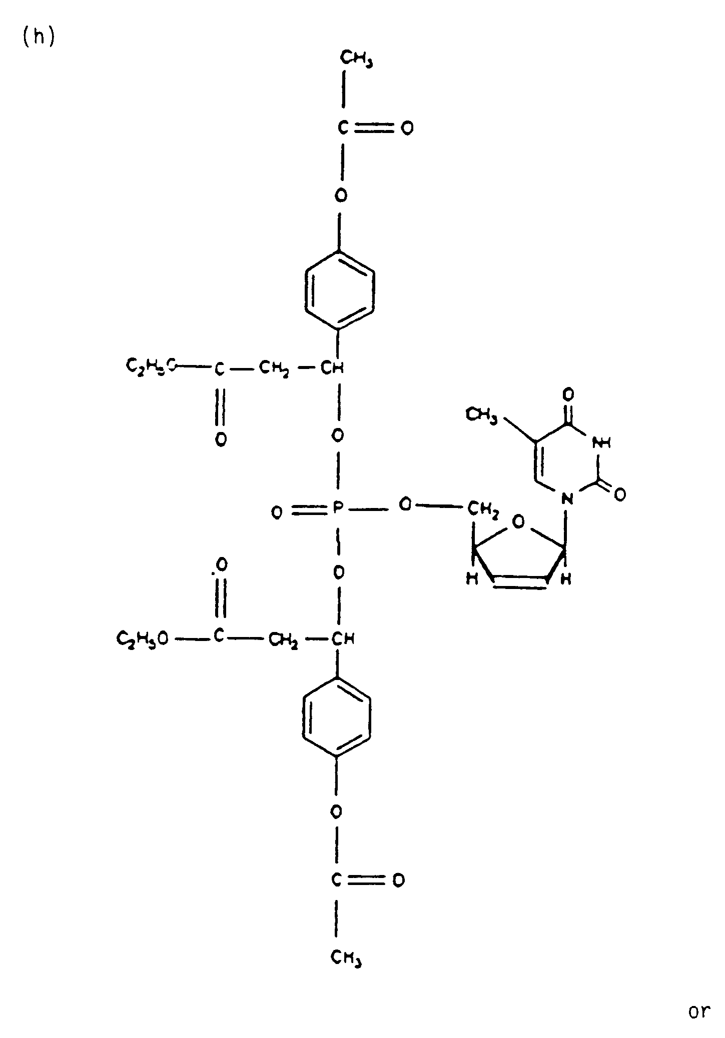

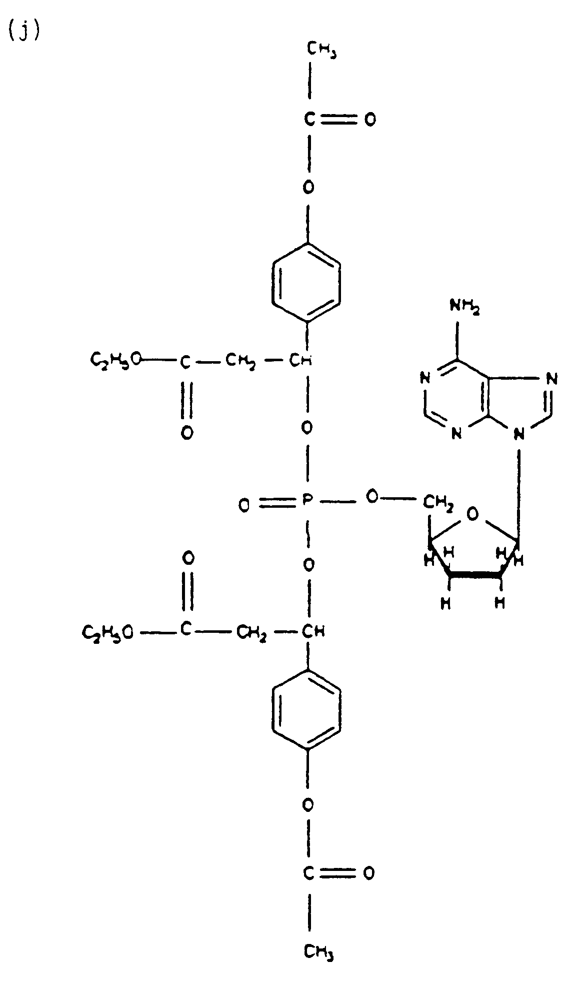

7. A process of preparing a prodrug to facilitate the delivery of phosphorous bearing parent drugs into cells and through biological membranes comprising the steps of:
a. Selecting a phosphorous bearing drug; and
b. Preparing a mono or bis phospho-ester from a suitably activated derivative of the parent drug and an alcohol of Structure A to yield a prodrug, wherein the Structure A comprises: 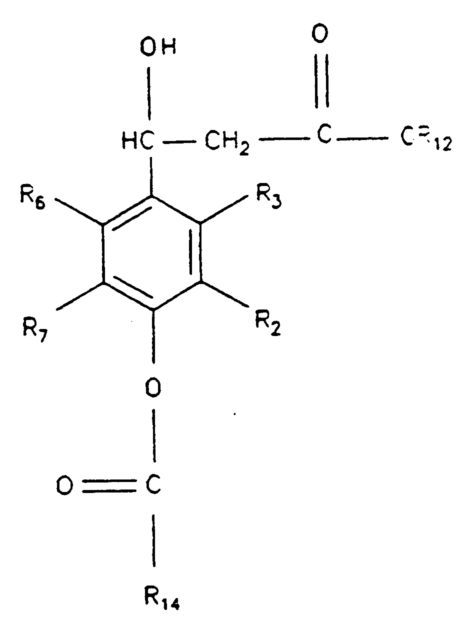 Wherein R2 and R7 can be hydrogen, a ( -CO₂R10 ) group , a methyl group , a halogen, or a methoxy group , or a hydroxymethyl group (HO-CH₂-); wherein R10 may be a methyl or ethyl group;
Wherein R3 and R6 can be hydrogen; a methyl group; a hydroxymethyl group (-CH₂-OH ); a halogen; a hydroxyethyl group (-CH₂-CH₂-OH );
Wherein R12 is a methyl, ethyl, phenyl or benzyl group;
Wherein R14 can be a group of the following structure: (-(CH₂)_{N}-CH₃) for N= 0-18; a phenyl group, wherein the phenyl group may in turn bear substituents, wherein R14 may be selected such that R14-CO₂H is; an amino acid; lactic acid; glycolic acid ( HO-CH₂-CO₂H); glyceric acid ( HO-CH₂-CH(OH)-CO₂H); or acetoacetic acid ( CH₃COCH₂-CO₂H); wherein R14 may be (-O-(CH₂)_{N}-CH₃) for N= 0-18; a phenyloxy- group, wherein the phenyloxy group may in turn bear substituents; and wherein R14 may be a group of the following such as: (-NH₂) ;(-NHCH3 ); or (-N(CH3)₂)

8. A process of Claim 7 wherein the Structure A is: 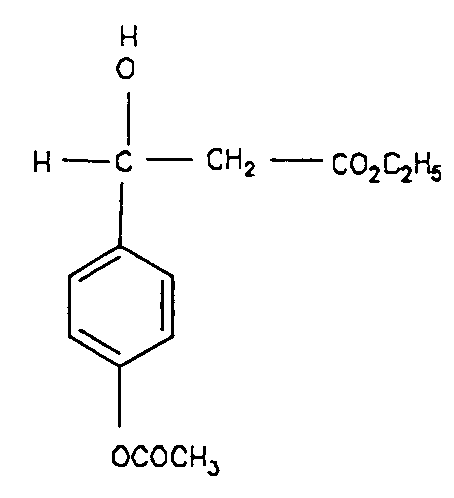 ethyl 3-hydroxy-3-(4-acetoxyphenyl)propionate

9. A prodrug prepared by a process according to any one of claims 1 to 8 for use in therapy in facilitating the delivery of phosphorous bearing drugs into cells and through biological membranes by contacting the cells and biological membranes with the prodrug.

10. Use of a prodrug prepared by a process according to any one of claims 1 to 8 for the manufacture of a medicament to facilitate the delivery of phosphorous bearing drugs into cells and through biological membranes by contacting the cells and biological membranes with the prodrug.

11. A processs for preparing a prodrug to facilitate the delivery of phosphorous bearing parent drugs into cells and through biological membranes, comprising the steps of:
a. Selecting a phosphorous bearing parent drug; and
b. Converting one or more of the hydroxy groups on each phosphorous of the parent drug into a group of the structure A to yield a prodrug, wherein the group "A" is a benzyl-oxy- derivative with one or more acyloxy groups in para or ortho positions relative to the phosphoester,and wherein the parent drug is liberated following conversion of the acyloxy group by esterase into the corresponding hydroxy group

12. Use of a prodrug compound for the manufacture of a phosphate or phosphonate bearing parent drug which is a mono or diester of the phosphate or phosphonate for use in therapy, in which therapy the prodrug is degraded to the parent phosphorous bearing drug by an elimination reaction to form a carbon-carbon double bond; and wherein the elimination reaction is triggered by the spontaneous or enzymatic unmasking of a group which is known to be strongly electron donating, e.g. a hydroxy group.

13. Use of a prodrug prepared by a process according to claim 11 for the manufacture of a medicament to facilitate the delivery of phosphorous bearing drugs into cells and through biological membranes by contacting the cells with the prodrug.

## Patentansprüche (Patentansprüche für folgende(n) Vertragsstaat(en): AT, BE, CH, LI, DE, DK, FR, GB, IT, LU, NL, SE)

1. Prodrug-Verbindung für einen Stammwirkstoff, wobei der Stammwirkstoff die allgemeine Struktur 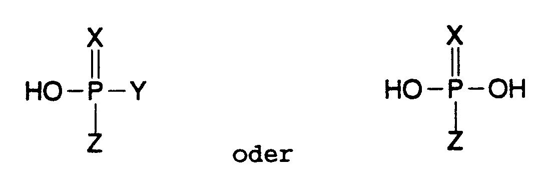 hat, worin X Sauerstoff (O) oder Schwefel (S) ist und Y und Z durch den Rest des Stammwirkstoffs definiert sind, und wobei der Stammwirkstoff keine Phosphorsäure ist und die Prodrug-Verbindung folgende Struktur hat: 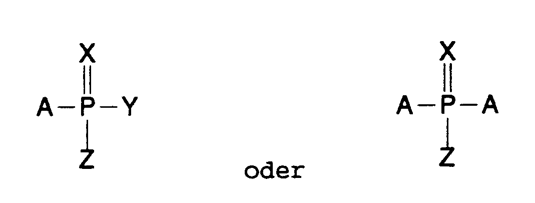 worin die Gruppe 'A' folgende Struktur hat: 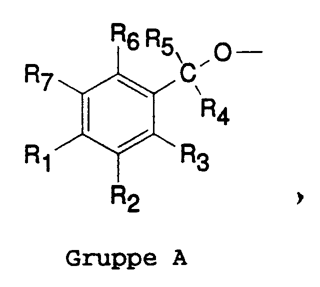 worin
R1 eine Acyloxygruppe (-O-CO-R8); eine (-O-CO₂-R8) Gruppe; eine (-O-C(O)-NH₂)-Gruppe; eine (-O-C-(O)-NHCH₃)-Gruppe; eine (-O-C(O)-N(CH₃)₂) Gruppe; Wasserstoff; ein Halogen; eine (-CO₂R9) Gruppe; eine Alkyl(z. B. Methyl)gruppe; oder eine Hydroxymethylgruppe (HO-CH₂-) sein kann;
R2 Wasserstoff; eine (-CO₂R10) Gruppe; eine Alkyl (z. B. Methyl)gruppe; ein Halogen; eine Methoxygruppe; eine Acyloxygruppe (-O-CO-R8); oder eine Hydroxymethylgruppe (HO-CH₂-) sein kann;
R3 eine Acyloxygruppe (-O-CO-R8); eine (-O-CO₂-R8) Gruppe; eine (-O-C(O)-NH₂) Gruppe; eine (-O-C(O)-NHCH₃) Gruppe; eine (-O-C(O)-N(CH₃)₂) Gruppe; Wasserstoff, eine Alkyl(z. B. Methyl)gruppe; eine Hydroxymethylgruppe (-CH₂-OH); ein Halogen; eine Hydroxyethylgruppe (-CH₂-CH₂-OH); oder eine (-CH₂-CO₂-R11) Gruppe sein kann;
R4 Wasserstoff; eine Alkyl(z. B. Methyl)gruppe; Methoxymethyl (-CH₂-O-CH₃), eine (-CH₂-CO₂-R12) Gruppe; eine (-CH₂-CO-CH₃) Gruppe; eine (-CH(CH₃)-CO₂-R12) Gruppe; eine (-CH₂-CO-NH₂) Gruppe; eine (-CH₂-NO₂) Gruppe; oder eine (-CH₂-SO₂-CH₃) Gruppe, oder ein Methylen sein kann, das mit einer funktionellen Gruppe substituiert ist, die eine Abspaltungsreaktion durch Herabsetzen der inneren Sperre zur Entfernung eines Protons vom Methylen begünstigt;
R5 Wasserstoff; eine Alkyl(z. B. Methyl)gruppe; Methoxymethyl (-CH₂-O-CH₃); eine (-CH₂-CO₂-R12) Gruppe; eine (-CH₂-CO-CH₃) Gruppe; eine (-CH(CH₃)-CO₂-R12) Gruppe; eine (-CH₂-CO-NH₂) Gruppe; eine (-CH₂-NO₂) Gruppe; oder eine (-CH₂-SO₂-CH₃) Gruppe, oder ein Methylen sein kann, das mit einer funktionellen Gruppe substituiert ist, die eine Abspaltungsreaktion durch Herabsetzen der inneren Sperre zur Entfernung eines Protons vom Methylen begünstigt;
R6 eine Acyloxygruppe (-O-CO-R8); eine (-O-CO₂-R8) Gruppe; eine (-O-C(O)-NH₂) Gruppe; eine (-O-C(O)-NHCH₃) Gruppe; eine (-O-C(O)-N(CH₃)₂) Gruppe; Wasserstoff; eine Alkyl(z. B. Methyl)gruppe; eine Hydroxymethylgruppe (-CH₂-OH); ein Halogen; eine Hydroxyethylgruppe (-CH₂-CH₂-OH); oder eine (-CH₂-CO₂-R11) Gruppe sein kann;
R7 Wasserstoff; eine (-CO₂-R10) Gruppe; eine Alkyl (z. B. Methyl)gruppe; ein Halogen; oder eine Methoxygruppe; eine Acyloxygruppe (-O-CO-R8); oder eine Hydroxymethylgruppe (HO-CH₂-) sein kann;
worin R8 eine Gruppe mit folgender Struktur sein kann: (-(CH₂)_{N}-CH₃), wobei N = 0 - 18 ist; R8 eine Phenylgruppe sein kann, wobei die Phenylgruppe ihrerseits Substituenten haben kann; R8 so gewählt sein kann, daß R8-CO₂H eine Aminosäure; Milchsäure; Glykolsäure (HO-CH₂-CO₂H); Glyzerinsäure (HO-CH₂-CH(OH)-CO₂H); oder Azetessigsäure (-CH₃COCH₂-CO₂H) ist;
worin R9 und R10, R11 und R12 eine Methyl-, Ethyl-, Phenyl- oder Benzylgruppe ist; und worin mindestens eine der folgenden Gruppen: R1; R3; R6 eine Acyloxygruppe (-O-CO-R8) oder eine andere Gruppe ist, die durch *in vivo* Biotransformation schließlich eine Hydroxygruppe am Phenylring ergibt.

2. Prodrug gemäß Anspruch 1, wobei der Stammwirkstoff
(a) ein Monophosphatesterwirkstoff; oder
(b) ein Phosphodiesterwirkstoff; oder
(c) ein Phosphonsäure- oder Phosphonatwirkstoff; oder
(d) ein Phosphinsäurewirkstoff; oder
(e) ein Nukleotidmonophosphatwirkstoff; oder
(f) ausgewählt ist aus der Gruppe bestehend aus:
9-(2-Phosphonylmethoxy-ethyl)adenin (PMEA);
9-(RS)-(3-Fluor-2-phosphonylmethoxypropyly)adenin (FPMPA),
9-(RS)-(3-Fluor-2-phosphonylmethoxypropyly)2,6, diaminopurin (FPMPDAP);
3'Azido-dideoxythymidin 5'monophosphat (AZT-Phosphat);
2',3'-Didehydro-2',3'dideoxythymidin 5'monophosphat (D4T-Phosphat);
2',3'-Dideoxyadenosin 5'monophosphat;
einem Carboxylatester von Phosphonoformiat.

3. Prodrug gemäß Anspruch 1, wobei die Struktur der Gruppe 'A' ausgewählt ist aus der Gruppe bestehend aus:

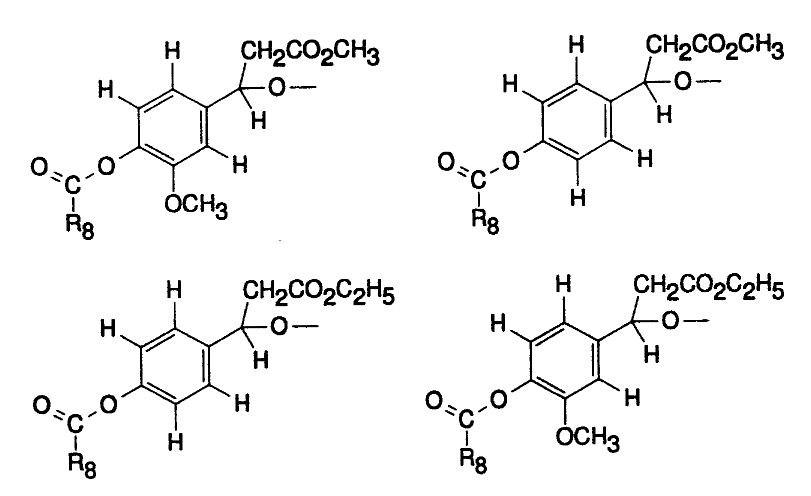

4. Prodrug gemäß Anspruch 3, wobei der Stammwirkstoff
(a) ein Nukleotidmonophosphat ist; oder
(b) aus folgender Gruppe ausgewählt ist:
9-(2-Phosphonylmethoxy-ethyl)adenin (PMEA);
9-(RS)-(3-Fluor-2-phosphonylmethoxypropyly)adenin (FPMPA),
9-(RS)-(3-Fluor-2-phosphonylmethoxypropyly)2,6, diaminopurin (FPMPDAP);
3'Azido-dideoxythymidin 5'monophosphat (AZT-Phosphat);
2',3'-Didehydro-2',3'dideoxythymidin 5'monophosphat (D4T-Phosphat);
2',3'-Dideoxyadenosin 5'monophosphat; oder
(c) ein Carboxylatester von Phosphonoformiat ist.

5. Prodrug gemäß Anspruch 1, wobei die Gruppe 'A' folgende Struktur hat:

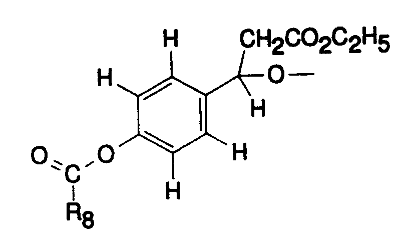

6. Prodrug gemäß Anspruch 5, wobei der Stammwirkstoff ein Nukleotidmonophosphat oder aus folgender Gruppe ausgewählt ist:
9-(2-Phosphonylmethoxy-ethyl)adenin (PMEA);
9-(RS)-(3-Fluor-2-phosphonylmethoxypropyly)adenin (FPMPA),
9-(RS)-(3-Fluor-2-phosphonylmethoxypropyly)2,6, diaminopurin (FPMPDAP);
3'Azido-dideoxythymidin 5'monophosphat (AZT-Phosphat);
2',3'-Didehydro-2',3'dideoxythymidin 5'monophosphat (D4T-Phosphat);
2',3'-Dideoxyadenosin 5'monophosphat;
ein Carboxylatester von Phosphonoformiat.

7. Prodrug gemäß Anspruch 1 mit folgender Struktur: 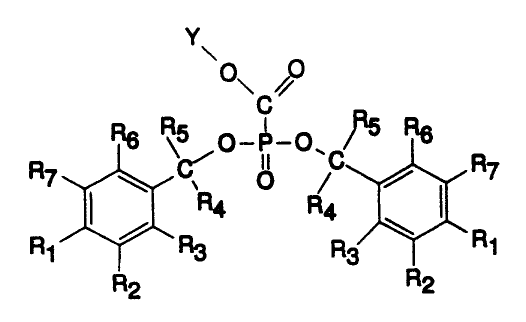 worin Y eine Benzyl- oder Phenylgruppe ist und die Benzyl- oder Phenylgruppe ihrerseits Substituenten haben kann.

8. Prodrug gemäß Anspruch 7 mit der Struktur:

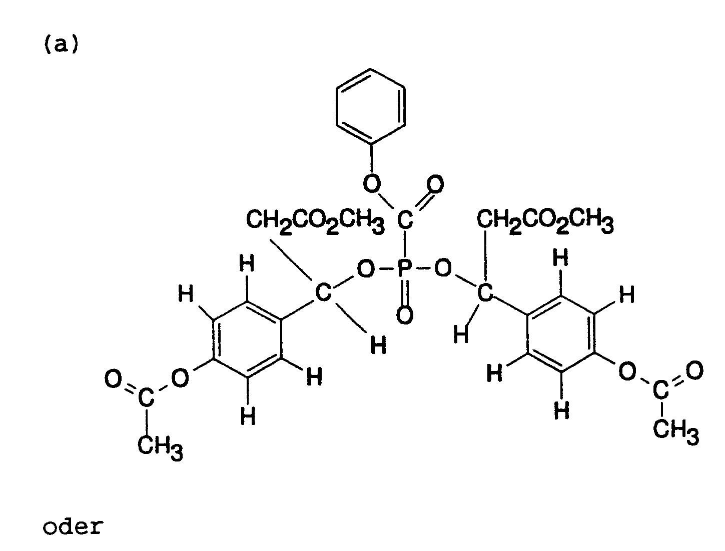

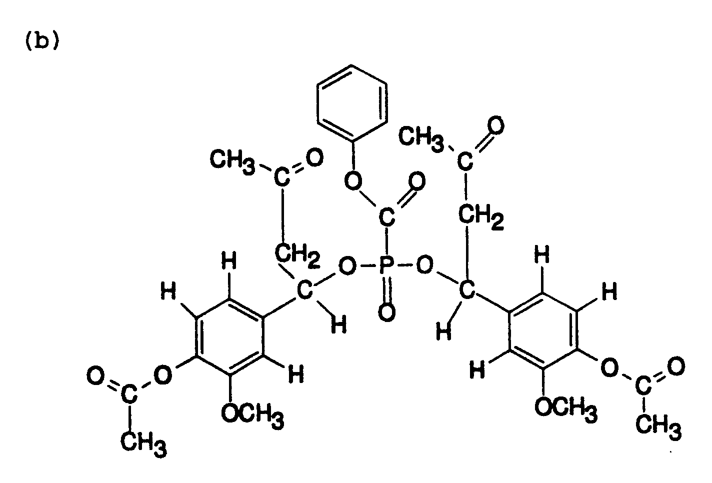

9. Prodrug gemäß Anspruch 1 mit folgender Struktur:

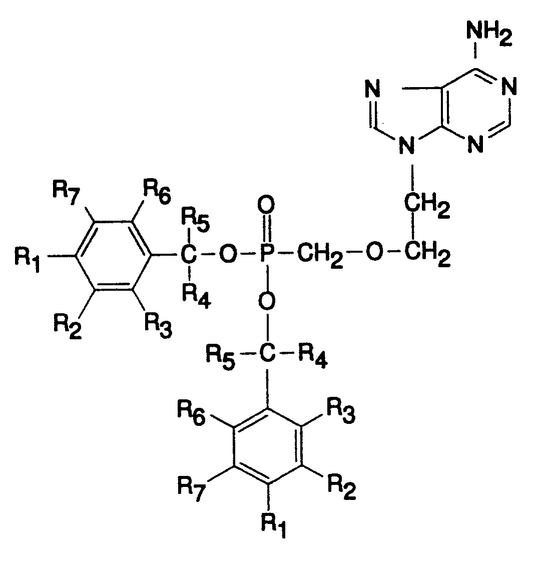

10. Prodrug gemäß Anspruch 9 mit folgender Struktur:

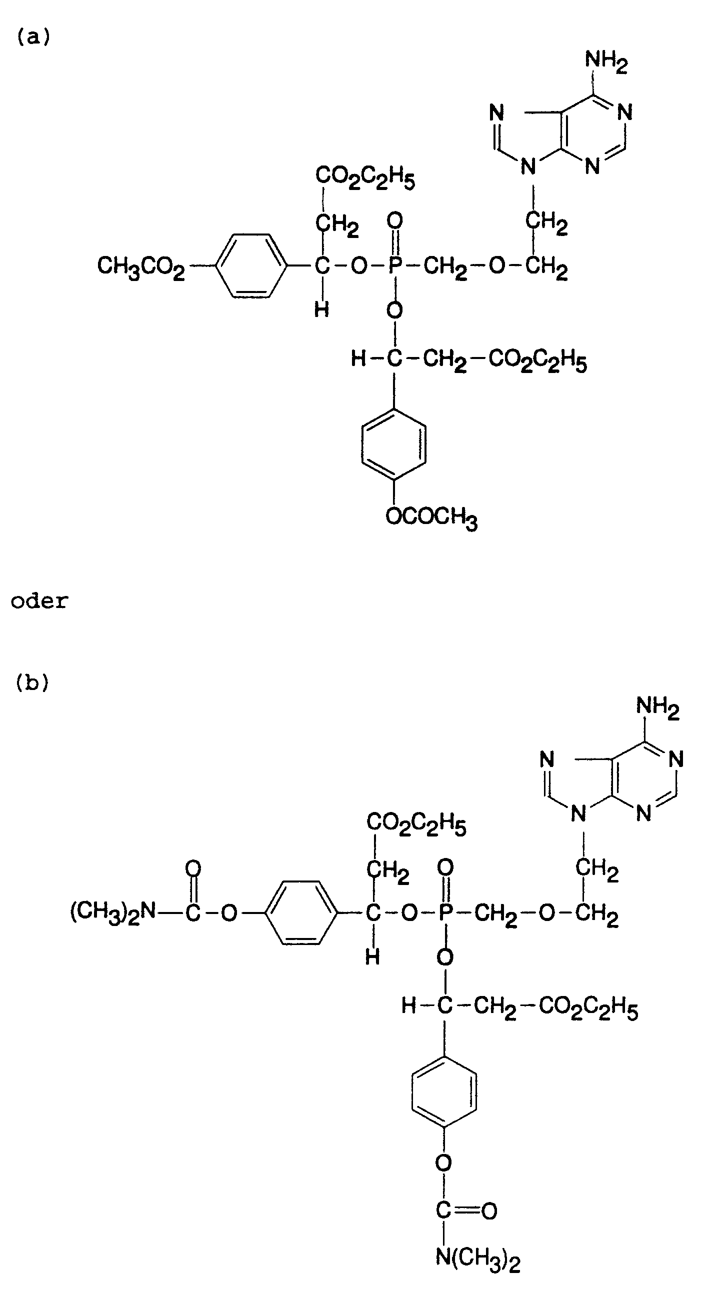

11. Prodrug gemäß Anspruch 1 mit folgenden Strukturen:

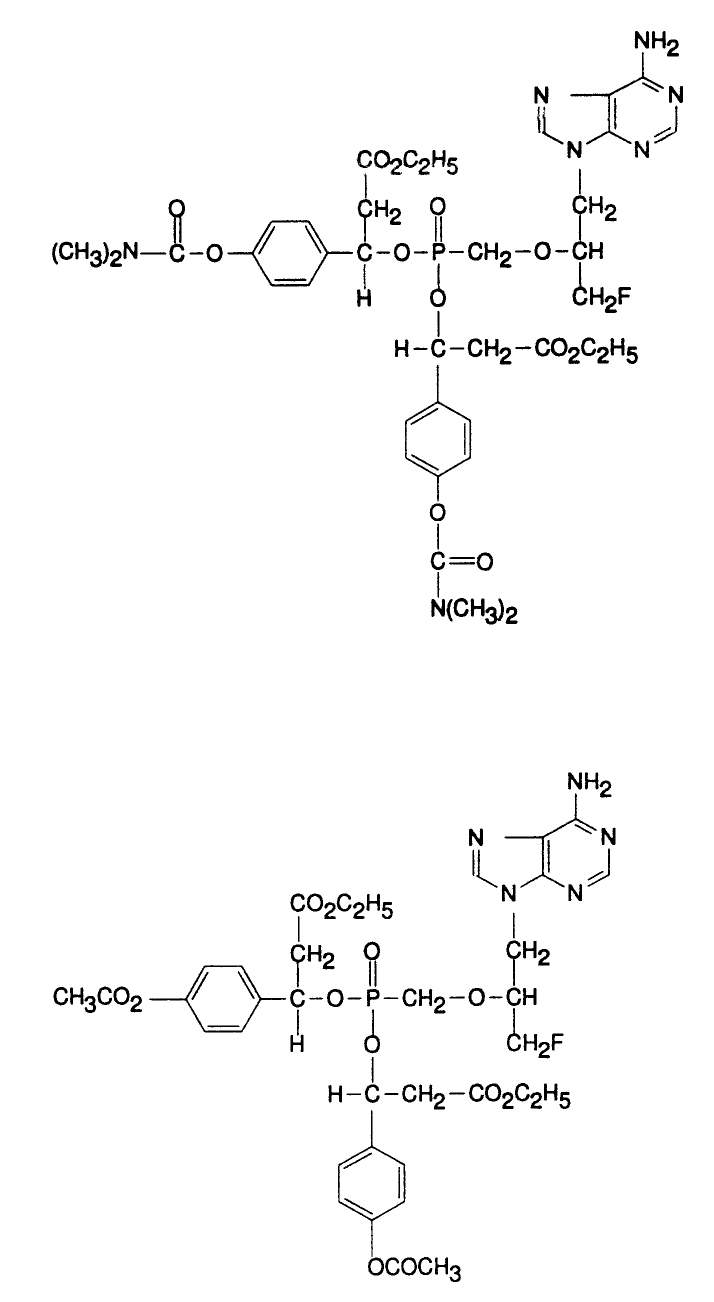

12. Prodrug gemäß Anspruch 1 mit folgender Struktur: 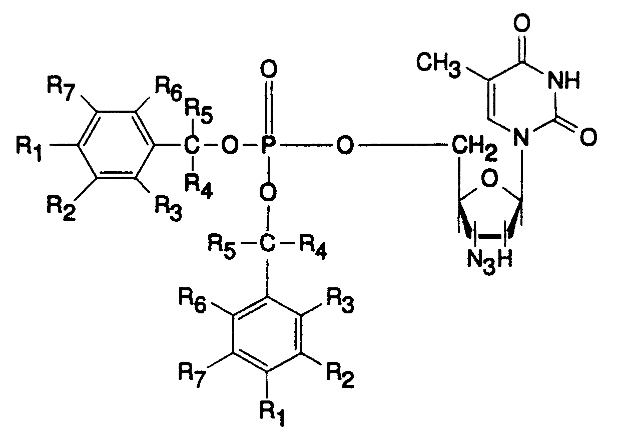 zum Beispiel:

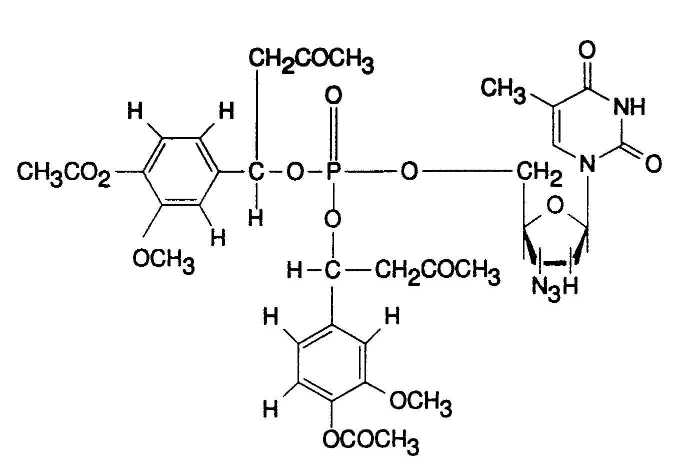

13. Prodrug gemäß Anspruch 1 mit folgender Struktur:

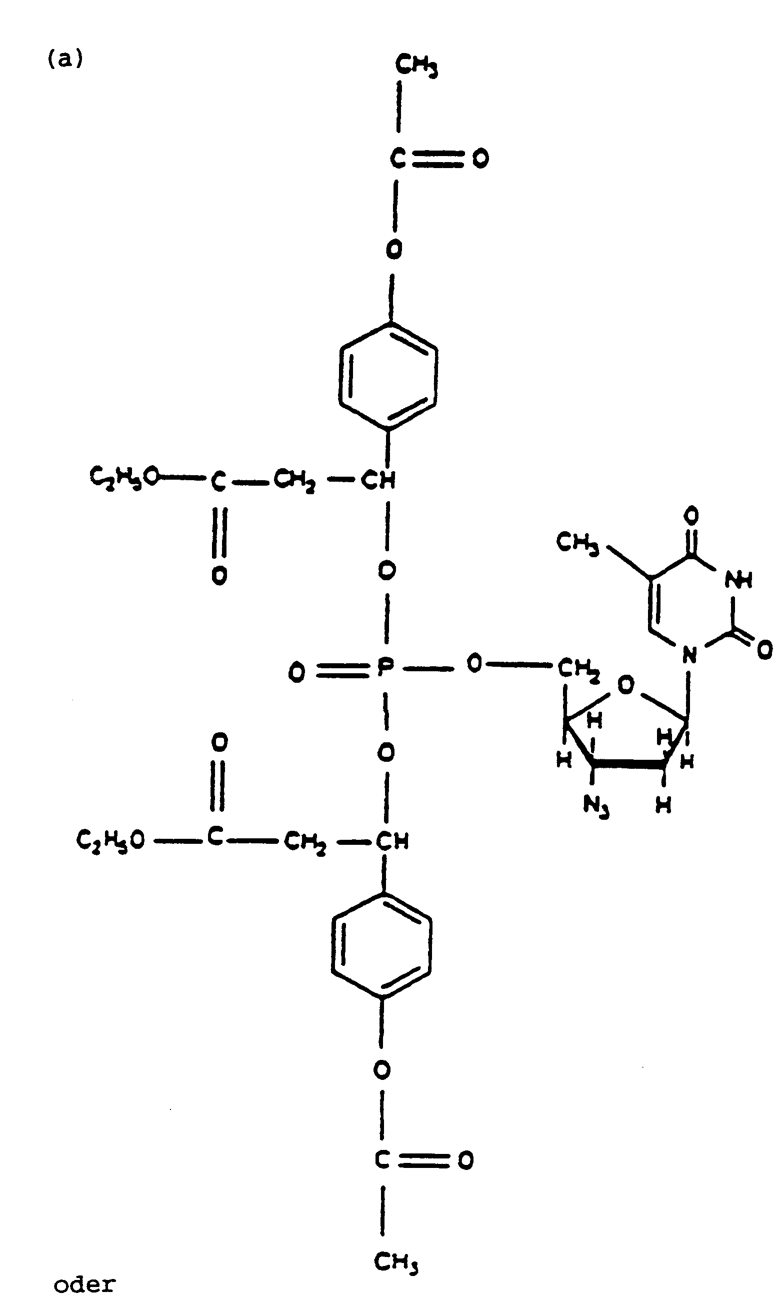

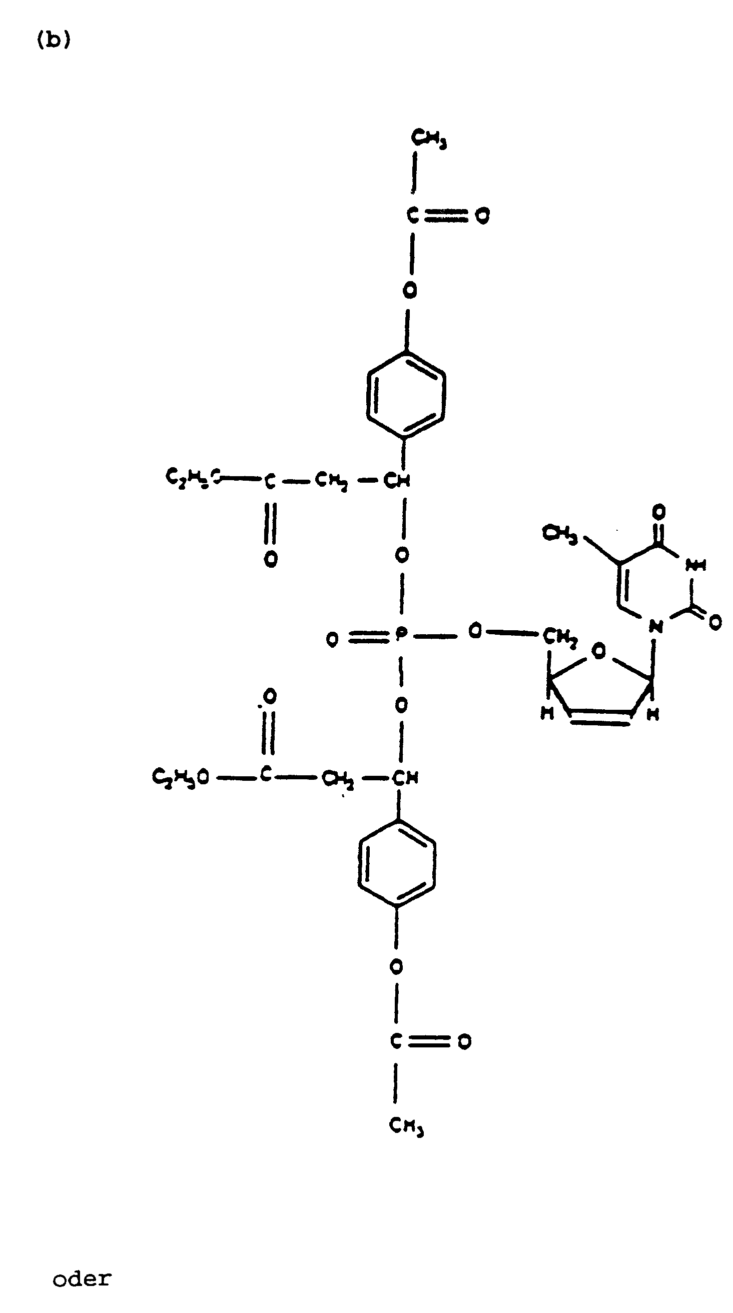

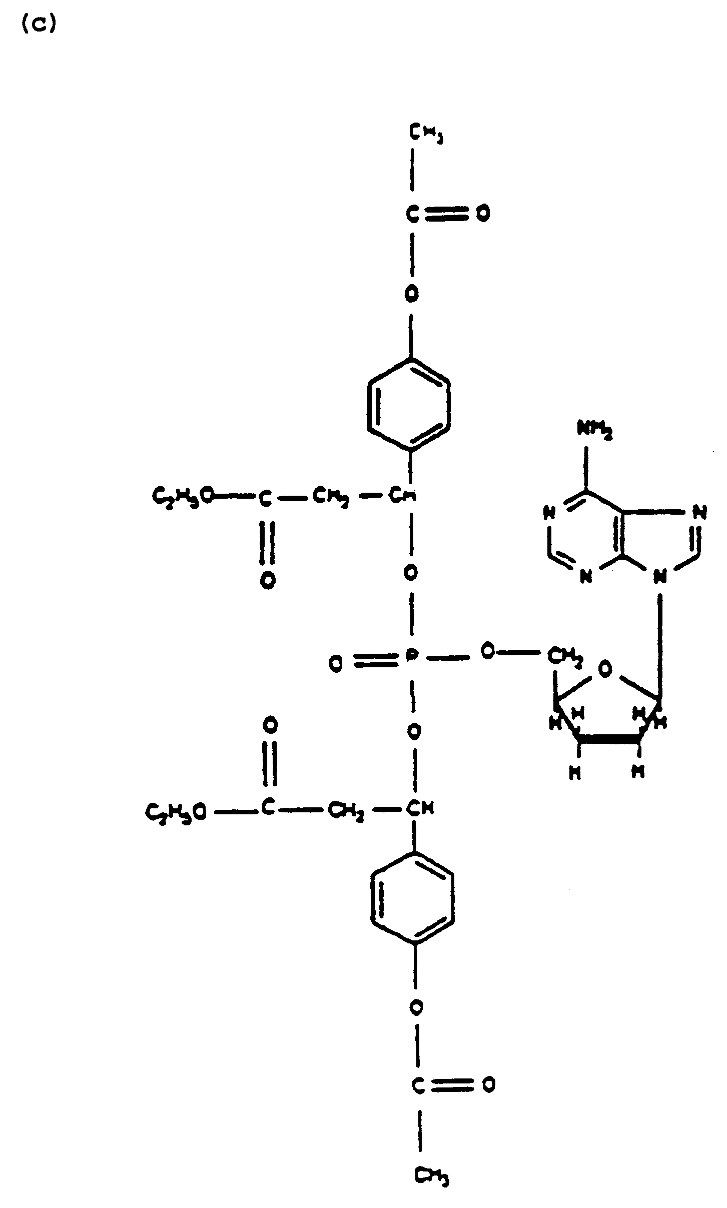

14. Verbindung zur Verwendung als Zwischenprodukt bei der Prodrug-Synthese mit folgender Struktur: 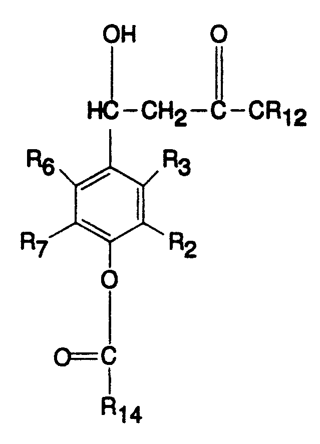 worin
R2 und R7 Wasserstoff, eine (-CO₂R10) Gruppe, eine Methylgruppe, ein Halogen oder eine Methoxygruppe oder eine Hydroxymethylgruppe (HO-CH₂-) sein können, wobei R10 eine Methyl- oder Ethylgruppe sein kann;
R3 und R6 Wasserstoff; eine Methylgruppe; eine Hydroxymethylgruppe (-CH₂-OH); ein Halogen; eine Hydroxyethylgruppe (-CH₂-CH₂-OH) sein können;
R12 eine Methyl-, Ethyl-, Phenyl- oder Benzylgruppe ist;
R14 eine Gruppe mit der Struktur (-(CH₂)_{N}-CH₃) sein kann, wobei N = 0 - 18 ist; eine Phenylgruppe sein kann, wobei die Phenylgruppe ihrerseits Substituenten haben kann; R14 so gewählt sein kann, daß R14-CO₂H eine Aminosäure; Milchsäure; Glykolsäure (HO-CH₂-CO₂H); Glyzerinsäure (HO-CH₂-CH(OH)-CO₂H); oder Azetessigsäure (CH₃COCH₂-CO₂H) sein kann;
worin R14 (-O-(CH₂)_{N}-CH₃) sein kann, wobei N = 0 - 18 ist; eine Phenyloxygruppe sein kann, wobei die Phenyloxygruppe ihrerseits Substituenten haben kann; und worin R14 eine der folgenden Gruppen sein kann: (-NH₂); (-NHCH₃); oder (-N(CH₃)₂).

15. Verbindung gemäß Anspruch 14 mit folgender Struktur: 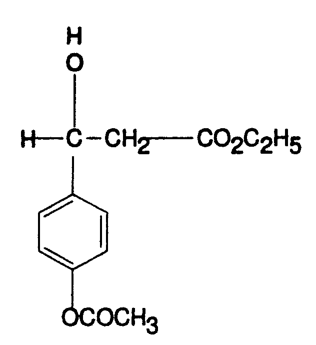 Ethyl-3-hydroxy-3-(4-acetoxyphenyl)propionat

16. Verfahren zur Herstellung einer Prodrug zur leichteren Abgabe phosphorhaltiger Stammwirkstoffe in Zellen hinein und durch Biomembranen, bestehend aus oder enthaltend folgende Schritte:
a) Auswahl eines phosphorhaltigen Stammwirkstoffs; und
b) Umwandlung einer oder mehrerer Hydroxygruppe(n) an jedem Phosphor des Stammwirkstoffs in eine Gruppe mit der Struktur A zur Bildung einer Prodrug, wobei die Struktur A besteht aus oder enthält: 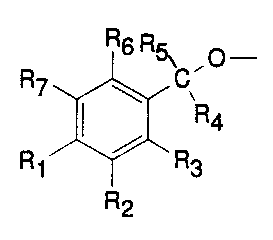 worin
R1 eine Acyloxygruppe (-O-CO-R8); eine (-O-CO₂-R8) Gruppe; eine (-O-C(O)-NH₂) Gruppe; eine (-O-C-(O)-NHCH₃) Gruppe; eine (-O-C(O)-N(CH₃)₂) Gruppe; Wasserstoff; ein Halogen; eine (-CO₂R9) Gruppe; eine Alkyl(z. B. Methyl)gruppe; oder eine Hydroxymethylgruppe (HO-CH₂-) sein kann;
R2 Wasserstoff; eine (-CO₂R10) Gruppe; eine Alkyl (z. B. Methyl) gruppe; ein Halogen; eine Methoxygruppe; eine Acyloxygruppe (-O-CO-R8); oder eine Hydroxymethylgruppe (HO-CH₂-) sein kann;
R3 eine Acyloxygruppe (-O-CO-R8); eine (-O-CO₂-R8) Gruppe; eine (-O-C(O)-NH₂) Gruppe; eine (-O-C(O)-NHCH₃) Gruppe; eine (-O-C(O)-N(CH₃)₂) Gruppe; Wasserstoff; eine Alkyl(z. B. Methyl)gruppe; eine Hydroxymethylgruppe (-CH₂-OH); ein Halogen; eine Hydroxyethylgruppe (-CH₂-CH₂-OH); oder eine (-CH₂-CO₂-R11) Gruppe sein kann;
R4 Wasserstoff; eine Alkyl(z. B. Methyl)gruppe; Methoxymethyl (-CH₂-O-CH₃), eine (-CH₂-CO₂-R12) Gruppe; eine (-CH₂-CO-CH₃) Gruppe; eine (-CH(CH₃)-CO₂-R12) Gruppe; eine (-CH₂-CO-NH₂) Gruppe; eine (-CH₂-NO₂) Gruppe; oder eine (-CH₂-SO₂-CH₃) Gruppe, oder ein Methylen sein kann, das mit einer funktionellen Gruppe substituiert ist, die eine Abspaltungsreaktion durch Herabsetzen der inneren Sperre zur Entfernung eines Protons vom Methylen begünstigt;
R5 Wasserstoff; eine Alkyl(z. B. Methyl)gruppe; Methoxymethyl (-CH₂-O-CH₃); eine (-CH₂-CO₂-R12) Gruppe; eine (-CH₂-CO -CH₃) Gruppe; eine (-CH(CH₃)-CO₂-R12) Gruppe; eine (-CH₂-CO-NH₂) Gruppe; eine (-CH₂-NO₂) Gruppe; oder eine (-CH₂-SO₂-CH₃) Gruppe, oder ein Methylen sein kann, das mit einer funktionellen Gruppe substituiert ist, die eine Abspaltungsreaktion durch Herabsetzen der inneren Sperre zur Entfernung eines Protons vom Methylen begünstigt;
R6 eine Acyloxygruppe (-O-CO-R8); eine (-O-CO₂-R8) Gruppe; eine (-O-C(O)-NH₂) Gruppe; eine (-O-C(O)-NHCH₃) Gruppe; eine (-O-C(O)-N(CH₃)₂) Gruppe; Wasserstoff; eine Alkyl(z. B. Methyl)gruppe; eine Hydroxymethylgruppe (-CH₂-OH); ein Halogen; eine Hydroxyethylgruppe (-CH₂-CH₂-OH); oder eine (-CH₂-CO₂-R11) Gruppe sein kann;
R7 Wasserstoff; eine (-CO₂-R10) Gruppe; eine Alkyl(z. B. Methyl)gruppe; ein Halogen; oder eine Methoxygruppe; eine Acyloxygruppe (-O-CO-R8); oder eine Hydroxymethylgruppe (HO-CH₂-) sein kann, worin R8 eine Gruppe mit folgender Struktur sein kann: (-(CH₂)_{N}-CH₃), wobei N = 0 - 18 ist; R8 eine Phenylgruppe sein kann, wobei die Phenylgruppe ihrerseits Substituenten haben kann; R8 so gewählt sein kann, daß R8-CO₂H eine Aminosäure; Milchsäure; Glykolsäure (HO-CH₂-CO₂H); Glyzerinsäure (HO-CH₂-CH(OH)-CO₂H); oder Azetessigsäure (-CH₃COCH₂-CO₂H) ist;
worin R9 und R10, R11 und R12 eine Methyl-, Ethyl-, Phenyl- oder Benzylgruppe ist; und worin mindestens eine der folgenden Gruppen: R1; R3; R6 eine Acyloxygruppe (-O-CO-R8) oder eine andere Gruppe ist, die durch *in vivo* Biotransformation schließlich eine Hydroxygruppe am Phenylring ergibt.

17. Verfahren gemäß Anspruch 16, wobei der Stammwirkstoff
(a) ein Monophosphatesterwirkstoff; oder
(b) ein Phosphodiesterwirkstoff; oder
(c) ein Phosphonsäurewirkstoff; oder
(d) ein Phosphonatwirkstoff; oder
(e) ein Phosphinsäurewirkstoff; oder
(f) ein Nukleotidmonophosphatwirkstoff; oder
(g) ausgewählt ist aus der Gruppe bestehend aus:
9-(2-Phosphonylmethoxy-ethyl)adenin (PMEA); 9-(RS)-(3-Fluor-2-phosphonylmethoxypropyly)adenin
(FPMPA),
9-(RS)-(3-Fluor-2-phosphonylmethoxypropyly)2,6, diaminopurin
3'Azido-dideoxythymidin 5'monophosphat (AZT-Phosphat);
2',3'-Didehydro-2',3'dideoxythymidin 5'monophosphat (D4T-Phosphat);
2',3'-Dideoxyadenosin 5'monophosphat;
einem Carboxylatester von Phosphonoformiat.

18. Verfahren gemäß Anspruch 16 oder 17, wobei
(a) die Struktur der Gruppe 'A' ausgewählt ist aus der Gruppe bestehend aus: 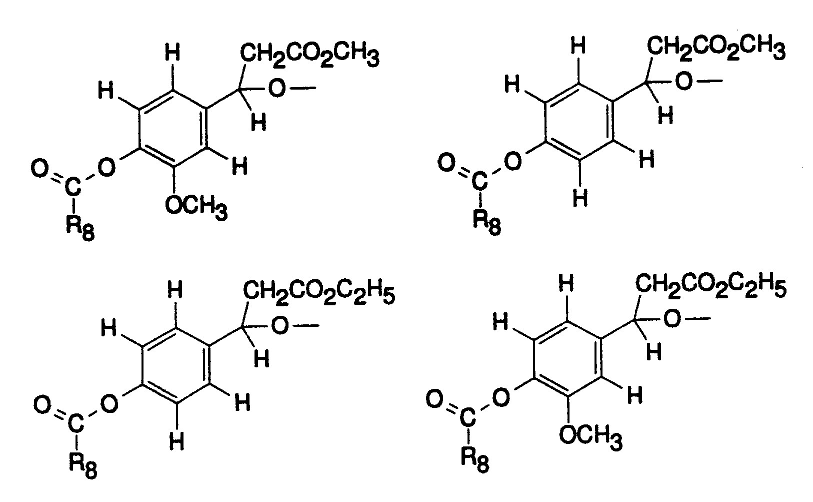 worin R8 eine Gruppe mit der Struktur (-(CH₂)_{N}-CH₃) sein kann, wobei N = 0 - 18 ist;
R8 eine Phenylgruppe sein kann, wobei die Phenylgruppe ihrerseits Substituenten haben kann;
R8 so gewählt sein kann, daß R8-CO₂H eine Aminosäure; Milchsäure; Glykolsäure (HO-CH₂-CO₂H); Glyzerinsäure (HO-CH₂-CH(OH)-CO₂H); oder Azetessigsäure (-CH₃COCH₂-CO₂H) ist; oder
worin R8 eine beliebige Gruppe ist, die so gewählt ist, daß die erhaltene Prodrug eine *in vivo* Biotransformation oder spontane Transformation durchmacht, um schließlich eine Hydroxygruppe am Phenylring zu ergeben; oder
(b) die Gruppe 'A' folgende Struktur hat:

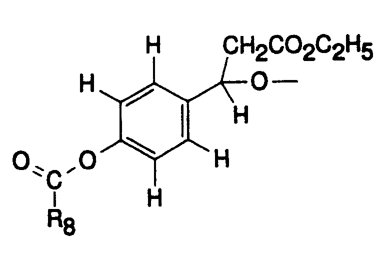

19. Verfahren gemäß Anspruch 16 zur Herstellung einer Prodrug zur leichteren Abgabe von Phosphonoformiat in Zellen hinein und durch Biomembranen, bestehend aus oder enthaltend die Herstellung einer Prodrug mit folgender Struktur: 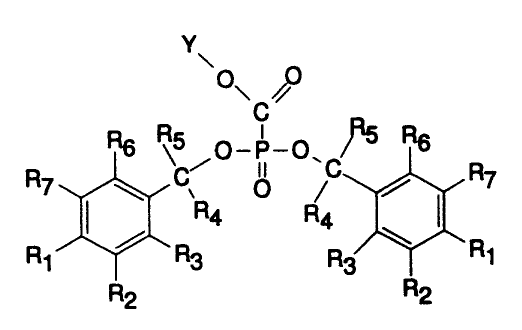 worin Y ein Benzyl, Phenyl ist, wobei die Benzyl- oder Phenylgruppe ihrerseits Substituenten haben kann.

20. Verfahren gemäß Anspruch 19, bestehend aus oder enthaltend die Herstellung einer Prodrug mit folgender Struktur:

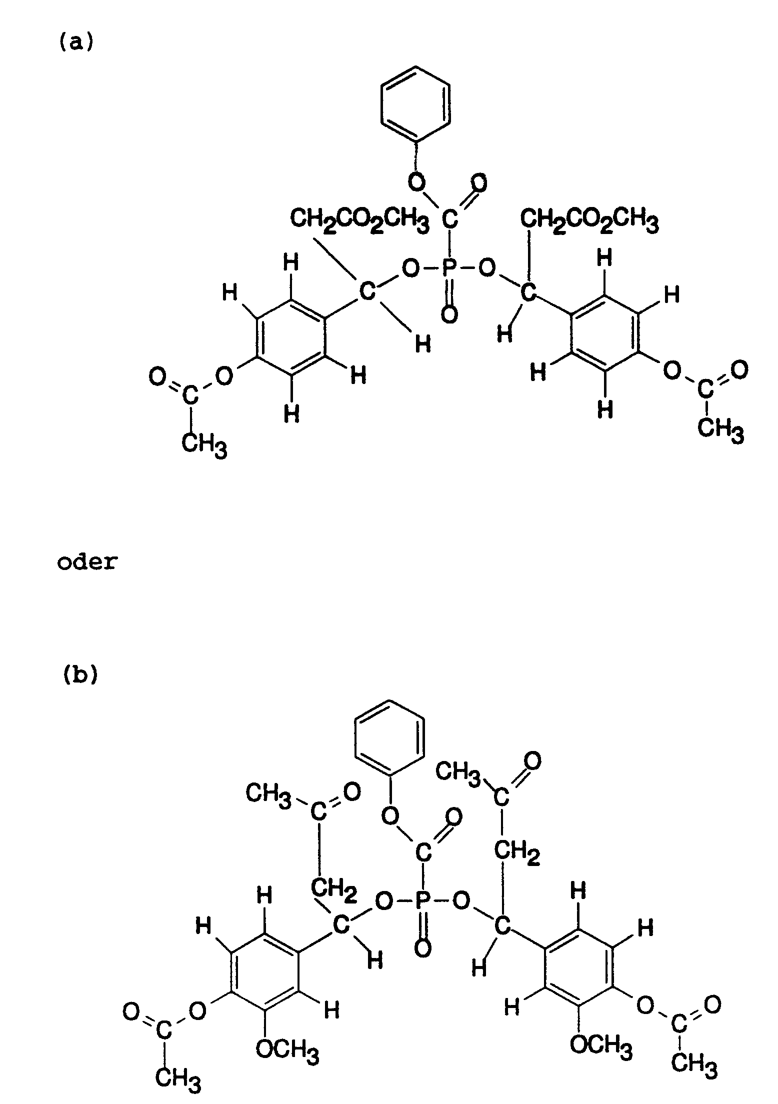

21. Verfahren gemäß Anspruch 16, wobei der phosphorhaltige Wirkstoff und die Struktur A so gewählt sind, daß folgende Prodrug erhalten wird:

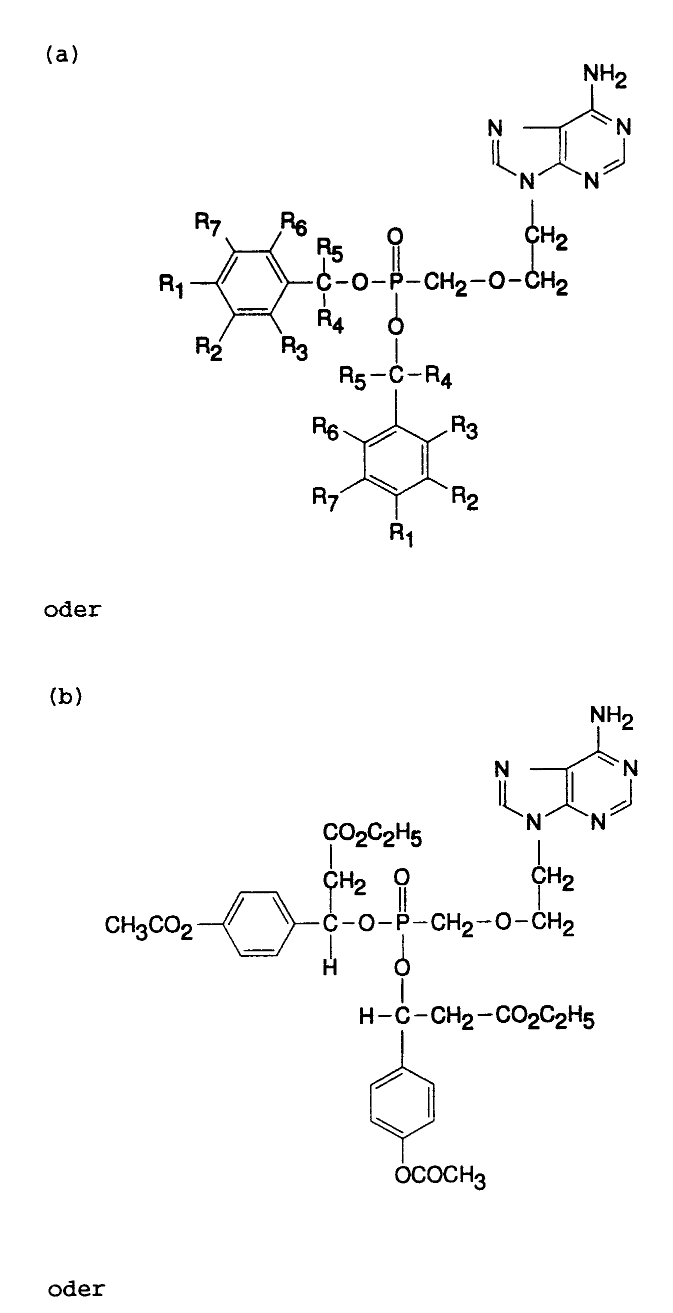

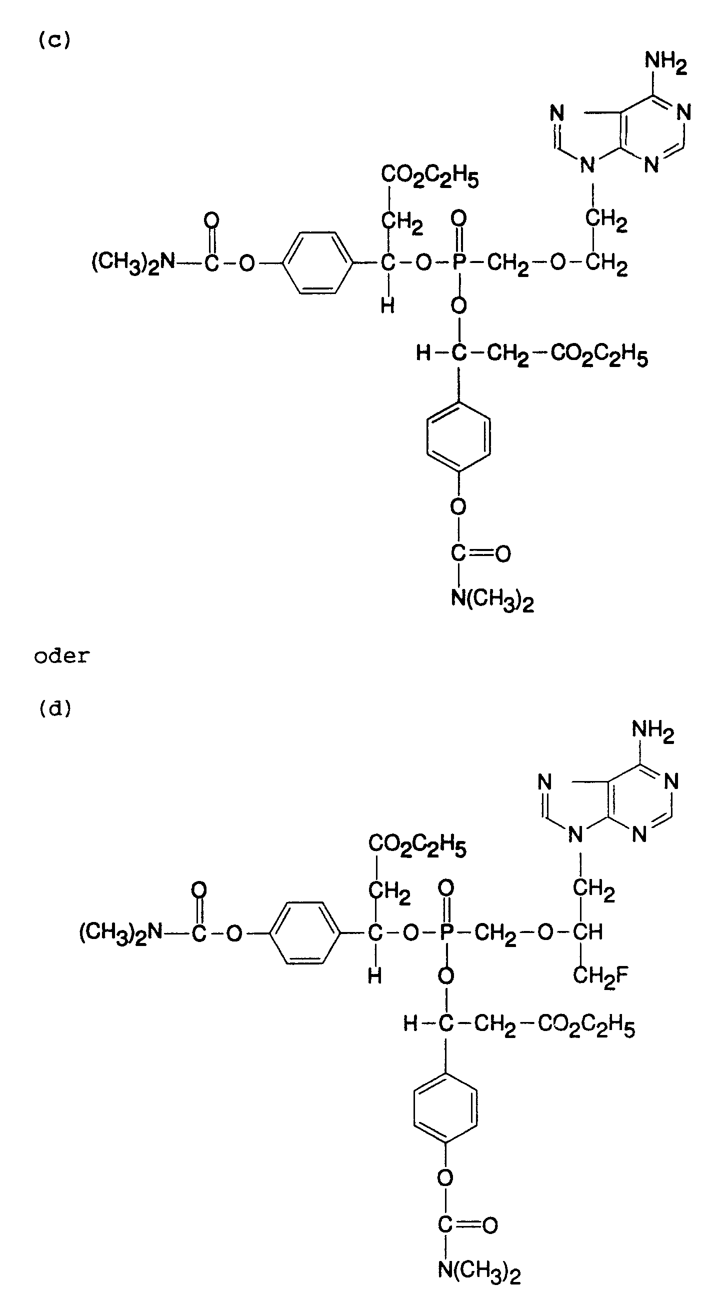

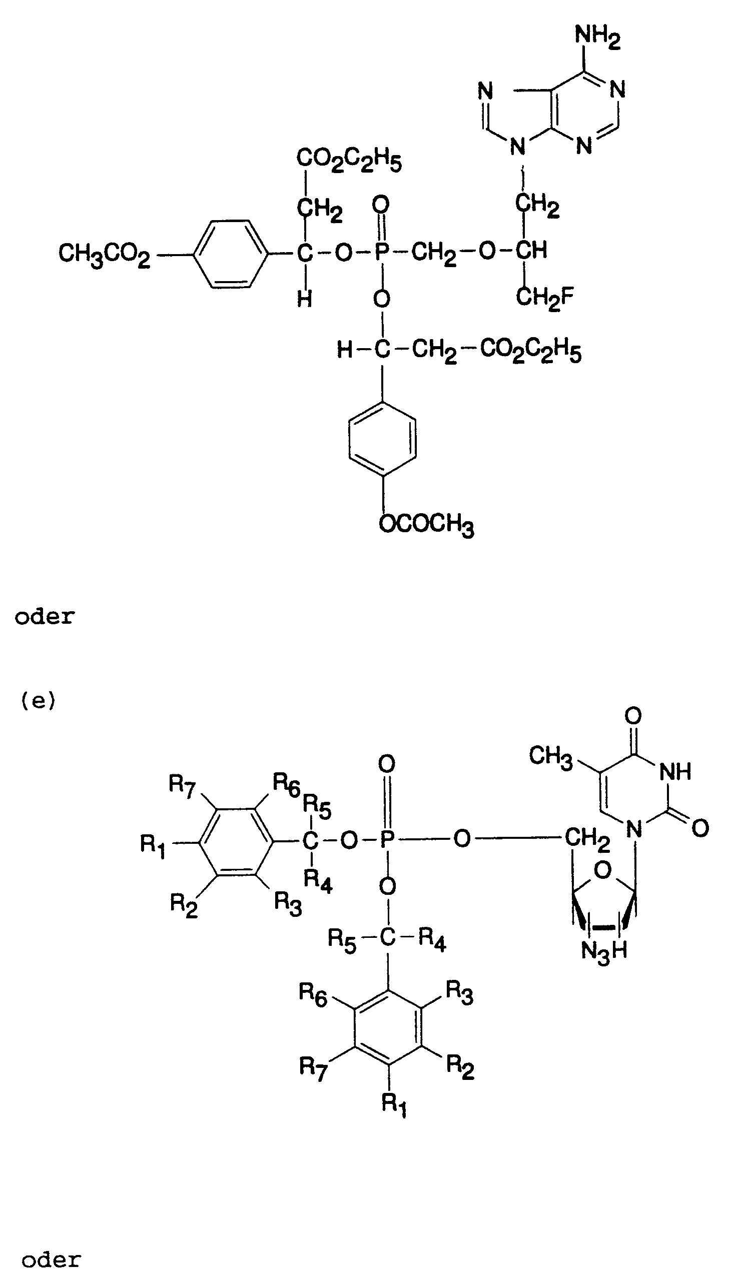

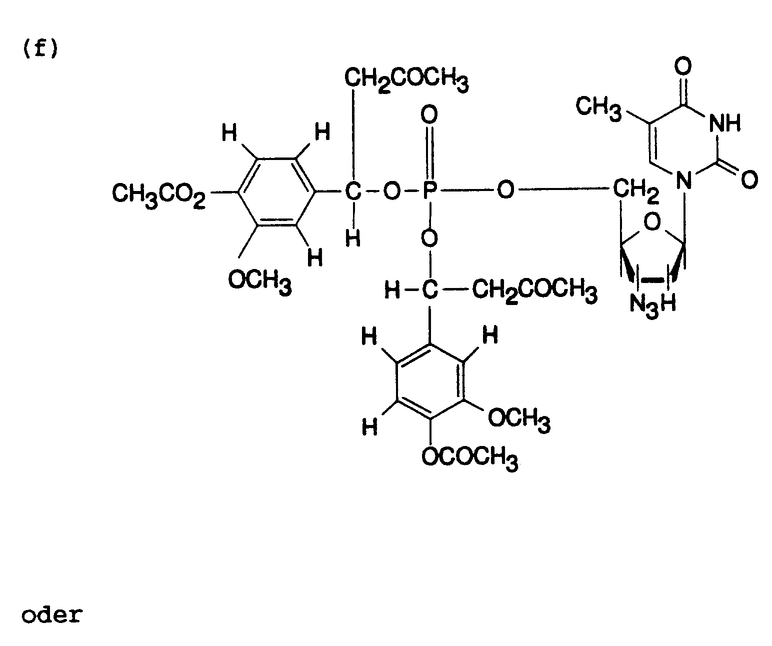

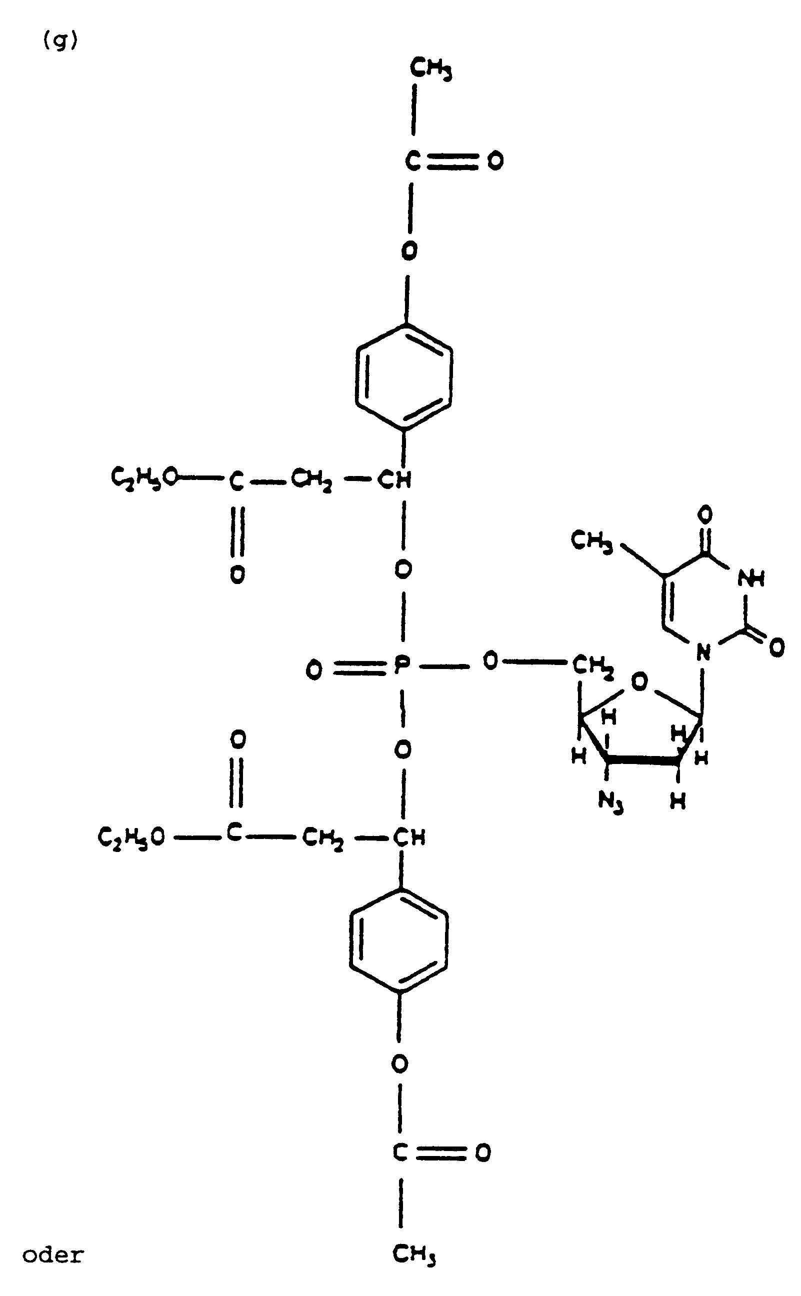

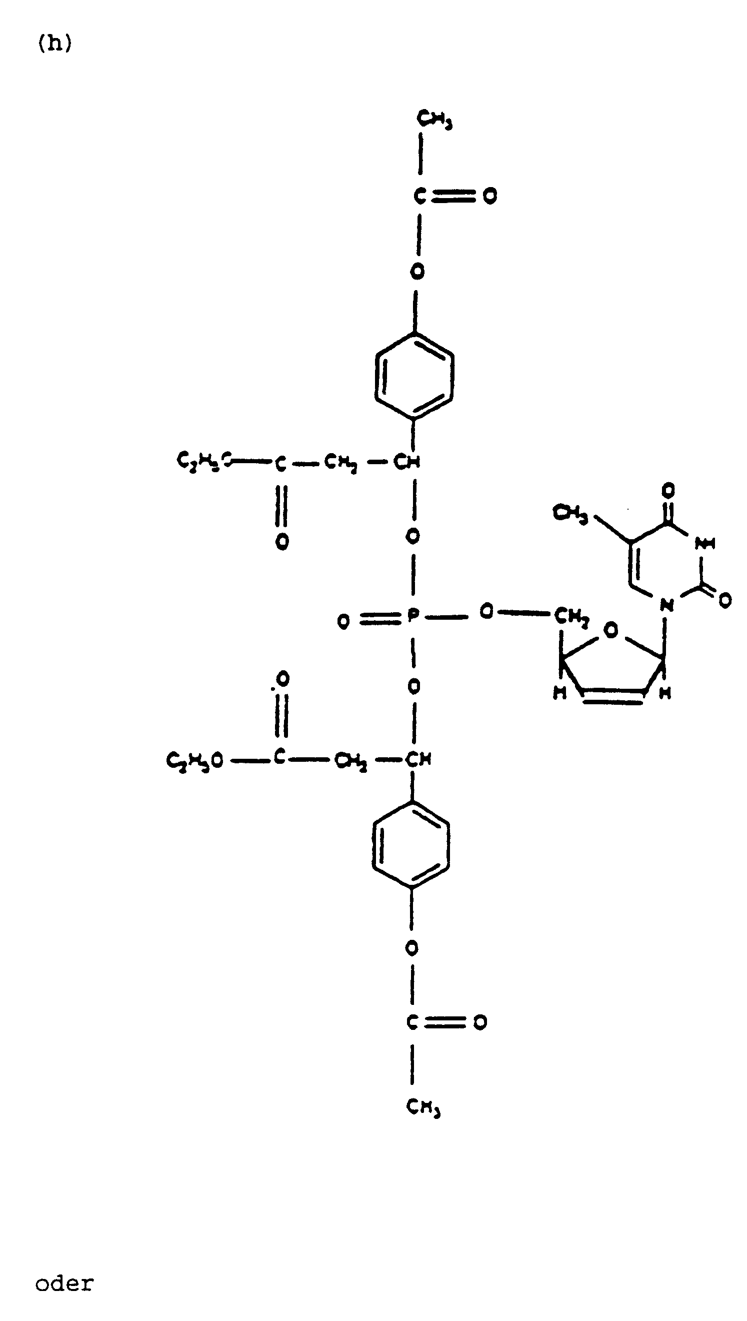

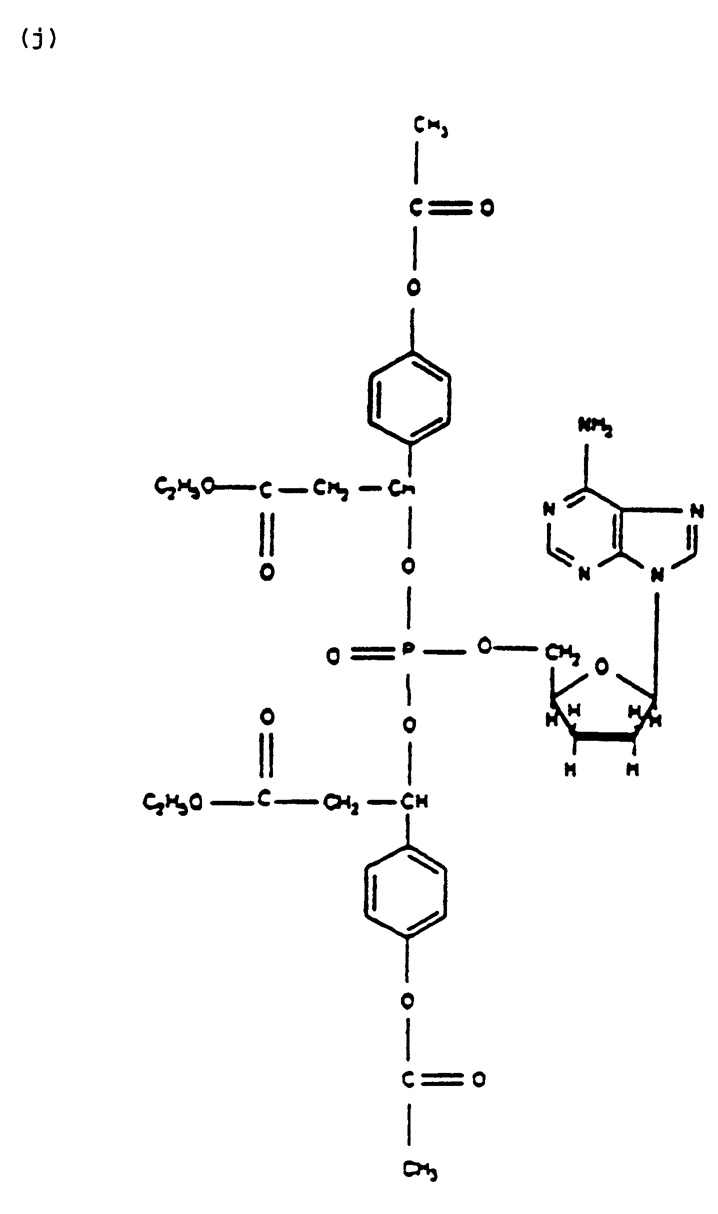

22. Verfahren zur Herstellung einer Prodrug zur leichteren Abgabe phosphorhaltiger Stammwirkstoffe in Zellen ninein und durch Biomembranen, bestehend aus oder enthaltend folgende Schritte:
a) Auswahl eines phosphorhaltigen Wirkstoffs; und
b) Herstellung eines Mono- oder Bisphosphoesters aus einem entsprechend aktivierten Derivat des Stammwirkstoffs und einem Alkohol mit der Struktur A zur Bildung einer Prodrug, wobei die Struktur A besteht aus oder enthält: 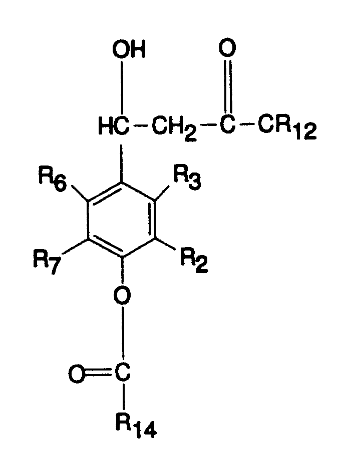 worin
R2 und R7 Wasserstoff, eine (-CO₂R10) Gruppe, eine Methylgruppe, ein Halogen oder eine Methoxygruppe oder eine Hydroxymethylgruppe (HO-CH₂-) sein können, wobei R10 eine Methyl- oder Ethylgruppe sein kann;
R3 und R6 Wasserstoff, eine Methylgruppe; eine Hydroxymethylgruppe (-CH₂-OH); ein Halogen; eine Hydroxyethylgruppe (-CH₂-CH₂-OH) sein können;
R12 eine Methyl-, Ethyl-, Phenyl- oder Benzylgruppe ist;
R14 eine Gruppe mit der Struktur (-(CH₂)_{N}-CH₃) sein kann, wobei N = 0 - 18 ist;
eine Phenylgruppe sein kann, wobei die Phenylgruppe ihrerseits Substituenten haben kann;
R14 so gewählt sein kann, daß R14-CO₂H eine Aminosäure; Milchsäure; Glykolsäure (HO-CH₂-CO₂H); Glyzerinsäure (HO-CH₂-CH(OH)-CO₂H); oder Azetessigsäure (-CH₃COCH₂-CO₂H) ist;
worin R14 (-O-(CH₂)_{N}-CH₃) sein kann, wobei N = 0 - 18 ist;
eine Phenyloxygruppe sein kann, wobei die Phenyloxygruppe ihrerseits Substituenten haben kann; und
worin R14 eine der folgenden Gruppen sein kann: (-NH₂); (-NHCH₃); oder (-N(CH₃)₂).

23. Verfahren gemäß Anspruch 22, wobei die Struktur A Ethyl-3-hydroxy-3-(4-acetoxyphenyl)propionat ist:

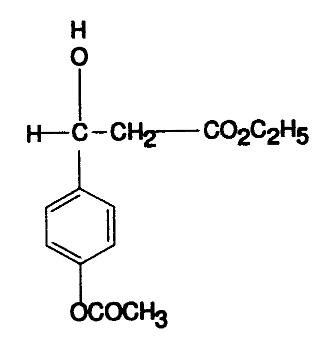

24. Prodrug, hergestellt nach einem Verfahren gemäß einem der Ansprüche 16 bis 23, für therapeutische Zwecke zur leichteren Angabe phosphorhaltiger Wirkstoffe in Zellen hinein und durch Biomembranen durch Kontaktierung der Zellen und Biomembranen mit der Prodrug.

25. Verwendung einer Prodrug, hergestellt nach einem Verfahren gemäß einem der Ansprüche 16 bis 23, zur Herstellung eines Medikaments zur leichteren Abgabe phosphorhaltiger Wirkstoffe in Zellen hinein und durch Biomembranen durch Kontaktierung der Zellen und Biomembranen mit der Prodrug.

26. Prodrug-Verbindung für einen Stammwirkstoff, wobei der Stammwirkstoff die allgemeine Struktur 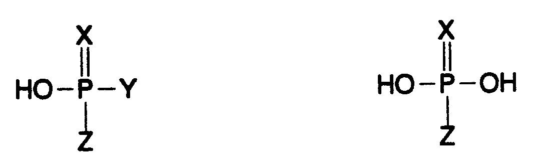 hat, worin X Sauerstoff (O) oder Schwefel (S) ist und Y und Z durch den Rest des Stammwirkstoffs definiert sind, und worin der Stammwirkstoff keine Phosphorsäure ist und die Prodrug-Verbindung folgende Struktur hat: 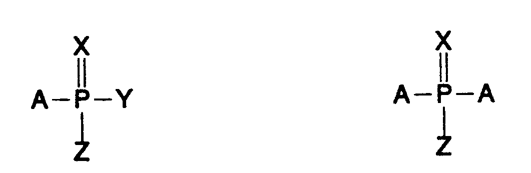 worin die Gruppe 'A' ein Benzyloxy-Derivat mit einer oder mehreren Acyloxygruppe(n) in Para- oder Ortho-Stellung zum Phosphoester ist und der Stammwirkstoff nach Umwandlung der Acyloxygruppe durch Esterase in die entsprechende Hydroxygruppe freigesetzt wird.

27. Verfahren zur Herstellung einer Prodrug zur leichteren Abgabe phosporhaltiger Stammwirkstoffe in Zellen hinein und durch Biomembranen, bestehend aus oder enthaltend folgende Schritte:
a) Auswahl eines phosphorhaltigen Stammwirkstoffs und
b) Umwandlung einer oder mehrerer Hydroxygruppe(n) an jedem Phosphor des Stammwirkstoffs in eine Gruppe mit der Struktur A zur Bildung einer Prodrug, wobei die Gruppe 'A' ein Benzyloxy-Derivat mit einer oder mehreren Acyloxygruppe(n) in Para- oder Ortho-Stellung zum Phosphoester ist und der Stammwirkstoff nach Umwandlung der Acyloxygruppe durch Esterase in die entsprechende Hydroxygruppe freigesetzt wird.

28. Verwendung einer Prodrug-Verbindung zur Herstellung eines phosphat- oder phosphonathaltigen Stammwirkstoffs, der ein Mono- oder Diester des Phosphats oder Phosphonats ist, für therapeutische Zwecke, wobei die Prodrug durch eine Abspaltungsreaktion zur Bildung einer Kohlenstoff-Kohlenstoff Doppelbindung zu dem phosphorhaltigen Stammwirkstoff abgebaut wird und die Abspaltungsreaktion durch spontane oder enzymatische Freilegung einer Gruppe ausgelöst wird, die dafür bekannt ist, daß sie in hohem Maße Elektronen abgibt, z. B. eine Hydroxygruppe.

29. Prodrug, hergestellt nach einem verfahren gemäß Anspruch 27, für therapeutische Zwecke zur leichteren Angabe phosphorhaltiger Wirkstoffe in Zellen hinein und durch Biomembranen durch Kontaktierung der Zellen mit der Prodrug.

30. Verwendung einer Prodrug, hergestellt nach einem Verfahren gemäß Anspruch 27, zur Herstellung eines Medikaments zur leichteren Abgabe phosphorhaltiger Wirkstoffe in Zellen hinein und durch Biomembranen durch Kontaktierung der Zellen mit der Prodrug.

## Patentansprüche (Patentansprüche für folgende(n) Vertragsstaat(en): ES, GR)

1. Verfahren zur Herstellung einer Prodrug zur leichteren Abgabe phosphorhaltiger Stammwirkstoffe in Zellen hinein und durch Biomembranen, bestehend aus oder enthaltend folgende Schritte:
a) Auswahl eines phosphorhaltigen Stammwirkstoffs; und
b) Umwandlung einer oder mehrerer Hydroxygruppe(n) an jedem Phosphor des Stammwirkstoffs in eine Gruppe mit der Struktur A zur Bildung einer Prodrug, wobei die Struktur A besteht aus oder enthält: 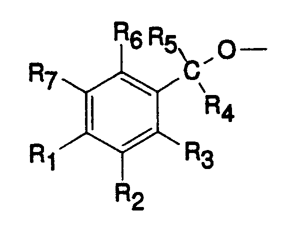 worin
R₁ eine Acyloxygruppe (-O-CO-R8); eine (-O-CO₂-R8) Gruppe;
eine (-O-C(O)-NH₂) Gruppe; eine (-O-C-(O)-NHCH₃) Gruppe; eine (-O-C(O)-N(CH₃)₂) Gruppe; Wasserstoff; ein Halogen; eine (-CO₂R9) Gruppe; eine Alkyl(z. B. Methyl)gruppe; oder eine Hydroxymethylgruppe (HO-CH₂-) sein kann;
R₂ Wasserstoff; eine (-CO₂R10) Gruppe; eine Alkyl (z. B. Methyl)gruppe; ein Halogen; eine Methoxygruppe; eine Acyloxygruppe (-O-CO-R8); oder eine Hydroxymethylgruppe (HO-CH₂-) sein kann;
R₃ eine Acyloxygruppe (-O-CO-R8); eine (-O-CO₂-R8) Gruppe; eine (-O-C(O)-NH₂) Gruppe; eine (-O-C(O)-NHCH₃) Gruppe; eine (-O-C(O)-N(CH₃)₂) Gruppe; Wasserstoff; eine Alkyl(z. B. Methyl)gruppe; eine Hydroxymethylgruppe (-CH₂-OH); ein Halogen; eine Hydroxyethylgruppe (-CH₂-CH₂-OH); oder eine (-CH₂-CO₂-R11) Gruppe sein kann;
R₄ Wasserstoff; eine Alkyl(z. B. Methyl)gruppe; Methoxymethyl (-CH₂-O-CH₃), eine (-CH₂-CO₂-R12) Gruppe; eine (-CH₂-CO-CH₃) Gruppe; eine (-CH(CH₃)-CO₂-R12) Gruppe; eine (-CH₂-CO-NH₂) Gruppe; eine (-CH₂-NO₂) Gruppe; oder eine (-CH₂-SO₂-CH₃) Gruppe, oder ein Methylen sein kann, das mit einer funktionellen Gruppe substituiert ist, die eine Abspaltungsreaktion durch Herabsetzen der inneren Sperre zur Entfernung eines Protons vom Methylen begünstigt;
R₅ Wasserstoff; eine Alkyl(z. B. Methyl)gruppe; Methoxymethyl (-CH₂-O-CH₃); eine (-CH₂-CO₂-R12) Gruppe; eine (-CH₂-CO-CH₃) Gruppe; eine (-CH(CH₃)-CO₂-R12) Gruppe; eine (-CH₂-CO-NH₂) Gruppe; eine (-CH₂-NO₂) Gruppe; oder eine (-CH₂-SO₂-CH₃) Gruppe, oder ein Methylen sein kann, das mit einer funktionellen Gruppe substituiert ist, die eine Abspaltungsreaktion durch Herabsetzen der inneren Sperre zur Entfernung eines Protons vom Methylen begünstigt;
R₆ eine Acyloxygruppe (-O-CO-R8); eine (-O-CO₂-R8) Gruppe; eine (-O-C(O)-NH₂) Gruppe; eine (-O-C(O)-NHCH₃) Gruppe; eine (-O-C(O)-N(CH₃)₂) Gruppe; Wasserstoff; eine Alkyl(z. B. Methyl)gruppe; eine Hydroxymethylgruppe (-CH₂-OH); ein Halogen; eine Hydroxyethylgruppe (-CH₂-CH₂-OH); oder eine (-CH₂-CO₂-R11) Gruppe sein kann;
R₇ Wasserstoff; eine (-CO₂-R10) Gruppe; eine Alkyl (z. B. Methyl)gruppe; ein Halogen; oder eine Methoxygruppe; eine Acyloxygruppe (-O-CO-R8); oder eine Hydroxymethylgruppe (HO-CH₂-) sein kann,
worin R8 eine Gruppe mit folgender Struktur sein kann: (-(CH₂)_{N}-CH₃), wobei N = 0 - 18 ist; R8 eine Phenylgruppe sein kann, wobei die Phenylgruppe ihrerseits Substituenten haben kann; R8 so gewählt sein kann, daß R8-CO₂H eine Aminosäure; Milchsäure; Glykolsäure (HO-CH₂-CO₂H); Glyzerinsäure (HO-CH₂-CH(OH)-CO₂H); oder Azetessigsäure (-CH₃COCH₂-CO₂H) ist;
worin R9 und R10, R11 und R12 eine Methyl-, Ethyl-, Phenyl- oder Benzylgruppe ist;
und
worin mindestens eine der folgenden Gruppen: R1; R3; R6 eine Acyloxygruppe (-O-CO-R8) oder eine andere Gruppe ist, die durch *in vivo* Biotransformation schließlich eine Hydroxygruppe am Phenylring ergibt.

2. Verfahren gemäß Anspruch 1, wobei der Stammwirkstoff
(a) ein Monophosphatesterwirkstoff; oder
(b) ein Phosphodiesterwirkstoff; oder
(c) ein Phosphonsäurewirkstoff; oder
(d) ein Phosphonatwirkstoff; oder
(e) ein Phosphinsäurewirkstoff; oder
(f) ein Nukleotidmonophosphatwirkstoff; oder
(g) ausgewählt ist aus der Gruppe bestehend aus:
9-(2-Phosphonylmethoxy-ethyl)adenin (PMEA);
9-(RS)-(3-Fluor-2-phosphonylmethoxypropyly)adenin (FPMPA),
9-(RS)-(3-Fluor-2-phosphonylmethoxypropyly)2,6, diaminopurin
3'Azido-dideoxythymidin 5'monophosphat (AZT-Phosphat);
2',3'-Didehydro-2',3'dideoxythymidin 5'monophosphat (D4T-Phosphat);
2',3'-Dideoxyadenosin 5'monophosphat;
einem Carboxylatester von Phosphonoformiat.

3. Verfahren gemäß Anspruch 1 oder 2, wobei
(a) die Struktur der Gruppe 'A' ausgewählt ist aus der Gruppe bestehend aus: 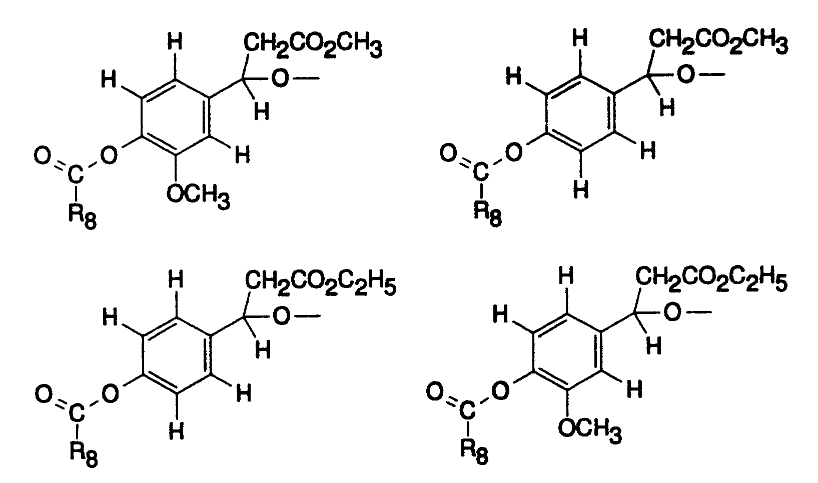 worin R8 eine Gruppe mit der Struktur (-(CH₂)_{N}-CH₃) sein kann, wobei N = 0 - 18 ist; R8 eine Phenylgruppe sein kann, wobei die Phenylgruppe ihrerseits Substituenten haben kann; R8 so gewählt sein kann, daß R8-CO₂H eine Aminosäure; Milchsäure; Glykolsäure (HO-CH₂-CO₂H); Glyzerinsäure (HO-CH₂-CH(OH)-CO₂H); oder Azetessigsäure (-CH₃COCH₂-CO₂H) ist; oder worin R8 eine beliebige Gruppe ist, die so gewählt ist, daß die erhaltene Prodrug eine *in vivo* Biotransformation oder spontane Transformation durchmacht, um schließlich eine Hydroxygruppe am Phenylring zu ergeben; oder
(b) die Gruppe 'A' folgende Struktur hat:

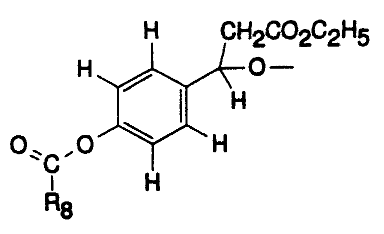

4. Verfahren gemäß Anspruch 1 zur Herstellung einer Prodrug zur leichteren Angabe von Phosphonoformiat in Zellen hinein und durch Biomembranen, bestehend aus oder enthaltend die Herstellung einer Prodrug mit folgender Struktur: 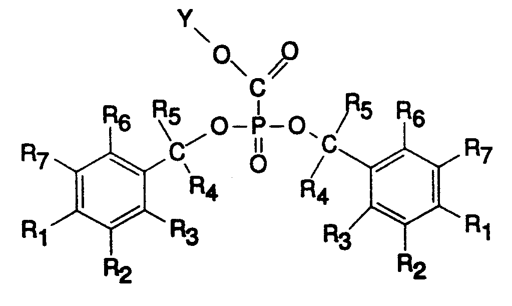 worin Y ein Benzyl, Phenyl ist, wobei die Benzyl- oder Phenylgruppe ihrerseits Substituenten haben kann.

5. Verfahren gemäß Anspruch 4, bestehend aus oder enthaltend die Herstellung einer Prodrug mit folgender Struktur:

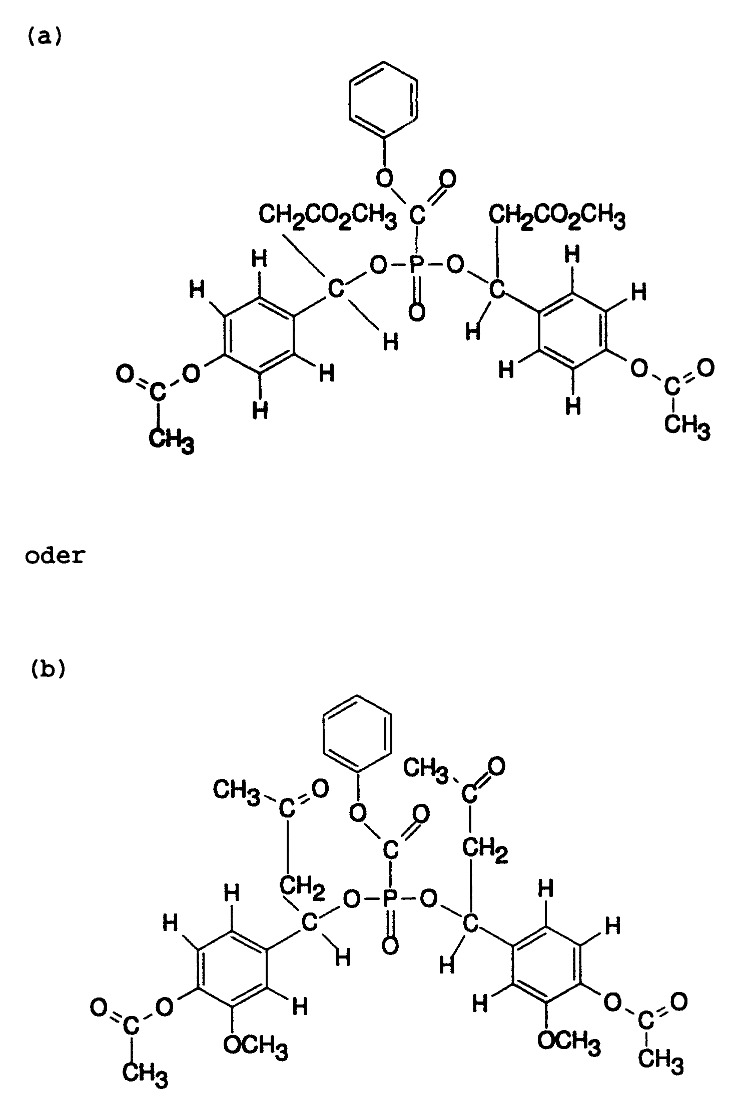

6. Verfahren gemäß Anspruch 1, wobei der phosphorhaltige Wirkstoff und die Struktur A so gewählt sind, daß folgende Prodrug erhalten wird:

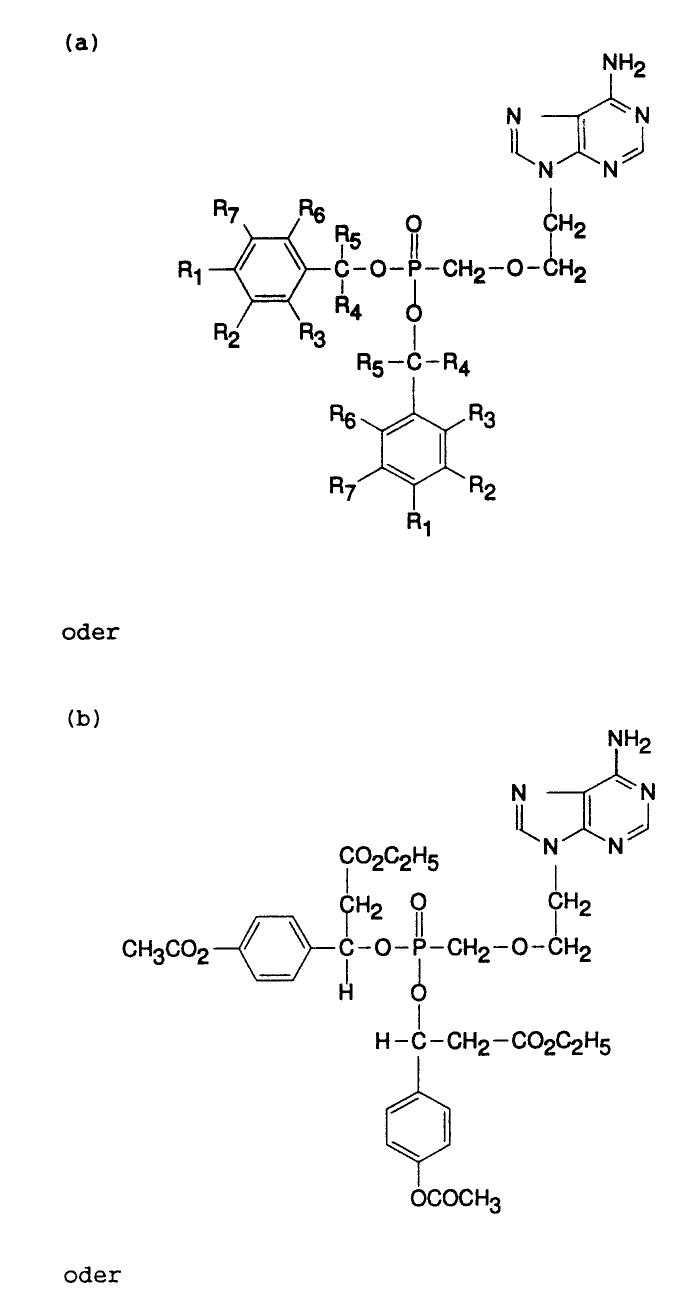

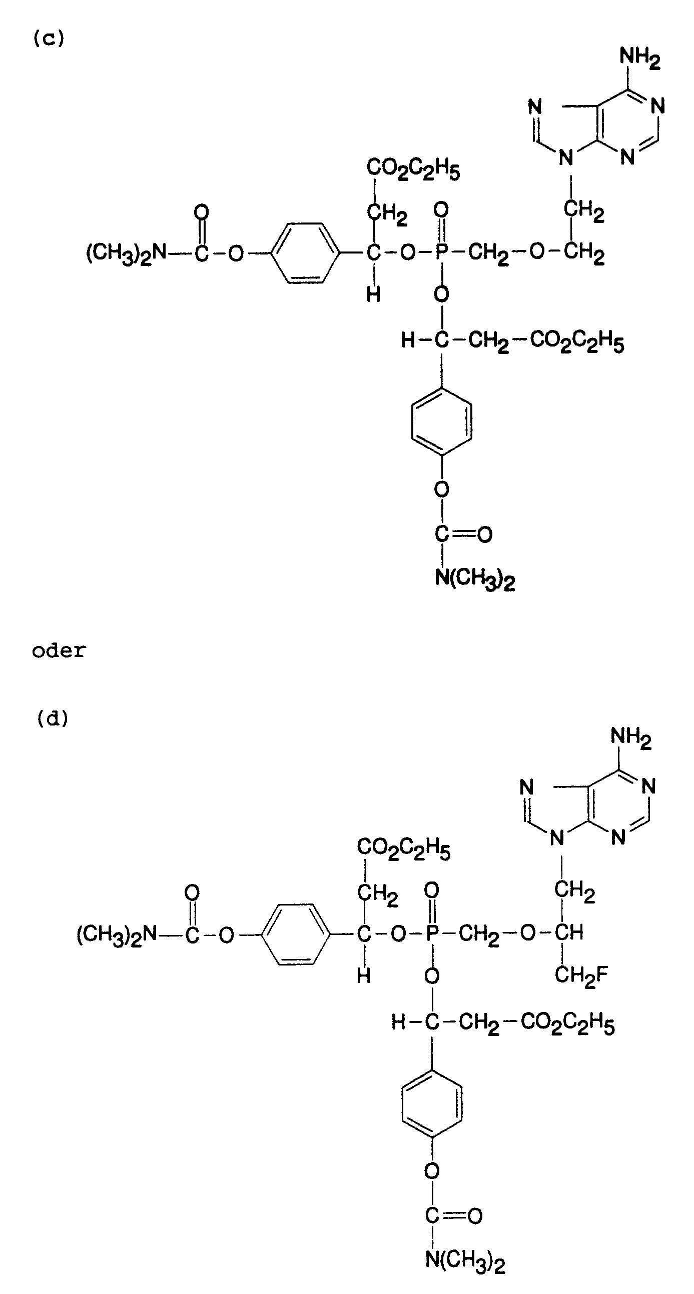

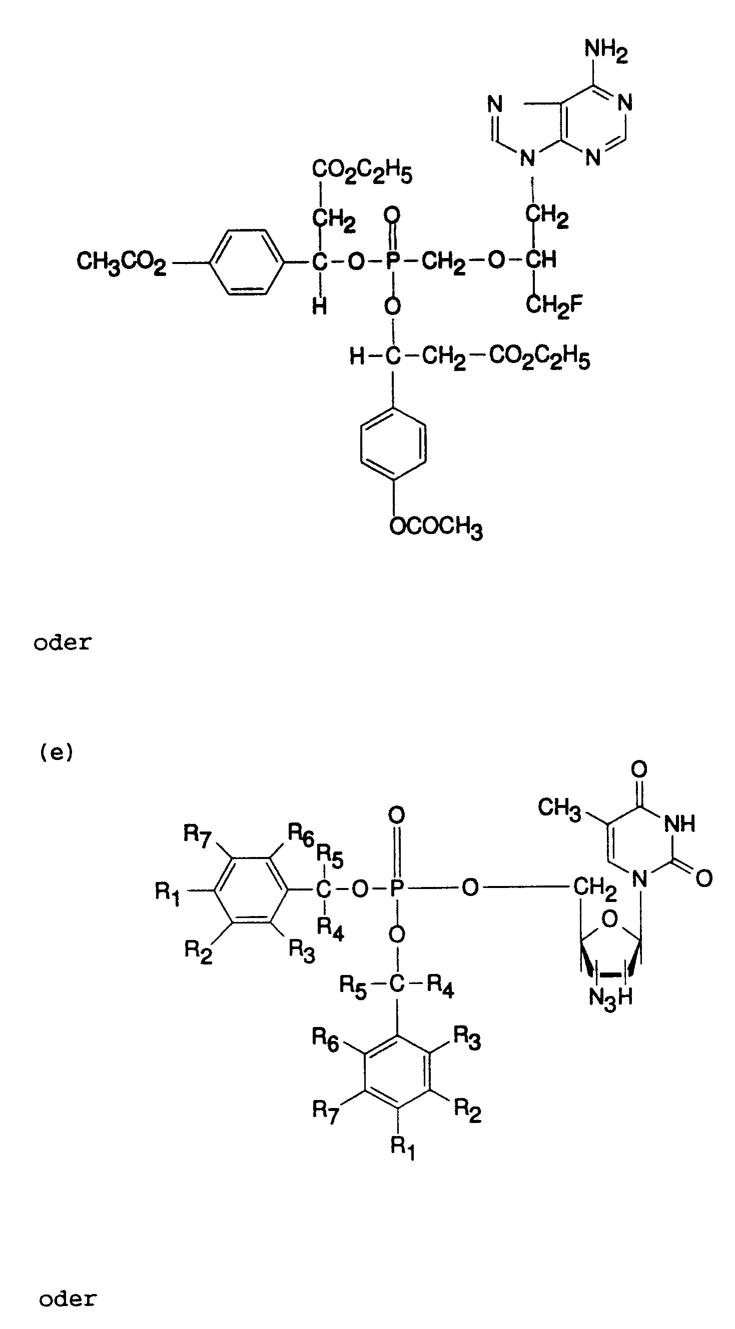

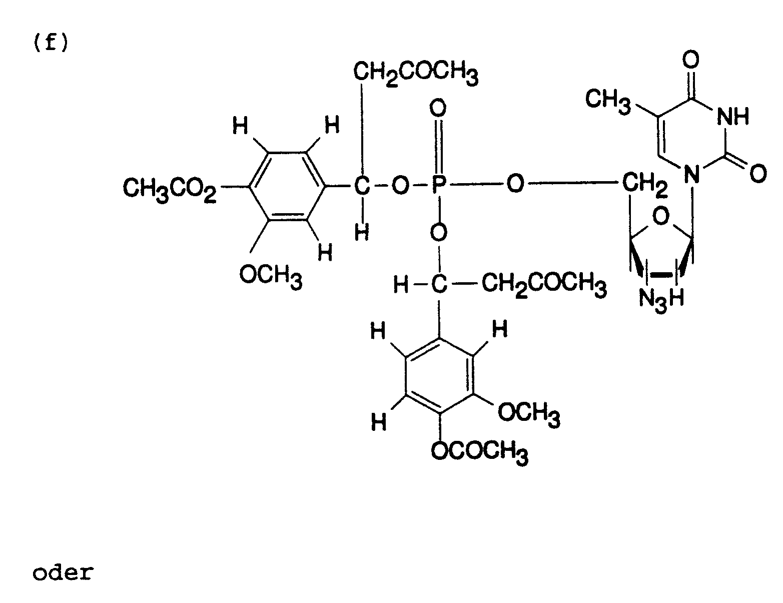

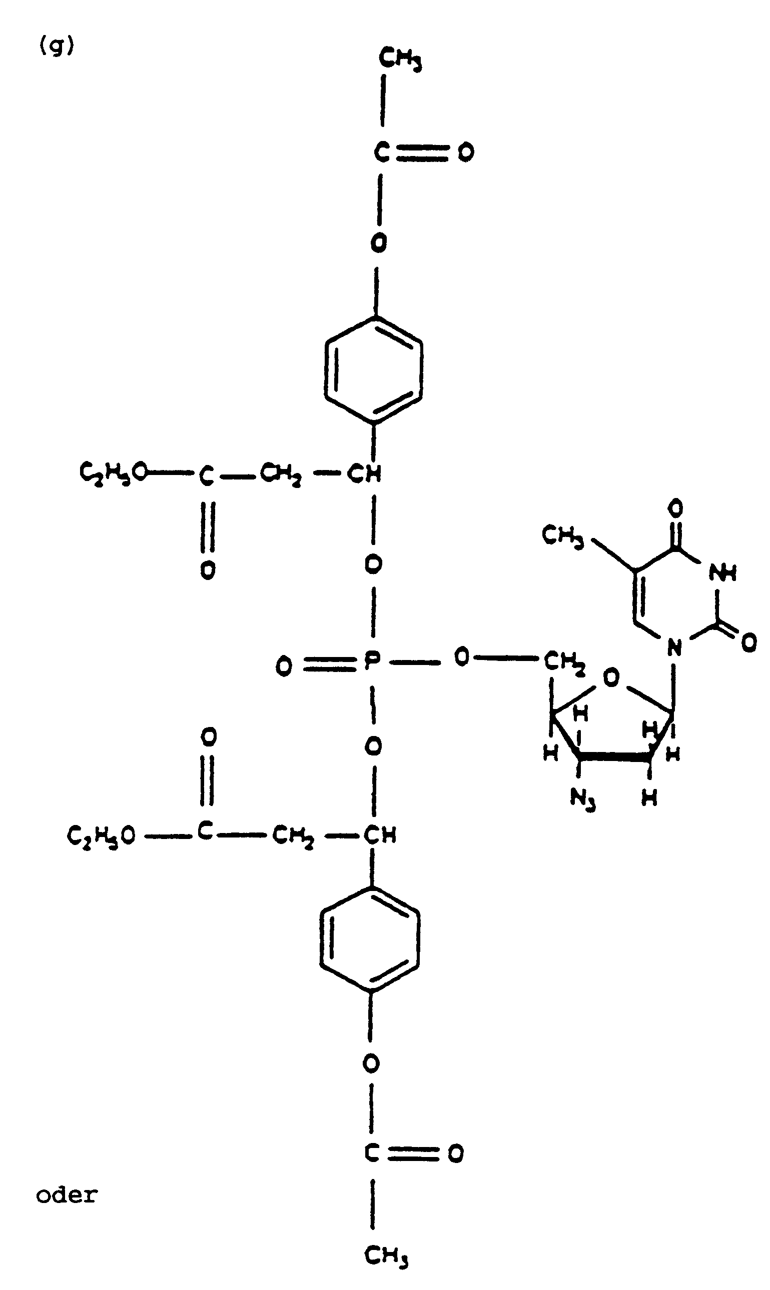

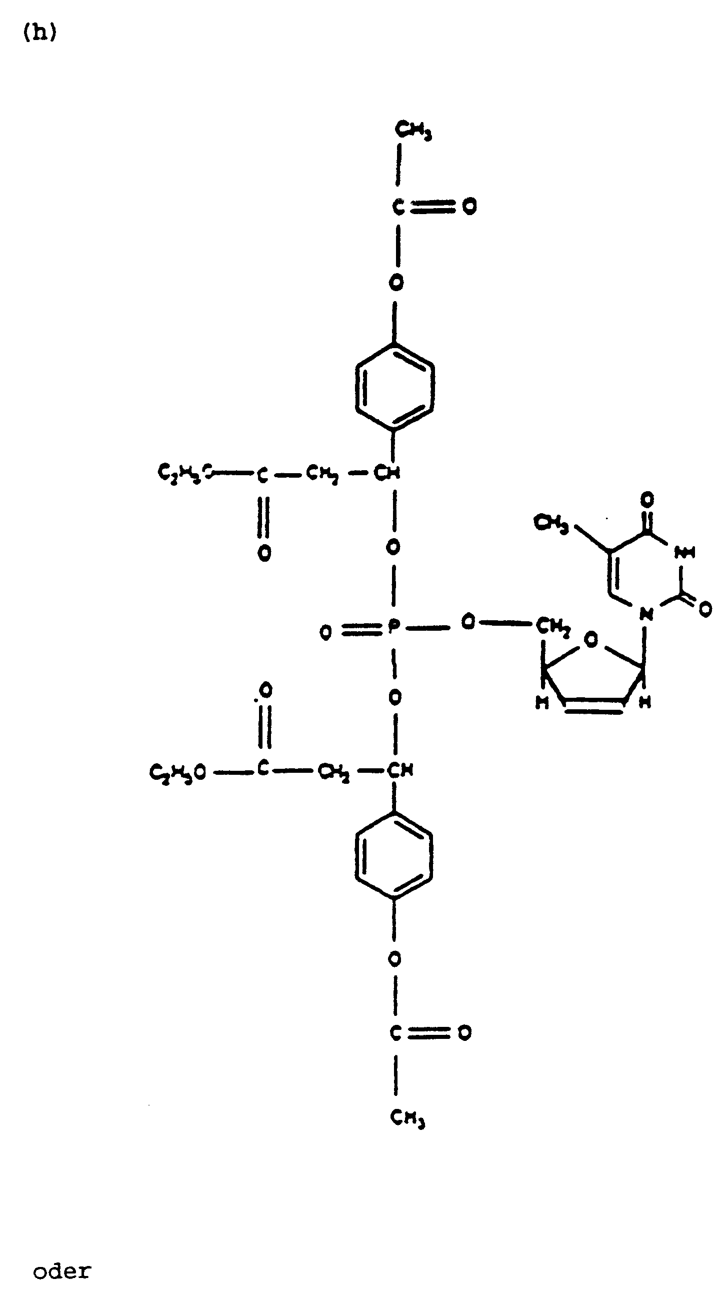

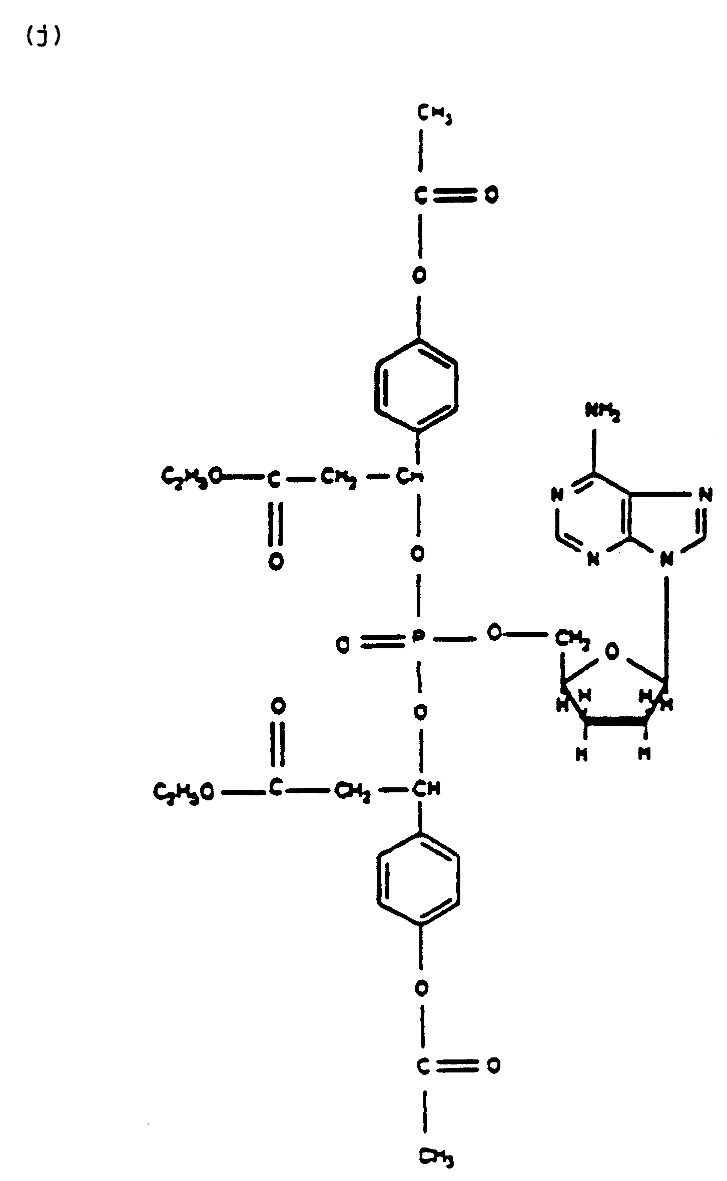

7. Verfahren zur Herstellung einer Prodrug zur leichteren Abgabe phosphorhaltiger Stammwirkstoffe in Zellen hinein und durch Biomembranen, bestehend aus oder enthaltend folgende Schritte:
a) Auswahl eines phosphorhaltigen Wirkstoffe; und
b) Herstellung eines Mono- oder Bisphosphoesters aus einem entsprechend aktivierten Derivat des Stammwirkstoffs und einem Alkohol mit der Struktur A zur Bildung einer Prodrug, wobei die Struktur A besteht aus oder enthält: 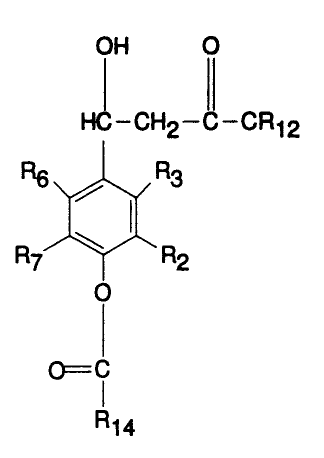 worin
R2 und R7 Wasserstoff, eine (-CO₂R10) Gruppe, eine Methylgruppe, ein Halogen oder eine Methoxygruppe oder eine Hydroxymethylgruppe (HO-CH₂-) sein können, wobei R10 eine Methyl- oder Ethylgruppe sein kann;
R3 und R6 Wasserstoff, eine Methylgruppe; eine Hydroxymethylgruppe (-CH₂-OH); ein Halogen; eine Hydroxyethylgruppe (-CH₂-CH₂-OH) sein können;
R12 eine Methyl-, Ethyl-, Phenyl- oder Benzylgruppe ist;
R14 eine Gruppe mit der Struktur (-(CH₂)_{N}-CH₃) sein kann, wobei N = 0 - 18 ist; eine Phenylgruppe sein kann, wobei die Phenylgruppe ihrerseits Substituenten haben kann;
R14 so gewählt sein kann, daß R14-CO₂H eine Aminosäure; Milchsäure; Glykolsäure (HO-CH₂-CO₂H); Glyzerinsäure (HO-CH₂-CH(OH)-CO₂H); oder Azetessigsäure (-CH₃COCH₂-CO₂H) ist;
worin R14 (-O-(CH₂)_{N}-CH₃) sein kann, wobei N = 0 - 18 ist; eine Phenyloxygruppe sein kann, wobei die Phenyloxygruppe ihrerseits Substituenten haben kann; und worin R14 eine der folgenden Gruppen sein kann: (-NH₂); (-NHCH₃); oder (-N(CH₃)₂).

8. Verfahren gemäß Anspruch 7, wobei die Struktur A Ethyl-3-hydroxy-3-(4-acetoxyphenyl)propionat ist:

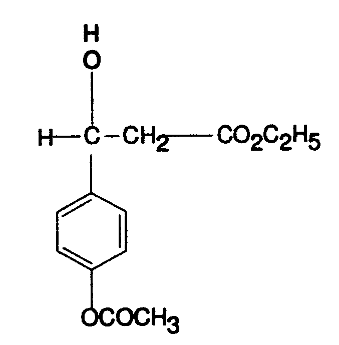

9. Prodrug, hergestellt nach einem Verfahren gemäß einem der Ansprüche 1 bis 8, für therapeutische Zwecke zur leichteren Abgabe phosphorhaltiger Wirkstoffe in Zellen hinein und durch Biomembranen durch Kontaktierung der Zellen und Biomembranen mit der Prodrug.

10. Verwendung einer Prodrug, hergestellt nach einem Verfahren gemäß einem der Ansprüche 1 bis 8, zur Herstellung eines Medikaments zur leichteren Abgabe phosphorhaltiger Wirkstoffe in Zellen hinein und durch Biomembranen durch Kontaktierung der Zellen und Biomembranen mit der Prodrug.

11. Verfahren zur Herstellung einer Prodrug zur leichteren Abgabe phosphorhaltiger Stammwirkstoffe in Zellen hinein und durch Biomembranen, bestehend aus oder enthaltend folgende Schritte:
a) Auswahl eines phosphorhaltigen Stammwirkstoffs; und
b) Umwandlung einer oder mehrerer Hydroxygruppe(n) an jedem Phosphor des Stammwirkstoffs in eine Gruppe mit der Struktur A zur Bildung einer Prodrug, wobei die Gruppe 'A' ein Benzyloxy-Derivat mit einer oder mehreren Acyloxygruppe(n) in Para- oder Ortho-Stellung zum Phosphoester ist und der Stammwirkstoff nach Umwandlung der Acyloxygruppe durch Esterase in die entsprechende Hydroxygruppe freigesetzt wird.

12. Verwendung einer Prodrug-Verbindung zur Herstellung eines phosphat- oder phosphonathaltigen Stammwirkstoffs, der ein Mono- oder Diester des Phosphats oder Phosphonats ist, für therapeutische Zwecke, wobei die Prodrug durch eine Abspaltungsreaktion zur Bildung einer Kohlenstoff-Kohlenstoff Doppelbindung zu dem phosphorhaltigen Stammwirkstoff abgebaut wird und die Abspaltungsreaktion durch spontane oder enzymatische Freilegung einer Gruppe ausgelöst wird, die dafür bekannt ist, daß sie in hohem Maße Elektronen abgibt, z. B. eine Hydroxygruppe.

13. Verwendung einer Prodrug, hergestellt nach einem Verfahren gemäß Anspruch 11, zur Herstellung eines Medikaments zur leichteren Abgabe phosphorhaltiger Wirkstoffe in Zellen hinein und durch Biomembranen durch Kontaktierung der Zellen mit der Prodrug.

## Revendications (Revendications pour l'(les) Etat(s) contractant(s) suivant(s): AT, BE, CH, LI, DE, DK, FR, GB, IT, LU, NL, SE)

1. Promédicament d'un médicament parent où le médicament parent a la structure générale : 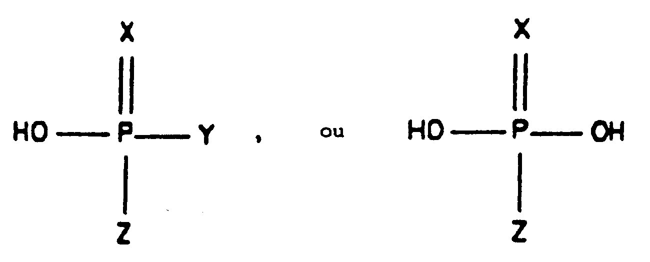 où X est un atome d'oxygène (O) ou de soufre (S) et Y et Z sont définis par le reste du médicament parent ; et où le médicament parent n'est pas l'acide phosphorique ; où le promédicament a la structure suivante : 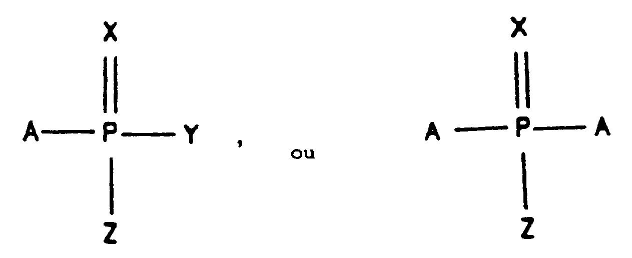 où le groupe "A" a la structure suivante : 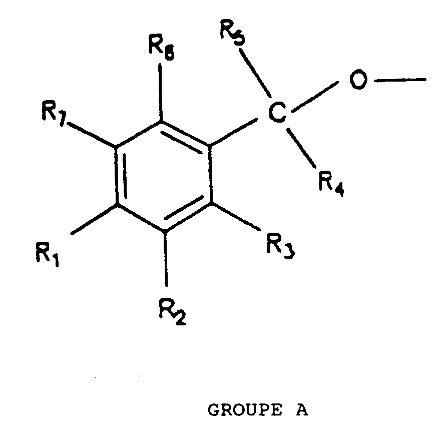 où
R₁ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un atome d'halogène ; un groupe (-CO₂R₉) ; un groupe alkyle (par exemple méthyle) ; ou un groupe hydroxyméthyle (HO-CH₂-) ;
R₂ peut être un atome d'hydrogène ; un groupe (-CO₂R₁₀) ; un groupe alkyle (par exemple méthyle) ; un atome d'halogène ; un groupe méthoxy ; un groupe acyloxy (-O-CO-R₈) ; ou un groupe hydroxyméthyle (HO-CH₂-) ;
R₃ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un groupe alkyle (par exemple méthyle) ; un groupe hydroxyméthyle (-CH₂-OH) ; un atome d'halogène ; un groupe hydroxyéthyle (-CH₂-CH₂-OH) ; ou un groupe (-CH₂-CO₂-R₁₁) ;
R₄ peut être un atome d'hydrogène ; un groupe alkyle (par exemple méthyle) ; un groupe méthoxyméthyle (-CH₂-O-CH₃) ; un groupe (-CH₂-CO₂-R₁₂) ; un groupe (-CH₂-CO-CH₃) ; un groupe (-CH(CH₃)-CO₂-R₁₂) ; un groupe (-CH₂-CO-NH₂) ; un groupe (-CH₂-NO₂) ; ou un groupe (-CH₂-SO₂-CH₃) ; ou un groupe méthylène substitué avec un groupe fonctionnel qui favorise une réaction d'élimination en abaissant la barrière intrinsèque pour l'enlèvement d'un proton dudit groupe méthylène ;
R₅ peut être un atome d'hydrogène ; un groupe alkyle (par exemple méthyle) ; un groupe méthoxyméthyle (-CH₂-O-CH₃) ; un groupe (-CH₂-CO₂-R₁₂) ; un groupe (-CH₂-CO-CH₃) ; un groupe (-CH(CH₃)-CO₂-R₁₂) ; un groupe (-CH₂-CO-NH₂) ; un groupe (-CH₂-NO₂) ; ou un groupe (-CH₂-SO₂-CH₃) ; ou un groupe méthylène substitué avec un groupe fonctionnel qui favorise une réaction d'élimination en abaissant la barrière intrinsèque pour l'enlèvement d'un proton dudit groupe méthylène ;
R₆ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un groupe alkyle (par exemple méthyle) ; un groupe hydroxyméthyle (-CH₂-OH) ; un atome d'halogène ; un groupe hydroxyéthyle (-CH₂-CH₂-OH) ; ou un groupe (-CH₂-CO₂-R₁₁) ;
R₇ peut être un atome d'hydrogène ; un groupe (-CO₂R₁₀) ; un groupe alkyle (par exemple méthyle) ; un atome d'halogène ; un groupe méthoxy ; un groupe acyloxy (-O-CO-R₈) ; ou un groupe hydroxyméthyle (HO-CH₂-) ;
où R₈ peut être un groupe de structure suivante : (-(CH₂)_{N}-CH₃) pour N = 0-18 ; R₈ peut être un groupe phényle, ce groupe phényle pouvant lui-même porter des substituants, où R₈ peut également être choisi de telle sorte que R₈-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; où R₉, et R₁₀, R₁₁, et R₁₂, sont un groupe méthyle, un groupe éthyle, un groupe phényle ou un groupe benzyle ; et
où au moins un des groupes suivants : R₁ ; R₃ ; et R₆ est un groupe acyloxy (-O-CO-R₈) ou un autre groupe qui subit une biotransformation in vivo pour former finalement un groupe hydroxy sur le cycle phényle.

2. Promédicament selon la revendication 1, où le médicament parent est
(a) un médicament ester monophosphate ; ou
(b) un médicament phosphodiester ; ou
(c) un médicament acide phosphonique ou un médicament phosphonate ; ou
(d) un médicament acide phosphinique ; ou
(e) un médicament nucléotide monophosphate ; ou est
(f) choisi dans le groupe constitué par :
la 9-(2-phosphonylméthoxyéthyl)adénine, (PMEA) ; la
9-(RS)-(3-fluoro-2-phosphonylméthoxypropyl)adénine, (FPMPA) ; la
9-(RS)-(3-fluoro-2-phosphonylméthoxypropyl)-2,6-diaminopurine, (FPMPDAP) ; le 3'-azido-didésoxythymidine 5'-monophosphate, (AZT-phosphate) ; le 2',3'-didéhydro-2',3'-didésoxythymidine 5'-monophosphate (D4T-phosphate) ; le 2',3'-didésoxyadénosine 5'-monophosphate ; et un ester carboxylate de phosphonoformiate.

3. Promédicament selon la revendication 1, où la structure du groupe "A" est choisie parmi l'ensemble constitué par :

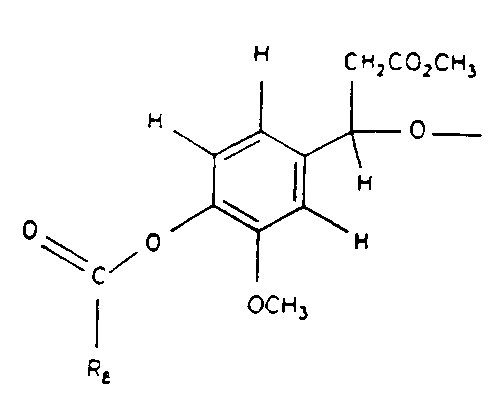

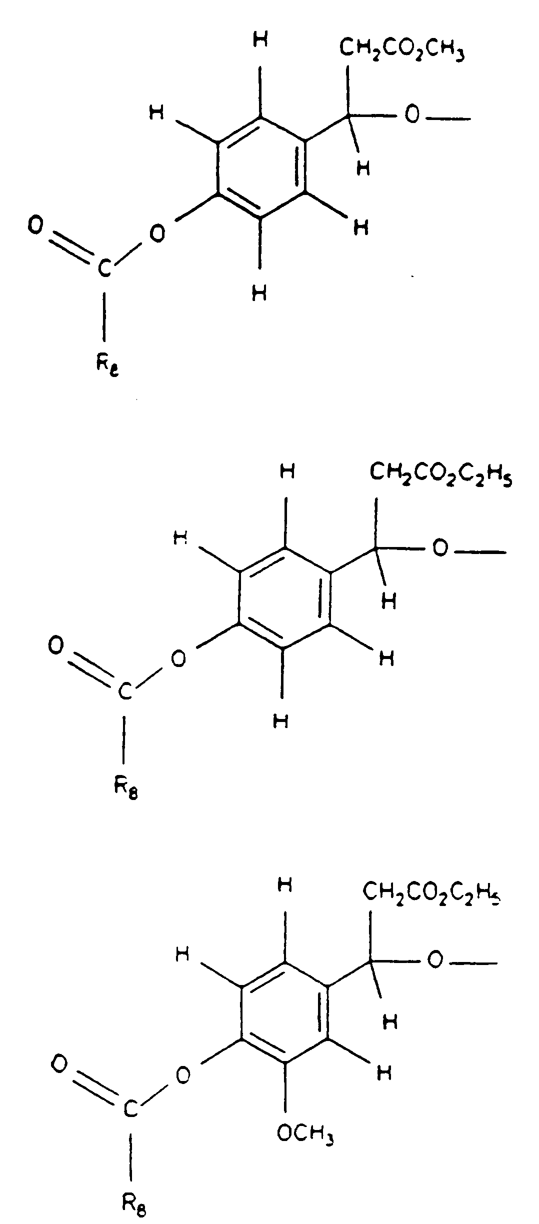

4. Promédicament selon la revendication 3, où le médicament parent est :
(a) un nucléotide monophosphate ; ou est
(b) choisi parmi le groupe suivant :
la 9-(2-phosphonylméthoxyéthyl)adénine, (PMEA) ; la
9-(RS)-(3-fluoro-2-phosphonylméthoxypropyl)adénine, (FPMPA) ; la
9-(RS)-(3-fluoro-2-phosphonylméthoxypropyl)-2,6-diaminopurine, (FPMPDAP) ; le 3'-azido-didésoxythymidine 5'-monophosphate, (AZT-phosphate) ; le 2',3'-didéhydro-2',3'-didésoxythymidine 5'-monophosphate (D4T-phosphate) ; le 2',3'-didésoxyadénosine 5'-monophosphate ; ou est
(c) un ester carboxylate de phosphonoformiate.

5. Promédicament selon la revendication 1 où le groupe "A" a la structure suivante :

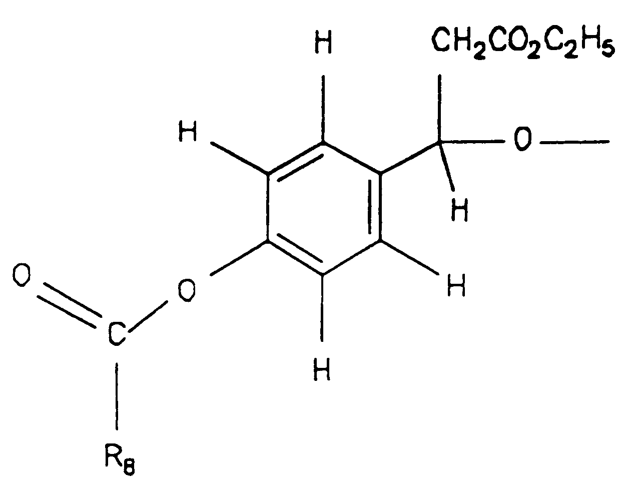

6. Promédicament selon la revendication 5, où le médicament parent est un nucléotide monophosphate ; ou
est choisi dans le groupe suivant :
la 9-(2-phosphonylméthoxyéthyl)adénine, (PMEA) ; la 9-(RS)-(3-fluoro-2-phosphonylméthoxypropyl)adénine, (FPMPA) ; la 9-(RS)-(3-fluoro-2-phosphonylméthoxypropyl)-2,6-diaminopurine, (FPMPDAP) ; le 3'-azido-didésoxythymidine 5'-monophosphate ; le 2',3'-didéhydro-2',3'-didésoxythymidine 5'-monophosphate (D4T-phosphate) ; le 2',3'-didésoxyadénosine 5'-monophosphate ; et un ester carboxylate de phosphonoformiate.

7. Promédicament selon la revendication 1 ayant la structure suivante : 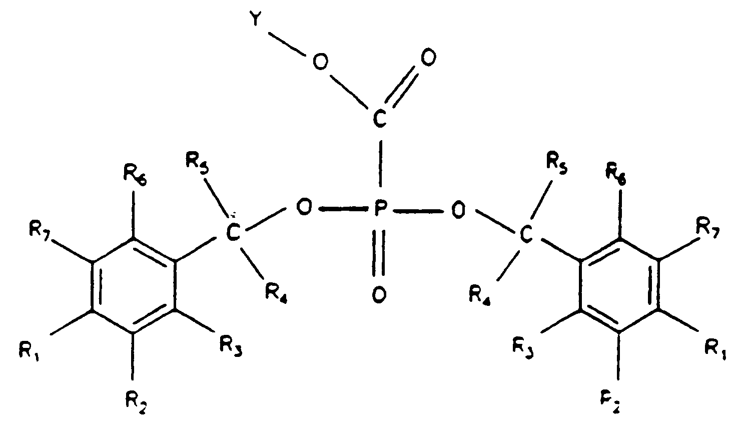 où Y est un groupe benzyle ou un groupe phényle et où le groupe benzyle ou le groupe phényle peut porter des substitants.

8. Promédicament selon la revendication 7 ayant la structure :

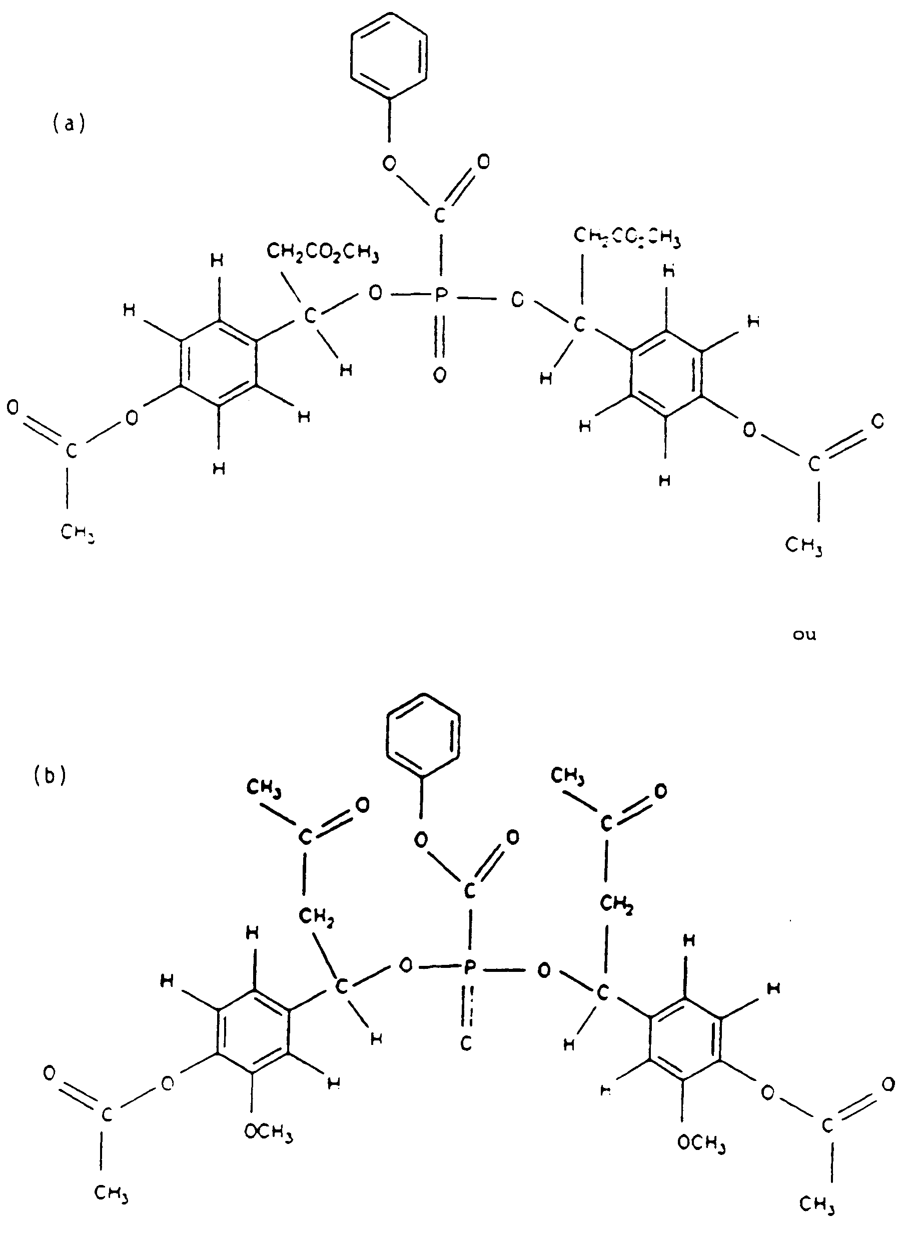

9. Promédicament selon la revendication 1 ayant la structure suivante :

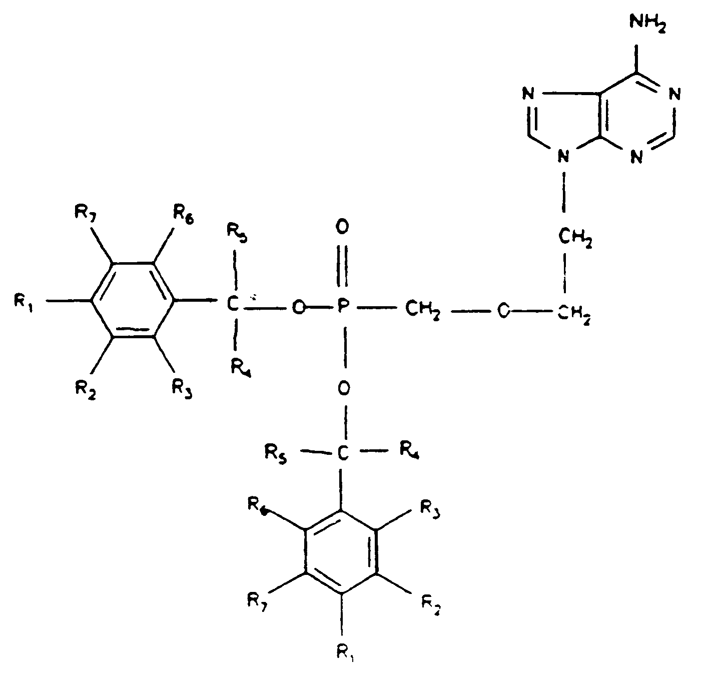

10. Promédicament selon la revendication 9 ayant la structure :

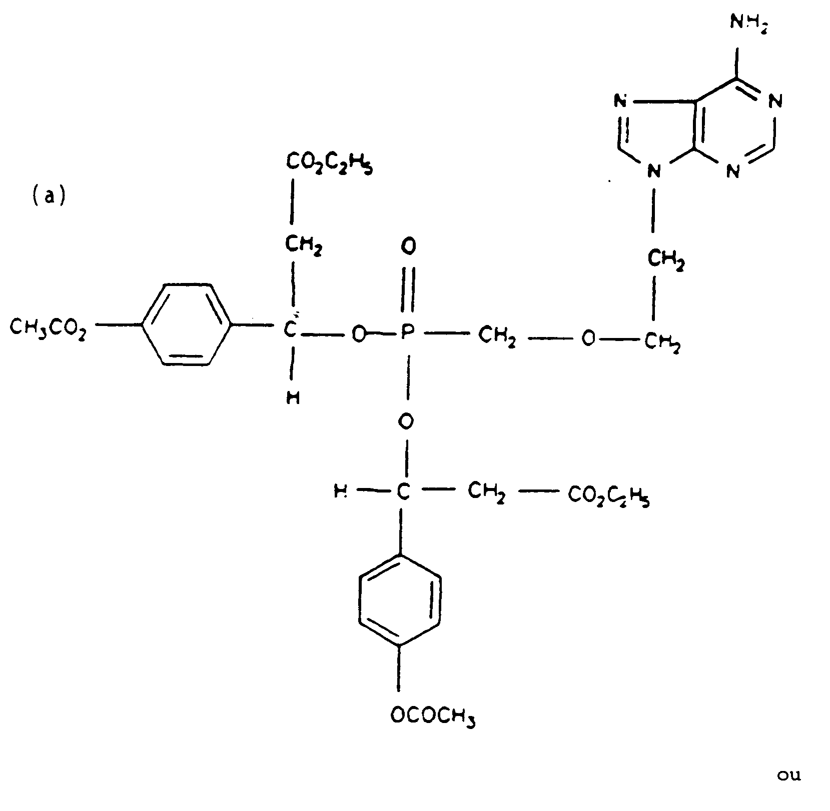

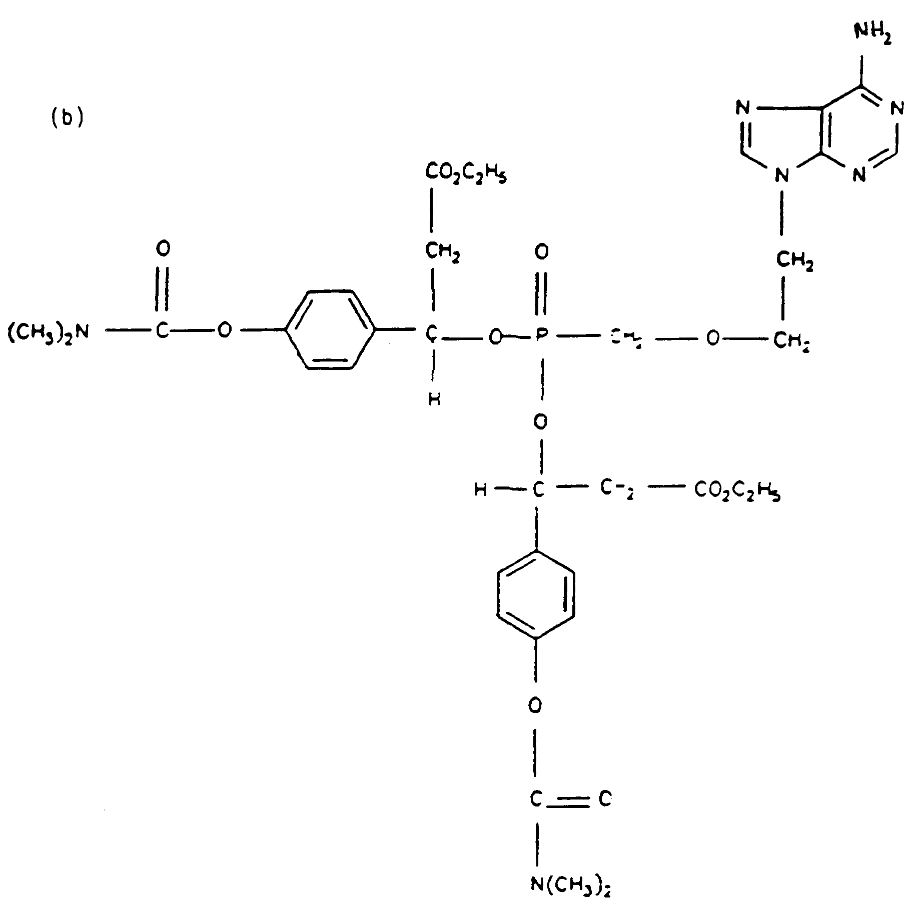

11. Promédicament selon la revendication 1 ayant les structures suivantes :

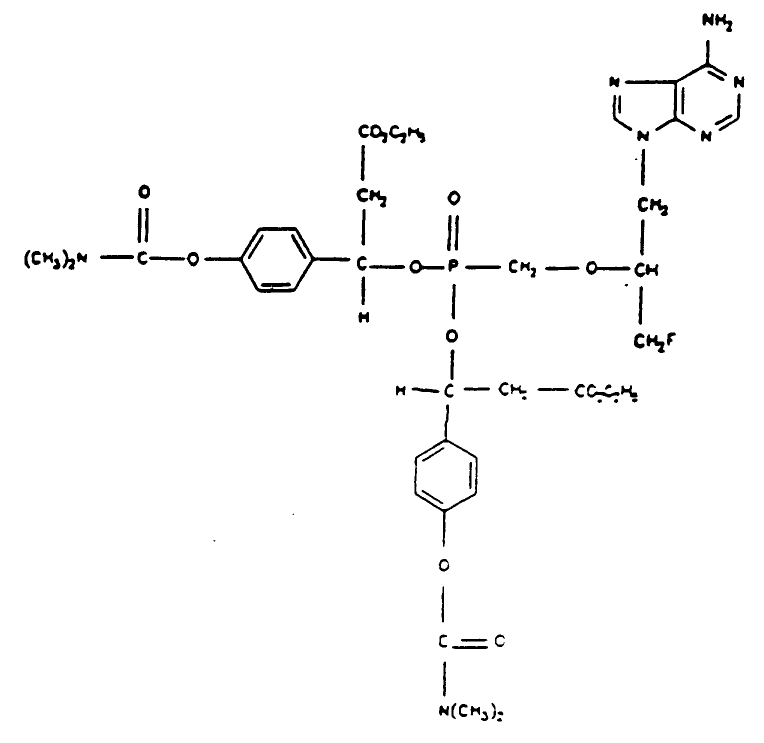

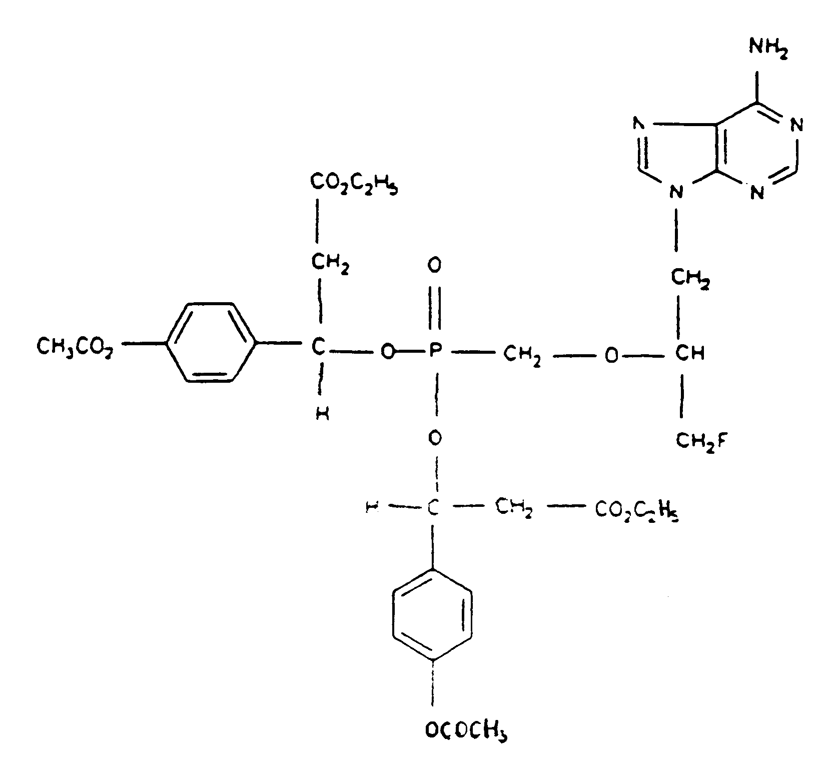

12. Promédicament selon la revendication 1, ayant la structure suivante : 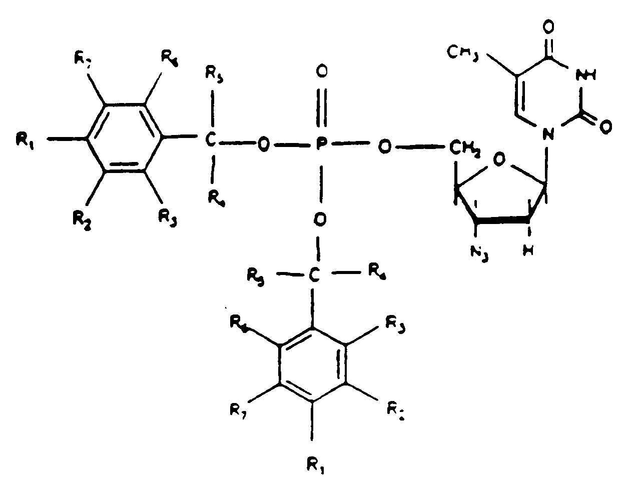 par exemple :

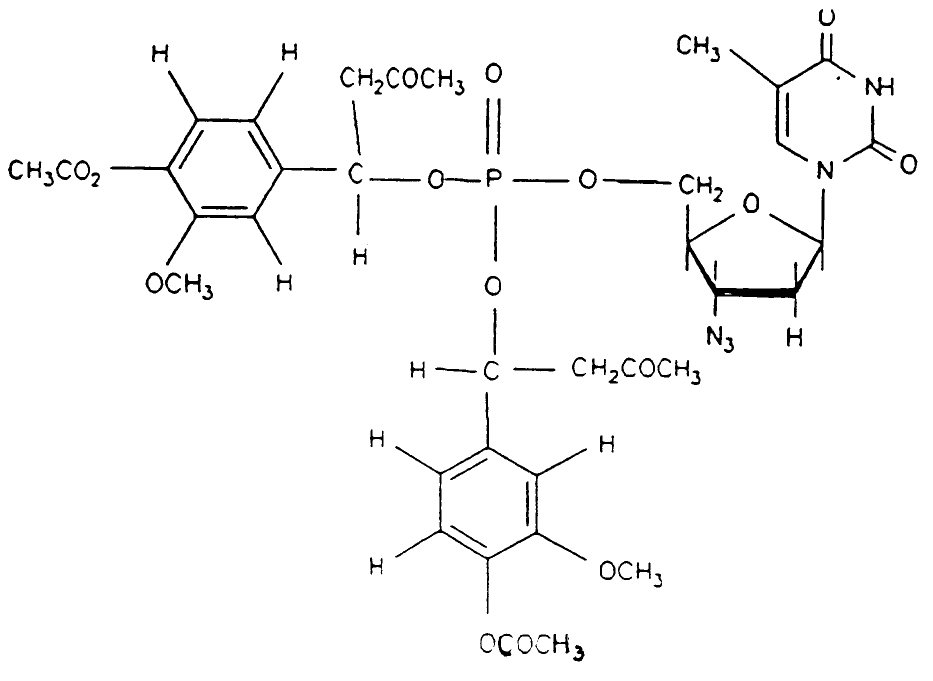

13. Promédicament selon la revendication 1, ayant la structure :

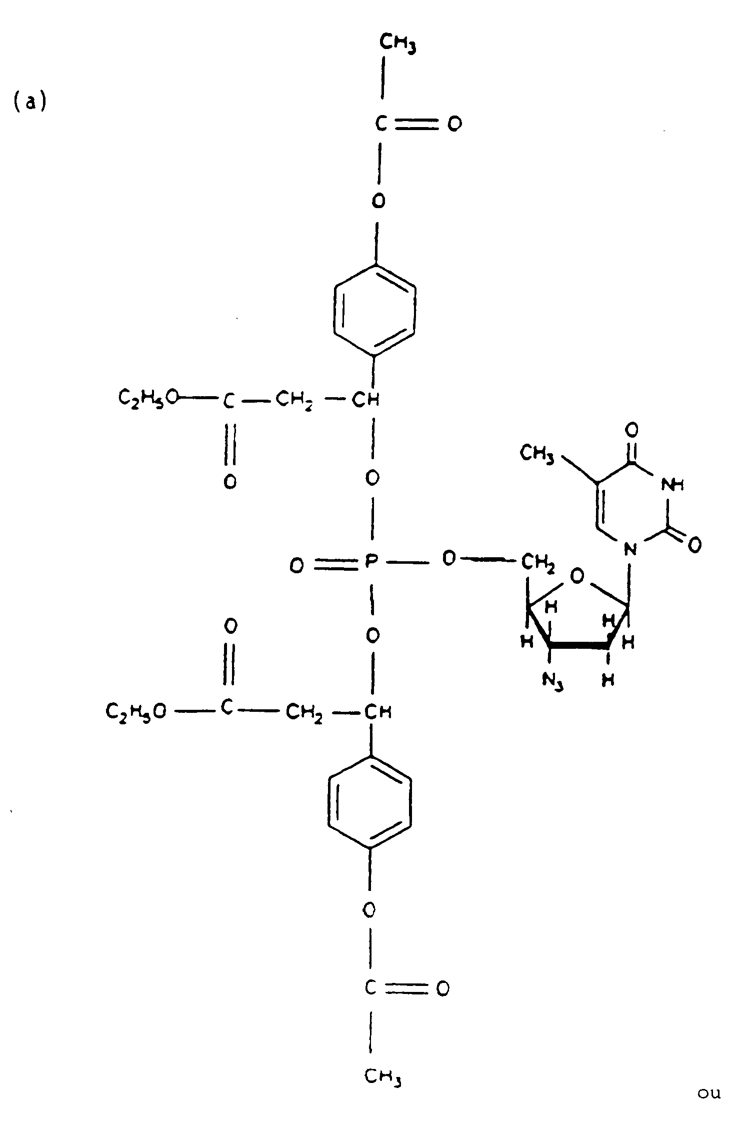

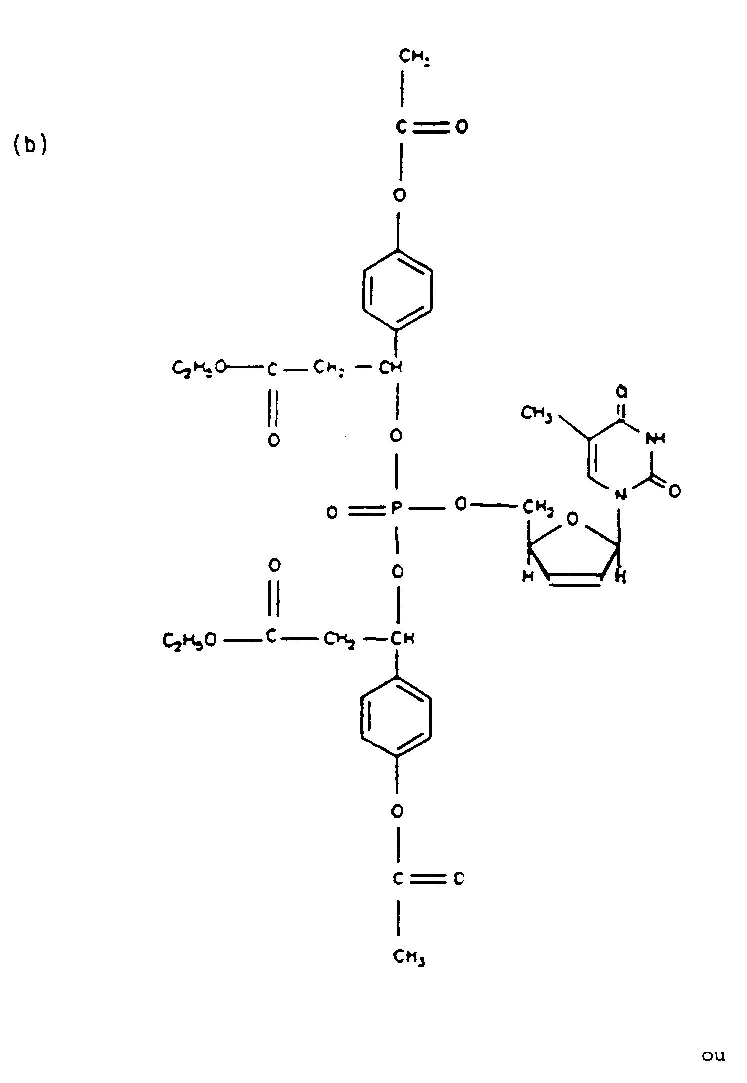

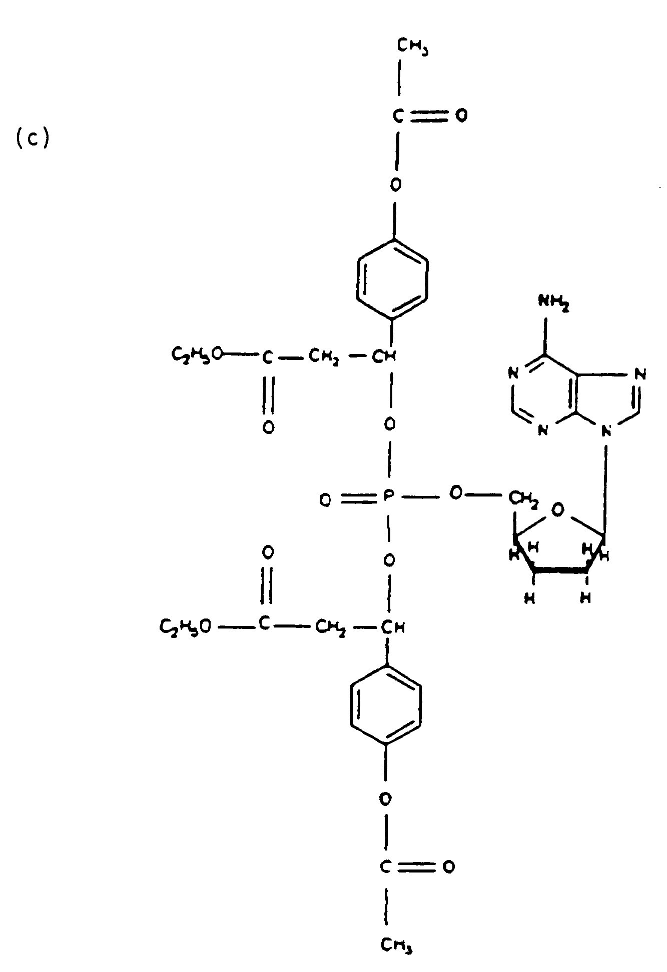

14. Composé utile comme intermédiaire dans la synthèse d'un promédicament, de structure suivante : 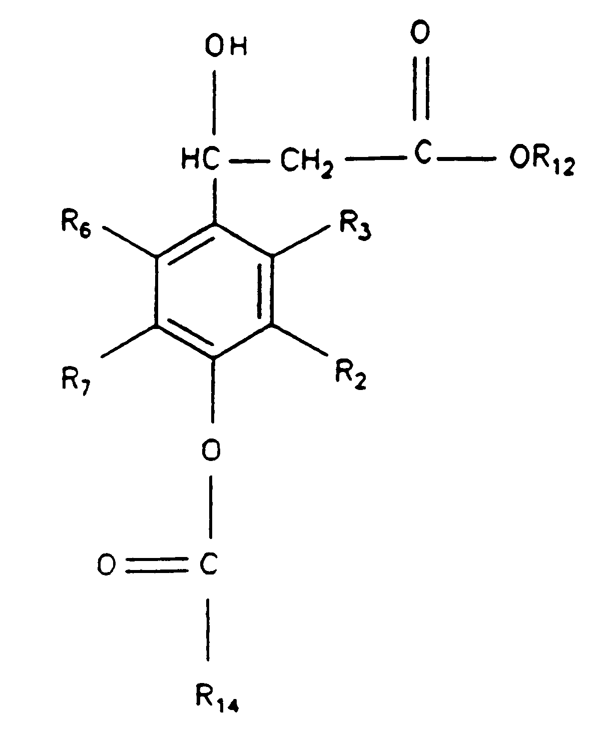 où R₂ et R₇ peuvent être un atome d'hydrogène, un groupe (-CO₂R₁₀), un groupe méthyle, un atome d'halogène ou un groupe méthoxy, ou un groupe hydroxyméthyle (HO-CH₂-) ; où R₁₀ peut être un groupe méthyle ou éthyle ;
où R₃ et R₆ peuvent être un atome d'hydrogène ; un groupe méthyle ; un groupe hydroxyméthyle (-CH₂-OH) ; un atome d'halogène ; ou un groupe hydroxyéthyle (-CH₂-CH₂-OH) ;
où R₁₂ est un groupe méthyle, éthyle, phényle ou benzyle ;
où R₁₄ peut être un groupe de structure suivante : (-(CH₂)_{N}-CH₃) pour N = 0-18 ; un groupe phényle, le groupe phényle pouvant lui-même porter des substituants ; où R₁₄ peut être choisi pour que R₁₄-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; où R₁₄ peut être (-O-(CH₂)_{N}-CH₃) pour N = 0-18 ; un groupe phényloxy, lequel groupe phényloxy peut lui-même porter des substituants ; et où R₁₄ peut être un des groupes suivants : (-NH₂) ; (-NHCH₃) ; ou (-N(CH₃)₂).

15. Composé selon la revendication 14 ayant la structure suivante : 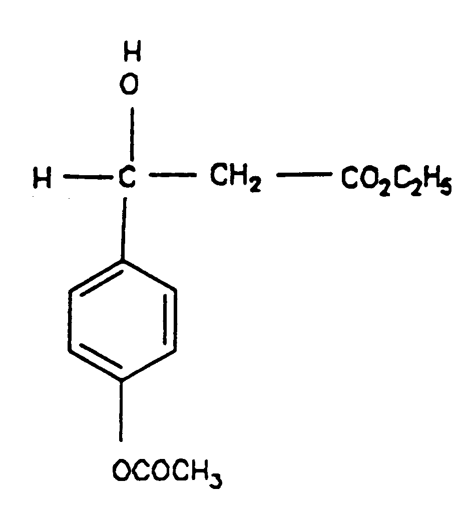 3-hydroxy-3-(4-acétoxyphényl)propionate d'éthyle

16. Procédé pour la préparation d'un promédicament pour faciliter l'apport des médicaments parents portant un groupe phosphoreux dans des cellules et à travers des membranes biologiques, comprenant les étapes consistant à :
a. choisir un médicament parent portant un groupe phosphoreux ; et
b. transformer un ou plusieurs des groupes hydroxy sur chaque groupe phosphoreux du médicament parent en un groupe de structure A pour obtenir un promédicament où la structure A comprend : 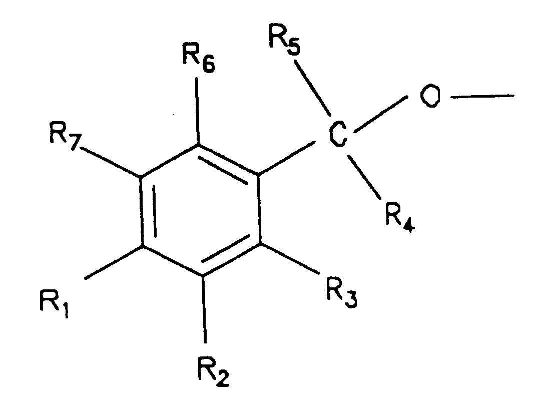 où
R₁ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un atome d'halogène ; un groupe (-CO₂R₉) ; un groupe alkyle (par exemple méthyle) ; ou un groupe hydroxyméthyle (HO-CH₂-) ;
R₂ peut être un atome d'hydrogène ; un groupe (-CO₂R₁₀) ; un groupe alkyle (par exemple méthyle) ; un atome d'halogène ; un groupe méthoxy ; un groupe acyloxy (-O-CO-R₈) ; ou un groupe hydroxyméthyle (HO-CH₂-) ;
R₃ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un groupe alkyle (par exemple méthyle) ; un groupe hydroxyméthyle (-CH₂-OH) ; un atome d'halogène ; un groupe hydroxyéthyle (-CH₂-CH₂-OH) ; ou un groupe (-CH₂-CO₂-R₁₁) ;
R₄ peut être un atome d'hydrogène ; un groupe alkyle (par exemple méthyle) ; un groupe méthoxyméthyle (-CH₂-O-CH₃) ; un groupe (-CH₂-CO₂-R₁₂) ; un groupe (-CH₂-CO-CH₃) ; un groupe (-CH(CH₃)-CO₂-R₁₂) ; un groupe (-CH₂-CO-NH₂) ; un groupe (-CH₂-NO₂) ; ou un groupe (-CH₂-SO₂-CH₃) ; ou un groupe méthylène substitué avec un groupe fonctionnel qui favorise une réaction d'élimination en abaissant la barrière intrinsèque pour l'enlèvement d'un proton dudit groupe méthylène ;
R₅ peut être un atome d'hydrogène ; un groupe alkyle (par exemple méthyle) ; un groupe méthoxyméthyle (-CH₂-O-CH₃) ; un groupe (-CH₂-CO₂-R₁₂) ; un groupe (-CH₂-CO-CH₃) ; un groupe (-CH(CH₃)-CO₂-R₁₂) ; un groupe (-CH₂-CO-NH₂) ; un groupe (-CH₂-NO₂) ; ou un groupe (-CH₂-SO₂-CH₃) ; ou un groupe méthylène substitué avec un groupe fonctionnel qui favorise une réaction d'élimination en abaissant la barrière intrinsèque pour l'enlèvement d'un proton dudit groupe méthylène ;
R₆ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un groupe alkyle (par exemple méthyle) ; un groupe hydroxyméthyle (-CH₂-OH) ; un atome d'halogène ; un groupe hydroxyéthyle (-CH₂-CH₂-OH) ; ou un groupe (-CH₂-CO₂-R₁₁) ;
R₇ peut être un atome d'hydrogène ; un groupe (-CO₂R₁₀) ; un groupe alkyle (par exemple méthyle) ; un atome d'halogène ; un groupe méthoxy ; un groupe acyloxy (-O-CO-R₈) ; ou un groupe hydroxyméthyle (HO-CH₂-) ;
où R₈ peut être un groupe de structure suivante : (-(CH₂)_{N}-CH₃) pour N = 0-18 ; R₈ peut être un groupe phényle, lequel groupe phényle peut lui-même porter des substituants, où R₈ peut également être choisi de telle sorte que R₈-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; où R₉, et R₁₀, R₁₁, et R₁₂, sont un groupe méthyle, un groupe éthyle, un groupe phényle ou un groupe benzyle ; et
où au moins un des groupes suivants : R₁ ; R₃ ; et R₆ est un groupe acyloxy (-O-CO-R₈) ou un autre groupe qui subit une biotransformation in vivo pour former finalement un groupe hydroxy sur le cycle phényle.

17. Procédé selon la revendication 16, où le médicament parent est
(a) un médicament ester monophosphate ; ou
(b) un médicament phosphodiester ; ou
(c) un médicament acide phosphonique ; ou
(d) un médicament phosphonate ; ou
(e) un médicament acide phosphinique ; ou
(f) un médicament nucléotide monophosphate ; ou est
(g) choisi dans le groupe constitué par :
la 9-(2-phosphonylméthoxyéthyl)adénine, (PMEA) ; la
9-(RS)-(3-fluoro-2-phosphonylméthoxypropyl)adénine, (FPMPA) ; la
9-(RS)-(3-fluoro-2-phosphonylméthoxypropyl)-2,6-diaminopurine ; le
3'-azido-didésoxythymidine 5'-monophosphate (AZT-phosphate) ; le
2',3'-didéhydro-2',3'-didésoxythymidine 5'-monophosphate (D4T-phosphate) ; le 2',3'-didésoxyadénosine 5'-monophosphate ; et un ester carboxylate de phosphonoformiate.

18. Procédé selon la revendication 16 ou la revendication 17, où
(a) la structure du groupe "A" est choisie dans l'ensemble constitué par : 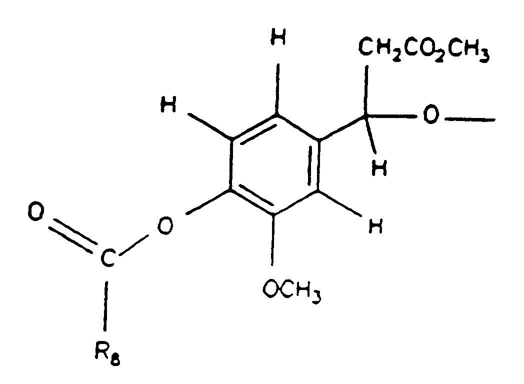 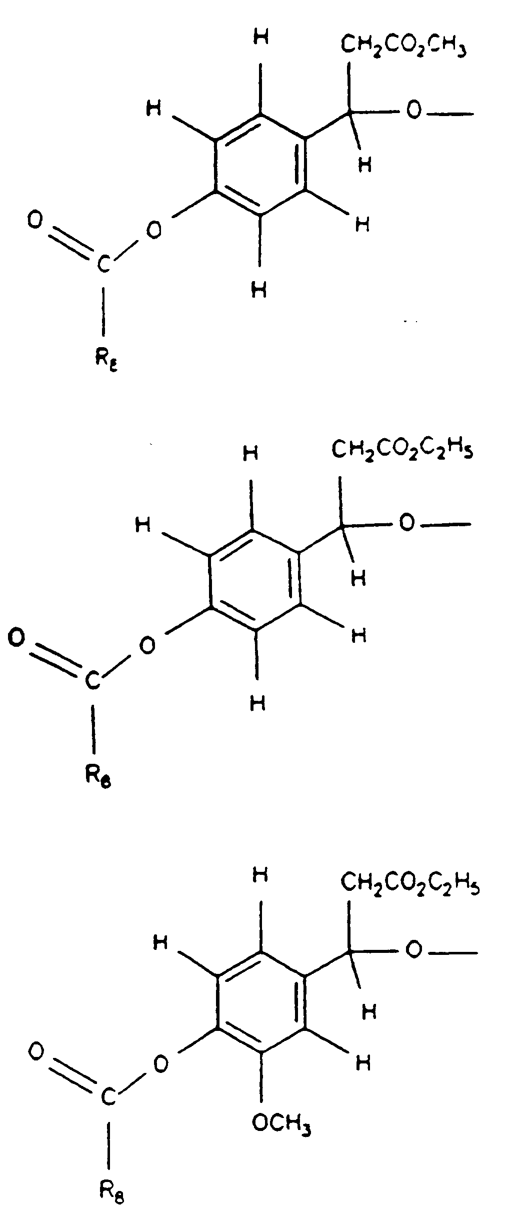 où R₈ peut être un groupe de structure suivante : (-(CH₂)_{N}-CH₃) pour N = 0-18 ; R₈ peut être un groupe phényle, lequel groupe phényle peut lui-même porter des substituants, où R₈ peut également être choisi de telle sorte que R₈-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; ou où R₈ est un groupe quelconque choisi de telle sorte que le promédicament obtenu subisse une biotransformation ou une transformation spontanée in vivo pour finalement former un groupe hydroxy sur le cycle phényle ; ou
(b) le groupe "A" a la structure suivante :

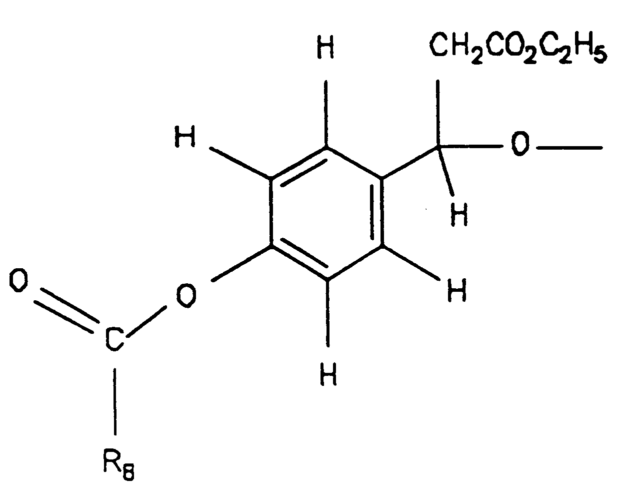

19. Procédé selon la revendication 16 pour la préparation d'un promédicament pour faciliter l'apport de phosphonoformiate dans des cellules et à travers des membranes biologiques, comprenant la préparation d'un promédicament de structure suivante : 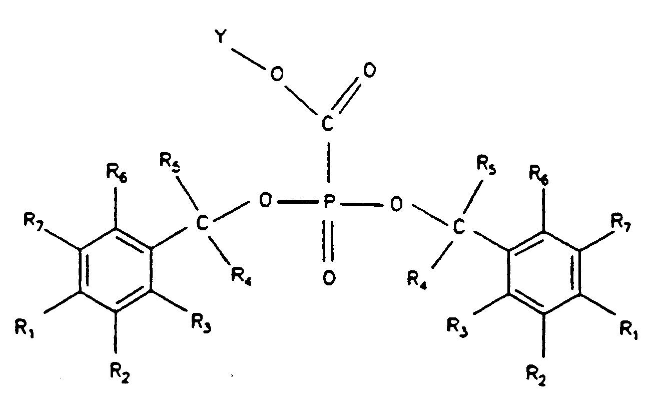 dans laquelle Y est un groupe benzyle ou phényle, le groupe benzyle ou phényle pouvant lui-même porter des substituants.

20. Procédé selon la revendication 19, qui comprend la préparation d'un promédicament de structure :

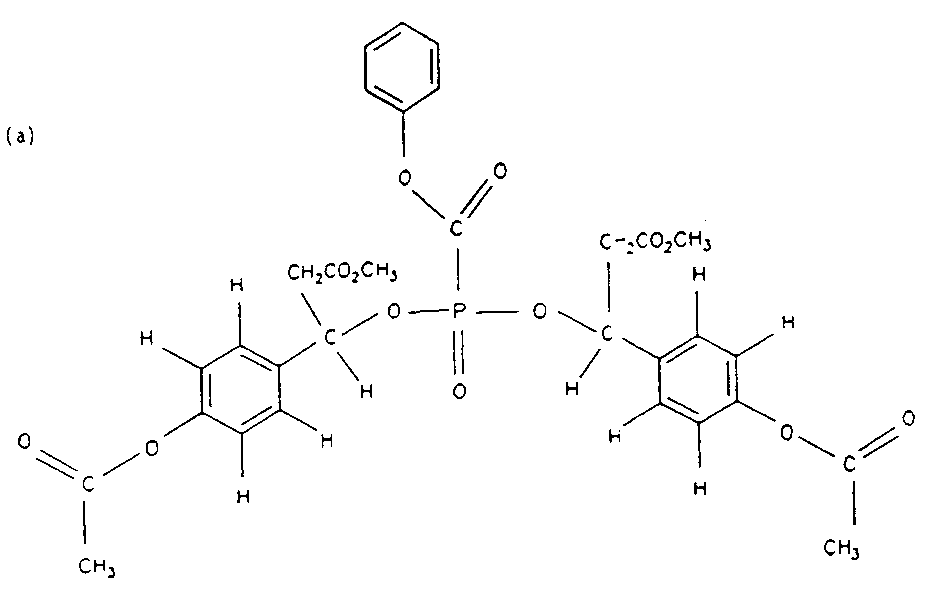

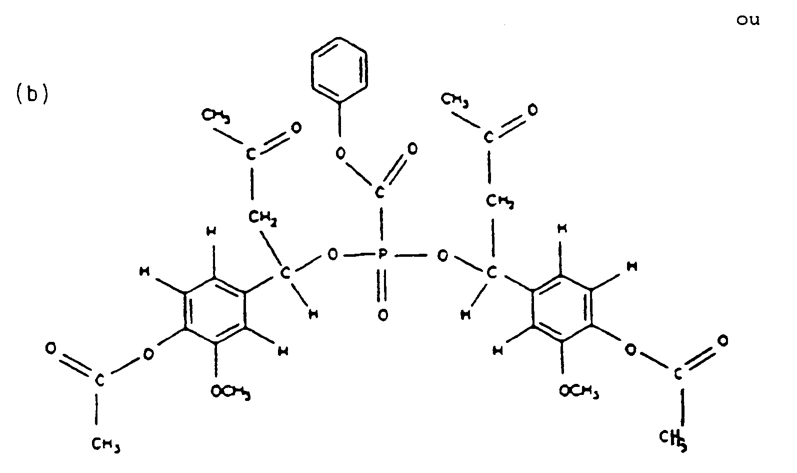

21. Procédé selon la revendication 16, où l'on choisit le médicament portant un groupe phosphoreux et la structure A de telle sorte que le promédicament soit :

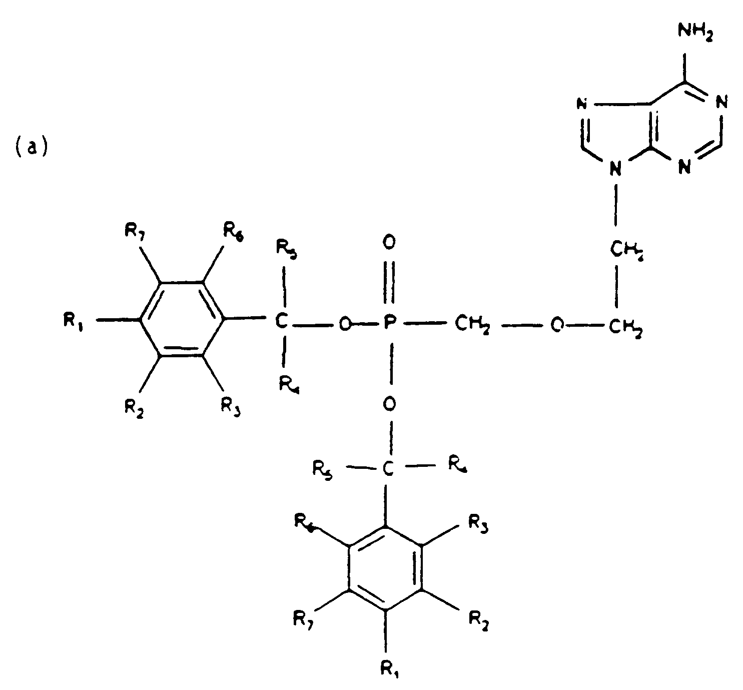

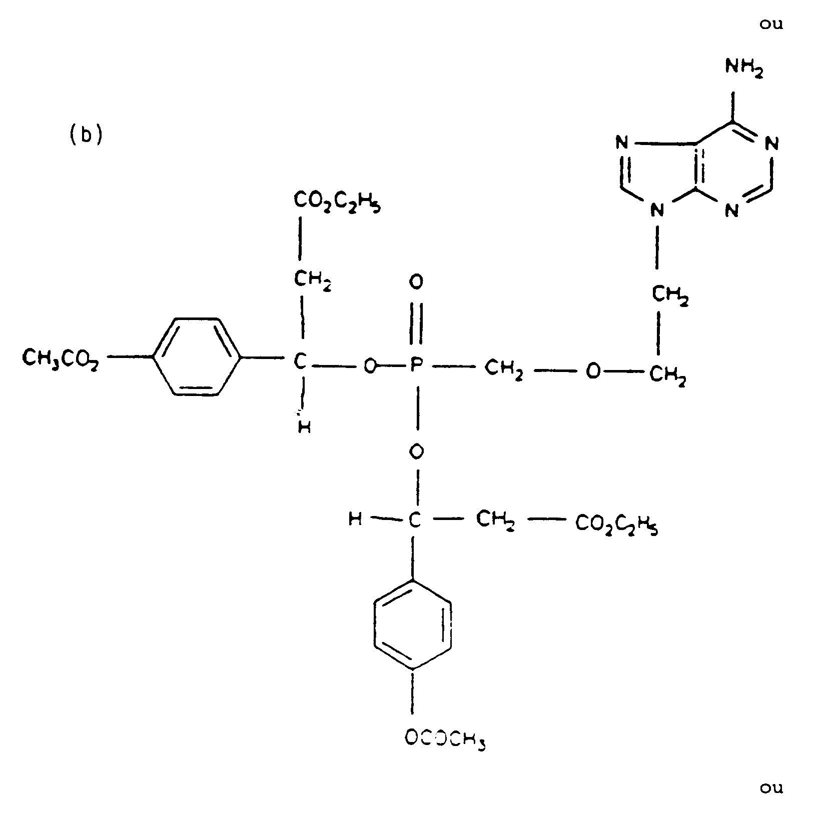

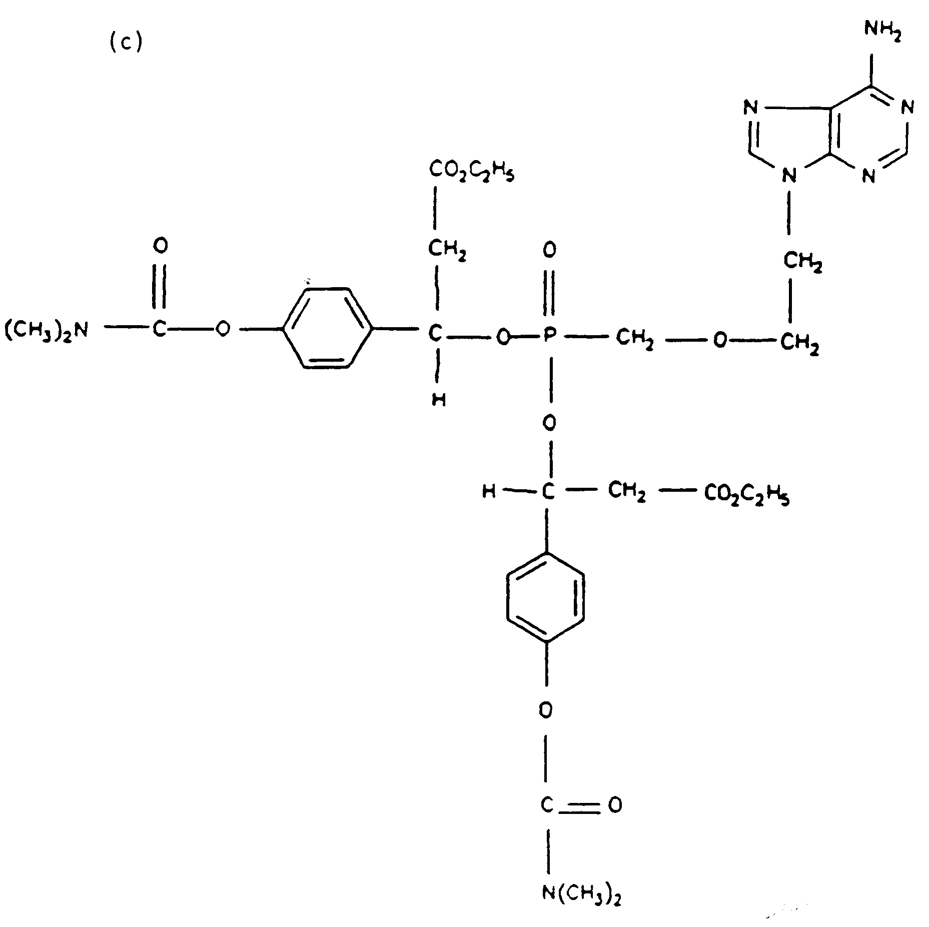

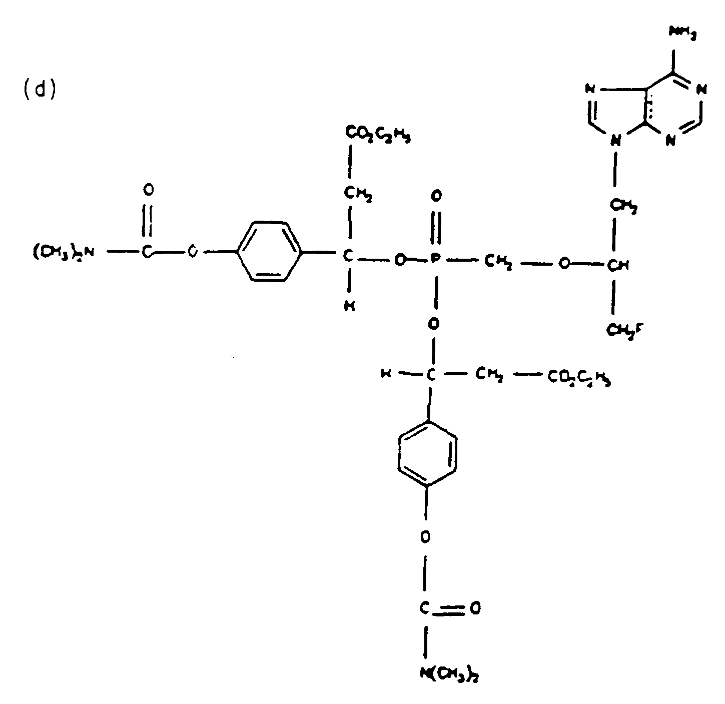

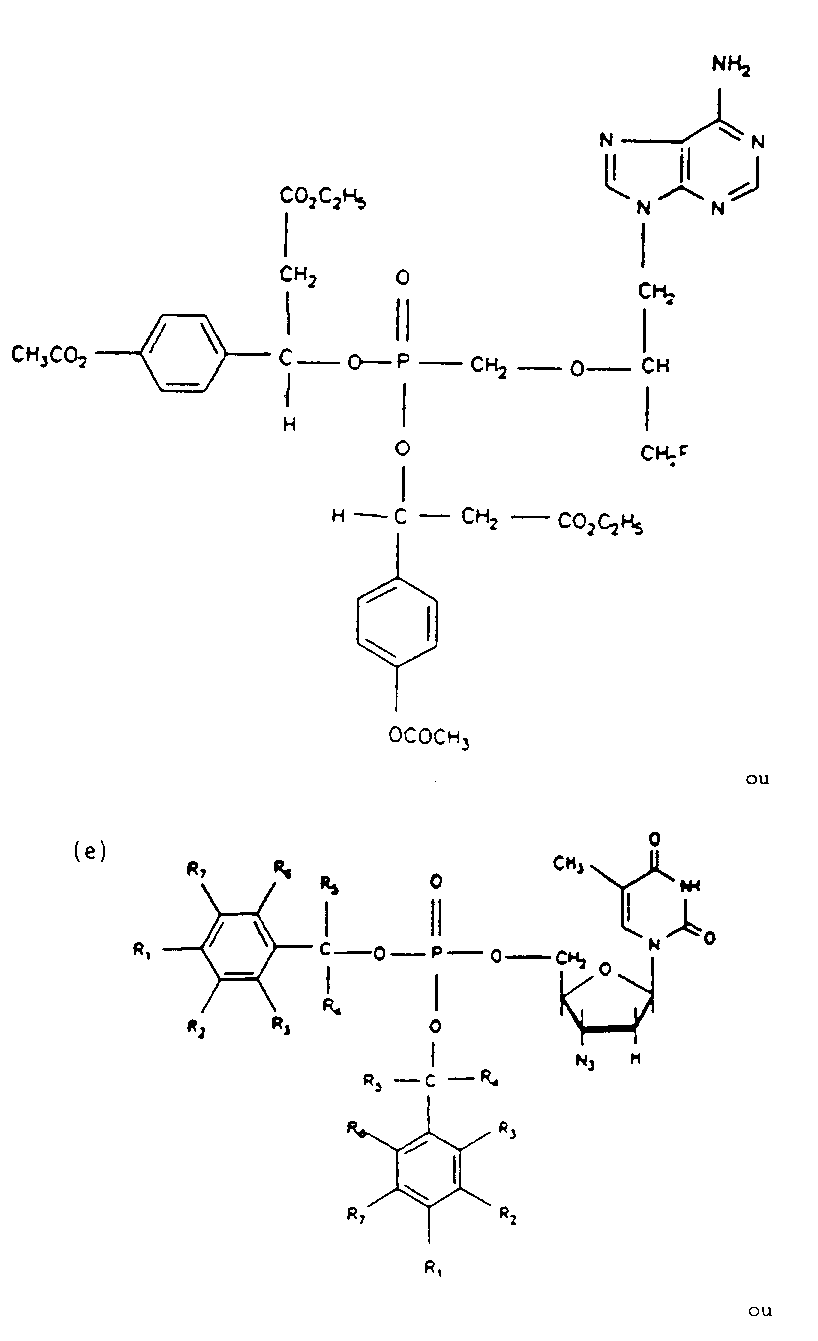

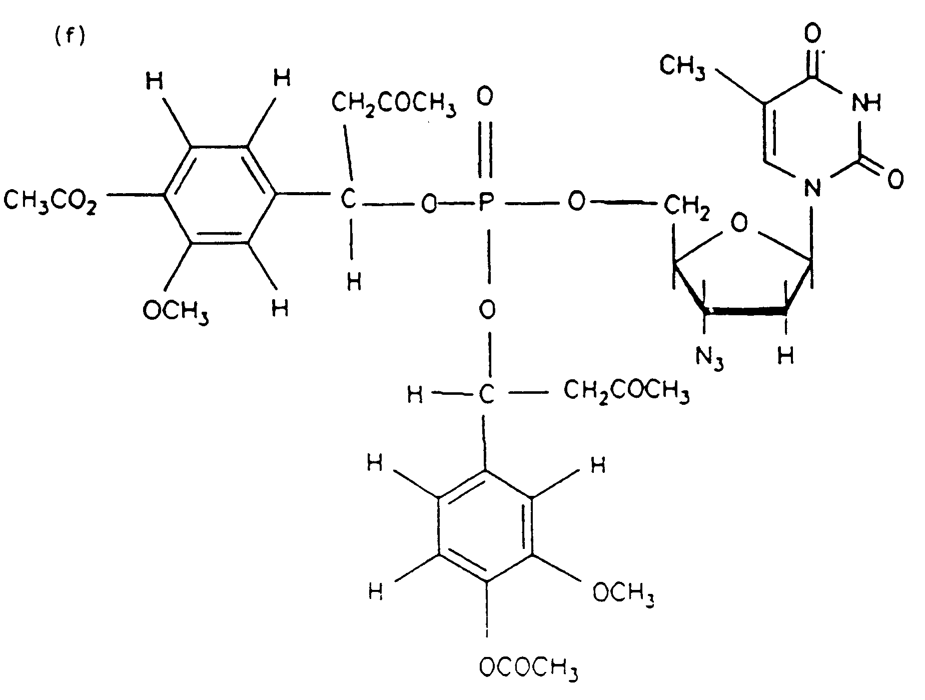

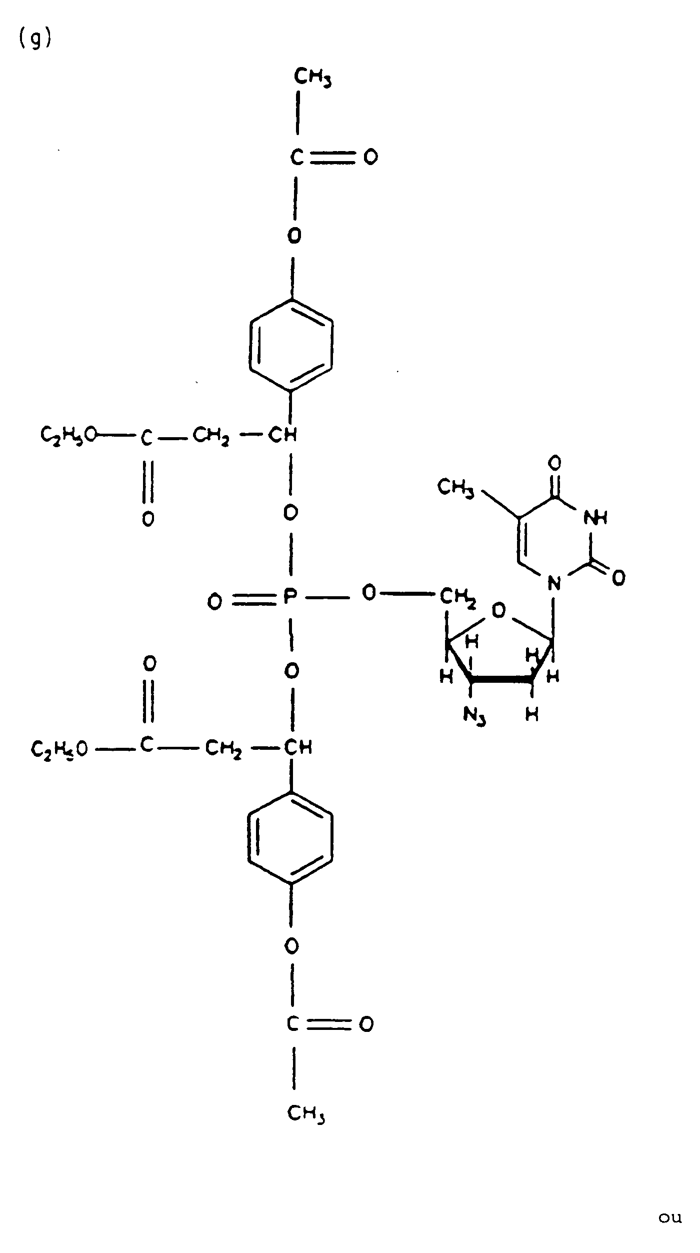

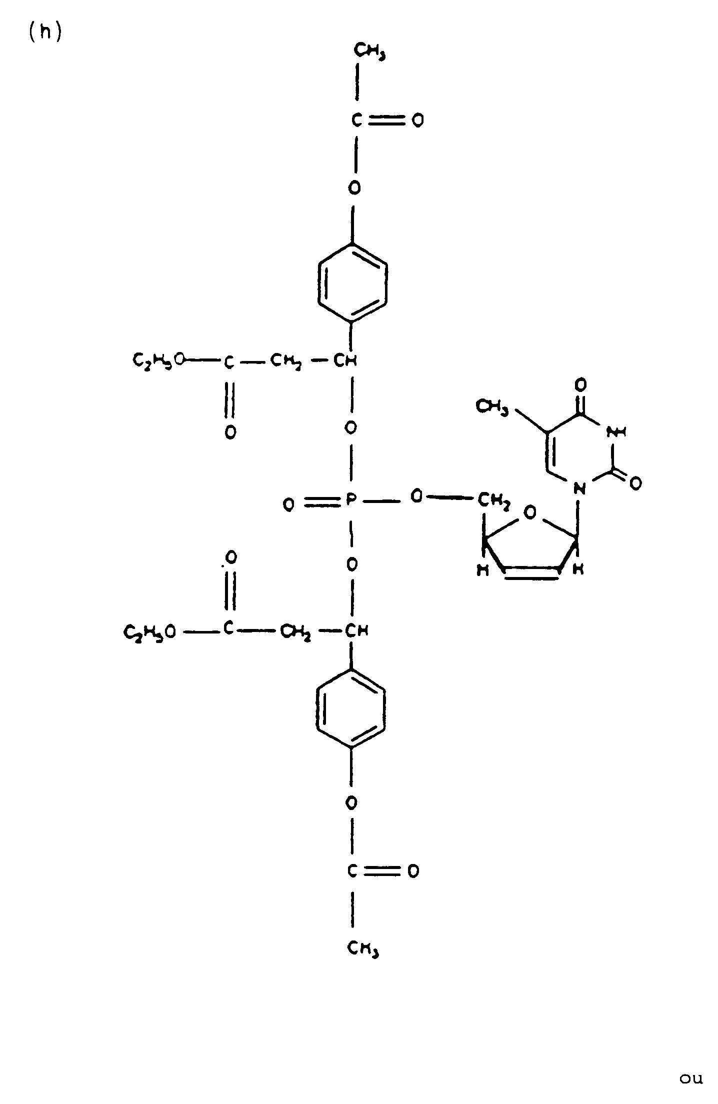

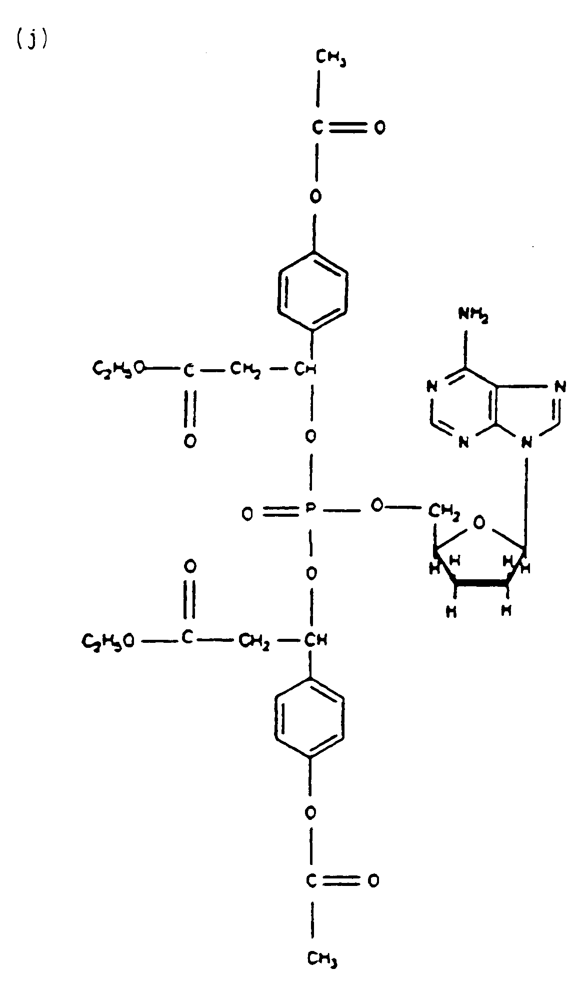

22. Procédé de préparation d'un promédicament pour faciliter l'apport de médicaments parents portant un groupe phosphoreux dans des cellules et à travers des membranes biologiques comprenant les étapes consistant à :
a. choisir un médicament portant un groupe phosphoreux ; et
b. préparer un mono- ou bis-phosphoester à partir d'un dérivé convenablement activé du médicament parent et d'un alcool de structure A pour obtenir un promédicament où la structure A comprend 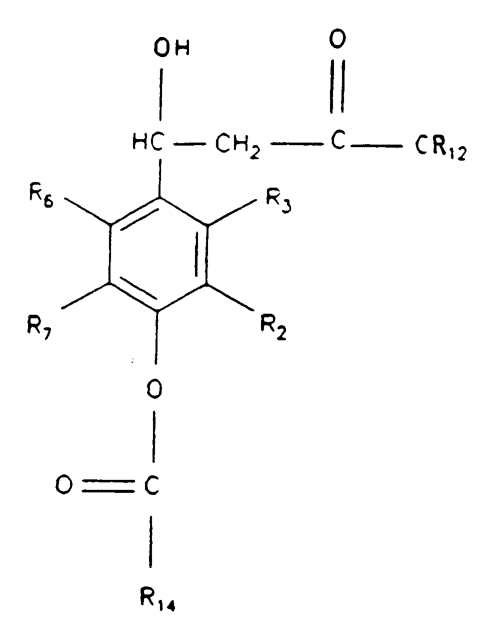 où R₂ et R₇ peuvent être un atome d'hydrogène, un groupe (-CO₂R₁₀), un groupe méthyle, un atome d'halogène ou un groupe méthoxy, ou un groupe hydroxyméthyle (HO-CH₂-) ; où R₁₀ peut être un groupe méthyle ou éthyle ;
où R₃ et R₆ peuvent être un atome d'hydrogène ; un groupe méthyle ;
un groupe hydroxyméthyle (-CH₂-OH) ; un atome d'halogène ; ou un groupe hydroxyéthyle (-CH₂-CH₂-OH) ;
où R₁₂ est un groupe méthyle, éthyle, phényle ou benzyle ;
où R₁₄ peut être un groupe de structure suivante : (-(CH₂)_{N}-CH₃) pour N = 0-18 ; un groupe phényle, le groupe phényle pouvant lui-même porter des substituants ; où R₁₄ peut être choisi pour que R₁₄-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; où R₁₄ peut être (-O-(CH₂)_{N}-CH₃) pour N = 0-18 ; un groupe phényloxy, ce groupe phényloxy pouvant lui-même porter des substituants ; et où R₁₄ peut être un des groupes suivants : (-NH₂) ; (-NHCH₃) ; ou (-N(CH₃)₂).

23. Procédé selon la revendication 22, où la structure A est : 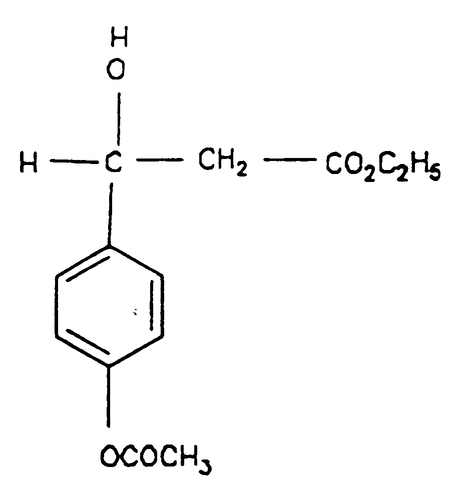 3-hydroxy-3-(4-acétoxyphényl)propionate d'éthyle

24. Promédicament préparé selon le procédé de l'une quelconque des revendications 16 à 23 pour l'utilisation en thérapeutique pour faciliter l'apport de médicaments portant un groupe phosphoreux dans les cellules et à travers les membranes biologiques, par contact des cellules et des membranes biologiques avec le promédicament.

25. Utilisation d'un promédicament préparé selon le procédé de l'une quelconque des revendications 16 à 23 pour la fabrication d'un médicament pour faciliter l'apport de médicaments portant un groupe phosphoreux dans les cellules et à travers les membranes biologiques, par contact des cellules et des membranes biologiques avec le promédicament.

26. Promédicament d'un médicament parent, où le médicament parent a la structure générale : 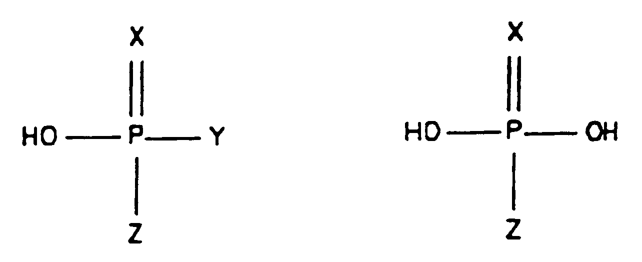 où X est un atome d'oxygène (O) ou de soufre (S) et Y et Z sont tels que définis par le reste du médicament parent ; et où le médicament parent n'est pas l'acide phosphorique ; où le promédicament a la structure suivante : 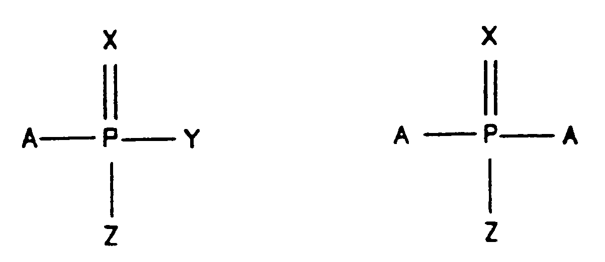 où le groupe "A" est un dérivé benzyloxy avec un ou plusieurs groupes acyloxy dans les positions para ou ortho relativement au phosphoester, et où le médicament parent est libéré après conversion du groupe acyloxy par une estérase en le groupe hydroxy correspondant.

27. Procédé pour préparer un promédicament pour faciliter l'apport de médicaments parents portant un groupe phosphoreux dans des cellules et à travers des membranes biologiques, comprenant les étapes consistant à :
a. choisir un médicament parent portant un groupe phosphoreux ; et
b. transformer un ou plusieurs des groupes hydroxy sur chaque groupe phosphoreux du médicament parent en un groupe de structure A pour obtenir un promédicament, où le groupe "A" est un dérivé benzyloxy avec un ou plusieurs groupes acyloxy dans les positions para ou ortho relativement au phosphoester, et où le médicament parent est libéré après conversion du groupe acyloxy par une estérase en le groupe hydroxy correspondant.

28. Utilisation d'un promédicament pour la fabrication d'un médicament parent portant un groupe phosphate ou phosphonate qui est un mono- ou un diester du phosphate ou du phosphonate, pour l'utilisation en thérapeutique, dans laquelle thérapeutique, le médicament est dégradé en le médicament parent portant un groupe phosphoreux par une réaction d'élimination pour former une double liaison carbone-carbone ; et où la réaction d'élimination est déclenchée par le démasquage spontané ou enzymatique d'un groupe que l'on sait être fortement donneur d'électrons, par exemple un groupe hydroxy.

29. Promédicament préparé selon le procédé de la revendication 27 pour l'utilisation en thérapeutique pour faciliter l'apport de médicaments portant un groupe phosphoreux dans des cellules et à travers des membranes biologiques par contact des cellules avec le promédicament.

30. Utilisation d'un promédicament préparé selon le procédé de la revendication 27 pour la fabrication d'un médicament pour faciliter l'apport de médicaments portant un groupe phosphoreux dans des cellules et à travers des membranes biologiques par contact des cellules avec le promédicament.

## Revendications (Revendications pour l'(les) Etat(s) contractant(s) suivant(s): ES, GR)

1. Procédé pour la préparation d'un promédicament pour faciliter l'apport des médicaments parents portant un groupe phosphoreux dans des cellules et à travers des membranes biologiques, comprenant les étapes consistant à :
a. choisir un médicament parent portant un groupe phosphoreux ; et
b. transformer un ou plusieurs des groupes hydroxy sur chaque groupe phosphoreux du médicament parent en un groupe de structure A pour obtenir un promédicament où la structure A comprend : 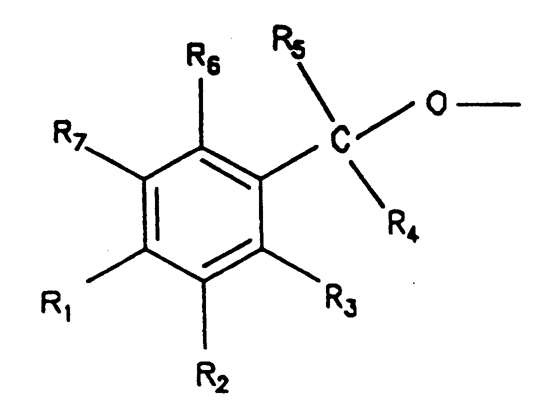 où
R₁ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un atome d'halogène ; un groupe (-CO₂R₉) ; un groupe alkyle (par exemple méthyle) ; ou un groupe hydroxyméthyle (HO-CH₂-) ;
R₂ peut être un atome d'hydrogène ; un groupe (-CO₂R₁₀) ; un groupe alkyle (par exemple méthyle) ; un atome d'halogène ; un groupe méthoxy ; un groupe acyloxy (-O-CO-R₈) ; ou un groupe hydroxyméthyle (HO-CH₂-) ;
R₃ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un groupe alkyle (par exemple méthyle) ; un groupe hydroxyméthyle (-CH₂-OH) ; un atome d'halogène ; un groupe hydroxyéthyle (-CH₂-CH₂-OH) ; ou un groupe (-CH₂-CO₂-R₁₁) ;
R₄ peut être un atome d'hydrogène ; un groupe alkyle (par exemple méthyle) ; un groupe méthoxyméthyle (-CH₂-O-CH₃) ; un groupe (-CH₂-CO₂-R₁₂) ; un groupe (-CH₂-CO-CH₃) ; un groupe (-CH(CH₃)-CO₂-R₁₂) ; un groupe (-CH₂-CO-NH₂) ; un groupe (-CH₂-NO₂) ; ou un groupe (-CH₂-SO₂-CH₃) ; ou un groupe méthylène substitué avec un groupe fonctionnel qui favorise une réaction d'élimination en abaissant la barrière intrinsèque pour l'enlèvement d'un proton dudit groupe méthylène ;
R₅ peut être un atome d'hydrogène ; un groupe alkyle (par exemple méthyle) ; un groupe méthoxyméthyle (-CH₂-O-CH₃) ; un groupe (-CH₂-CO₂-R₁₂) ; un groupe (-CH₂-CO-CH₃) ; un groupe (-CH(CH₃)-CO₂-R₁₂) ; un groupe (-CH₂-CO-NH₂) ; un groupe (-CH₂-NO₂) ; ou un groupe (-CH₂-SO₂-CH₃) ; ou un groupe méthylène substitué avec un groupe fonctionnel qui favorise une réaction d'élimination en abaissant la barrière intrinsèque pour l'enlèvement d'un proton dudit groupe méthylène ;
R₆ peut être un groupe acyloxy (-O-CO-R₈) ; un groupe (-O-CO₂-R₈) ; un groupe (-O-C(O)-NH₂) ; un groupe (-O-C(O)-NHCH₃) ; un groupe (-O-C(O)-N(CH₃)₂) ; un atome d'hydrogène ; un groupe alkyle (par exemple méthyle) ; un groupe hydroxyméthyle (-CH₂-OH) ; un atome d'halogène ; un groupe hydroxyéthyle (-CH₂-CH₂-OH) ; ou un groupe (-CH₂-CO₂-R₁₁) ;
R₇ peut être un atome d'hydrogène ; un groupe (-CO₂R₁₀) ; un groupe alkyle (par exemple méthyle) ; un atome d'halogène ; un groupe méthoxy ; un groupe acyloxy (-O-CO-R₈) ; ou un groupe hydroxyméthyle (HO-CH₂-) ;
où R₈ peut être un groupe de structure suivante : (-(CH₂)_{N}-CH₃) pour N = 0-18 ; R₈ peut être un groupe phényle, lequel groupe phényle peut lui-même porter des substituants, où R₈ peut également être choisi de telle sorte que R₈-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; où R₉, et R₁₀, R₁₁, et R₁₂, sont un groupe méthyle, un groupe éthyle, un groupe phényle ou un groupe benzyle ; et
où au moins un des groupes suivants : R₁ ; R₃ ; et R₆ est un groupe acyloxy (-O-CO-R₈) ou un autre groupe qui subit une biotransformation in vivo pour former finalement un groupe hydroxy sur le cycle phényle.

2. Procédé selon la revendication 1, où le médicament parent est
(a) un médicament ester monophosphate ; ou
(b) un médicament phosphodiester ; ou
(c) un médicament acide phosphonique ; ou
(d) un médicament phosphonate ; ou
(e) un médicament acide phosphinique ; ou
(f) un médicament nucléotide monophosphate ; ou est
(g) choisi dans le groupe constitué par :
la 9-(2-phosphonylméthoxyéthyl)adénine, (PMEA) ; la
9-(RS)-(3-fluoro-2-phosphonylméthoxypropyl)adénine, (FPMPA) ; la
9-(RS)-(3-fluoro-2-phosphonylméthoxypropyl)-2,6-diaminopurine ; le 3'-azido-didésoxythymidine 5'-monophosphate, (AZT-phosphate) ; le
2',3'-didéhydro-2',3'-didésoxythymidine 5'-monophosphate (D4T-phosphate) ; le 2',3'-didésoxyadénosine 5'-monophosphate ; et un ester carboxylate de phosphonoformiate.

3. Procédé selon la revendication 1 ou la revendication 2, où
(a) la structure du groupe "A" est choisie dans l'ensemble constitué par : 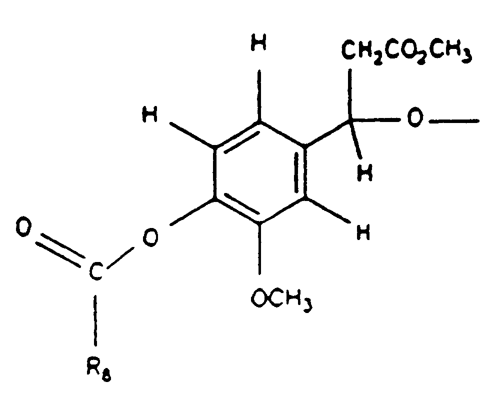 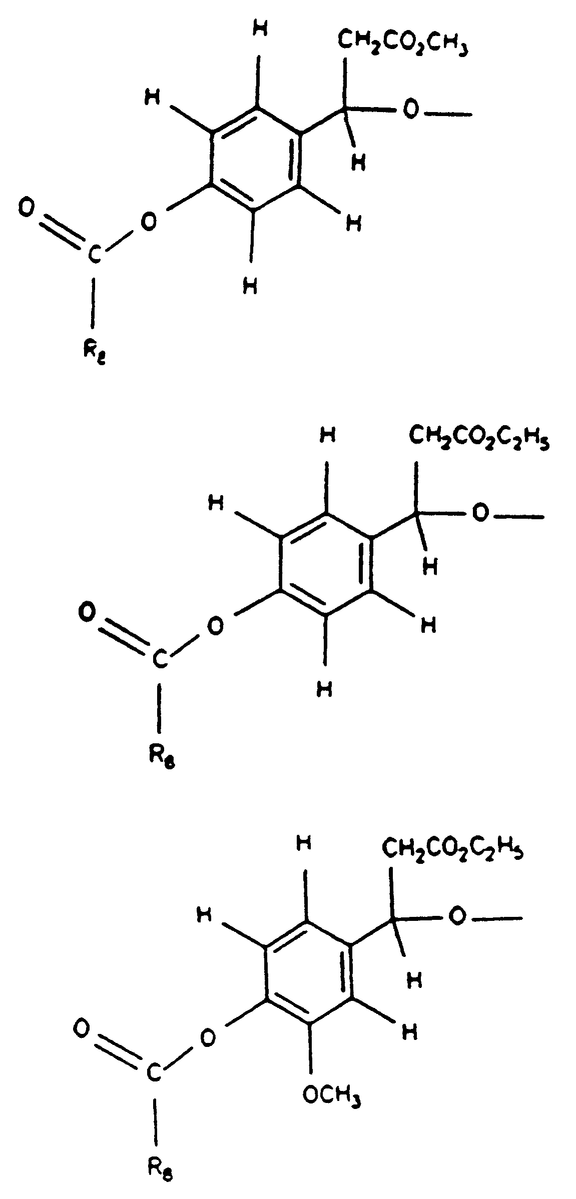 où R₈ peut être un groupe de structure suivante : (-(CH₂)_{N}-CH₃) pour N = 0-18 ; R₈ peut être un groupe phényle, lequel groupe phényle peut lui-même porter des substituants, où R₈ peut également être choisi de telle sorte que R₈-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; ou où R₈ est un groupe quelconque choisi de telle sorte que le promédicament obtenu subisse une biotransformation ou une transformation spontanée in vivo pour finalement former un groupe hydroxy sur le cycle phényle ; ou
(b) le groupe "A" a la structure suivante :

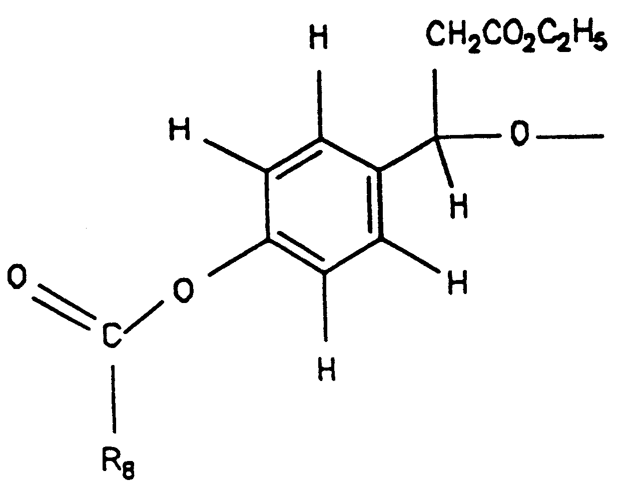

4. Procédé selon la revendication 1 pour la préparation d'un promédicament pour faciliter l'apport de phosphonoformiate dans des cellules et à travers des membranes biologiques, comprenant la préparation d'un promédicament de structure suivante : 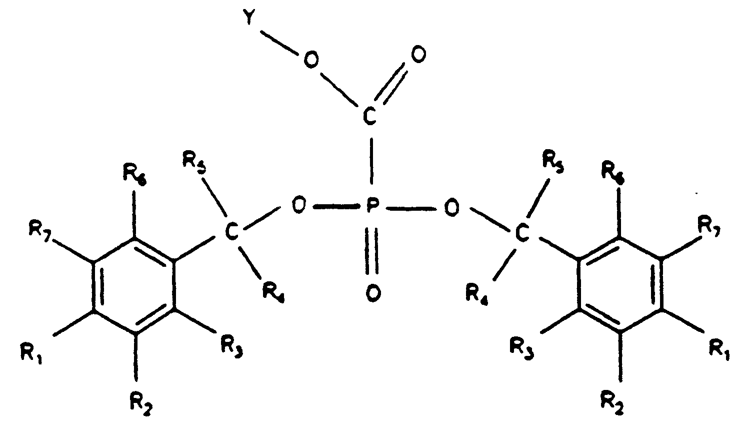 dans laquelle Y est un groupe benzyle ou phényle, le groupe benzyle ou phényle pouvant lui-même porter des substituants.

5. Procédé selon la revendication 4, qui comprend la préparation d'un promédicament de structure :

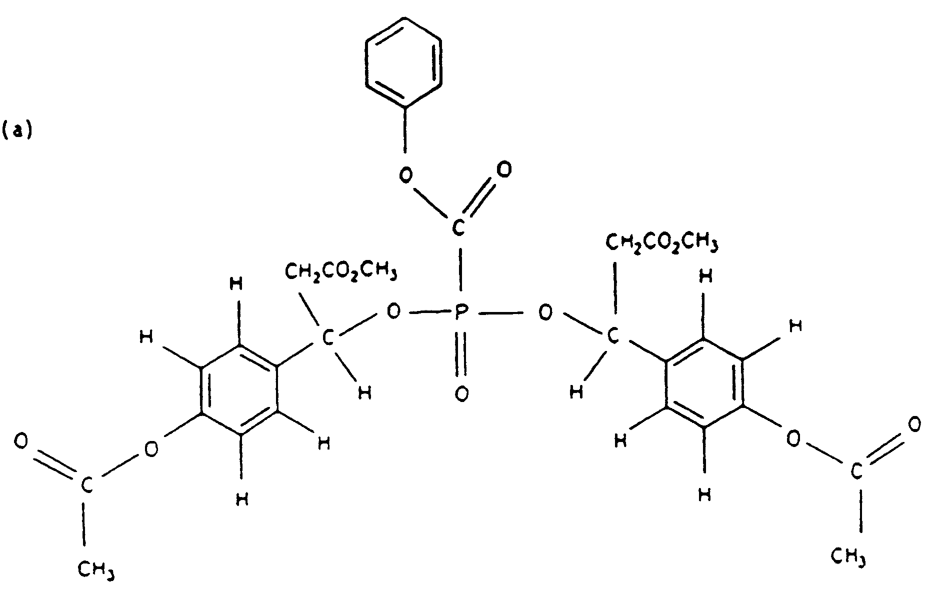

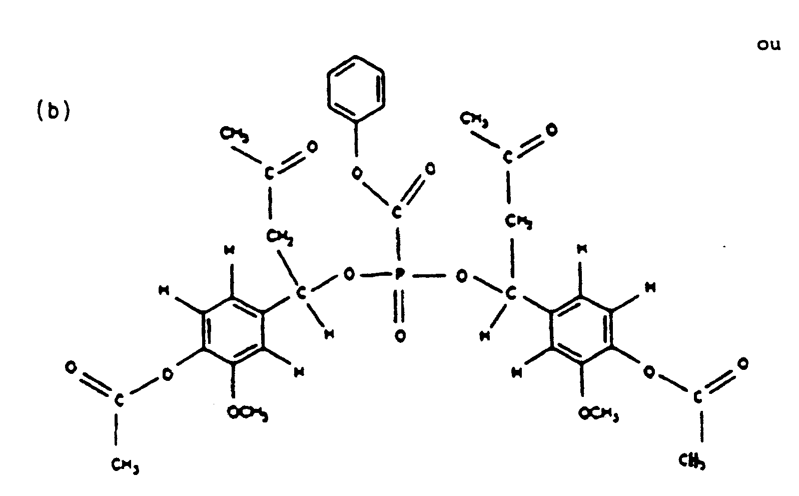

6. Procédé selon la revendication 1, où l'on choisit le médicament portant un groupe phosphoreux et la structure A de telle sorte que le promédicament soit :

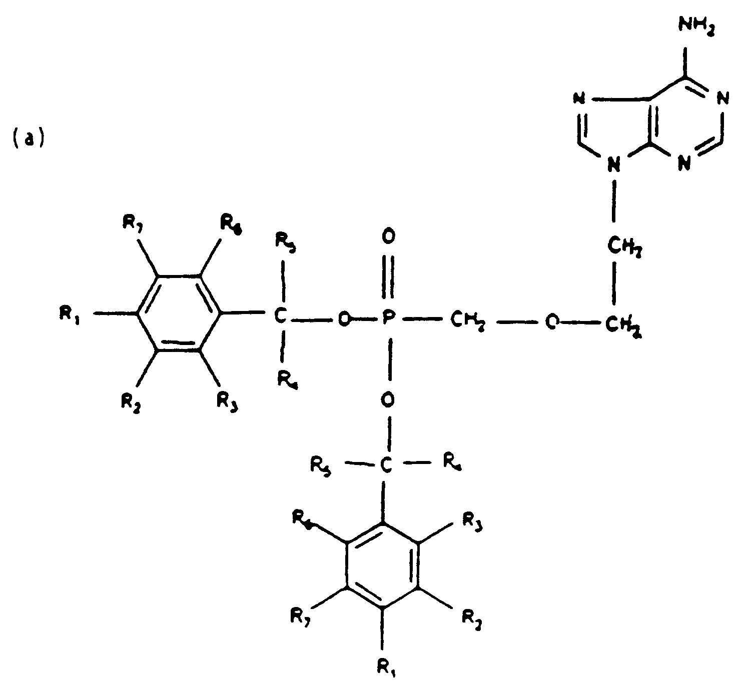

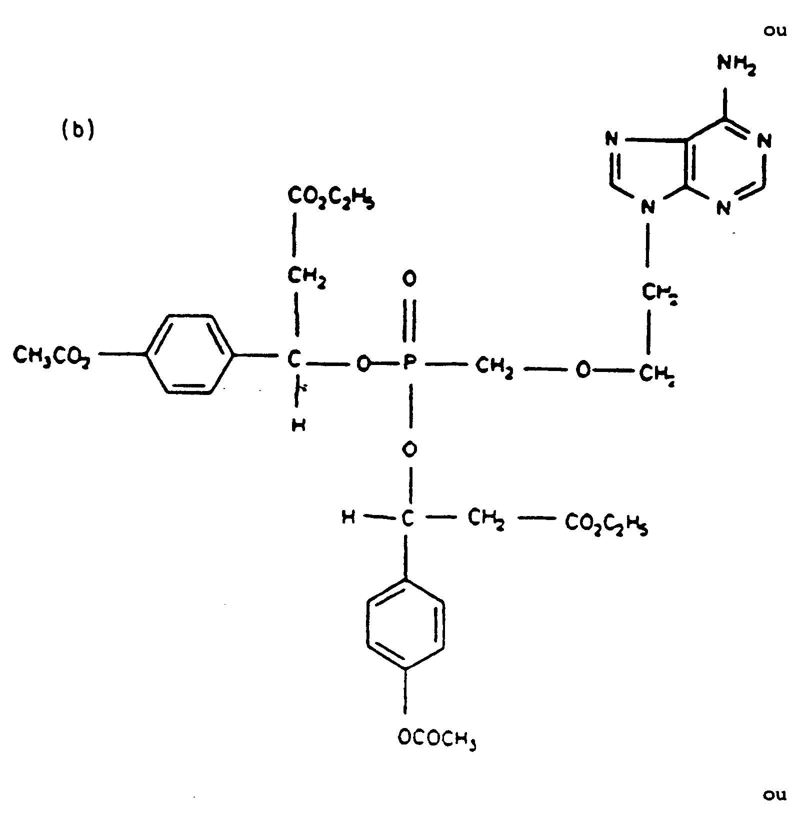

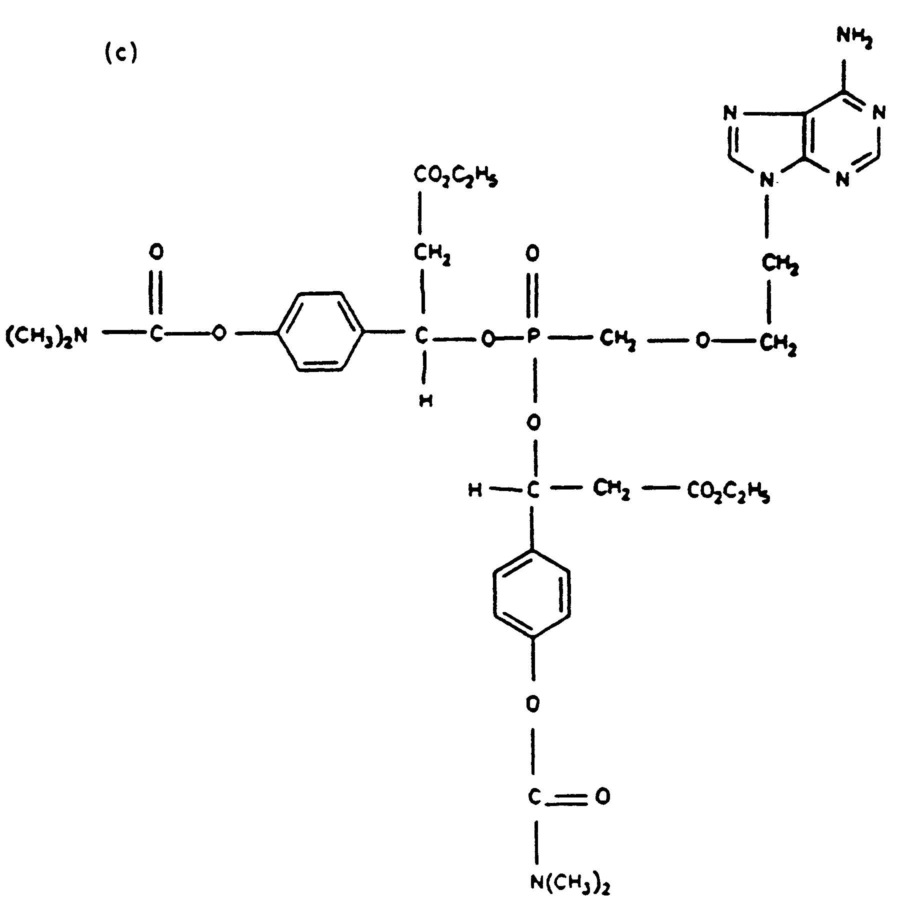

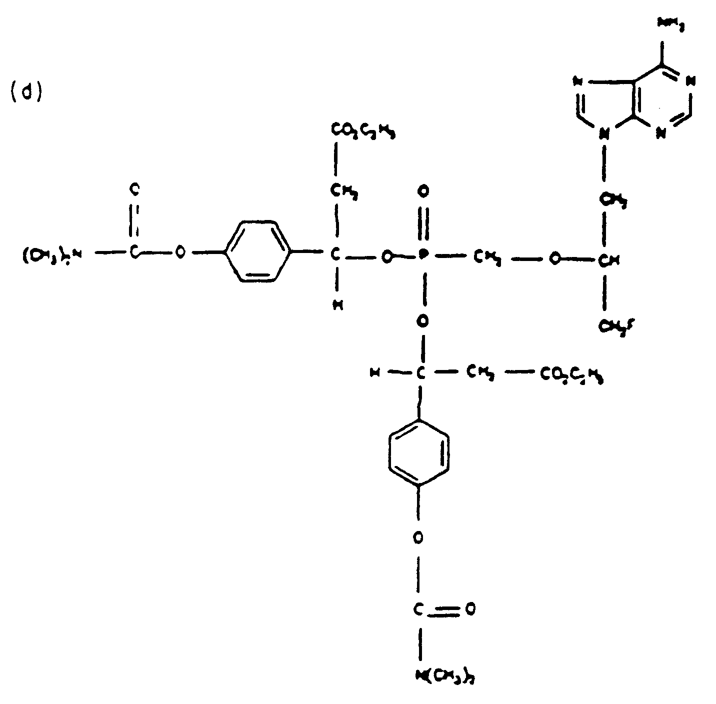

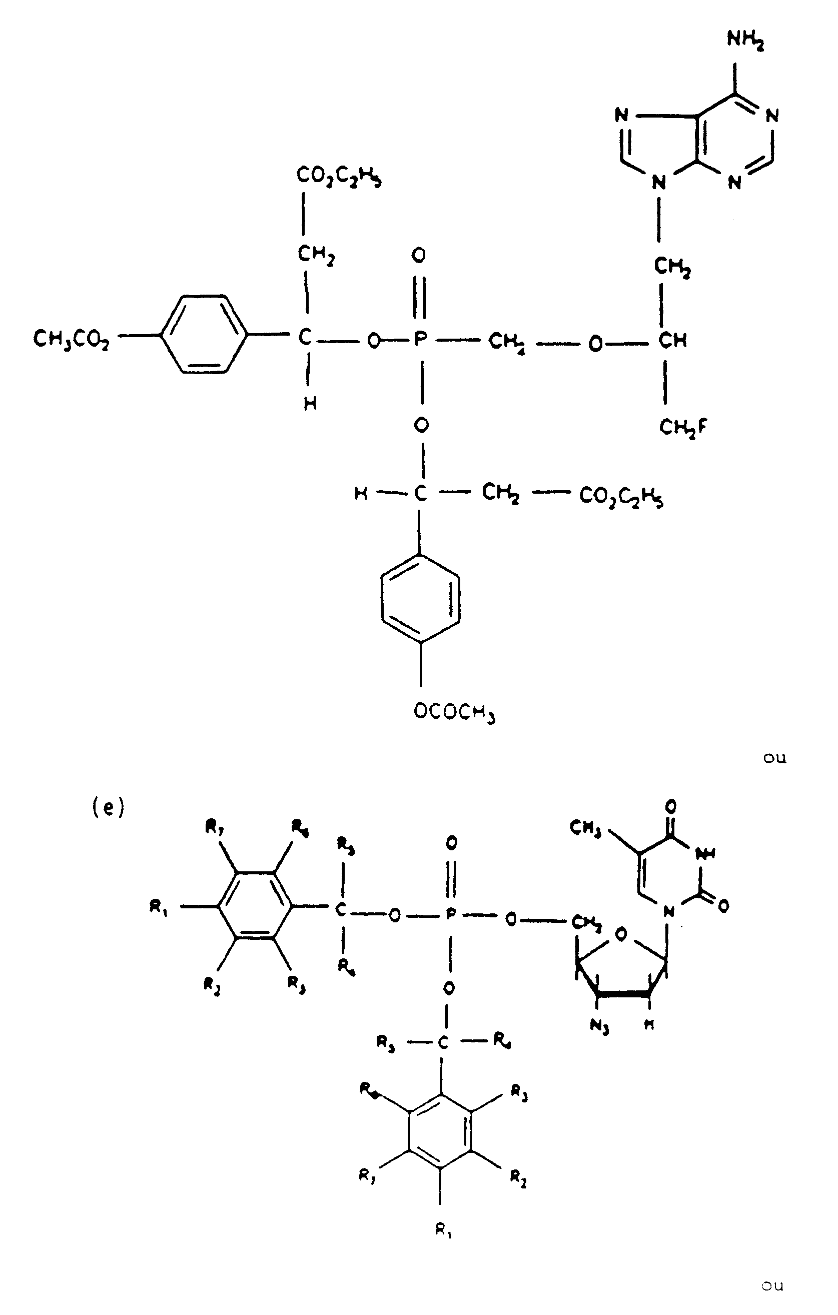

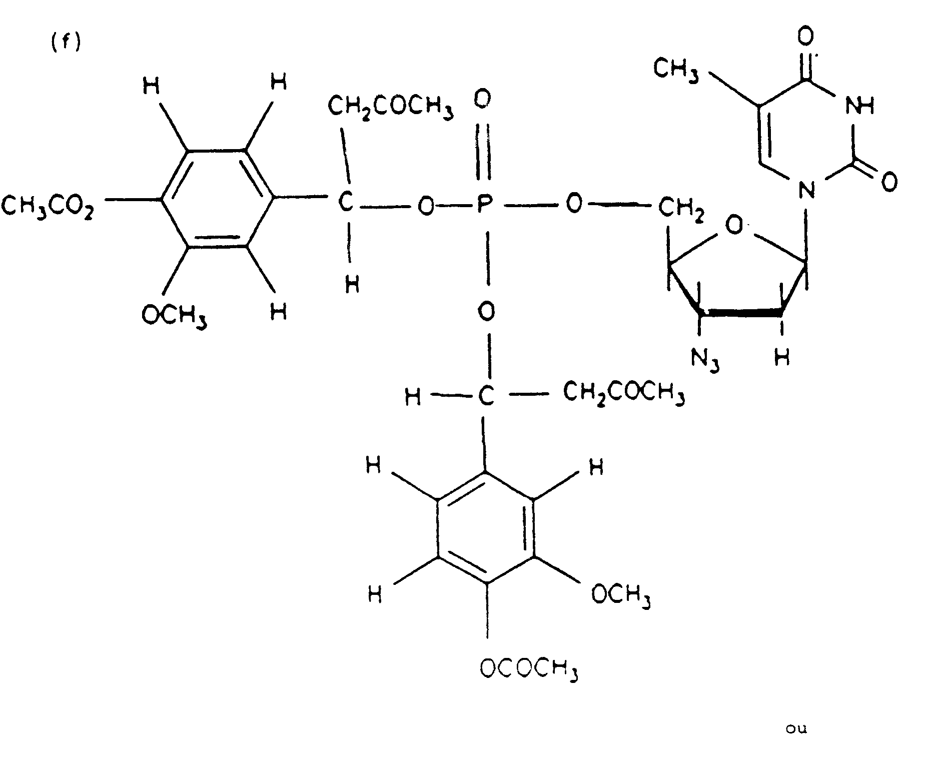

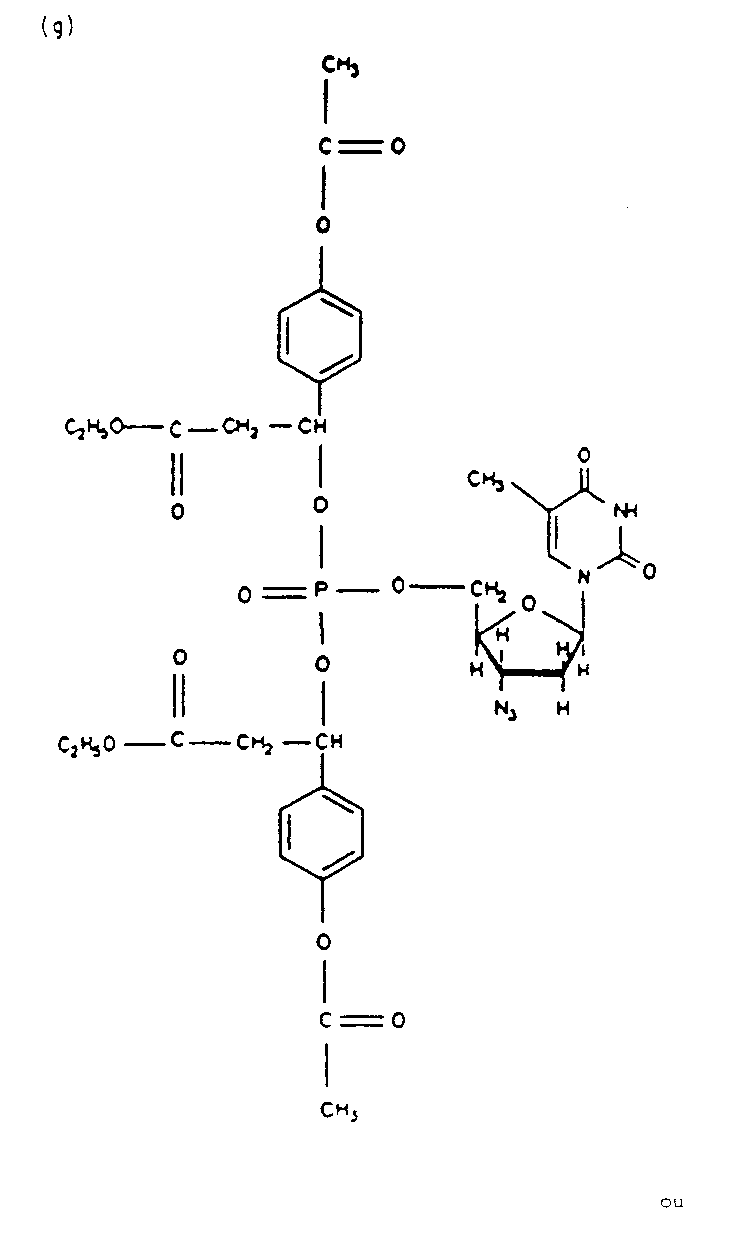

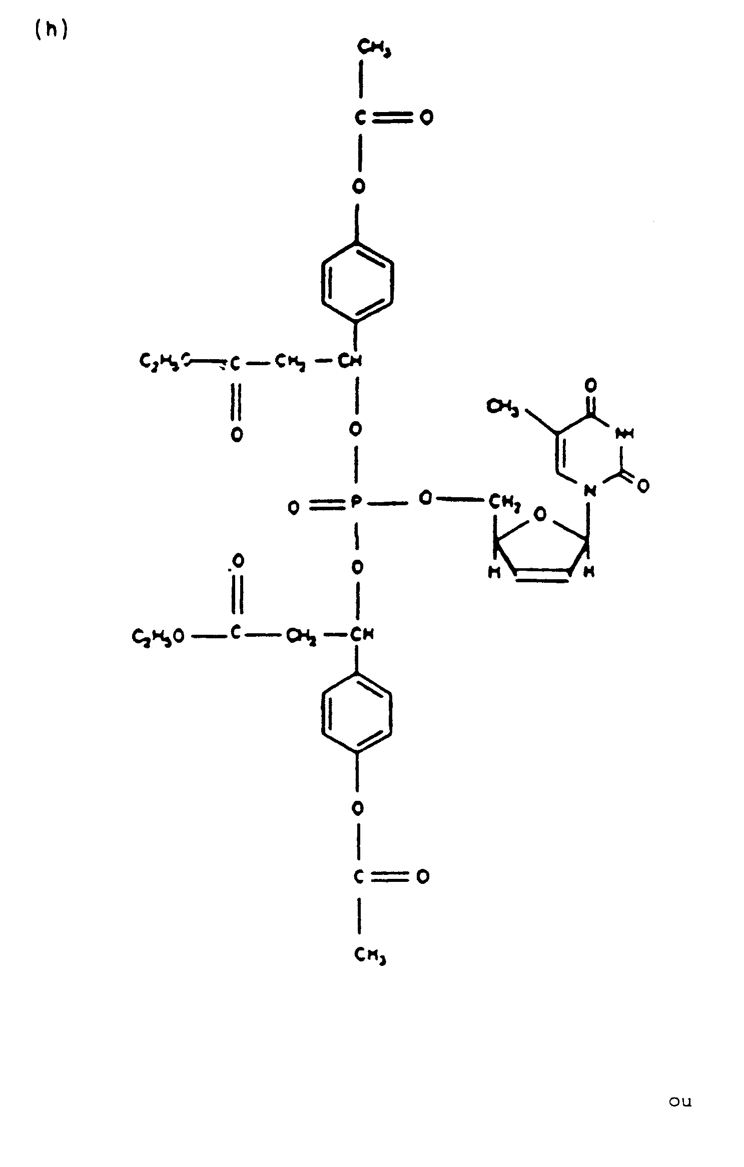

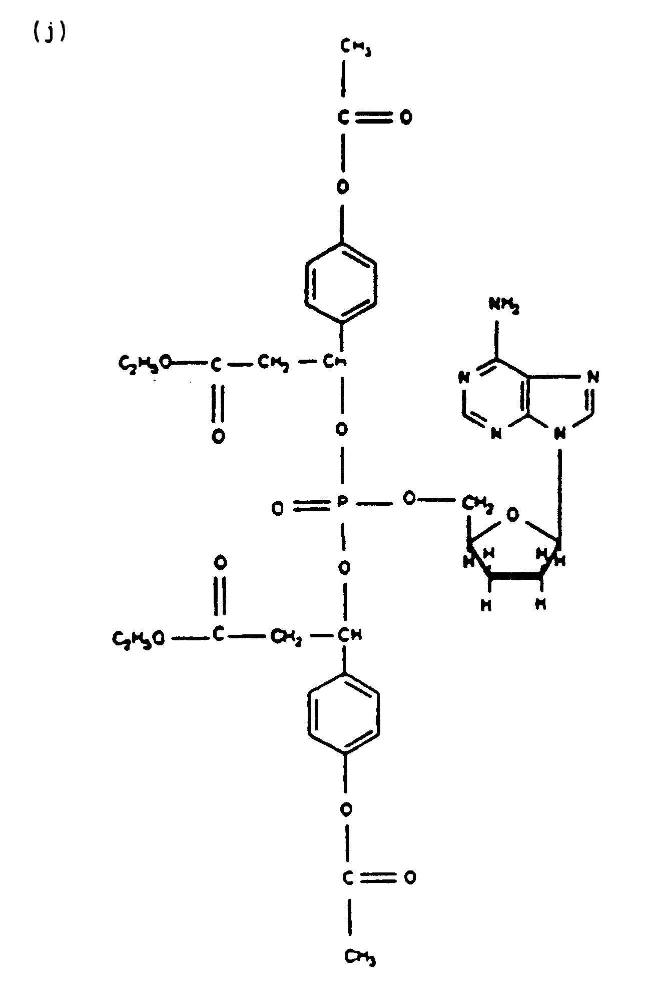

7. Procédé de préparation d'un promédicament pour faciliter l'apport de médicaments parents portant un groupe phosphoreux dans des cellules et à travers des membranes biologiques comprenant les étapes consistant à :
a. choisir un médicament portant un groupe phosphoreux ; et
b. préparer un mono- ou bis-phosphoester à partir d'un dérivé convenablement activé du médicament parent et d'un alcool de structure A pour obtenir un promédicament où la structure A comprend 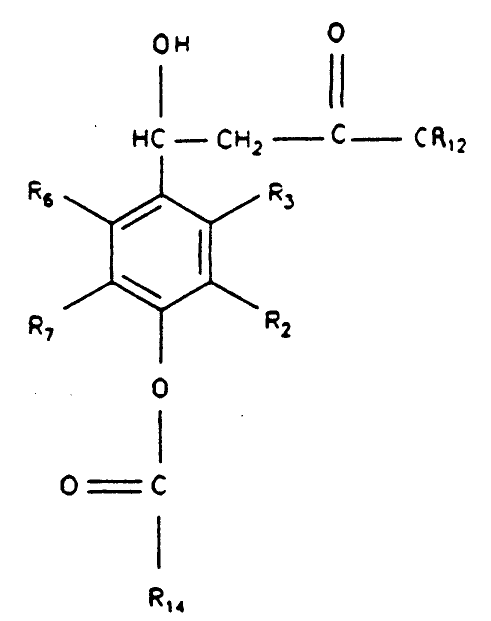 où R₂ et R₇ peuvent être un atome d'hydrogène, un groupe (-CO₂R₁₀), un groupe méthyle, un atome d'halogène ou un groupe méthoxy, ou un groupe hydroxyméthyle (HO-CH₂-) ; où R₁₀ peut être un groupe méthyle ou éthyle ;
où R₃ et R₆ peuvent être un atome d'hydrogène ; un groupe méthyle ; un groupe hydroxyméthyle (-CH₂-OH) ; un atome d'halogène ; ou un groupe hydroxyéthyle (-CH₂-CH₂-OH) ;
où R₁₂ est un groupe méthyle, éthyle, phényle ou benzyle ;
où R₁₄ peut être un groupe de structure suivante : (-(CH₂)_{N}-CH₃) pour N = 0-18 ; un groupe phényle, le groupe phényle pouvant être lui-même porter des substituants ; où R₁₄ peut être choisi pour que R₁₄-CO₂H soit : un amino-acide ; l'acide lactique ; l'acide glycolique (HO-CH₂-CO₂H) ; l'acide glycérique (HO-CH₂-CH(OH)-CO₂H) ; ou l'acide acétoacétique (CH₃COCH₂-CO₂H) ; où R₁₄ peut être (-O-(CH₂)_{N}-CH₃) pour N = 0-18 ; un groupe phényloxy, ce groupe phényloxy pouvant lui-même porter des substituants ; et où R₁₄ peut être un des groupes suivants : (-NH₂) ; (-NHCH₃) ; ou (-N(CH₃)₂).

8. Procédé selon la revendication 7, où la structure A est : 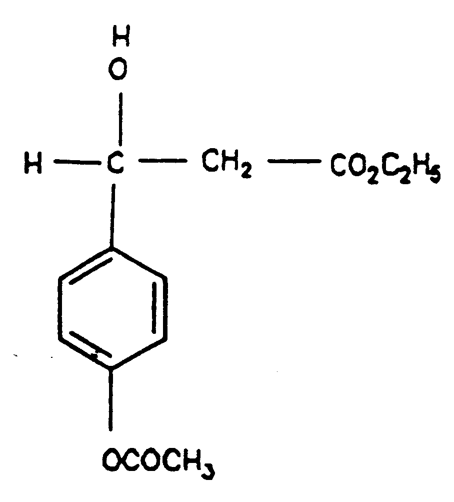 3-hydroxy-3-(4-acétoxyphényl)propionate d'éthyle

9. Promédicament préparé selon le procédé de l'une quelconque des revendications 1 à 8 pour l'utilisation en thérapeutique pour faciliter l'apport de médicaments portant un groupe phosphoreux dans les cellules et à travers les membranes biologiques, par contact des cellules et des membranes biologiques avec le promédicament.

10. Utilisation d'un promédicament préparé selon le procédé de l'une quelconque des revendications 1 à 8 pour la fabrication d'un médicament pour faciliter l'apport de médicaments portant un groupe phosphoreux dans les cellules et à travers les membranes biologiques, par contact des cellules et des membranes biologiques avec le promédicament.

11. Procédé pour préparer un promédicament pour faciliter l'apport de médicaments parents portant un groupe phosphoreux dans des cellules et à travers des membranes biologiques, comprenant les étapes consistant à :
a. choisir un médicament parent portant un groupe phosphoreux ; et
b. transformer un ou plusieurs des groupes hydroxy sur chaque groupe phosphoreux du médicament parent en un groupe de structure A pour obtenir un promédicament, où le groupe "A" est un dérivé benzyloxy avec un ou plusieurs groupes acyloxy dans les positions para ou ortho relativement au phosphoester, et où le médicament parent est libéré après conversion du groupe acyloxy par une estérase en le groupe hydroxy correspondant.

12. Utilisation d'un promédicament pour la fabrication d'un médicament parent portant un groupe phosphate ou phosphonate qui est un mono- ou un diester du phosphate ou du phosphonate, pour l'utilisation en thérapeutique, dans laquelle thérapeutique, le médicament est dégradé en le médicament parent portant un groupe phosphoreux par une réaction d'élimination pour former une double liaison carbone-carbone ; et où la réaction d'élimination est déclenchée par le démasquage spontané ou enzymatique d'un groupe que l'on sait être fortement donneur d'électrons, par exemple un groupe hydroxy.

13. Utilisation d'un promédicament préparé selon le procédé de la revendication 11 pour la fabrication d'un médicament pour faciliter l'apport de médicaments portant un groupe phosphoreux dans des cellules et à travers des membranes biologiques par contact des cellules avec le promédicament.
